# EUROPEAN PATENT SPECIFICATION

(11) **EP 0 372 982 B1**
(45) Date of publication and mention of the grant of the patent: **02.07.1997**
(21) Application number: 89312805.8
(22) Date of filing: 08.12.1989
(51) Int. Cl.: C07D 207/34, A01N 43/36

(54) **Pyrrole insecticides**
Pyrrole enthaltende Insektizide
Insecticides contenant des pyrroles

(30) Priority: 09.12.1988 US 282439; 16.11.1989 US 435362
(43) Date of publication of application: 13.06.1990
(73) Proprietor: RHONE-POULENC AGROCHIMIE, 69009 Lyon (FR)
(72) Inventor: Timmons, Philip, Durham North Carolina 27705 (US); Outcalt, Russel, Cary North Carolina 27511 (US); Cramp, Susan, Dagenham Essex RM10 7XS (GB); Kwiatkowski, Patricia, Raleigh North Carolina 27614 (US); Lopes, Anibal, Raleigh North Carolina 27604 (US); Sinodis, David, Cary North Carolina 27511 (US); Cain, Paul, Cary North Carolina 27511 (US)
(74) Representative: Bentham, Stephen

(56) References cited:
- DE-A- 2 016 393
- GB-A- 2 189 242
- JOURNAL OF THE CHEMICAL SOCIETY, PERKIN TRANSACTIONS I, vol. 4, no. 19, 1975, pages 1910-1913; A. BRODRICK et al.: "1H-pyrrolo 2,3-b pyridines. Part III. A novel synthetic route from 1-substituted 2-aminopyrroles"

## Description

The invention relates to new compounds of the chemical family of pyrroles, as well as intermediate products for the preparation of these compounds, and to processes for preparing these compounds and intermediates thereto. The invention also relates to the application of said compounds in agriculture, especially as pesticides for controlling arthropods, preferably as insecticides and acaricides; the invention also relates to agrochemical compositions useful to control arthropods, especially insects and arachnids.

Many pyrazoles (two nitrogen atom-containing heterocyclic formula) are well known as insecticides. Also some compounds containing the pyrrole group (one nitrogen atom containing formula) are known as insecticides. However, they usually contain also another chemical group in their formula which is well known to have insecticidal properties per se, such as a pyrethroid group, or a carbamate group, or some organophosphoric group. Simple substituted pyrrole derivatives have been described as agrochemical compounds, for example in British patent 2,189,242, but for fungicidal use.

The new pyrrole compounds of the invention have the formula (I) 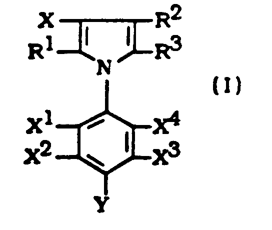 wherein:
- X: may be a halogen atom, or a group cyano, cyanato, thiocyanato, haloalkyl, alkoxy, haloalkoxy, alkylthio, alkylsulfinyl, alkylsulfonyl, haloalkylthio, haloalkylsulfinyl, haloalkylsulfonyl, alkenylthio, alkenylsulfinyl, alkenylsulfonyl, haloalkenylthio, haloalkenylsulfinyl, haloalkenylsulfonyl, haloalkylcarbonyl, haloalkylthiocarbonyl, phenylthio, phenylsulfinyl, phenylsulfonyl, heteroarylthio, heteroarylsulfinyl, or heteroarylsulfonyl; wherein the phenyl groups being optionally substituted with halogen, cyano or haloalkyl groups and the heteroaryl groups being monocyclic five or six membered, containing one or two heteroatoms such as oxygen, sulfur or nitrogen in any combination and being optionally substituted with halogen, nitro, cyano or haloalkyl groups; and wherein the halo-substitution in all the aforementioned groups consists of one or more halogen atoms, which are the same or different, from mono up to complete poly substitution and the alkyl, haloalkyl, alkenyl, haloalkenyl, alkoxy, and haloalkoxy groups being linear or branched chains, and having generally less than 10 carbon atoms, preferably less than 5 carbon atoms;
- R¹, R², and R³: may be chosen from: the same set of substituents as described for X; a hydrogen atom; an alkyl group; and a substituent of which no more than one of them (R¹, R², R³) may be selected from the group consisting of formyl, hydroxyiminoalkylidenyl, alkoxyiminoalkylidenyl, azido, amino, alkylamino, dialkylamino, aralkylamino, aminocarbonylamino, alkylcarbonylamino, haloalkylcarbonylamino, wherein the halo-substitution in the aforementioned groups consists of one or more halogen atoms, which are the same or different, from mono up to complete poly substitution and the alkyl groups being linear or branched chains, and having generally less than 10 carbon atoms, preferably less than 5 carbon atoms;
- Y: may be a halogen atom or a group cyano, alkyl, haloalkyl, alkoxy or haloalkoxy; wherein the halo-substitution in these groups consists of one or more halogen atoms, which are the same or different, from mono up to complete poly substitution and the alkyl, haloalkyl, alkoxy and haloalkoxy groups being linear or branched chains, and having generally less than 10 carbon atoms, preferably less than 5 carbon atoms; or
Y is a hydrogen atom when:
X is a halogen atom or a group R⁵S(O)n, in which n is 0, 1 or 2 and R⁵ is alkyl or haloalkyl; and the alkyl carbon chains and the halo-substitution are as defined above;
R¹ and R³ are each a hydrogen atom; and
R² is cyano;
- X¹, X², X³ and X⁴: are individually selected from the same set of substituents as described for Y or a hydrogen atom; with the following
provisos:
at least one of R¹, R², and R³ is selected from the same set of substituents as described for X;
if X⁴ and X¹ are H, and X is halogen or cyano, then R² is different from X;
if X⁴ and X¹ are H, and Y is methyl, then X is different from bromo; and with the exclusion of the compound wherein Y⁴ = X¹ = Y = R¹ = X = R² = R³ = Cl and at the same time X³ = X² = H.

More specific new pyrrole compounds of formula (I) of the invention are the following compounds wherein:
- X: may be a halogen atom, or a group cyano, haloalkyl, alkoxy, haloalkoxy, alkylthio, alkylsulfinyl, alkylsulfonyl, haloalkylthio, haloalkylsulfinyl or haloalkylsulfonyl; wherein the halo-substitution in all the aforementioned groups is mono substitution or up to as much as complete poly substitution and the alkyl, haloalkyl, alkoxy, and haloalkoxy groups being linear or branched chains, and having generally less than 10 carbon atoms, preferably less than 5 carbon atoms;
- R¹, R², and R³: may be chosen from: the same set of substituents as described for X; a hydrogen atom; an alkyl group; and a substituent of which no more than one of them (R¹, R², R³) may be selected from the group consisting of formyl, amino, alkylamino and dialkylamino; wherein the halo-substitution in the aforementioned groups is mono substitution or up to as much as complete poly substitution and the alkyl, haloalkyl, alkoxy and haloalkoxy groups being linear or branched chains, and having generally less than 10 carbon atoms, preferably less than 5 carbon atoms;
- Y: may be a halogen atom or a group cyano, alkyl, haloalkyl, alkoxy or haloalkoxy; wherein the halo-substitution in these groups is mono substitution or up to as much as complete poly substitution and the alkyl, haloalkyl, alkoxy and haloalkoxy groups being linear or branched chains, and having generally less than 10 carbon atoms, preferably less than 5 carbon atoms; and
- X¹, X², X³ and X⁴: are as defined above, and
the same provisos apply as previously defined.

Generally preferred compounds of formula (I) which are of interest as insecticides and acaricides are compounds of formula (I) wherein:
- X is: a halogen atom or a group R⁵S(O)ₙ in which n is 0, 1, or 2 and R⁵ is alkyl, haloalkyl, alkenyl, or haloalkenyl;
- R¹ is: a hydrogen atom, a halogen atom or alkylthio;
- R² is: cyano;
- R³ is: a hydrogen atom or a halogen atom;
- Y is: a hydrogen atom, a halogen atom, haloalkyl or haloalkoxy with the proviso of
- Y is: hydrogen as defined above in formula (I); and
- X¹,X²,X³ and X⁴ are: individually selected from the group consisting of a hydrogen atom, a halogen atom, C₁₋₃, alkyl, C₁₋₃ alkoxy, and C₁₋₃ alkylthio.

More specific compounds of general formula (I) which are preferred and are of particular interest are the compounds:
A) Compounds of formula (II), with high levels of insecticidal activity, 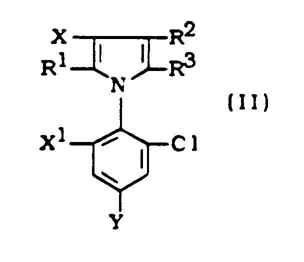 wherein:
   - X is: R⁵S(O)ₙ, in which n is 0, 1 or 2 and R⁵ is CH₃, CF₃, CF₂Cl, CFCl₂, CF₂Br, CHF₂, CHCl₂ or CHClF;
   - R² is: cyano;
   - R¹ is: H, F, Cl or Br;
   - R³ is: H, F, Cl or Br;
   - X¹ is: H or Cl; and
   - Y is: CF₃ or CF₃O.

   Among these compounds, preferred compounds are:
   1-(2,6-dichloro-4-trifluoromethylphenyl)-2-chloro-3-cyano-4-(trifluoromethylthio)pyrrole;
   1-(2,6-dichloro-4-trifluoromethoxyphenyl)-2-chloro-3-cyano-4-(trifluoromethylsulfonyl)pyrrole;
   1-(2,6-dichloro-4-trifluoromethylphenyl)-2-chloro-3-cyano-4-(dichlorofluoromethylthio)pyrrole;
   1-(2,6-dichloro-4-trifluoromethylphenyl)-2-chloro-3-cyano-4-(dichlorofluoromethylsulfinyl)pyrrole;
   1-(2,6-dichloro-4-trifluoromethylphenyl)-2-chloro-3-cyano-4-(dichlorofluoromethylsulfonyl)pyrrole;
   1-(2,6-dichloro-4-trifluoromethylphenyl)-2-chloro-3-cyano-4-(chlorodifluoromethylsulfonyl)pyrrole;
   1-(2,6-dichloro-4-trifluoromethylphenyl)-2-chloro-3-cyano-4-chlorodifluoromethylsulfinyl)pyrrole;
   1-(2,6-dichloro-4-trifluoromethylphenyl)-2-chloro-3-cyano-4-(chlorodifluoromethylthio)pyrrole;
   1-(2,6-dichloro-4-trifluoromethylphenyl)-2-bromo-3-cyano-4-(trifluoromethylsulfinyl)pyrrole;
   1-(2,6-dichloro-4-trifluoromethylphenyl)-2-bromo-3-cyano-4-(trifluoromethylsulfonyl)pyrrole;
   1-(2,6-dichloro-4-trifluoromethylphenyl)-2-bromo-3-cyano-4-(dichlorofluoromethylthio)pyrrole;
   1-(2,6-dichloro-4-trifluoromethylphenyl)-2-bromo-3-cyano-4-(dichlorofluoromethylsulfonyl)pyrrole;
   1-(2,6-dichloro-4-trifluoromethylphenyl)-2-bromo-3-cyano-4-(dichlorofluoromethylsulfinyl)pyrrole;
   1-(2,6-dichloro-4-trifluoromethylphenyl)-2-bromo-3-cyano-4-(chlorodifluoromethylthio)pyrrole;
   1-(2,6-dichloro-4-trifluoromethylphenyl)-2-bromo-3-cyano-4-(chlorodifluoromethylsulfinyl)pyrrole;
   1-(2,6-dichloro-4-trifluoromethylphenyl)-2-chloro-3-cyano-4-(dichlorofluoromethylsulfonyl)-5-bromopyrrole;
   1-(2,6-dichloro-4-trifluoromethylphenyl)-2-bromo-3-cyano-4-(chlorodifluoromethylsulfonyl)pyrrole;
   1-(2,6-dichloro-4-trifluoromethoxyphenyl)-2-chloro-3-cyano-4-(dichlorofluoromethylsulfinyl)pyrrole;
   1-(2,6-dichloro-4-trifluoromethoxyphenyl)-2-chloro-3-cyano-4-(dichlorofluoromethylsulfonyl)pyrrole;
   1-(2-chloro-4-trifluoromethylphenyl)-2-chloro-3-cyano-4-(dichlorofluoromethylsulfonyl)pyrrole;
   1-(2-chloro-4-trifluoromethylphenyl)-2-chloro-3-cyano-4-(dichlorofluoromethylsulfinyl)pyrrole;
   1-(2-chloro-4-trifluoromethylphenyl)-2-bromo-3-cyano-4-(dichlorofluoromethylsulfonyl)pyrrole;
   1-(2,6-dichloro-4-trifluoromethylphenyl)-2-chloro-3-cyano-4-(dichlorofluoromethylthio)-5-methylthiopyrrole;
   1-(2,6-dichloro-4-trifluoromethylphenyl)-2-chloro-3-cyano-4-(bromodifluoromethylthio)pyrrole;
   1-(2,6-dichloro-4-trifluoromethylphenyl)-2-chloro-3-cyano-4-(bromodifluoromethylsulfinyl)pyrrole;
   1-(2,6-dichloro-4-trifluoromethylphenyl)-2-chloro-3-cyano-4-(bromodifluoromethylsulfonyl)pyrrole;
   1-(2,6-dichloro-4-trifluoromethylphenyl)-2-chloro-3-cyano-4-(methylsulfinyl)pyrrole; or
   1-(2,6-dichloro-4-trifluoromethylphenyl)-2-chloro-3-cyano-4-(methylsulfonyl)pyrrole.
B) Other compounds of formula (II), with high insecticidal activity, wherein:
   X is R⁵S(O)ₙ, in which n is 0, 1 or 2 and R⁵ is CH₃, CF₃, CF₂Cl or CFCl₂:
   R² is cyano;
   R¹ is H, F, Cl, Br or NH₂;
   R³ is H, F, Cl, Br, CF₃ or CN;
   X¹ is H or Cl; and
   Y is CF₃ or CF₃O.

   Compounds of the invention which are preferred for their insecticidal activity are:
   1-(2,6-dichloro-4-trifluoromethylphenyl)-2-chloro-3-cyano-4-(trifluoromethylsulfonyl)pyrrole;
   1-(2,6-dichloro-4-trifluoromethylphenyl)-2-chloro-3-cyano-4-(trifluoromethylsulfinyl)pyrrole;
   1-(2,6-dichloro-4-trifluoromethylphenyl)-2-chloro-3-cyano-4-trifluoromethylsulfinyl-5-bromopyrrole;
   1-(2,6-dichloro-4-trifluoromethylphenyl)-2-chloro-3-cyano-4-trifluoromethylsulfonyl-5-bromopyrrole;
   1-(2,6-dichloro-4-trifluoromethylphenyl)-2-chloro-3-cyano-4-(trifluoromethylthio)pyrrole;
   1-(2,6-dichloro-4-trifluoromethylphenyl)-2-bromo-3-trifluoromethylthio-4-cyano-5-chloropyrrole;
   1-(2,6-dichloro-4-trifluoromethylphenyl)-2-[(trifluoromethyl)carbonylamino]-3-trifluoromethylthio-4-cyano-5-chloropyrrole;
   1-(2,6-dichloro-4-trifluoromethylphenyl)-2-methylcarbonylamino)-3-trifluoromethythio-4-cyano-5-chloropyrrole;
   1-(2,6-dichloro-4-trifluoromethylphenyl)-2-amino-3-trifluoromethylthio-4-cyano-5-chloropyrrole;
   1-(2-chloro-4-trifluoromethylphenyl)-2-amino-3-trifluoromethylthio-4-cyano-5-chloropyrrole;
   1-(2,6-dichloro-4-trifluoromethylphenyl)-2-amino-3-dichlorofluoromethylthio-4-cyano-5-chloropyrrole;
   1-(2,6-dichloro-4-trifluoromethylphenyl)-2,4-bis(trifluoromethylthio)-3-cyano-5-aminopyrrole;
   1-(2,6-dichloro-4-trifluoromethylphenyl)-2-amino-3-trifluoromethylthio-4-cyano-5-bromopyrrole;
   1-(2,6-dichloro-4-trifluoromethylphenyl)-2-amino-3-trifluoromethylthio-4-cyanopyrrole;
   1-(4-trifluoromethylphenyl)-2-amino-3-trifluoromethylthio-4-cyano-5-bromopyrrole;
   1-(2-chloro-4-trifluoromethylphenyl)-2-amino-3-trifluoromethylthio-4-cyano-5-bromopyrrole;
   1-(2,6-dichloro-4-trifluoromethylphenyl)-2-chloro-3-cyano-4-(dichlorofluoromethylthio)pyrrole;
   1-(2,6-dichloro-4-trifluoromethylphenyl)-2-chloro-3-cyano-4-(dichlorofluoromethylsulfinyl)pyrrole;
   1-(2,6-dichloro-4-trifluoromethylphenyl)-2-chloro-3-cyano-4-(dichlorofluoromethylsulfonyl)pyrrole;
   1-(2,6-dichloro-4-trifluoromethylphenyl)-3-cyano-4-(dichlorofluoromethylthio)pyrrole;
   1-(2,6-dichloro-4-trifluoromethylphenyl)-3-cyano-4-(dichlorofluoromethylsulfinyl)pyrrole; or
   1-(2,6-dichloro-4-trifluoromethoxyphenyl)-2-chloro-3-cyano-4-(trifluoromethylsulfonyl)pyrrole.
C) Compounds of formula (I), with surprisingly high levels of acaricidal activity, wherein:
   - X is: a halogen atom, or a group R⁵(O)ₙ, in which n is 0, 1, or 2 and R⁵ is: alkyl, preferably C₁₋₄ alkyl; haloalkyl, preferably trihalomethyl preferably in which halo is F, Cl, Br or combinations thereof, e.g. CF₃, CCl₃, CF₂Cl, CFCl₂ or CF₂Br; alkenyl; or haloalkenyl;
   - R¹ and R³ are: each a hydrogen atom;
   - R² is: cyano;
   - Y is: a hydrogen atom or a halogen atom, preferably Cl or Br; and
   - X^{1,} X², X³ and X⁴ are: individually selected from the group consisting of: hydrogen; halogen; C₁₋₃ alkyl; C₁₋₃ alkoxy; and C₁₋₃ alkylthio; and preferably, X¹ and X⁴ are individually H, F, Cl, Br or CH₃ and X² and X³ are each hydrogen.

   Among these compounds, the more preferred compounds are:
   1-(4-bromo-2,6-dichlorophenyl)-3-cyano-4-(chlorodifluoromethyl-thio) pyrrole;
   1-(4-bromo-2,6-dichlorophenyl)-3-cyano-4-(trifluoromethylthio)-pyrrole;
   1-(2,4,6-trichlorophenyl)-3-cyano-4-(chlorodifluoromethylthio)-pyrrole;
   1-(2,4,6-trichlorophenyl)-3-cyano-4-(chlorodifluoromethylsulfinyl)-pyrrole;
   1-(2,4,6-trichlorophenyl)-3-cyano-4-(dichlorofluoromethylthio)-pyrrole;
   1-(2,4,6-trichlorophenyl)-3-cyano-4-(dichlorofluoromethylsulfinyl)-pyrrole;
   1-(2,4,6-trichlorophenyl)-3-cyano-4-(dichlorofluoromethylsulfonyl)-pyrrole;
   1-(4-bromo-2,6-dichlorophenyl)-3-cyano-4-(dichlorofluoromethylthio)pyrrole;
   1-(4-bromo-2,6-dichlorophenyl)-3-cyano-4-(dichlorofluoromethylsulfinyl)pyrrole;
   1-(4-bromo-2,6-dichlorophenyl)-3-cyano-4-(dichlorofluoromethylsulfonyl)pyrrole;
   1-(2,4,6-trichlorophenyl)-3-cyano-4-(trifluoromethylthio)pyrrole;
   1-(2,4,6-trichlorophenyl)-3-cyano-4-(trifluoromethyl-sulfinyl)pyrrole;
   1-(2,4,6-trichlorophenyl)-3-cyano-4-(trifluoromethylsulfonyl)-pyrrole;
   1-(2,4,6-trichlorophenyl)-3-cyano-4-(trichloromethylthio)pyrrole;
   1-(2,4-dichlorophenyl)-3-cyano-4-(dichlorofluoromethylthio)-pyrrole;
   1-(2,4,6-trichlorophenyl)-3-cyano-4-chloropyrrole;
   1-(2,4,6-trichlorophenyl)-3-cyano-4-(chlorodifluoromethylsulfonyl)-pyrrole;
   1-(2,6-dichlorophenyl)-3-cyano-4-(dichlorofluoromethylthio)-pyrrole;
   1-(4-bromo-2,6-dichlorophenyl)-3-cyano-4-(trifluoromethylsulfinyl)pyrrole;
   1-(4-bromo-2,6-dichlorophenyl)-3-cyano-4-(trifluoromethylsulfonyl)pyrrole;
   1-(4-bromo-2,6-dichlorophenyl)-3-cyano-4-(chlorodifluoromethylsulfinyl)pyrrole;
   1-(4-bromo-2,6-dichlorophenyl)-3-cyano-4-(chlorodifluoromethylsulfonyl)pyrrole;
   1-(4-bromo-2,6-dimethylphenyl)-3-cyano-4-(trifluoromethylsulfinyl)pyrrole;
   1-(4-bromo-2,6-dimethylphenyl)-3-cyano-4-(trifluoromethylsulfonyl)pyrrole; or
   1-(4-bromo-2,6-difluorophenyl)-3-cyano-4-(dichlorofluoromethylthio)pyrrole.
D) Other preferred compounds of formula (I), are compounds of formula (I) wherein:
   - R¹ is: a hydrogen atom or a halogen atom such as chlorine or bromine;
   - R² is: cyano;
   - X is: a haloalkylthio group or a haloalkysulfinyl or haloalkylsulfonyl group, preferably CF₃S(O)ₙ, wherein n is 0, 1, or 2;
   - X¹ and X⁴: are other than a hydrogen atom;
   - X² and X³: are each a hydrogen atom; and
   - Y is: haloalkyl or haloalkoxy.

An objective of the present invention is to provide new insecticides and acaricides of the pyrrole family and intermediate compounds to make them.

Another objective of the present invention is to provide very active compounds. These and other objectives of the invention are totally or partially obtained with the new compounds hereafter defined.

### DESCRIPTION OF THE PREFERRED EMBODIMENTS

### METHODS OR PROCESSES OF SYNTHESIS

The compounds of general formula (I) can be prepared by the application or adaptation of known methods (ie. methods heretofore used or described in the chemical literature): generally pyrrole ring formation followed where necessary by changing substituents. It is to be also understood that, in the description of the following process methods the sequences for the introduction of the various groups on the pyrrole ring may be performed in a different order and that suitable protecting groups may be required as will be apparent to those skilled in the art. Also compounds of general formula (I) may be converted by known methods into other compounds of general formula (I).

In the following description of process methods when symbols appearing in formulae are not specifically defined, it is to be understood that they are "as hereinbefore defined" in accordance with the first definition of each symbol in this specification. The term "protection" shall include conversion to a suitable non-reactive group which may be reconverted when desired, as well as the addition of groups which render the functionality non-reactive. Within the process definitions, unless otherwise stated, amino refers to the unsubstituted amino group.

The invention embraces particular intermediate compounds, useful to make certain of the herein contemplated compounds. Such preferred intermediate compounds, prepared as described herein, have the formula (IIIa) 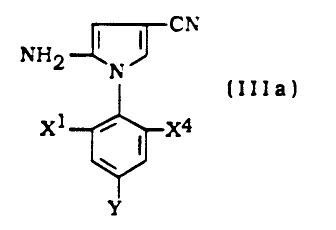 wherein Y is H, Cl, Br, CF₃ or OCF₃ and X¹ and X⁴ are individually H, Cl, F, CH₃ or SCH₃ with the proviso: if X¹ and X⁴ are each H, then Y is other than H or Cl. Such compounds defined by the proviso are not claimed by the present invention, but yet are still useful as intermediates to prepare compounds of formula (I) of the invention.

Compounds of formula (IIIa) more specifically preferred as intermediates are those wherein Y is CF₃ or OCF₃, X¹ is H or Cl, and X⁴ is Cl.

### METHOD 1

According to a feature of the invention, the compounds of formula (I) in which R¹ and R² are a hydrogen atom, X is a cyano group, and R³ is an amino (NH₂) group and X¹, X², X³, X⁴ and Y have the same definitions as that shown in the general definition of the invention, that is to say the compounds of formula (III) 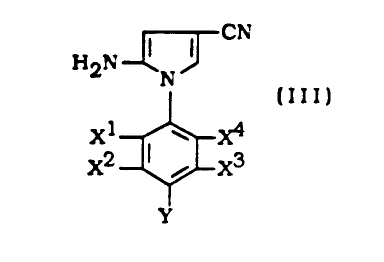 may be prepared from dicyanopropene derivatives of formula (IV) 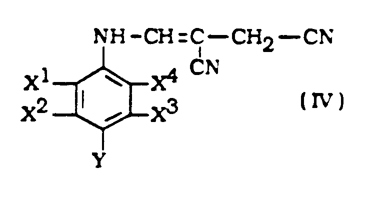 (in which the various symbols are as hereinbefore defined) by reaction with a basic agent, preferably an alkali agent such as a tertiary amine or a hydroxide or carbonate of an alkali metal. The reaction is advantageously carried out between -80 and 150°C, preferably 40 to 100°C. Solvents may be used, such as liquid alcohols, hydrocarbons, halohydrocarbons, ethers, ketones, amides such as N-methyl pyrrolidone, or water.

### METHOD 2

According to another feature of the invention, the compounds of formula (IV) may be prepared from an aniline derivative of formula (V) wherein X¹, X², X³, X⁴ and Y have the same definitions as shown in the general definition of the invention, 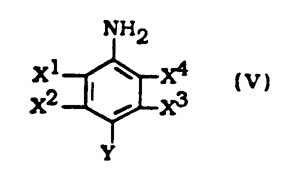 by reaction with formyl succinonitrile or an alkali metal salt of formyl succinonitrile. This reaction is generally carried out in an organic solvent or in water at a temperature between 10 and 120^{o}C, preferably at reflux temperature.

Formyl succinonitrile is a known compound, generally made by acidification of its alkaline salt which is obtained by reaction of succinonitrile with a lower alkyl formate in the presence of an alkaline agent, according to K. Gewald, *Z.Chem.* **1961**,*1*,349.

### METHOD 3

Compounds of general formula (I) in which X is halogen, R¹ is amino, R² is cyano and R³ is hydrogen and the other substituents have the meaning described in the general description of the invention may be prepared by treating compounds of general formula (III) with halogenating agents such as sulfuryl chloride. N-chlorosuccinimide, N-bromosuccinimide, N-ioidosuccinimide, pyridinium bromide perbromide or molecular fluorine, chlorine, bromine or iodine. Suitable organic solvents for these transformations include dichloromethane and acetonitrile. The reactions are carried out between -80^{o} C and + 80^{o} C, preferably between -30^{o} C and +25^{o} C. It may be advantageous to protect the amino group as the trifluoroacetamide derivative during treatment with elemental fluorine.

### METHOD 4

A) Compounds of general formula (I) in which X is a cyano group, R¹ is amino, R² is cyano and R³ is hydrogen and the other substituents have the meanings described in the general description of the invention, may be obtained from the corresponding compounds in which X is a group C=NOH by dehydration with agents such as acetic anhydride, cyanuric chloride or P₂O₅. With certain of these dehydrating agents it may be necessary to protect the amino group with a suitable protecting group.
B) The intermediate compounds above in which X is a group C=NOH may be obtained by condensation of hydroxylamine with the corresponding compounds in which X is formyl.
C) The intermediate compounds in which X is a formyl group (of formula (VI) shown hereinafter), may be obtained by hydrolysis of the corresponding compounds in which X is a bis(alkylthio)methyl or bis(arylthio)methyl group or treatment with a suitable alkyl nitrite followed by hydrolysis according to E. Fujita, K. Ichikawa and K. Fuji, Tetrahedron Letters 1978, 3561. Protection of the amino function with an appropriate protecting group may be necessary during the reaction with alkyl nitrites.
D) The intermediate compounds of general formula (I) in which X is a bis(alkylthio)methyl or bis(arylthio)methyl group, and the other groups are defined as above may be prepared by reaction of a compound of formula (III) with a tris(alkylthio)methane or tris(arylthio) methane in the presence of a Lewis acid, preferably a sulfonium salt such as dimethyl(methylthio)sulfonium tetrafluoroborate. General conditions for such transformations may be found in Synthesis 1984, 166.

### METHOD 5

A) Useful intermediate compounds of general formula (I) in which X is hydroxy, R¹ is an optionally protected amino group, R² is a cyano group, R³ is a hydrogen atom and X¹, X², X³, X⁴ and Y have the definitions shown in the general definition of the invention may be prepared from the corresponding compounds in which X is halogen by conversion to a Grignard reagent or a lithium dervative by standard methods followed by treatment with oxodiperoxymolybdenum(pyridine)(hexamethylphosphoric triamide) (MoOPH) by procedures similar to those described by N. J. Lewis *et. al.* in *J. Org. Chem.* **1977**, *42*, 1479 . It may be necessary to convert the cyano group in the compound above in which X is halogen to an appropriately protected derivative (for example an oxazoline derivative of the corresponding compound in which the cyano group has been hydrolyzed to a carboxylic acid ) prior to formation of the Grignard reagent or lithium derivative. Alternatively, the Grignard reagent or lithium derivative described above may be reacted with a trialkyl borate followed by oxidation with hydrogen peroxide by a procedure analogous to that described by M. F. Hawthorne in *J. Org. Chem.* **1957**,*22*, 1001 or R. W. Hoffmann and K. Ditrich in *Synthesis* **1983**, 107.
B) Compounds of general formula (I) in which X is cyanato, R¹ is amino , R² is a cyano group, R³ is a hydrogen atom and X¹, X², X³, X⁴ and Y have the definitions shown in the general definition of the invention may be prepared from the corresponding compounds in which X is hydroxy, R¹ is an optionally protected amino group, R³ is a hydrogen atom and X¹, X², X³, X⁴ and Y have the definitions shown in the general definition of the invention by treatment with cyanogen halides in the presence of a base by methods similar to those described by D. Martin and M. Bauer in *Org. Synth.* **61**, 35, followed by a deprotection step, if necessary.
C) Compounds of general formula (I) in which X is alkoxy, R¹ is amino , R² is a cyano group, R³ is a hydrogen atom and X¹, X², X³, X⁴ and Y have the definitions shown in the general definition of the invention may be prepared from the corresponding compounds in which X is hydroxy, R¹ is an optionally protected amino group, R³ is a hydrogen atom and X¹, X², X³, X⁴ and Y have the definitions shown in the general definition of the invention by treatment with an alkyl halide, alkyl sulfonate dialkyl sulfate and the like, optionally in the presence of a base in a solvent such as acetone or dimethylformamide at a temperature between 25 ^{o}C and the reflux temperature of the solvent followed by a deprotection step, if necessary.
D) Compounds of general formula (I) in which X is haloalkoxy, R¹ is amino , R² is a cyano group, R³ is a hydrogen atom and X¹, X², X³, X⁴ and Y have the definitions shown in the general definition of the invention may be prepared from the corresponding compounds in which X is hydroxy, R¹ is an optionally protected amino group, R³ is a hydrogen atom and X¹, X², X³, X⁴ and Y have the definitions shown in the general definition of the invention by various haloalkylation methods described in *Syntheses of Fluoroorganic Compounds;* Knunyants, I. L. and Yakobson, G. G., Ed.; Springer-Verlag; Berlin, 1985; pp 263 - 269, followed by a deprotection step, if necessary.

### METHOD 6

Compounds of general formula (I) in which X is a haloalkyl group, R¹ is an amino group, R² is a cyano group, R³ is a hydrogen atom and X¹, X², X³, X⁴ and Y have the definitions shown in the general definition of the invention may be prepared from the corresponding compounds in which X is a formyl group, a carboxylic acid function or a halogen and the amino group is optionally protected. For example, treatment of the formyl compounds with diethylaminosulfur trifluoride in a manner analogous to that described by W. J. Middleton in *J. Org. Chem.* **1975**, *40*, 574 provides compounds of general formula (I) in which X is a difluoromethyl group and the other substituents are defined as above. Oxidation of the above mentioned intermediate compounds of general formula (I) in which X is formyl with oxidizing agents such as chromium trioxide in sulfuric acid (Jones' reagent) provides intermediate compounds of general formula (I) in which X is a carboxylic acid function, R¹ is an amino group, R² is a cyano group, R³ is a hydrogen atom and X¹, X², X³, X⁴ and Y have the definitions shown in the general definition of the invention. It may be advantageous to protect the amino function as, for example, the trifluoroacetamide derivative during such oxidation reactions. Reaction of the compounds above in which X is a carboxylic acid group with sulfur tetrafluoride as described by G. A Boswell *et. al. Org. React.* **1974** *21*, 1-124 provides compounds in which X is a trifluoromethyl group and the other groups are defined as above.

Alternatively, compounds of general formula (I) in which X is trifluoromethyl, R¹ is an amino group, R² is a cyano group, R³ is a hydrogen atom and X¹, X², X³, X⁴ and Y have the definitions shown in the general definition of the invention may be prepared from compounds of general formula (I) in which X is halogen, preferably iodine, and the other substituents are as defined above by reaction with trifluoromethyl copper under conditions similar to those described by D. J. Burton and D. M. Wiemers in *J. Am. Chem. Soc.* **1986**, *108*, 832.

### METHOD 7

Compounds of general formula (I) in which X is a bromomethyl or chloromethyl group , R¹ is an amino group, R² is a cyano group, R³ is a hydrogen atom and X¹, X², X³, X⁴ and Y have the definitions shown in the general definition of the invention may be obtained by treatment of the corresponding intermediate compounds in which X is a methyl group and the amino group is optionally protected, with *N*-bromosuccinimide or *N*-chlorosuccinimide in solvents such as carbon tetrachloride at temperatures between O ⁰C and the reflux temperature of the solvent. The compounds above in which X is a methyl group may be obtained from the intermediate compounds of general formula (I) in which X is formyl and the other substituents are as described above by sequential treatment with *p*-toluenesulfonylhydrazine and sodium cyanoborohydride, according to a method similar to that described in *J. Am. Chem. Soc.* **1971**, *93*, 1793.

### METHOD 8

A) Compounds of general formula (I) in which X is haloalkylcarbonyl, R¹ is an amino group, R² is a cyano group, R³ is a hydrogen atom and X¹, X², X³, X⁴ and Y have the definitions shown in the general definition of the invention, that is to say compounds of formula (VIII), may be obtained by sequential treatment of the corresponding compounds (VI) in which the amino group is optionally protected, with a haloalkylmetal derivative to provide compounds of formula (VII) in which X is a haloalkylcarbinol, followed by oxidation according to the method of R. J. Linderman and D. M. Graves described in *Tetrahedron Lett.* **1987**, *28*, 4259 and a deprotection step, if necessary. Suitable haloalkylmetal derivatives include perfluoroalkyl lithium derivatives prepared according to P. G. Gassman and N. J. O'Reilly, *J. Org. Chem.* **1987**, *52*, 2481-2490 or trimethyltrifluoromethylsilane prepared and used according to G. A. Olah *et. al. J. Am. Chem. Soc.* **1989**, *111*, 393. References to other haloalkyl metal derivatives may also be found in this reference. With trimethyltrifluoromethylsilane, this process may be illustrated as follows: 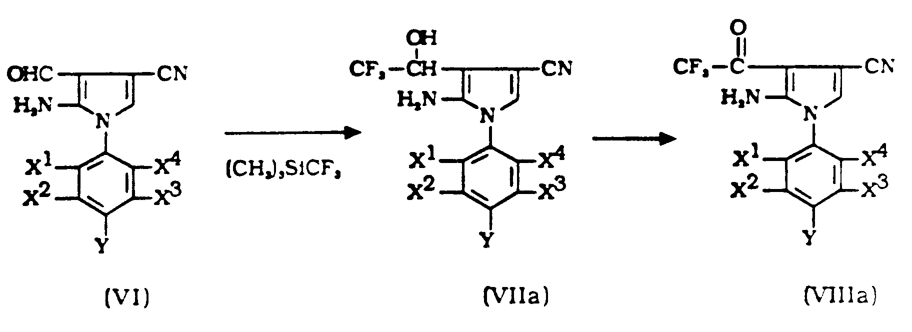
B) The compounds of formula (VIII) may be converted to compounds of general formula (I) in which X is a haloalkylthiocarbonyl group, R¹ is an amino group, R² is a cyano group, R³ is a hydrogen atom and X¹, X², X³, X⁴ and Y have the definitions shown in the general definition of the invention by treatment with [2,4-bis(4-methoxyphenyl)-1,3-dithia-2,4-diphosphetane-2,4-disulfide] Lawesson's Reagent).

### METHOD 9

The compounds of formula (VII) may be converted to other compounds of general formula (I) in which X represents a-haloalkyl-a-halomethyl groups by treatment with halogenating agents such as thionyl chloride or hydrogen bromide. It may be advantageous to protect the amino function as , for example, a trifluoroacetamide derivative to prevent halogenation of the pyrrole ring during such halogenations.

### METHOD 10

A) Compounds of general formula (I) in which X is thiocyanato, R¹ is an amino group, R² is a cyano group, R³ is a hydrogen atom and X¹, X², X³, X⁴ and Y have the definitions shown in the general definition of the invention may be prepared by treatment of compounds of formula (III) with MSCN wherein M is an alkali metal in the presence of bromine in a solvent such as methanol.
B) Compounds of general formula (I) in which X is an alkylthio , haloalkylthio, alkenylthio, haloalkenylthio or phenylthio group, R¹ is an amino group, R² is a cyano group and R³ is a hydrogen atom and X¹, X², X³, X⁴ and Y have the definitions shown in the general definition of the invention and the phenyl groups being constituted and/or substituted as described therein, i.e. compounds of formula (IX) (vide infra), may be prepared by reaction of a compound of formula (III) with a sulfenyl halide RSHal in which R is an alkyl, haloalkyl, alkenyl, haloalkenyl, phenyl group as defined above and Hal is a halogen atom, in a liquid reaction medium. Preferably an organic solvent, e.g. dichloromethane, is used at a temperature of - 100^{o} C to +100^{o} C, preferably -80^{o} C to +25^{o} C. This reaction may be optionally carried out in the presence of an acid acceptor such as a tertiary amine, e.g. pyridine. The alkylsulfenyl chlorides may be prepared according to S. Thea and G. Cevasco, *Tetrahedron Letters*, **1988**, 2865. When a sulfenyl chloride is used, the process may be represented by the following equation:

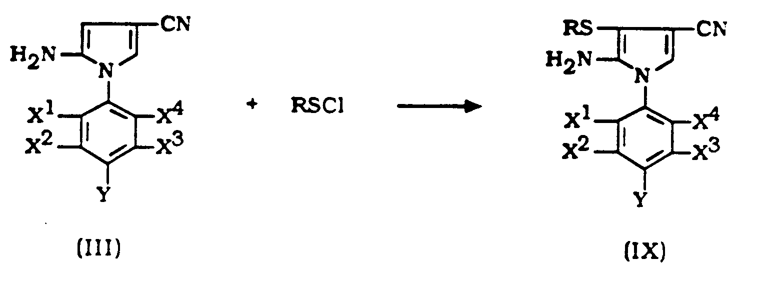

### METHOD 11

Compounds of general formula (I) in which X is thiocyanato, R¹ is an amino group, R² is a cyano group, R³ is a hydrogen atom and X¹, X², X³, X⁴ and Y have the definitions shown in the general definition of the invention may be further converted to compounds of general formula (I) in which X is an alkylthio group, R¹ is an amino group, R² is a cyano group, R³ is a hydrogen atom and X¹, X², X³, X⁴ and Y have the definitions shown in the general definition of the invention by treatment with a base such as sodium or potassium hydroxide in the presence of e.g. an alkyl halide or dialkyl sulfate in a solvent.

### METHOD 12

Compounds of formula (IX) may be oxidized to provide compounds of formula (X) in which X, (vide infra), is a group RS(O)ₙ, wherein n is 1 or 2 and R is as hereinbefore defined. The oxidizing agents which may be used include hydrogen peroxide, peroxyacetic acid, trifluoroperoxyacetic acid and m-chloroperoxybenzoic acid in solvents such as dichloromethane, acetic acid or trifluoroacetic acid at temperatures between -40^{o} C and +80^{o} C, preferably 0^{o} C to 25^{o}C. The appropriate reaction conditions, i.e. temperature, length of reaction and amount of oxidant may be changed to provide the sulfinyl (n=1) or sulfonyl (n=2) derivatives as desired. It is also possible to prepare the sulfonyl derivatives from the sulfinyl compounds, as is apparent to those skilled in the art. With certain haloalkylthio groups, for example trifluoromethylthio, it may be beneficial to protect the amino function as, for example, the trifluoroacetamide derivative. If, for example, trifluoroperoxyacetic acid is chosen as the oxidant, the process may be represented by the following equation:

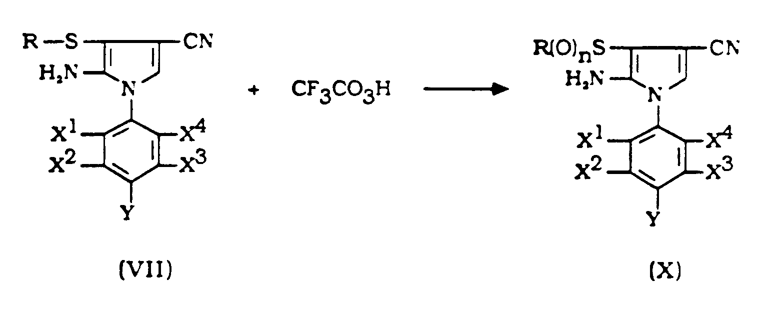

### METHOD 13

Compounds of general formula (I) in which R³ is halogen. R¹ is amino, R² is cyano, and X, X¹, X², X³, X⁴ and Y have the meanings given in the general definition of the invention may be prepared by treatment of compounds of general formula (I) in which R¹ is amino, R² is cyano, R³ is hydrogen and X, X¹, X², X³, X⁴ and Y have the definitions shown in the general definition of the invention, i.e. compounds of formula (XI) shown hereinafter by treatment with halogenating agents under similar conditions as described in METHOD 3. The general transformation is as shown below:

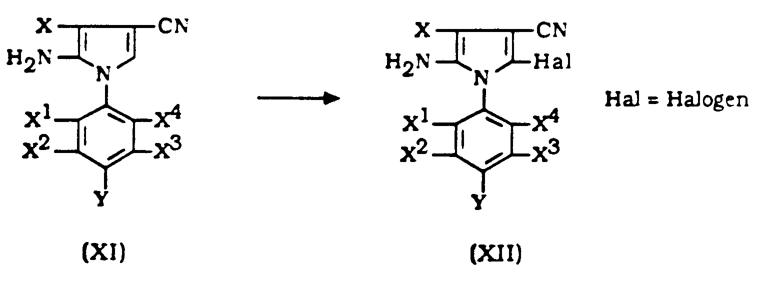

### METHOD 14

Compounds of general formula (I) in which R³ is a bis(alkylthio)methyl or bis(arylthio)methyl group, R¹ is amino, R² is cyano and X, X¹, X², X³, X⁴ and Y have the meanings given in the general definition of the invention may be prepared by reaction of a compound of formula (XI) with a tris(alkylthio)methane or tris(arylthio)methane, (R^{a}S)₃CH (wherein R^{a} is alkyl or aryl) in the presence of a Lewis acid, preferably a sulfonium salt, in a solvent, at a temperature between 0^{o}C and the reflux temperature of the solvent, optionally in the presence of an acid acceptor such as pyridine. A more preferred process employs acetonitrile as solvent at 25 ^{o}C with tris(methylthio)methane as the tris(alkylthio)methane and dimethyl(methylthio)sulfonium tetrafluoroborate as the Lewis acid without an acid acceptor. The process may be generally represented as shown below:

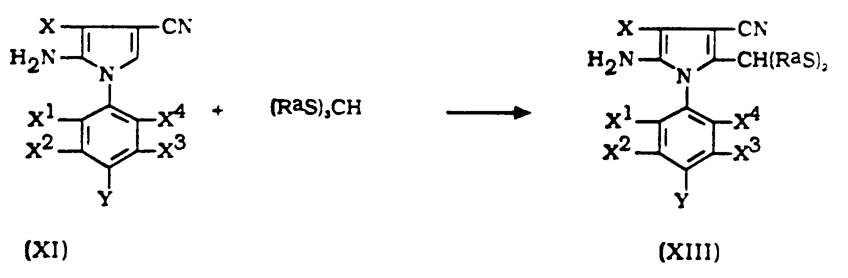

### METHOD 15

Useful intermediate compounds of general formula (I) in which R³ is formyl, R¹ is amino, R² is cyano and X, X¹, X², X³, X⁴ and Y have the meanings given in the general definition of the invention, i.e. compounds of formula (XIV) shown hereinafter, may be prepared by hydrolyzing compounds of formula (XIII) or
by treating with alkyl nitrites under similar conditions as discussed in METHOD 4C The process may be generally represented as follows:

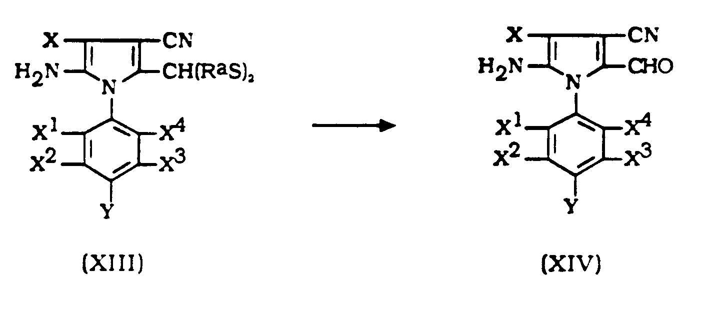

### METHOD 16

Intermediate compounds of general formula (I) in which R³ is hydroxyiminoalkylidenyl or alkoxyiminoalkylidenyl, R¹ is amino, R² is cyano and X, X¹, X², X³, X⁴ and Y have the meanings given in the general definition of the invention, useful as intermediates, may be prepared by condensation of a compound of general formula (I) in which R³ is alkylcarbonyl or formyl, R¹ is amino, R² is cyano and X, X¹, X², X³, X⁴ and Y have the meanings given in the general definition of the invention with hydroxylamine or an *O*-alkylhydroxylamine or their acid addition salts in a solvent such as ethanol. The compounds above in which R³ is alkylcarbonyl are prepared in the same manner as compounds (XIV) using ultimately a 1,1,1-tris(alkylthio or arylthio)alkane as a starting material.

### METHOD 17

Useful intermediate compounds of general formula (I) in which R³ is amino, R¹ is amino, R² is cyano and X, X¹, X², X³, X⁴ and Y have the meanings given in the general definition of the invention, useful as intermediates, may be prepared by reduction of the corresponding compounds in which R³ is nitro with, for example, hydrogen in the presence of a noble metal catalyst such as platinum or palladium or with hydrazine and Raney nickel. *With certain combinations of R*^{*2*} *and X substituents, the compounds of formula (I) in which R*^{*1*} *and R*^{*2*} *are simultaneously amino may have limited stability and may require protection of one of the amino groups with a suitable protecting group*. The compounds of general formula (I) in which R³ is nitro, R¹ is amino, R² is cyano and X, X¹, X², X³, X⁴ and Y have the meanings given in the general definition of the invention may be prepared by nitration of compounds of formula (XI) through the action of nitric acid and sulfuric acid or nitric acid in acetic anhydride or other nitrating agents. It may be advantageous during certain nitration reactions to protect the amino function in (XI) with a suitable protecting group such as a acetyl or trifluoroacetyl.

### METHOD 18

Various derivatives, useful as intermediates, of a compound of formula (XV) (*vide infra*) may be prepared by the following methods:
A) Compounds of general formula (I) in which R³ is alkylamino, dialkylamino or aralkylamino, R¹ is amino, R² is cyano and X, X¹, X², X³, X⁴ and Y have the meanings given in the general definition of the invention may be prepared from the corresponding compounds in which R³ is amino and the other substituents are defined as above by alkylation with alkyl or aralkyl halides or sulfonates in organic solvents such as ethanol, acetonitrile, toluene.
   The production of mono- or dialkylated products may be controlled through manipulation of stoichiometry or reaction conditions. Alternatively, if monoalkylamine products are desired, other methods such as the conversion of the amino group to alkoxyalkylideneimino group by treatment with an alkylorthoester followed by reduction may be employed. If the desired final products contain R¹ as the unsubstituted amino group, it may be necessary to protect the amino group prior to such treatment with a suitable protecting group. In such cases, a compound of general formula (I) in which R³ is amino, R¹ is a suitably protected amino group and the other substituents are as defined above, that is to say a compound of formula (XV), is prepared and used as a reactant. The protecting group in (XV) is normally added prior to the nitro reduction step discussed in METHOD 17. Depending on the subsequent transformation intended for the amino group at R³, various protecting groups may be chosen as will be seen in the following examples.
B) Compounds of general formula (I) in which R³ is aminocarbonylamino, R¹ is amino, R² is cyano and X, X¹, X², X³, X⁴ and Y have the meanings given in the general definition of the invention may be prepared from compounds of formula (XV) by treatment with phosgene followed by ammonia followed by a deprotection step.
C) Compounds of general formula (I) in which R³ is alkylcarbonylamino, haloalkylcarbonylamino, or arylcarbonylamino, R¹ is amino, R² is cyano and X, X¹, X², X³, X⁴ and Y have the meanings given in the general definition of the invention, may be prepared by reaction of a compound of formula (XV) with acid chlorides of formula R^{b}(C=O)Cl or acid anhydrides of formula [R^{b}(C=O)]₂O in which R^{b} is an alkyl, haloalkyl or aryl group as defined in the first definition of general formula (I) followed by a deprotection step. Solvents such as acetonitrile may be used and acid acceptors such as pyridine may be employed under appropriate conditions.
D) Likewise, compounds of general formula (I) in which R³ is alkylsulfonylamino or haloalkylsulfonylamino, R¹ is amino, R² is cyano and X, X¹, X², X³, X⁴ and Y have the meanings given in the general definition of the invention may be prepared from protected amino compounds of formula (XV) by reaction with alkyl or haloalkyl sulfonyl halides or sulfonic acid anhydrides under appropriate conditions followed by a deprotection step. Suitable amino protecting groups in such reactions include the alkoxyalkylideneimino group obtained by treatment of the amino compound with an alkylorthoformate. Deprotection procedures for such groups typically involve aqueous hydrolysis.
E) Compounds of general formula (I) in which R³ is alkylaminocarbonylamino or arylaminocarbonylamino, R¹ is amino, R² is cyano and X, X¹, X², X³, X⁴ and Y have the meanings given in the general definition of the invention may be prepared from a suitably protected amino compound of formula (XV) by reaction with an alkyl or aryl isocyanate in which the alkyl or aryl groups are as defined in the first definition of the invention, followed by deprotection. Proper conditions for formation of ureas are described by J. March in "Advanced Organic Chemistry" McGraw-Hill publ.(1985), p.802 and references cited therein. Suitable amino protecting groups in such reactions include the alkoxyalkylideneimino group with deprotection as described previously.
F) Compounds of general formula (I) in which R³ is alkoxycarbonylamino or haloalkyoxycarbonylamino, R¹ is amino, R² is cyano and X, X¹, X², X³, X⁴ and Y have the meanings given in the general definition of the invention may be prepared from a suitably protected amino compound of formula (XV) by reaction with an alkylchloroformate or haloalkylchloroformate followed by a deprotection step.
G) Compounds of general formula (I) in which R³ is alkylideneimino or benzylideneimino, R¹ is amino, R² is cyano and X, X¹, X², X³, X⁴ and Y have the meanings given in the general definition of the invention may be prepared by condensation of a suitably protected amino compound of formula (XV) with an alkyl or aryl aldehyde in which the alkyl or aryl groups are as defined in the first definition of the invention, followed by a deprotection step. Proper conditions for formation of Schiff's bases will be selected for the condensation step as described by J. March in *ibid*. p.1165 and references cited therein. Suitable amino protecting groups include acetyl or trifluoroacetyl and deprotection may be accomplished by alkaline hydrolysis or other methods as described by T. W. Greene in "Protective Groups in Organic Synthesis" J. Wiley publ. (1981) p. 254 and references cited therein.
H) Compounds of general formula (I) in which R³ is alkoxyalkylideneimino, R¹ is amino, R² is cyano and X, X¹, X², X³, X⁴ and Y have the meanings given in the general definition of the invention may be prepared from a protected amino compound of formula (XV) by condensation with an alkylorthoester followed by a deprotection reaction. Suitable protecting groups include amide or carbamate derivatives as discussed by T. W. Greene, *ibid*, p. 223 and 249.
I) Compounds of general formula (I) in which R³ is dialkylaminoalkylideneimino , R¹ is amino, R² is cyano and X, X¹, X², X³, X⁴ and Y have the meanings given in the general definition of the invention may be prepared by reacting a compound of formula (XV) with a dialkylacetal derivative of an *N,N*-dialkylformamide as described by T. W. Greene, *ibid*, p. 275., followed by a deprotection step. Alternatively, reaction of (XV) with an *N,N*-dialkylformamide in the presence of an agent such as phosphorous oxychloride under Vilsmeier conditions. Suitable protecting groups include amide or carbamate derivatives.
J) Compounds of general formula (I) in which R³ is alkylthioalkylideneimino, R¹ is amino, R² is cyano and X, X¹, X², X³, X⁴ and Y have the meanings given in the general definition of the invention may be prepared by reacting a compound of formula (XV) with a tris(alkylthio)methane in pyridine as solvent, optionally in the presence of a suitable catalyst, for example dimethyl(methylthio)sulfonium tetrafluorborate.
K) Compounds of general formula (I) in which R³ is azido, R¹ is amino, R² is cyano and X, X¹, X², X³, X⁴ and Y have the meanings given in the general definition of the invention may be prepared by reacting a compound of formula (XV) with *p*-toluenesulfonyl azide under conditions described by J. March *ibid*., p. 573 and references cited therein, followed by a deprotection step. Alternatively, the compounds described above in which R³ is azido may be prepared from a compound of formula (XV) by conversion of the amino group to a diazonium salt followed by reduction to a hydrazino group followed by treatment with nitrous acid to give the azide followed by a deprotection step.

### METHOD 19

A) Useful intermediate compounds of general formula (I) in which R³ is phenyl substituted as described in the general definition of the invention, R¹ is amino, R² is cyano and X, X¹, X², X³, X⁴ and Y have the meanings given in the general definition of the invention may be prepared from a compound of formula (XI) in which the amino group is optionally protected with a suitable protecting group, by treatment with an appropriately substituted phenyl diazonium salt under the conditions of the Gomberg-Bachmann reaction as described by M. Swainsbury in *Tetrahedron* **1980**, *36*, 3327-3359 and references cited therein.
B) Alternatively, the intermediate compounds of general formula (I) in which R³ is phenyl substituted as described in the general definition of the invention, R¹ is amino, R² is cyano and X, X¹, X², X³, X⁴ and Y have the meanings given in the general definition of the invention may be prepared from a compound of formula (XII) in which the amino group is optionally protected with a suitable protecting group by treatment with an appropriately substituted phenyl halide, preferably a bromide or iodide in the presence of copper under the conditions of the Ullmann reaction as described by M. Swainsbury *ibid*.
C) Alternatively, the intermediate compounds of general formula (I) in which R³ is phenyl substituted as described in the general definition of the invention, R¹ is amino, R² is cyano and X, X¹, X², X³, X⁴ and Y have the meanings given in the general definition of the invention may be prepared from a compound of formula (XII), preferably a bromide or iodide, by treatment with an appropriately substituted phenyl boronic acid in the presence of palladium (0) under conditions similar to those described by V. Snieckus *et. al.* in *Tetrahedron Letters* **1988**, *29*, 2135 and references cited therein.

### METHOD 20

Compounds of general formula (I) in which R³ is a cyano group, R¹ is amino, R² is cyano and X, X¹, X², X³, X⁴ and Y have the meanings given in the general definition of the invention may be prepared from the compounds of general formula (I) in which R³ is hydroxyiminomethylidenyl or alkoxyiminomethylidenyl described in METHOD 16 by dehydration according to procedures described in METHOD 4A.

### METHOD 21

Compounds of general formula (I) in which R³ is haloalkylcarbonyl or haloalkylthiocarbonyl, R¹ is amino, R² is cyano and X, X¹, X², X³, X⁴ and Y have the meanings given in the general definition of the invention may be prepared from an optionally amino-protected compound of formula (XIV) by the procedures discussed in METHOD 8 followed by a deprotection step, as necessary.

### METHOD 22

A) Compounds of general formula (I) in which R³ is haloalkyl, R¹ is amino, R² is cyano and X, X¹, X², X³, X⁴ and Y have the meanings given in the general definition of the invention may be prepared from compounds of formula (XII) or (XIV) in which the amino group is optionally protected, by the procedures described in METHODS 6, 7 and 9 with deprotection as necessary.
B) Compounds of general formula (I) in which R³ is alkyl, R¹ is amino, R² is cyano and X, X¹, X², X³, X⁴ and Y have the meanings given in the general definition of the invention may be prepared from compounds of formula (XIV) in which the amino group is optionally protected, by reaction with a Grignard reagent derived from an alkyl halide or an alkyllithium to produce a carbinol, followed by a dehydration step to produce a compound in which R³ is alkenyl, followed by reduction. Compounds of general formula (I) in which R³ is methyl and the other substituents are as described above may be prepared from compounds of formula (XIV) by the procedure described in METHOD 7.

### METHOD 23

Compounds of general formula (I) in which R³ is thiocyanato, alkylthio, haloalkylthio, alkenylthio, haloalkenylthio or phenylthio, R¹ is amino, R² is cyano and X, X¹, X², X³, X⁴ and Y have the meanings given in the general definition of the invention and the phenyl groups being constituted and/or substituted as described therein, i.e. compounds of formula (XVI) (vide infra), may be prepared from compounds of formula (XI) under similar conditions as described in METHOD 10. The overall process may be represented by the following equation:

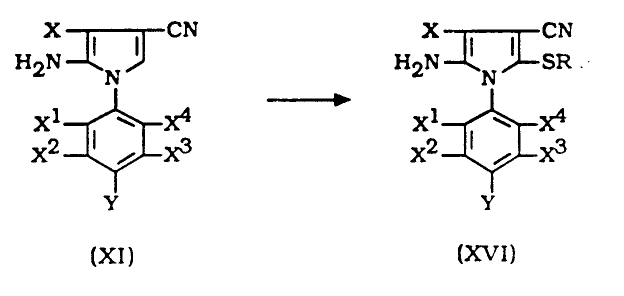

Compounds of formula (XVI) in which R represents alkyl may also be prepared from compounds of general formula (I) in which R³ is thiocyanato and the other substituents are defined as in (XVI) by treatment with alkyl halides or the like by procedures similar to those described in METHOD 11.

### METHOD 24

Compounds of general formula (I) in which R³ is alkylsulfinyl, alkylsulfonyl, alkenylsulfinyl, alkenylsulfonyl, haloalkylsulfinyl, haloalkylsulfonyl, haloalkenylsufinyl, haloalkenylsulfonyl, phenylsulfinyl or phenylsulfonyl R¹ is amino, R² is cyano and X, X¹, X², X³, X⁴ and Y have the meanings given in the general definition of the invention and the phenyl groups being constituted and/or substituted as described therein may be prepared by oxidizing compounds of formula (XVI) according to similar methods as those described in METHOD 12. In those instances when X is an RS group which may undergo undesired competitive oxidation, sulfenylation according to the above procedures may be carried out on a compound of general formula (I) in which X is halogen, preferably bromine or iodine, R¹ is amino, R² is cyano , R³ is hydrogen and the other substituents have the meanings given in the general description of the invention, followed by oxidation , followed by treatment with an alkyllithium by a procedure similar to those described by C. Kruse *et. al*., in *Heterocycles* **1989**, *29*, 79, followed by an aqueous quench to give a compound of formula (XVII). Compound (XVII) may then be sulfenylated to give compounds of formula (XVIII). The overall process may be represented as follows:

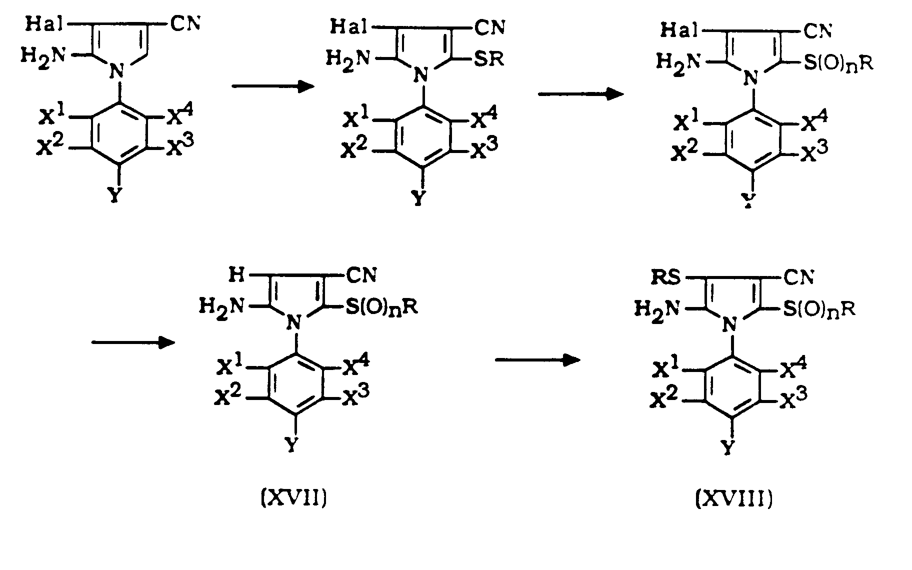

### METHOD 25

Compounds of general formula (I) in which R³ is cyanato, alkoxy or haloalkoxy, R¹ is amino, R² is cyano and X, X¹, X², X³, X⁴ and Y have the meanings given in the general definition of the invention may be prepared from compounds of formula (XII) in which the amino group is optionally protected, by procedures similar to those described in METHOD 5, followed by deprotection as required.

### METHOD 26

A) Compounds of general formula (I) in which R¹ is hydrogen, R² is cyano and R³, X, X¹, X², X³, X⁴ and Y have the meanings given in the general definition of the invention, i.e. compounds of formula (XX) (vide infra), may be prepared from compounds of general formula (I) in which R¹ is amino, R² is cyano and R³, X, X¹, X², X³, X⁴ and Y have the meanings given in the general definition of the invention, i.e. compounds of formula (XIX) (vide infra), diazotization, preferably with an alkyl nitrite such as *t*-butyl nitrite in an inert solvent such as tetrahydrofuran or acetonitrile. This reaction may be conducted between -80^{o} C and the reflux temperature of the solvent, preferably between 0^{o}C and 25^{o}C.
B) Compounds of general formula (I) in which R¹ halogen, R² is cyano and R³, X, X¹, X², X³, X⁴ and Y have the meanings given in the general definition, i.e. compounds of general formula (XXI) (vide infra), of the invention may be prepared from a compound of formula (XIX) by diazotization with an alkyl nitrite, for example *t*-butyl nitrite, in the presence of a halogen atom donor such as bromoform, carbon tetrachloride, anhydrous cupric chloride or iodine.
C) Compounds of general formula (I) in which R¹ is thiocyanato, alkylthio, haloalkylthio, alkenylthio, haloalkenylthio or phenylthio, R² is cyano and R³, X X¹, X², X³, X⁴ and Y have the meanings given in the general definition and the phenyl groups being constituted and/or substituted as described therein. i.e., compounds of formula (XXII) (vide infra), may be prepared from a compound of formula (XIX) by treatment with an alkyl nitrite in the presence of (SCN)₂ or a disulfide of formula RSSR in which R is an alkyl, haloalkyl, alkenyl, haloalkenyl or phenyl group as defined above. The reaction is typically performed in a solvent such as chloroform at 0 ^{o}C with one to five equivalents of the alkyl nitrite and two to five equivalents of the disulfide.

The overall processes may be illustrated as shown below:

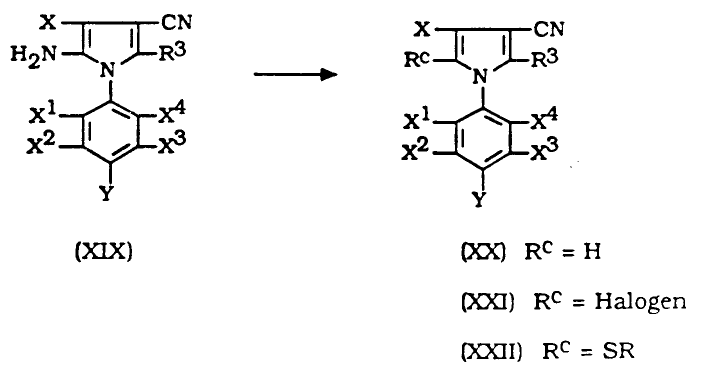

### METHOD 27

Alternatively, many of the compounds of formula (XXII) may be prepared from a compound of general formula (XX) in which R³ is amino by a procedure similar to that described in METHOD 10. Conversion of the amino group to other functional groups of the invention may then be effected by one of the procedures previously described.

### METHOD 28

Compounds of general formula (I) in which R¹ is alkylsulfinyl, alkylsulfonyl, alkenylsulfinyl, alkenylsulfonyl, haloalkylsulfinyl, haloalkylsulfonyl, haloalkenylsulfinyl, haloalkenylsulfonyl, phenylsulfinyl or phenylsulfonyl R² is cyano and R³, X, X¹, X², X³, X⁴ and Y have the meanings given in the general definition and the phenyl groups being constituted and/or substituted as described therein may be prepared by oxidation of compounds of formula (XXII) according to methods described in METHOD 24. If the X or R³ groups are also SR groups which are to be maintained at the sulfide oxidation level, a similar strategy as that described in METHOD 24 can be adopted to provide the desired compounds.

### METHOD 29

Compounds of general formula (I) in which R¹ is alkylamino, dialkylamino, aralkylamino, aminocarbonylamino, alkylcarbonylamino, haloalkylcarbonylamino, arylcarbonylamino, alkylsulfonylamino, haloalkylsulfonylamino, alkylaminocarbonylamino, arylaminocarbonylamino, alkoxycarbonylamino, haloalkoxycarbonylamino, alkylideneimino, benzylideneimino, alkoxyalkylideneimino, dialkylaminoalkylideneimino, alkylthioalkylideneimino or azido, R² is cyano and R³, X, X¹, X², X³, X⁴ and Y have the meanings given in the general definition of the invention may be prepared from a compound of general formula (XIX) by procedures similar to those described in METHOD 18.

### METHOD 30

A) Compounds of general formula (I) in which R¹ is formyl, R² is cyano and R³, X, X¹, X², X³, X⁴ and Y have the meanings given in the general definition of the invention may be prepared from a compound of general formula (XIX) by treatment with sodium nitrite , HC=NOH, copper sulfate and HCl in a manner analogous to that described by W. F. Beech *J. Chem. Soc*. **1954**, 1297. If R³ is an amino group in (XIX), suitable protection may be provided. The compound above in which R¹ is formyl may be converted to a compound in which R¹ is a bis(alkylthio)methyl or bis(arylthio)methyl group by standard methods of thioacetalization as described by T. W. Greene *ibid.,*, p. 130 and references cited therein. Alternatively, compounds of general formula (I) in which R¹ is bis(alkylthio)methyl or bis(arylthio)methyl, R² is cyano, R³ is amino and X, X¹, X², X³, X⁴ and Y have the meanings given in the general definition of the invention may be prepared from a compound of general formula (XX) in which R³ is amino by a procedure similar to that described in METHOD 14. Conversion of the bis(alkylthio)methyl or bis(arylthio)methyl group to formyl may be effected by procedures analogous to those described in METHOD 15. Conversion of the amino function to other functional groups of the invention may be effected by one the procedures previously described.
B) Compounds of general formula (I) in which R¹ is hydroxyiminoalkylidenyl or alkoxyiminoalkylidenyl, R² is cyano and R³, X, X¹, X², X³, X⁴ and Y have the meanings given in the general definition of the invention may be prepared from compounds of general formula (I) in which R¹ is alkylcarbonyl or formyl and the other substituents are as defined above by procedures similar to those described in METHOD 16. The compounds above in which R¹ is alkylcarbonyl may be prepared from the corresponding compounds in which R¹ is halogen by conversion to a Grignard reagent or lithium derivative, with optional protection of the cyano group as discussed in METHOD 5, followed by reaction with an aliphatic acid chloride or anhydride or alternatively condensation with an aliphatic aldehyde followed by oxidation. Alternatively, the compounds in which R¹ is alkylcarbonyl may be prepared from the corresponding compounds in which R¹ is formyl by reaction with alkyl Grignard reagent, followed by oxidation.
C) Compounds of general formula (I) in which R¹ is cyano, R² is cyano and R³, X, X¹, X², X³, X⁴ and Y have the meanings given in the general definition of the invention may be prepared from the corresponding compounds in which R¹ is hydroxyiminomethylidenyl or alkoxyiminomethylidenyl by procedures similar to those described in METHOD 4A).
D) Compounds of general formula (I) in which R¹ is haloalkylcarbonyl or haloalkylthiocarbonyl, R² is cyano and R³, X, X¹, X², X³, X⁴ and Y have the meanings given in the general definition of the invention may be prepared from a compound of general formula (I) in which R¹ is formyl and the other substituents are defined as above by treatment under conditions similar to those described in METHOD 8.
E) Compounds of general formula (I) in which R¹ is haloalkyl or alkyl, R² is cyano and R³, X, X¹, X², X³, X⁴ and Y have the meanings given in the general definition of the invention may be prepared from a compound of general formula (I) in which R¹ is formyl or halogen and the other substituents are defined as above by treatment under conditions similar to those described in METHOD 22.

### METHOD 31

Compounds of general formula (I) in which R¹ is phenyl or heteroaryl substituted as described in the general definition of the invention, R² is cyano and R³, X, X¹, X², X³, X⁴ and Y have the meanings given in the general definition of the invention may be prepared from compounds of general formula (I) in which R¹ is halogen and the other substituents are as defined above by procedures similar to those described in METHODS 19B) and 19C).

### METHOD 32

Compounds of general formula (I) in which R¹ is cyanato, alkoxy or haloalkoxy, R² is cyano and R³, X, X¹, X², X³, X⁴ and Y have the meanings given in the general definition of the invention may be prepared from compounds of formula (XXI) by procedures similar to those described in METHOD 5.

### METHOD 33

Compounds of general formula (I) in which R² is formyl, R¹, R³, X, X¹, X², X³, X⁴ and Y have the meanings given in the general definition of the invention, i.e. compounds of formula (XXIV) (vide infra), may be prepared from compounds of formula (I) in which R² is cyano, R¹, R³, X, X¹, X², X³, X⁴ and Y have the meanings given in the general definition of the invention, i.e. compounds of formula (XXIII) (vide infra), by treatment with a reducing agent, preferably diisobutylaluminum hydride in a solvent, preferably a 1:1 mixture of toluene and hexane by a procedure similar to that described by S. Trofimenko in *J. Org. Chem.* **1964**, *29*, 3046. The overall process is as shown below:

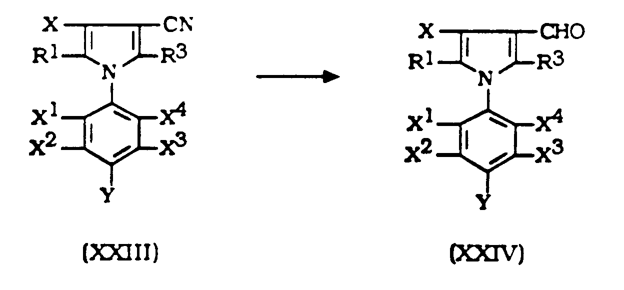

### METHOD 34

Useful intermediate compounds of general formula (I) in which R² is a carboxylic acid function, R¹, R³, X, X¹, X², X³, X⁴ and Y have the meanings given in the general definition of the invention, i.e. compounds of formula (XXV) (vide infra), may be prepared by oxidation of the compounds of formula (XXIV) with Jones' reagent. The overall process is as follows:

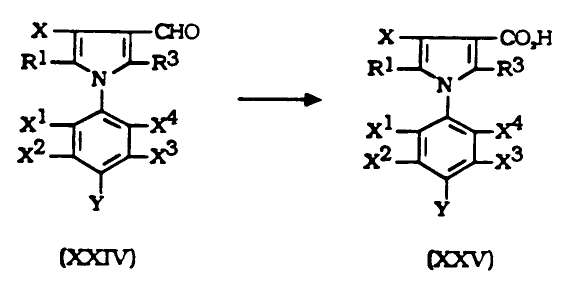

### METHOD 35

Compounds of general formula (I) in which R² is hydroxyiminoalkylidenyl, alkoxyiminoalkylidenyl, haloalkylcarbonyl, haloalkylthiocarbonyl, alkyl, bis(alkylthio)methyl, bis(arylthio)methyl haloalkyl, and R¹, R³, X, X¹, X², X³, X⁴ and Y have the meanings given in the general definition of the invention may be prepared from compounds of formula (XXIV) or compounds of general formula (I) in which R² is halogen and the other substituents are defined as above, whose preparation is described in METHOD 38, by procedures similar to those described in METHOD 30 A), B), D) and E).

### METHOD 36

Compounds of general formula (I) in which R² is amino, R¹, R³, X, X¹, X², X³, X⁴ and Y have the meanings given in the general definition of the invention, i.e. compounds of formula (XXVII) (vide infra) may be prepared from the compounds of formula (XXV) by treatment with diphenylphosphoryl azide in the presence of an organic base such as triethylamine in an alcoholic solvent such as *tert*.-butanol to produce a carbamate (XXVI) followed by hydrolysis. Other methods of producing (XXVII) from (XXV) *via* a Curtius rearrangement include conversion to the acid chloride followed by reaction with azide ion and treatment with an alcohol as described by J. March in "Advanced Organic Chemistry" McGraw-Hill publ.(1985). p.984. The overall transformation is as follows:

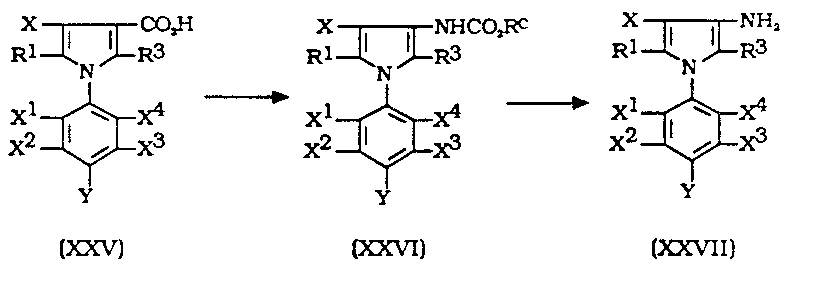

### METHOD 37

Compounds of general formula (I) in which R² is alkylamino, dialkylamino, aralkylamino, aminocarbonylamino, alkylcarbonylamino, haloalkylcarbonylamino, arylcarbonylamino, alkylsulfonylamino, haloalkylsulfonylamino, alkylaminocarbonylamino, arylaminocarbonylamino, alkoxycarbonylamino, haloalkoxycarbonylamino, alkylideneimino, benzylideneimino, alkoxyalkylideneimino, dialkylaminoalkylideneimino, alkylthioalkylideneimino or azido, R¹, R³, X, X¹, X², X³, X⁴ and Y have the meanings given in the general definition of the invention may be prepared from the compounds of formula (XXVII) by procedures similar to those described in METHOD 18.

### METHOD 38

Compounds of general formula (I) in which R² is hydrogen, halogen, thiocyanato, alkylthio, haloalkylthio, alkenylthio, haloalkenylthio or phenylthio R¹, R³, X, X¹, X², X³, X⁴ and Y have the meanings given in the general definition of the invention may be prepared from the compounds of formula (XXVII) by procedures similar to those described in METHOD 26. Alternatively, the compounds of general formula (I) in which R² is hydrogen, R¹, R³, X, X¹, X², X³, X⁴ and Y have the meanings given in the general definition of the invention may be prepared from compounds of formula (XXV) by heating with 48% HBr in glacial acetic acid at reflux or heating in a high boiling solvent such as decalin or quinoline in the presence of copper.

### METHOD 39

Compounds of general formula (I) in which R² is alkylsulfinyl, alkylsulfonyl, alkenylsulfinyl, alkenylsulfonyl, haloalkylsulfinyl, haloalkylsulfonyl, haloalkenylsulfinyl, haloalkenylsulfonyl, phenylsulfinyl or phenylsulfonyl R¹, R³, X, X¹, X², X³, X⁴ and Y have the meanings given in the general definition of the invention may be prepared by oxidizing compounds of general formula (I) in which R² is alkylthio, haloalkylthio, alkenylthio, haloalkenylthio or phenylthio by procedures similar to those described in METHOD 24.

### METHOD 40

Compounds of general formula (I) in which R² is phenyl or heteroaryl substituted as described in the general definition of the invention, R¹, R³, X, X¹, X², X³, X⁴ and Y have the meanings given in the general definition of the invention may be prepared from compounds of general formula (I) in which R² is halogen and the other substituents are as defined above by procedures similar to those described in METHODS 19B and 19C.

### METHOD 41

Compounds of formula (I) in which R² is cyanato, alkoxy or haloalkoxy, R¹, R³, X, X¹, X², X³, X⁴ and Y have the meanings given in the general definition of the invention may be prepared from compounds of general formula (I) in which R² is halogen and the other substituents are as defined above by procedures similar to those described in METHOD 5.

In a global manner the process invention may be defined as described below:
P₁. A process for the preparation of compounds of formula 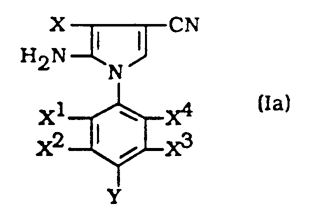 wherein X¹, X², X³, X⁴ and Y have the same meaning as in method one, X is halogen, trifluoromethyl, cyano, alkylthio, alkylsulfinyl, alkylsulfonyl, haloalkylthio, haloalkylsulfinyl or haloalkylsulfonyl, wherein a compound of formula 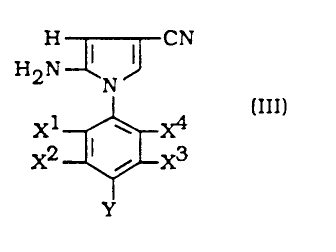 where amino is optionally protected:
   (a) is reacted with a halogenating agent, optionally in the presence of a solvent to obtain a compound of formula (Ia) wherein X is halogen, then optionally reacting said compound with trifluoromethyl copper in a known manner to get compounds of formula (Ia) where X is trifluoromethyl;
   (b) is reacted with a tris(alkylthio)methane or tris(arylthio)methane in the presence of a Lewis acid, then the obtained compound of formula (XXXVI) where X is bis(alkylthio)methyl or bis(arylthio)methyl is reacted with a suitable alkyl nitrite followed by hydrolysis, in order to obtain the compound of formula (XXXVII) where X is formyl, then bringing the said compound into contact with hydroxylamine followed by dehydration with suitable agents such as P₂O₅ in a known manner in order to obtain the compound of formula (Ia) where X is a cyano group;
   (c) is reacted with a compound of formula MSCN, M being an alkali metal, in the presence of bromine, in a solvent such as methanol in order to obtain the compound of formula (Ia) where X is a thiocyanato group and then reacting said compound with an alkyl halide or dialkyl sulfate in the presence of a base such as NaOH or KOH in a solvent to get a compound of formula (Ia) where X is alkylthio; or
   (d) is reacted with a sulfenyl halide of formula RSHal, in which R is an alkyl, haloalkyl or phenyl radical, Hal is a halogen atom in an organic liquid reaction medium, optionally in the presence of an acid acceptor such as a tertiary amine in order to get a compound of formula (Ia) where X is alkylthio or haloalkylthio, then optionally oxidizing in a known manner the obtained compound, in order to get a compound of formula (Ia) where X is RS(O)ₙ wherein n equals 1 or 2 depending on reaction conditions.
P₂. A process for the preparation of compounds of formula (Ia) wherein X¹, X², X³, X⁴ and Y have the same meaning as in method one, X is alkoxy, haloalkoxy,
   wherein a compound of formula: 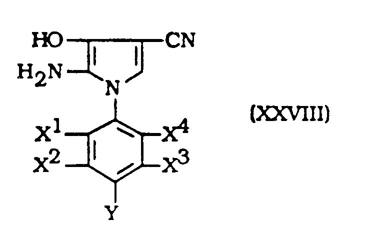 where the amino and cyano groups are suitably protected if necessary:
   (a) is reacted with an alkylating agent, optionally in the presence of a base to obtain compounds of formula (Ia) where X is alkoxy; or
   (b) is haloalkylated in a known manner, according to Syntheses *of Fluoroorganic Compounds*; Knunyants, I. L. and Yakobson, G. G., Ed.; Springer-Verlag: Berlin. 1985; pp 263 - 269, to obtain compounds of formula (Ia) where X is haloalkoxy.
P₃. A process for the preparation of compounds of formula (Ia) wherein X¹, X², X³, X⁴ and Y have the same meaning as in method one and X is haloalkyl [CF₂H, CF₃, BrCH₂, ClCH₂], or a-haloalkyl-a-halomethyl,
   wherein a compound of formula: 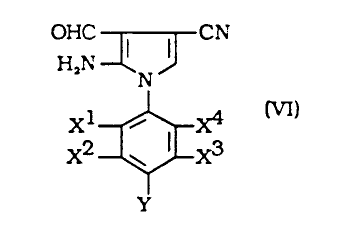 where amino and cyano are suitably protected if needed:
   (a) is reacted with a fluorinating agent such as diethylaminosulfur trifluoride in a known manner in order to obtain the compound of formula (Ia) wherein X is a difluoromethyl group;
   (b) is reacted with a suitable oxidizing agent such as chromium trioxide in sulfuric acid to provide compounds where X is a carboxylic acid group, then submitting the said compounds to a fluorinating agent such as sulfur tetrafluoride in a known manner to get the compound of formula (Ia) where X is trifluoromethyl;
   (c) is reacted under Wolff-Kishner conditions, or a variant such as treatment with *p*-toluenesulfonylhydrazide followed by sodium cyanoborohydride, to obtain a compound where X is a methyl group then submitting the said compound to a halogenating agent such as *N*-bromosuccinimide or *N*-chlorosuccinimide in a suitable solvent in order to obtain a compound of formula (Ia) where X is bromomethyl or chloromethyl; or
   (d) is reacted sequentially with a haloalkyl metal derivative or trifluoromethyltrimethylsilane to provide compounds of formula (Ia) where X is haloalkylcarbinol, then the compound where X is haloalkylcarbinol is reacted with halogenating agents such as thionyl chloride or hydrogen bromide in order to get compounds of formula (Ia) where X is α-haloalkyl-α-halomethyl, all the previous steps having been followed by a deprotection step if needed.
P₄. A process for the preparation of compounds of formula (Ib) 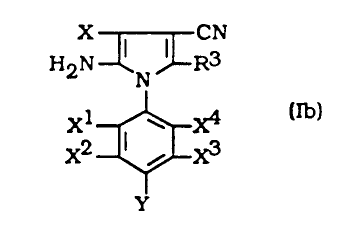 where X, X¹, X², X³, X⁴ and Y have the same meaning as in method one and, R³ is halogen, formyl, haloalkyl, alkyl, alkylthio, haloalkylthio, alkylsulfinyl or alkylsulfonyl, wherein a compound of formula (Ia) in which X, cyano and amino are optionally protected in a suitable manner if required:
   (a) is reacted according to method P₁(a) to get compounds of formula (Ib) where R³ is halogen, then said compound is optionally reacted according to method P₁(a) to get compounds of formula (Ib) where R³ is trifluoromethyl;
   (b) is reacted according to method P₁(b) to get first a compound of formula (Ib) where R³ is a bis(alkylthio)methyl group or a bis(arylthio)methyl group and then a compound of formula (Ib) where R³ is formyl;
   (c) is reacted according to method P₁(c,d) in order to get compounds of formula (Ib) where R³ is alkylthio or haloalkythio, then optionally oxidized according to method P₁(d) in order to get the compounds of formula (Ib) where R³ is alkylsulfinyl, alkylsulfonyl, haloalkylsulfinyl or haloalkylsulfonyl, with the proviso that X is not an RS group which may undergo undesired oxidation, and when X is a halogen, optional treatment with an alkylithium in a known manner followed by an aqueous quench and sulfenylation according to method P₁(c,d) in order to get compounds of formula (Ib) where X is alkylthio or haloalkylthio.
P₅. A process for the preparation of compounds of formula (Ib) wherein X, X¹, X², X³, X⁴ and Y have the same meaning as in method one and R³ is cyano or alkyl wherein a compound of formula 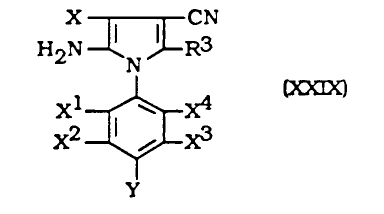 in which R³ is formyl or alkylcarbonyl and X, cyano and amino are protected in a suitable manner if required:
   (a) is condensed with hydroxylamine or *O*-alkylhydroxylamine or their addition salts in a solvent such as ethanol in order to obtain a compound of formula (Ib) where R³ is hydroxyiminoalkylidenyl or alkoxyiminoalkylidenyl, and the elements of water or an alcohol are eliminated according to method P₁(b) to obtain a compound of formula (Ib) where R³ is a cyano group;
   (b) is reacted, when R³ is formyl, according to method P₃(a,b,c,d) to obtain a compound of formula (Ib) where R³ is a methyl or haloalkyl group; or
   (c) is reacted, when R³ is formyl, with a Grignard reagent derived from an alkyl halide or an alkyllithium to produce a carbinol, followed by a dehydration step to produce a compound where R³ is alkenyl, followed by reduction in order to get compounds of formula (Ib) where R³ is alkyl, followed optionally by a deprotection step.
P₆. A process for the preparation of compounds of formula 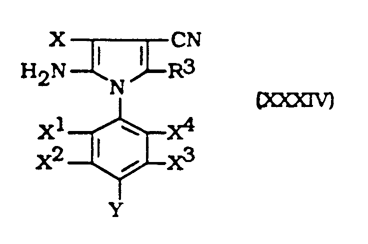 useful as intermediate compounds wherein X, X¹, X², X³, X⁴ and Y are as defied in method one and R³ is amino, alkylamino or dialkylamino, wherein a compound of formula 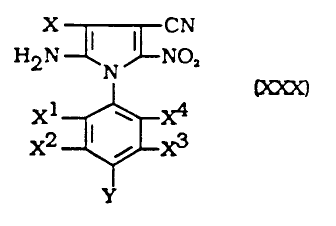 where the amino group is protected in a suitable manner, is reduced in order to get compounds of formula (XXXIV) where R³ is amino, said compounds being reacted:
   with appropriate alkylating agents in organic solvents, the mono or disubstituted amino group being obtained depending on the stoichiometric ratio or reaction conditions or through conversion of the amino group to an alkoxyalkylideneimino followed by reduction in order to obtain compounds of formula (XXXIV) where R³ is alkylamino or dialkylamino.
P₇. A process of preparation of compound of formula (Ib) wherein X, X¹, X², X³, X⁴, Y have the same meaning as in method one and R³ is alkoxy or haloalkoxy wherein a compound of formula 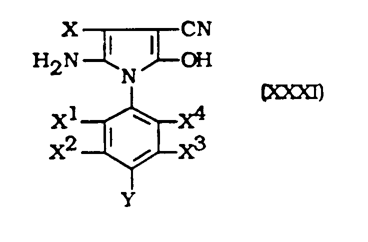 in which amino, cyano and X, are optionally protected in a suitable manner is reacted according to method P₂ (a), (b), (c) to obtain a compound of formula (Ib) wherein R³ is alkoxy or haloalkoxy.
P₈. A process for the preparation of compounds of formula 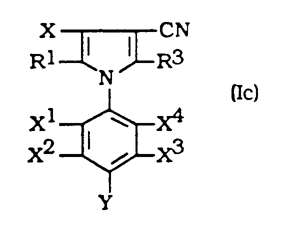 wherein X, R³, X¹, X², X³, X⁴, Y have the same meaning as in method one and R¹ is hydrogen, halogen, alkylthio, haloalkylthio, alkylsulfinyl, alkylsulfonyl, haloalkylsulfinyl, haloalkylsulfonyl, alkylamino, dialkylamino, formyl, haloalkyl or alkyl, wherein a compound of formula 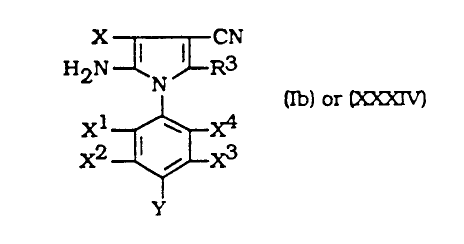 prepared according to methods P₄ to P₇ in which the amino group is deprotected after protecting the X, R³ or cyano group if required:
   (a) is reacted with a diazotization agent, preferably with an alkyl nitrite, in an inert solvent to obtain a compound of formula (Ic) where R¹ is H;
   (b) is reacted with a diazotization agent, preferably with an alkyl nitrite, in the presence of a halogen donor to obtain compound of formula (Ic) where R¹ is halogen;
   (c) is reacted with a diazotization agent, preferably with an alkyl nitrite, in the presence of (SCN)₂ or a disulfide in a solvent such as chloroform to obtain compounds of formula (Ic) where R¹ is alkylthio or haloalkylthio, then optionally oxidized according to method P₁(d) to get a compound of formula (Ic) where R¹ is alkylsulfinyl or alkylsulfonyl;
   (d) is reacted according to method P₆ in order to get compounds of formula (Ic) where R¹ is alkylamino or dialkylamino; or
   (e) is reacted with sodium nitrite and formaldoxime, copper sulfate and HCl in a known manner in order to get compounds of formula (Ic) where R¹ is formyl, then optionally (i) reacted according to method P₅ so as to obtain a compound of formula (Ic) where R¹ is cyano or (ii) is reacted according to method P₃(a-d) to obtain a compound of formula Ic where R¹ is haloalkyl or alkyl followed if needed by a deprotection step.
P₉. A process for the preparation of compounds of formula (Ic) wherein X, R³, X¹, X², X³, X⁴, Y have the same meaning as in method one and R¹ is alkoxy or haloalkoxy, wherein a compound of formula 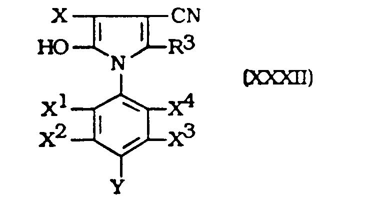 in which X, cyano or R³ are optionally protected in a known manner, is reacted according to method P₂(a or b) to obtain a compound of formula (Ic) where R¹ is alkoxy or haloalkoxy followed by an optional deprotection step.
P₁₀. A process for the preparation of compounds of formula (I) wherein X, R¹, R³, X¹, X², X³, X⁴, Y have the same meaning as in method one, and R² is CHO, wherein a compound of formula (Ic) is treated with a reducing agent, preferably diisobutylaluminum hydride, in a solvent to provide the compound in which R² is CHO, said compound being optionally oxidized in a known manner to get the corresponding compound of formula (XXXV) 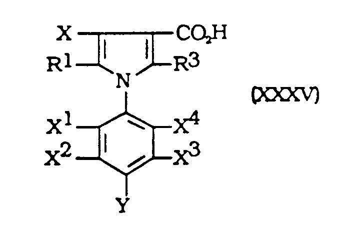
P₁₁. A process for the preparation of compounds of formula (I) wherein X, R¹, R³, X¹, X², X³, X⁴, Y have the same meaning as in method one and R² is alkyl, haloalkyl or cyano, wherein a compound of formula (I) in which R² is CHO, after having optionally protected X, R¹, or R³ if required in a known manner is reacted according to method P₃(a,b,c,d), P₅(a,b) or P₈(e), followed by a deprotection step if required.
P₁₂ A process for the preparation of compounds of formula (I) wherein X, R¹, R³, X¹, X², X³, X⁴, Y have the same meaning as in method one, and R² is amino, alkylamino, dialkylamino, hydrogen, halogen, alkylthio, haloalkylthio, alkylsulfinyl, alkylsulfonyl, haloalkylsulfinyl, haloalkylsulfonyl, or trifluoromethyl wherein a compound of formula (XXXV) after having optionally protected X, R¹, or R³ if required in a known manner is reacted under conditions for the Curtius rearrangement, for example by conversion to an acid chloride followed by reaction with an alkali metal azide, or with diphenyl phosphoryl azide in the presence of an organic base such as triethylamine in an alcoholic solvent to produce a carbamate which may then be hydrolyzed to obtain the corresponding compound in which R² is amino which is then optionally reacted according to method P₆(a-k) or P₈(a-c), then when R² is halogen, optionally reacted according to method P₄(a), followed by a deprotection step if necessary.
P₁₃. A process for the preparation of compounds of formula (I) wherein X, R¹, R³, X¹, X², X³, X⁴, Y have the same meaning as in method one and R² is alkoxy or haloalkoxy, wherein a compound of formula 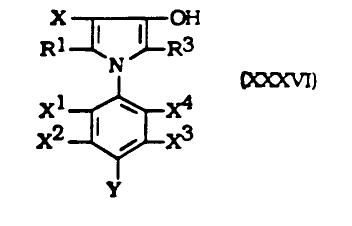 after having optionally protected the X, R¹, or R³ groups if required, is reacted according to method P₇ followed by a deprotection step if necessary.
P₁₄. A process for the preparation of compounds of formula (XXVIII), (XXXI), (XXXVI) and (XXXVII) according to methods P₂, P₇, P₁₀ or P₁₃, wherein the corresponding halogenated compound according to methods P₁(a), P₄(a), P₈(b) or P₁₂, after having protected the amino group if present, is converted to a Grignard reagent or lithium derivative, followed by reaction with a trialkyl borate and oxidation in a known manner, followed by a deprotection step if necessary.
P₁₅. A process for the preparation of a compound of formula 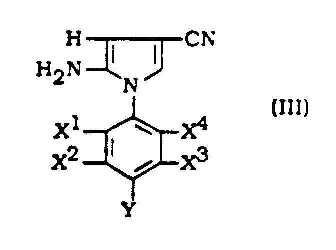 in which X¹, X², X³, X⁴, Y have the same meaning as in formula (I), wherein a dicyanopropene derivative of formula 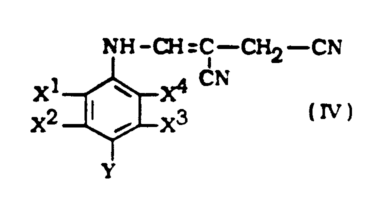 is reacted with a basic agent.
P₁₆. The invention is also related to compounds of formula (III) and (XXVIII) to (XXXVIII), wherein the various substituents have the same meaning as in previous methods, with the exclusion of compounds wherein R¹ = R² = H, R³ = amino, X = cyano, X³ = X⁴ = H and: X¹ = methyl, methoxy or chlorine and X² = Y = H; X² = methyl, methoxy or chlorine and X¹ = Y = H; Y = methyl methoxy or chlorine and X¹ = X² = H. especially useful as intermediate compounds for preparing compounds of formula (I) according to process methods P₁ to P₁₅.

### OTHER METHODS

Compounds of formula (I) wherein X is a perhaloalkylthio group, are additionally prepared by the following process of chlorosulfonation, reduction to a disulfide and finally free radical promoted reduction. The process is as follows:
A) Compounds of the general formula (XXXIX) (vide infra) wherein the definitions of R¹, R², R³, Y, X¹, X², X³, and X⁴ are those herein above defined in formula (I), can be prepared from compounds of the general formula (XXXVIII) (vide infra) wherein X is hydrogen, by treatment with chlorosulfonic acid either neat or in the presence of an organic solvent such as chloroform, dichloromethane, carbon tetrachloride, or dimethylformamide at a reaction temperature from 0^{o}C to 150^{o}C. A more specific example would be the preparation of a compound of the general formula (XXXIX) (vide infra) in which R¹ is amino, alkylcarbonylamino, or haloalkylcarbonylamino, R² is cyano, and R³ is hydrogen, from compounds of the general formula (XXXVIII) in which R¹ is amino, alkylcarbonylamino, or haloalkylcarbonylamino, R² is cyano, R³ is hydrogen, and X is hydrogen, by treatment with chlorosulfonic acid. A representative procedure for chlorosulfonation of an aromatic compound is given in J. March, "Advanced Organic Chemistry," McGraw-Hill publ. (1968), p. 402. 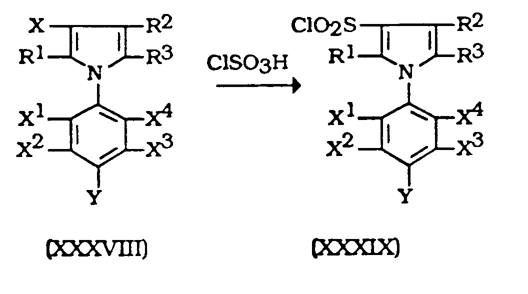
   Compounds of the general formula (XL) (vide infra) wherein the definitions of R¹, R² Y, X¹, X², X³, and X⁴ are those herein above defined in formula (I), can be prepared from compounds of the general formula (XXXIX) wherein R³ is hydrogen, by treatment with a chlorinating agent such as chlorine, N-chlorosuccinimide or sulfuryl chloride, in an organic solvent such as diethyl ether, acetonitrile, or dichloromethane at a reaction temperature from -70^{o}C to 25^{o}C. A more specific example would be the preparation of a compound of the general formula, (XL) in which R¹ is amino and R² is cyano, by treatment of a compound of the general formula (XXXIX) in which R¹ is amino and R² is cyano, and R³ is hydrogen, with sulfuryl chloride in diethyl ether at -40^{o}C. 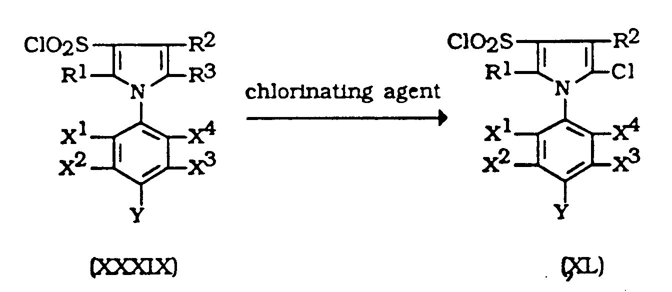
B) Compounds of the general formula (XLI) (vide infra) wherein the definitions of R¹, R², Y, X¹, X², X³, and X⁴ are those herein above defined in formula (I), can be prepared from compounds of the general formula (XXXIX) by treatment with a reducing agent, such as triphenylphosphine, in the presence of an organic solvent, such as tetrahydrofuran, toluene, or dichloromethane, at a reaction temperature from 0^{o}C to 110^{o}C. A more specific example would be the preparation of a compound of the general formula (XLI) in which R¹ is hydrogen or amino, R² is cyano, and R³ is hydrogen or Cl, from a compound of the general formula (XXXIX) wherein R¹ is hydrogen or amino, R² is cyano, and R³ is hydrogen or Cl, by treatment with triphenylphosphine in tetrahydrofuran at 25^{o}C. A representative example of a procedure for the reduction of toluene to p-tolyldisulfide is provided in G. A. Olah et al. J. Org. Chem. *1980*, **45**, 4792. 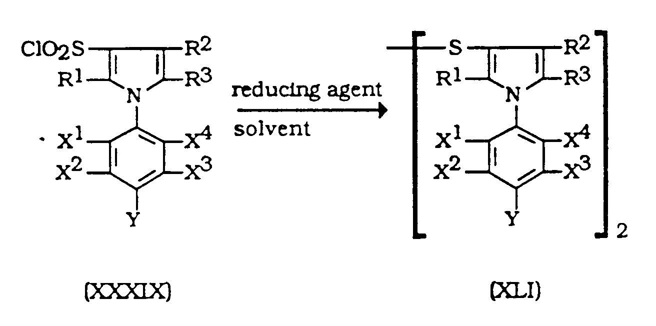
C) Compounds of the general formula (I) wherein the definitions of R¹, R², Y, X¹, X², X³, and X⁴ are those herein above defined in formula (I), and X is a perhaloalkylthio group, R⁶S, in which R⁶ is CFR⁷R⁸ and R⁷ is F, Cl, or Br, and R⁸ is F, Cl, Br or a perfluoroalkyl group, can be prepared from the reaction of a compound of the general formula (XLI) and a perhaloalkane compound of the general formula (XLII), ZCFR⁷R⁸, wherein Z is Cl, Br, or I, R⁷ is F, Cl or Br, and R⁸ is F, Cl, Br or a perfluoroalkyl group, with a reducing agent which can promote the formation of the free radical CFR⁷R⁸ from ZCFR⁷R⁸, preferably chosen from the metals zinc, cadmium, aluminum, manganese, or a compound with an oxide of sulfur, eg., the dithionites or the hydroxymethylsulfinates. The metal dithionite e.g. alkali dithionite or alkaline earth, corresponds to the general formula (XLIII), Mₙ(S₂O₄), in which n can be 1 or 2 depending upon the valence of the metal M. When one uses a dithionite of the general formula (XLIII) or a hydroxymethylsulfinate, one needs to add a base, eg. chosen from among the alkaline hydroxides, alkaline earth hydroxides, ammonia, triethylbenzylammonium, or the salts of weak acids such as disodium phosphate, sodium metabisulfite, sodium hydrogen sulfite, or sodium borate. The reation is generally performed in a solvent (which can solubilize the dithionite or the hydroxymethylsulfinate and the compound (XLII), ZCFR⁷R⁸), such as acetonitrile formamide, dimethylformamide, dimethylacetamide, hexamethylphosphoramide, N-methylpyrrolidone, dimethylsulfoxide, or sulfolane, at a reaction temperature from 20^{o}C to 85^{o}C. The alkaline dithionite can be added to the reaction mixture as a saturated solution in water or formamide. It is also possible to add the dithionite as a solid. When one is working with a gas which is only slightly soluble in the reaction solvent, the reaction pressure can be increased e.g. from one atmosphere to 50 atmospheres. A more specific example would be the preparation of a compound of the general formula (I) in which R¹ is hydrogen or amino, R² is cyano, R³ is hydrogen or Cl, and X is a perhaloalkylthio group, R⁶S, in which R⁶ is CFR⁷R⁸ and R⁷ is F, Cl or Br and R⁸ is F, Cl, Br or a perfluoroalkyl group, from the reaction of a compound of the general formula (XLI) in which R¹ is hydrogen or amino, R² is cyano, and R³ is hydrogen or Cl, and a compound of the general formula (XLII), ZCFR⁷R⁸, wherein Z is Cl, Br or I, R⁷ is F or Cl, Br, and R⁸ is F, Cl, Br or a perfluoroalkyl group, with sodium dithionite and disodium phosphate in dimethylformamide at 25^{o}C. The reaction is represented by the following equation: 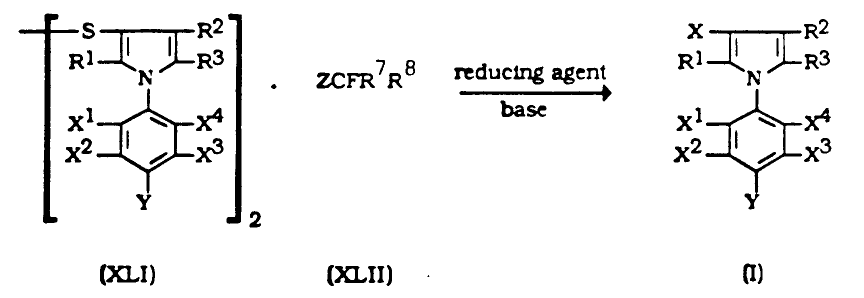
   The intermediate chlorosulfonyl compound of formula (XXXIX) and the intermediate disulfide compound of formula (XLI) are an additional part of the present invention.

### REPRESENTATIVE COMPOUNDS OF THE INVENTION

Specific representative pyrrole compounds (RPC) which are contemplated in the invention are compounds of formula (I) wherein R² is cyano and the other substituents have the meanings as described in **TABLE 1** (RPC-No's 1-389).

Other specific representative pyrrole compounds (RPC) which are included in the invention are compounds of formula (I) wherein X² and X³ are hydrogen, X¹ and X⁴ are chloro, Y is CF₃, and X, R¹, R² and R³ have the meanings as described in **TABLE 2** (RPC-No's 390-491).

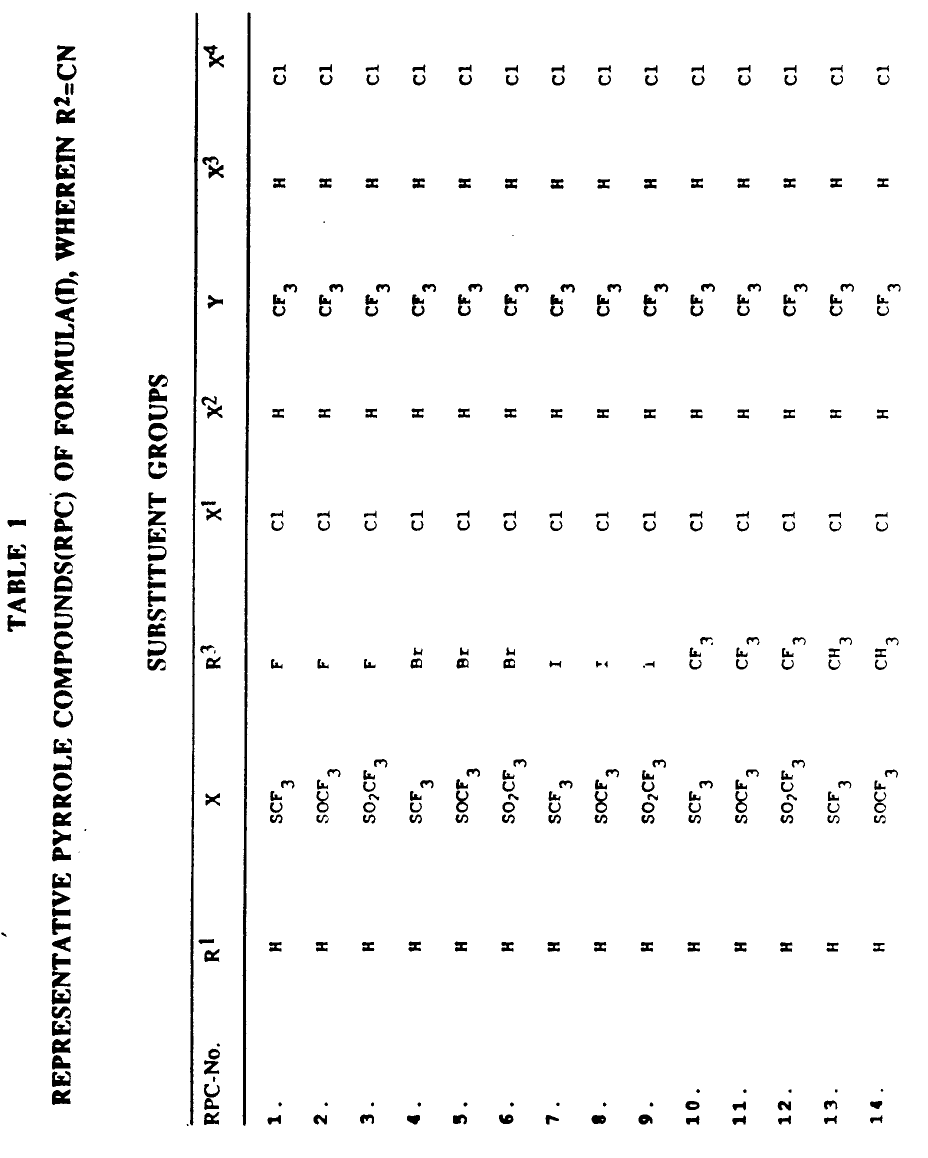

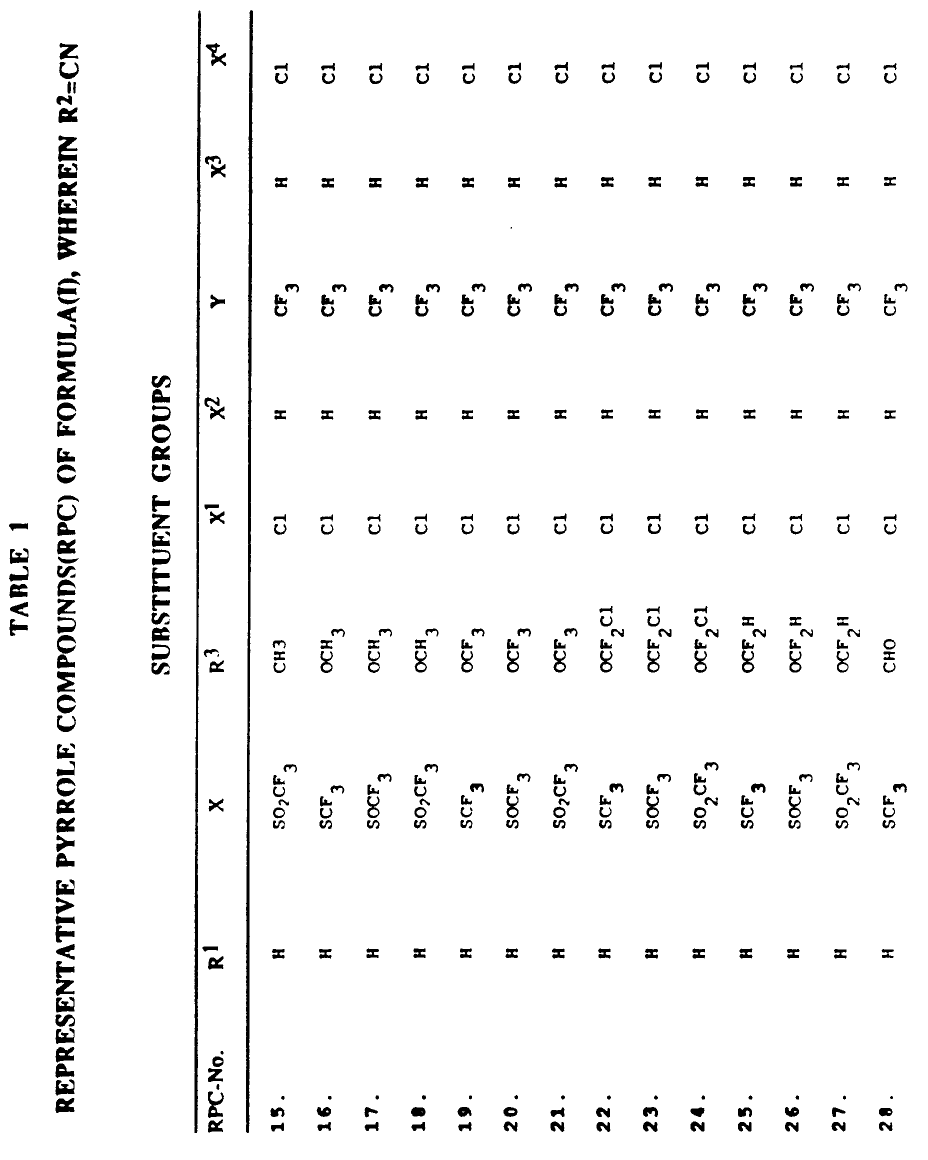

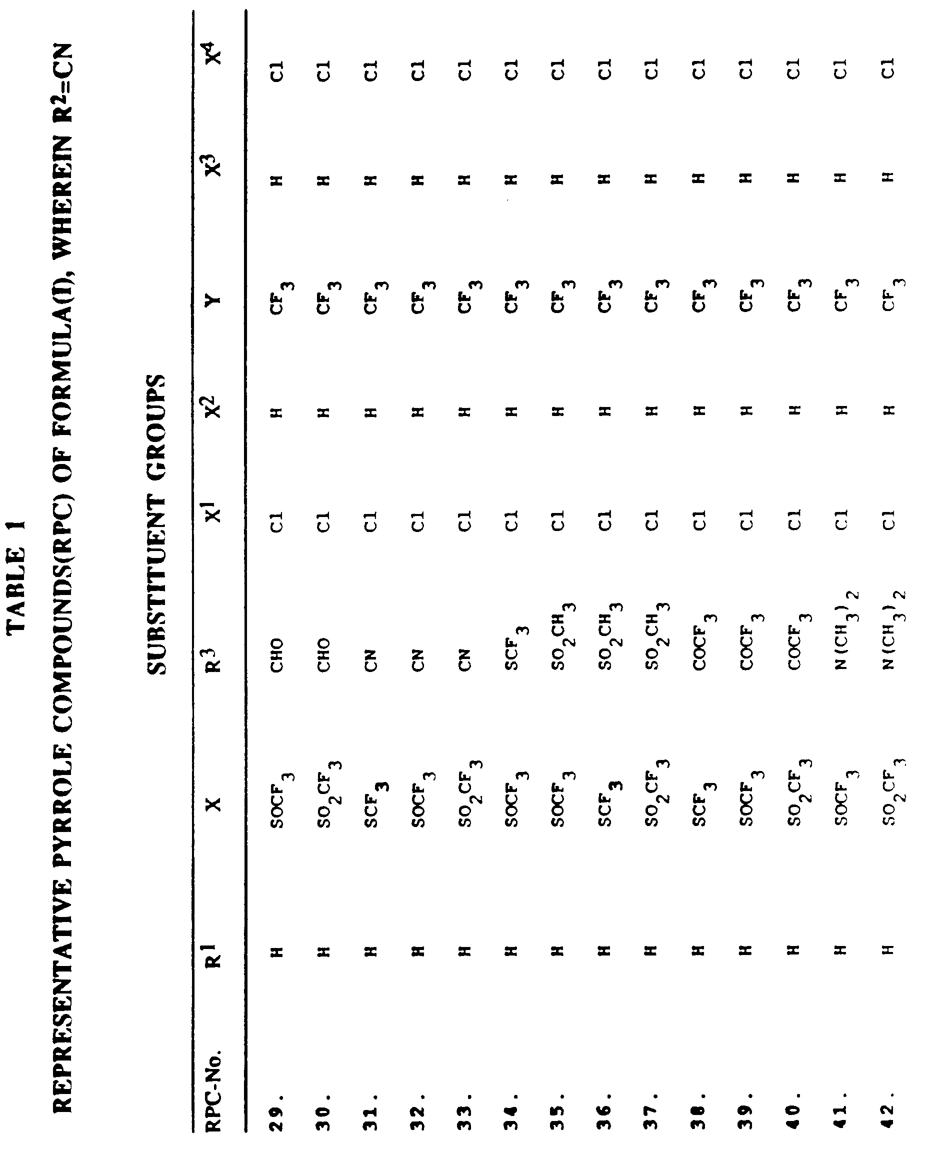

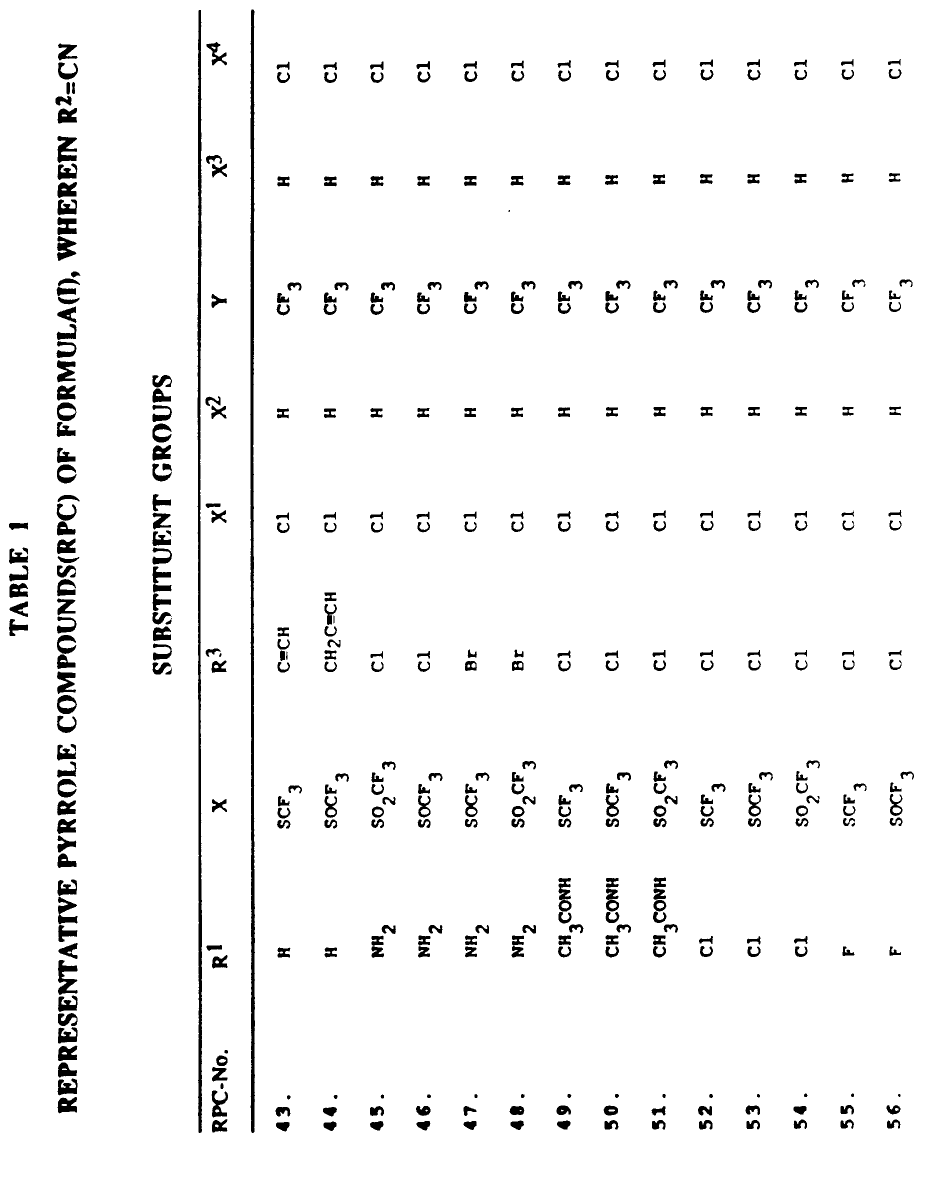

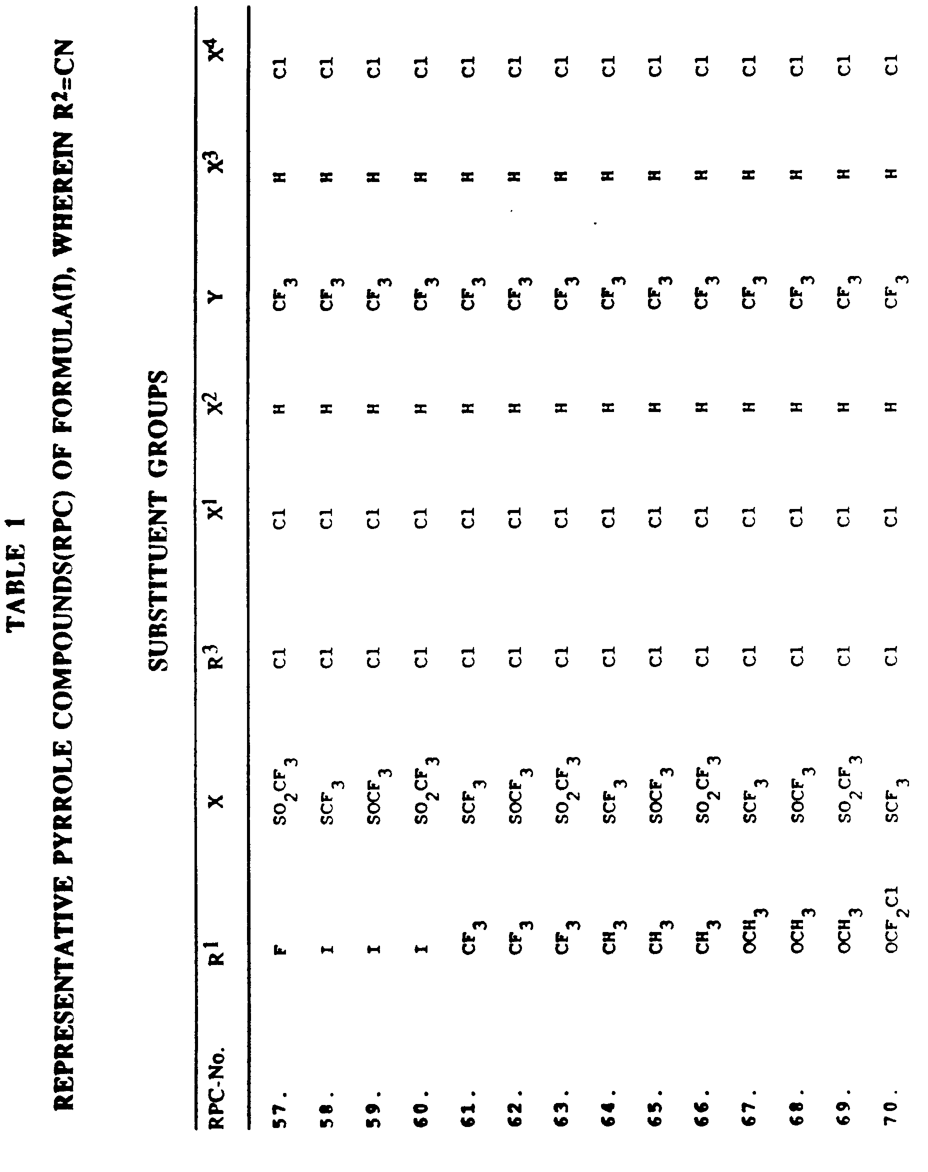

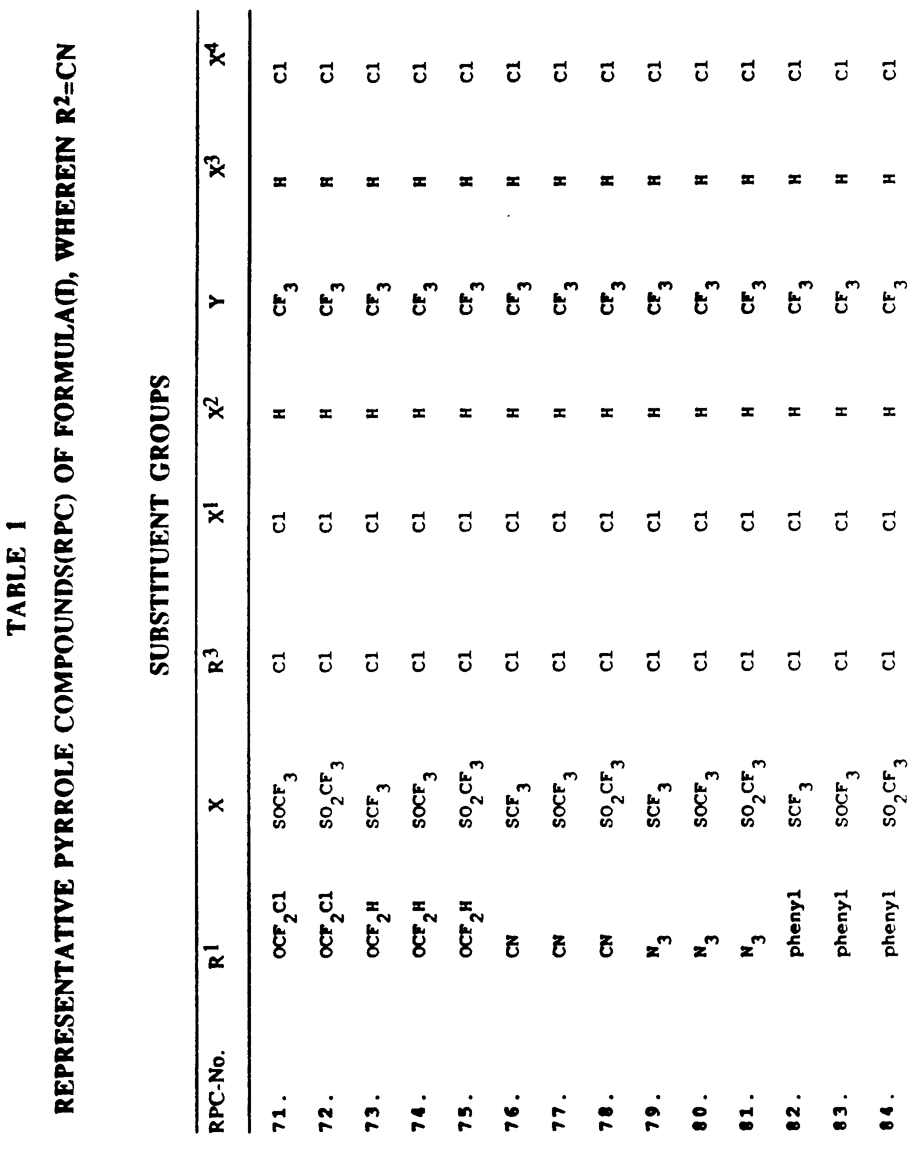

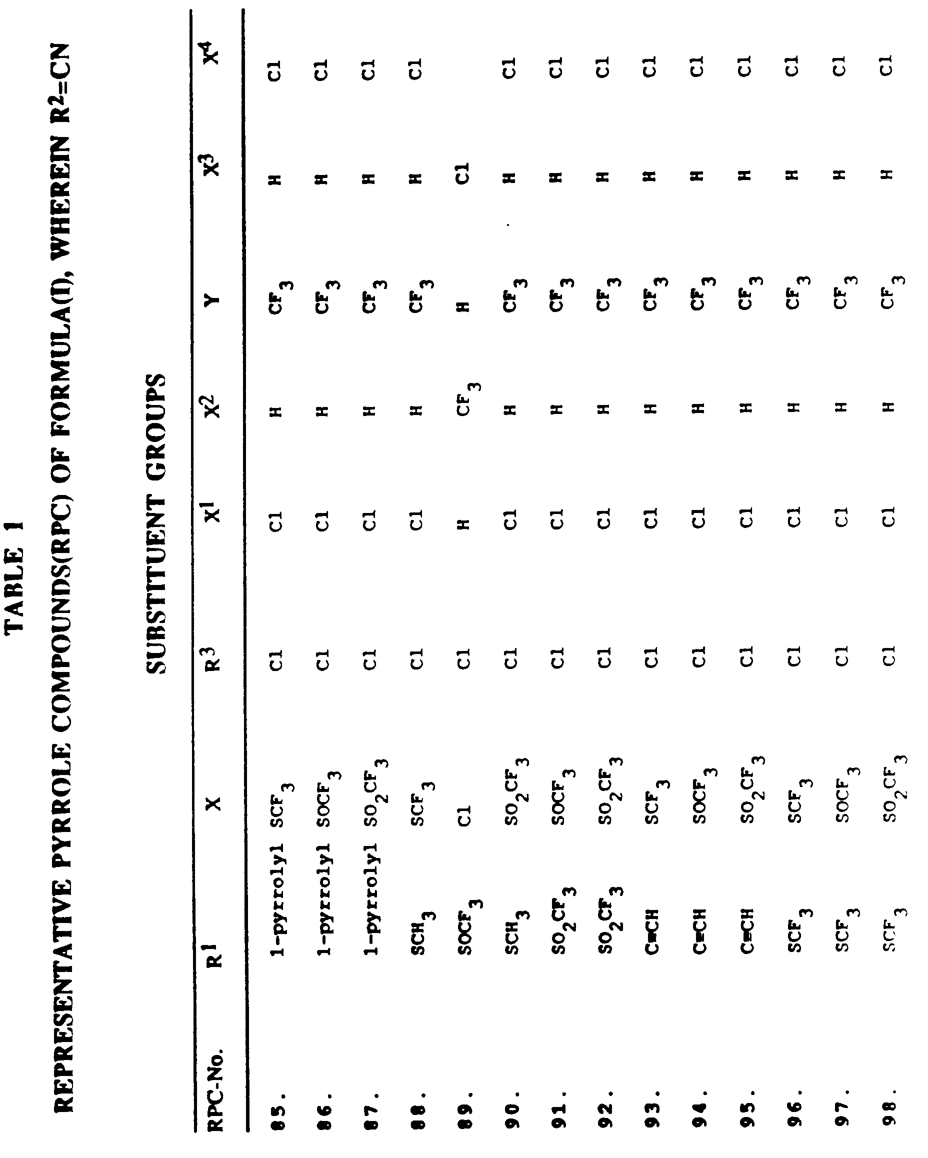

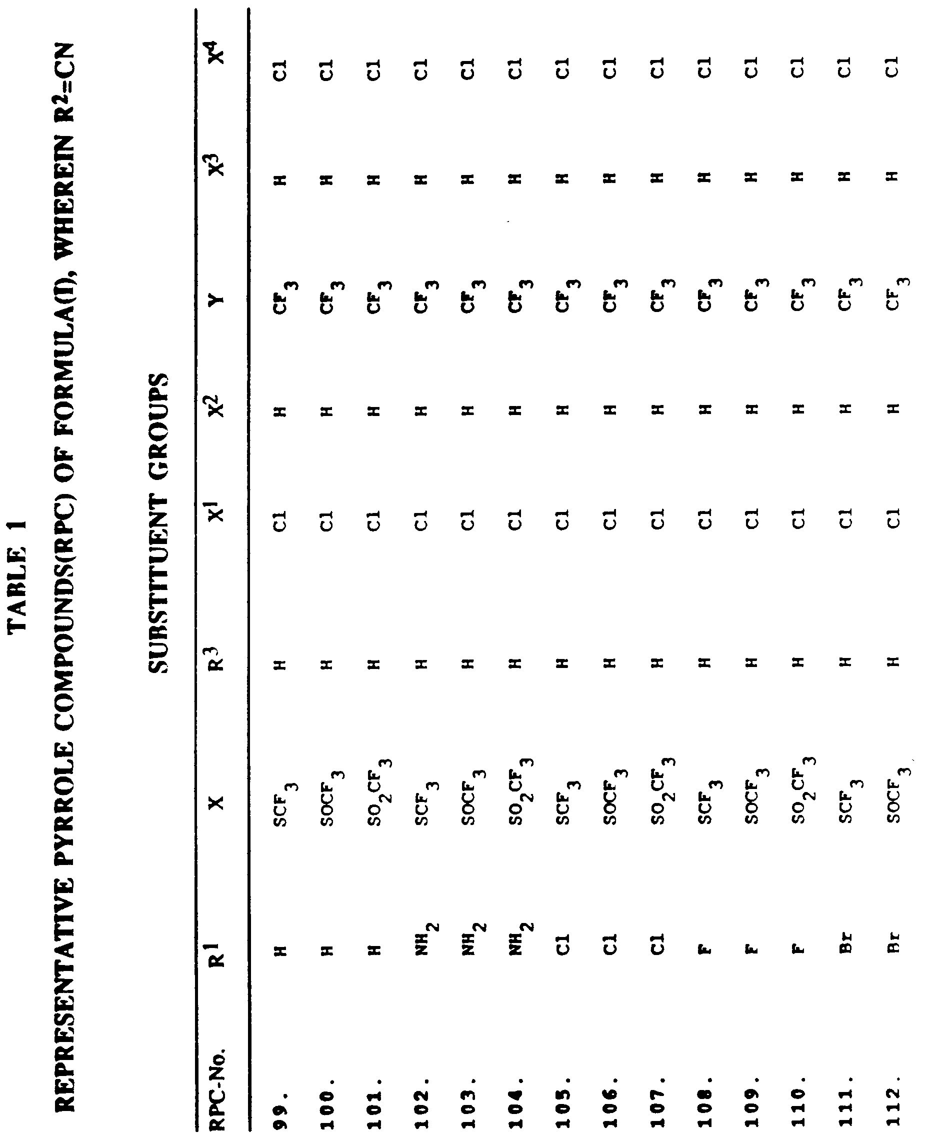

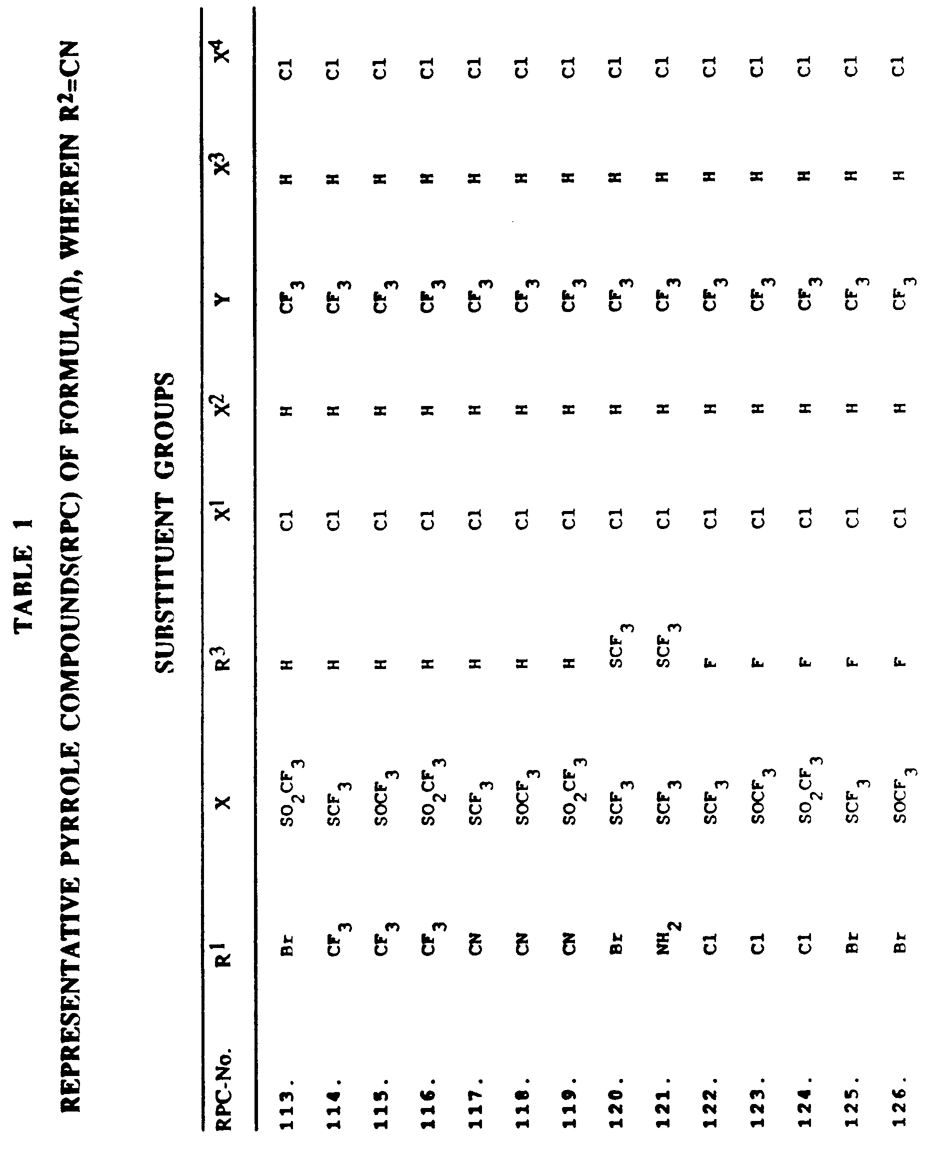

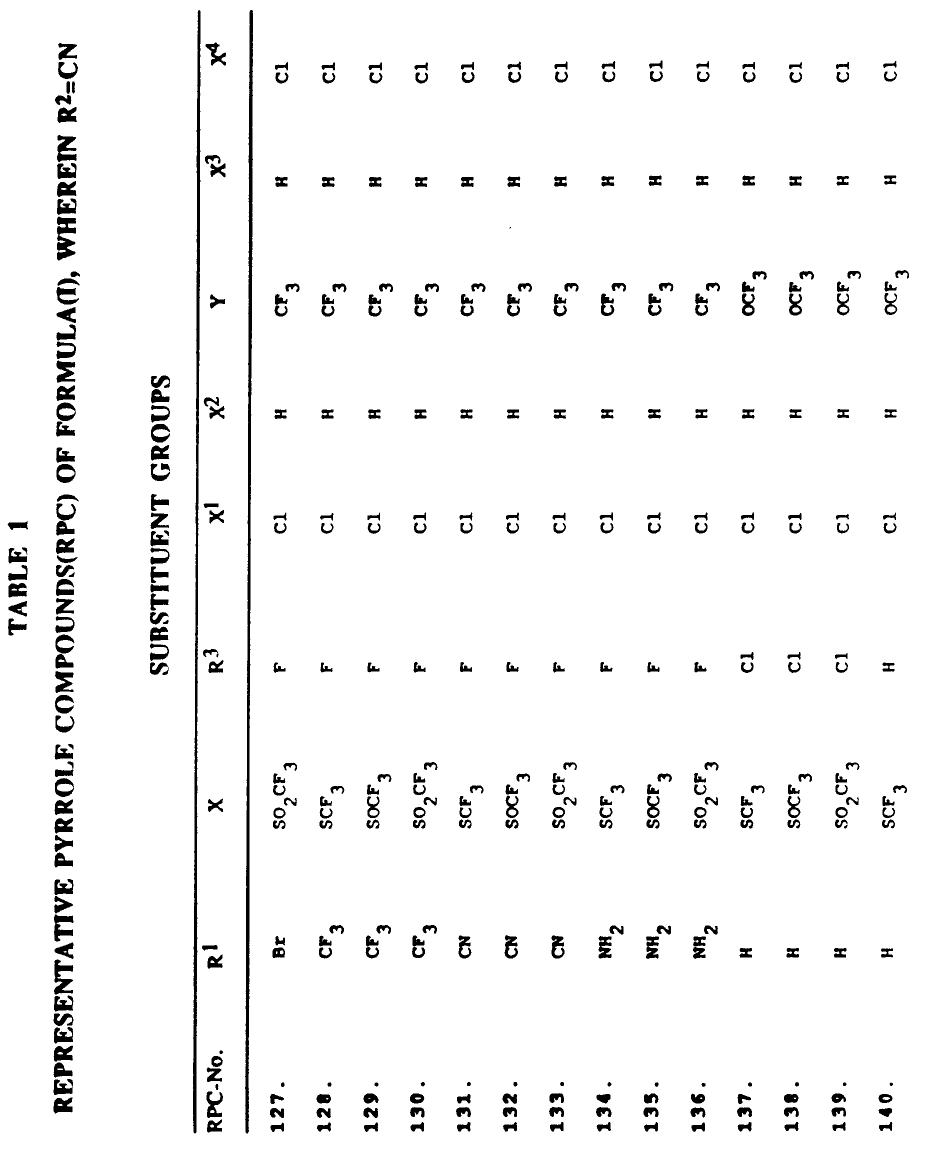

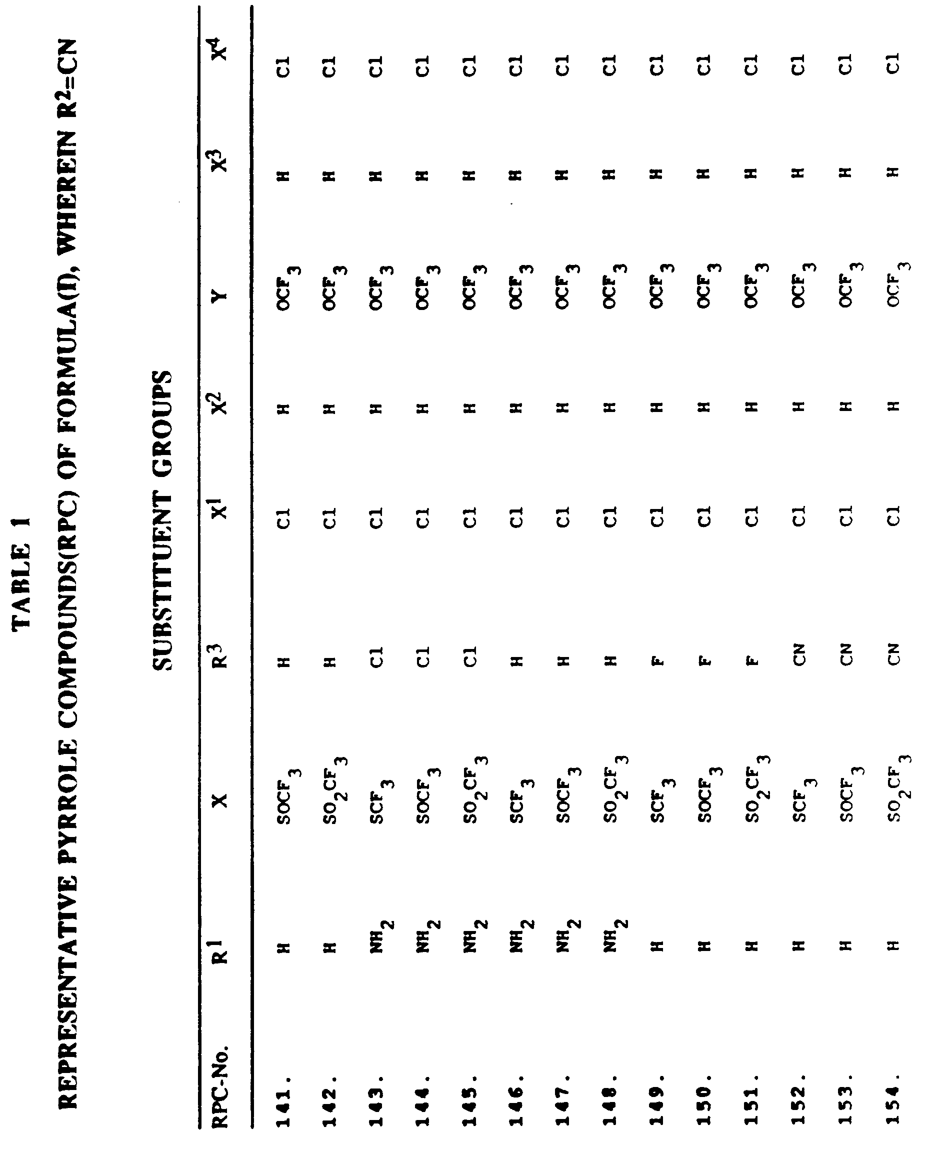

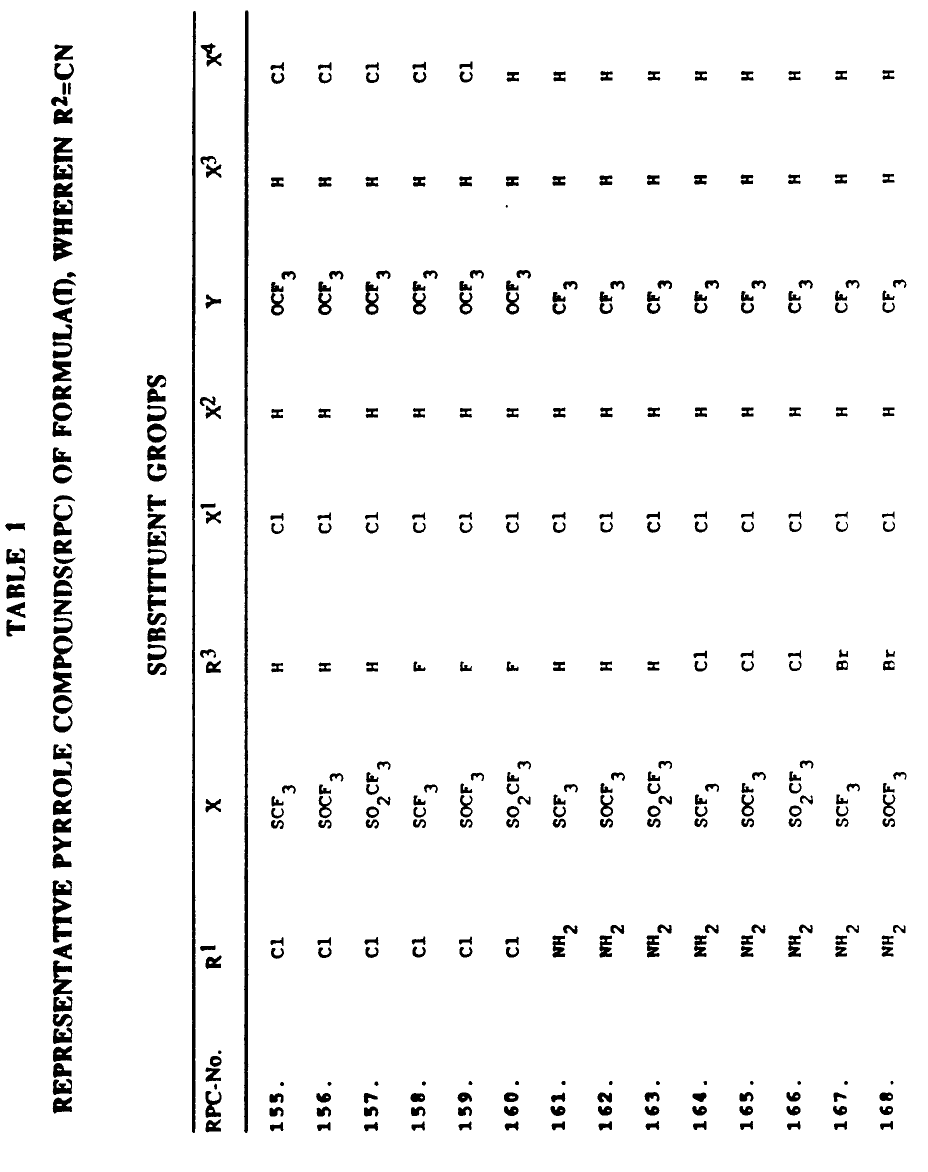

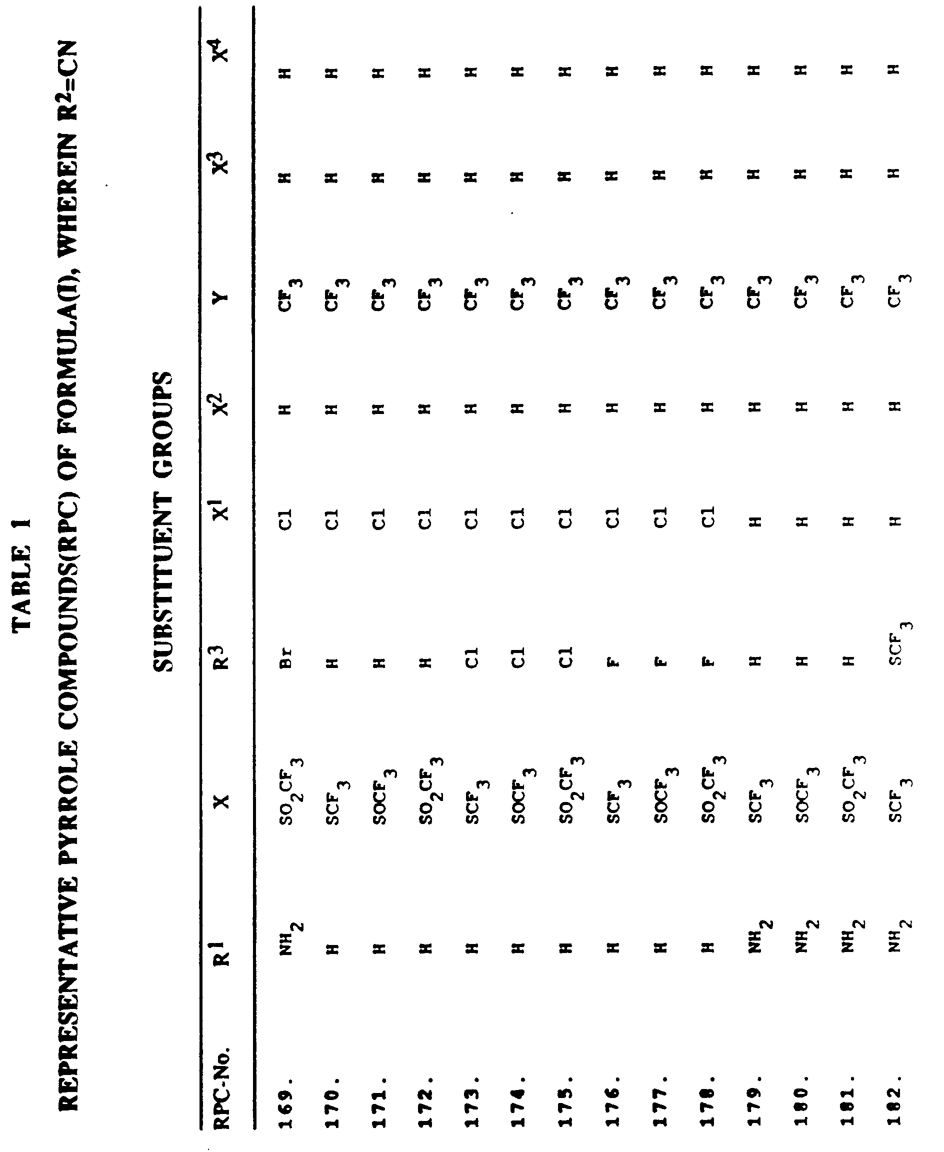

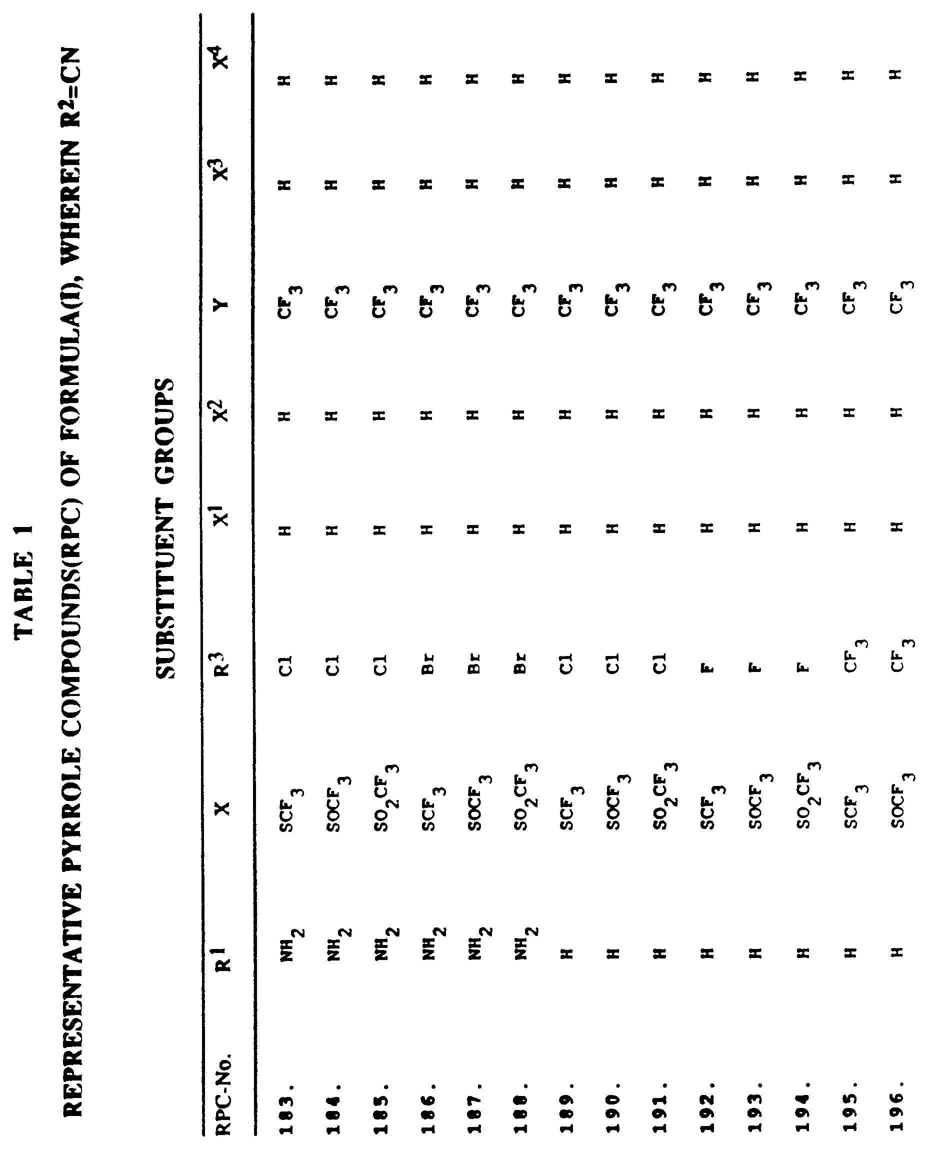

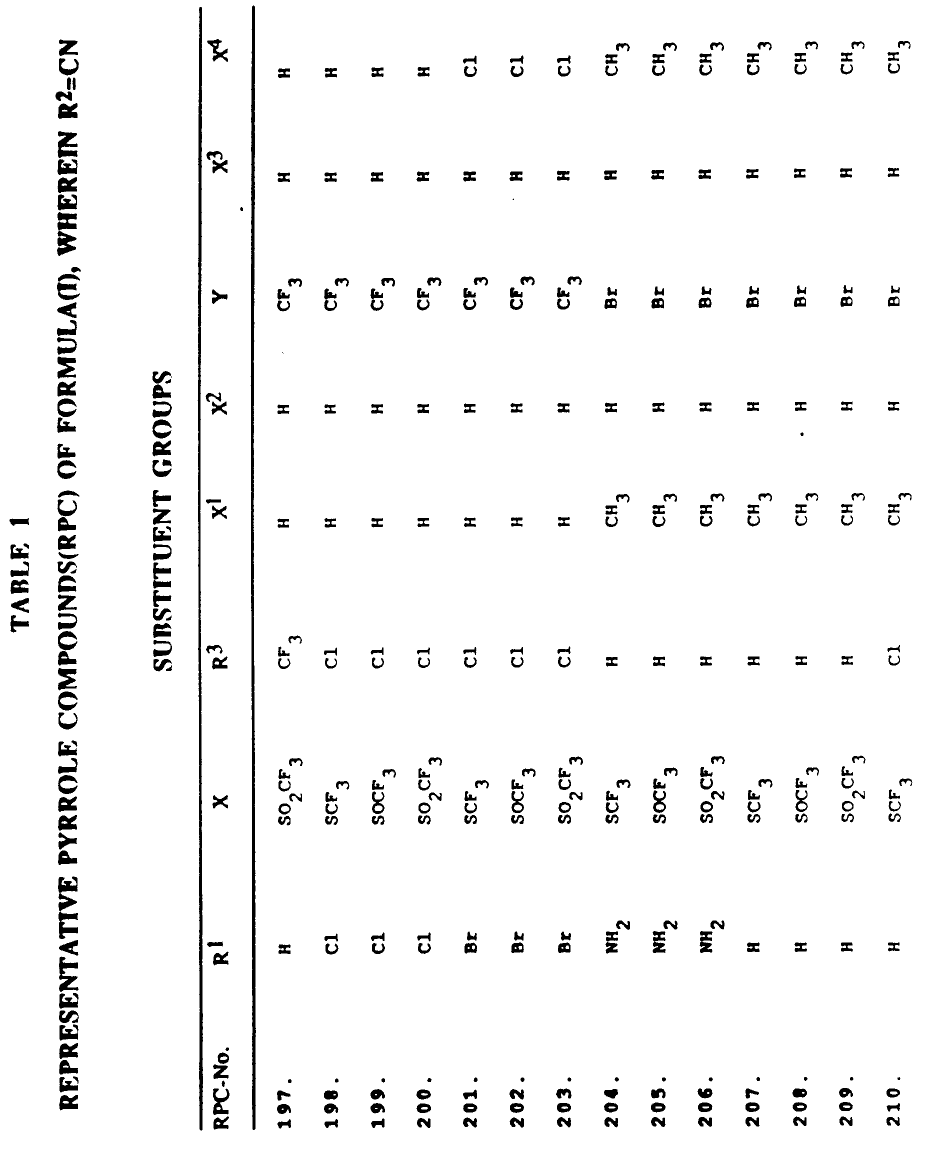

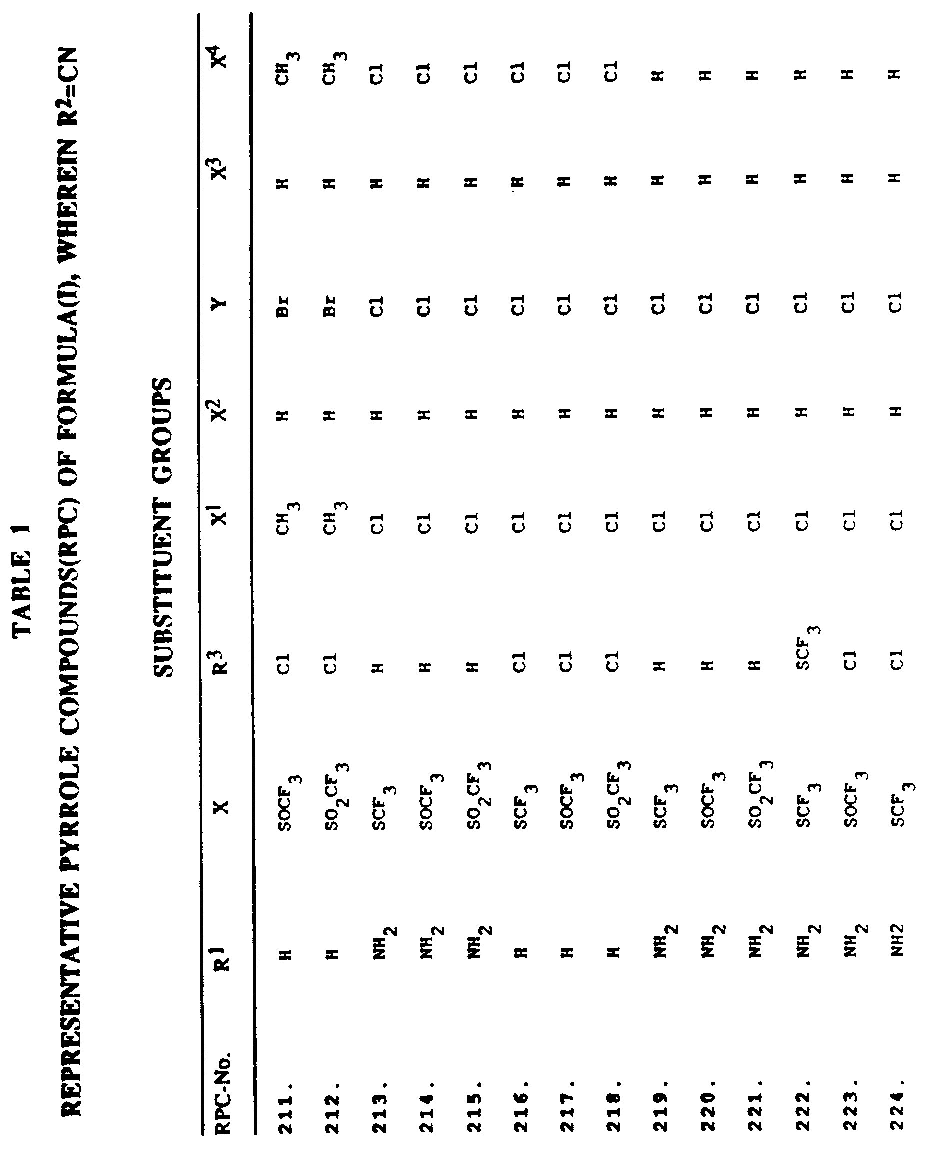

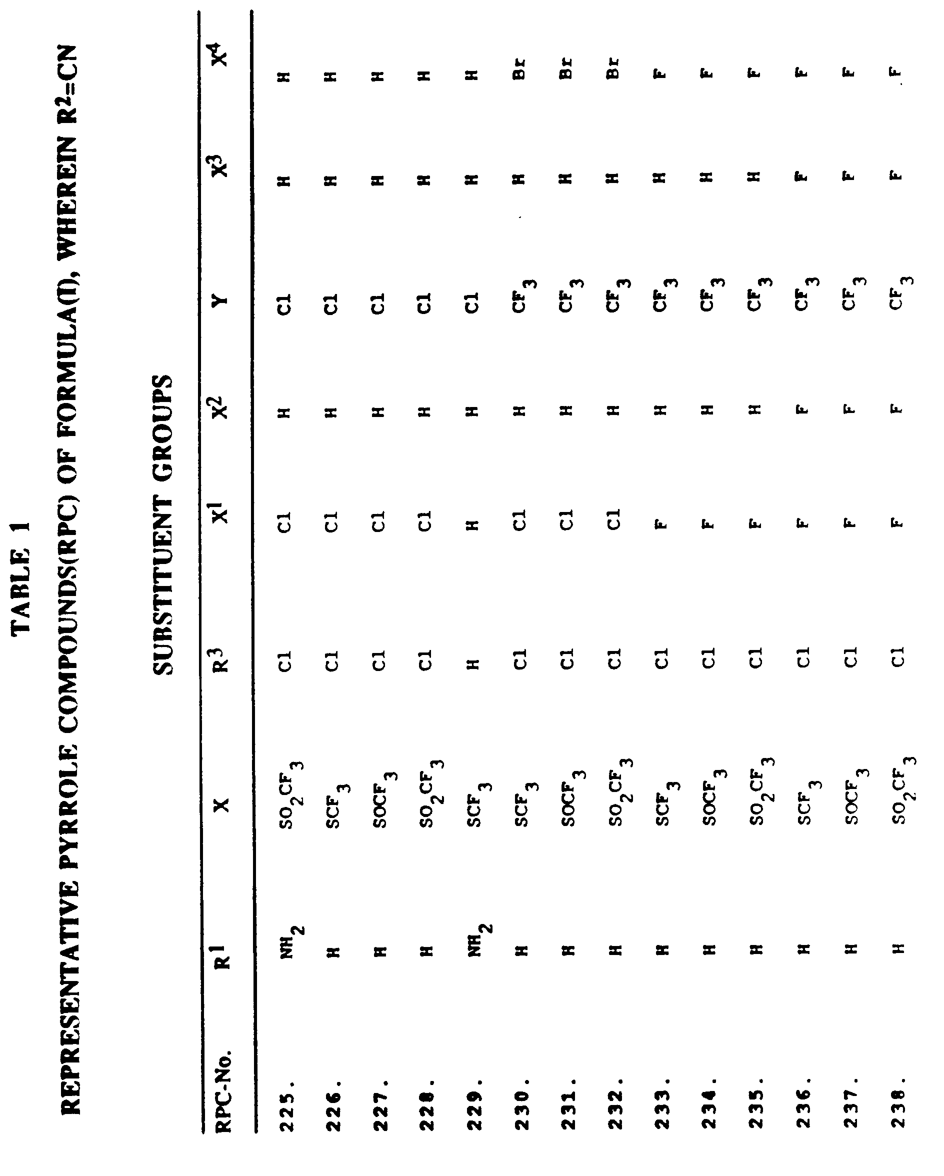

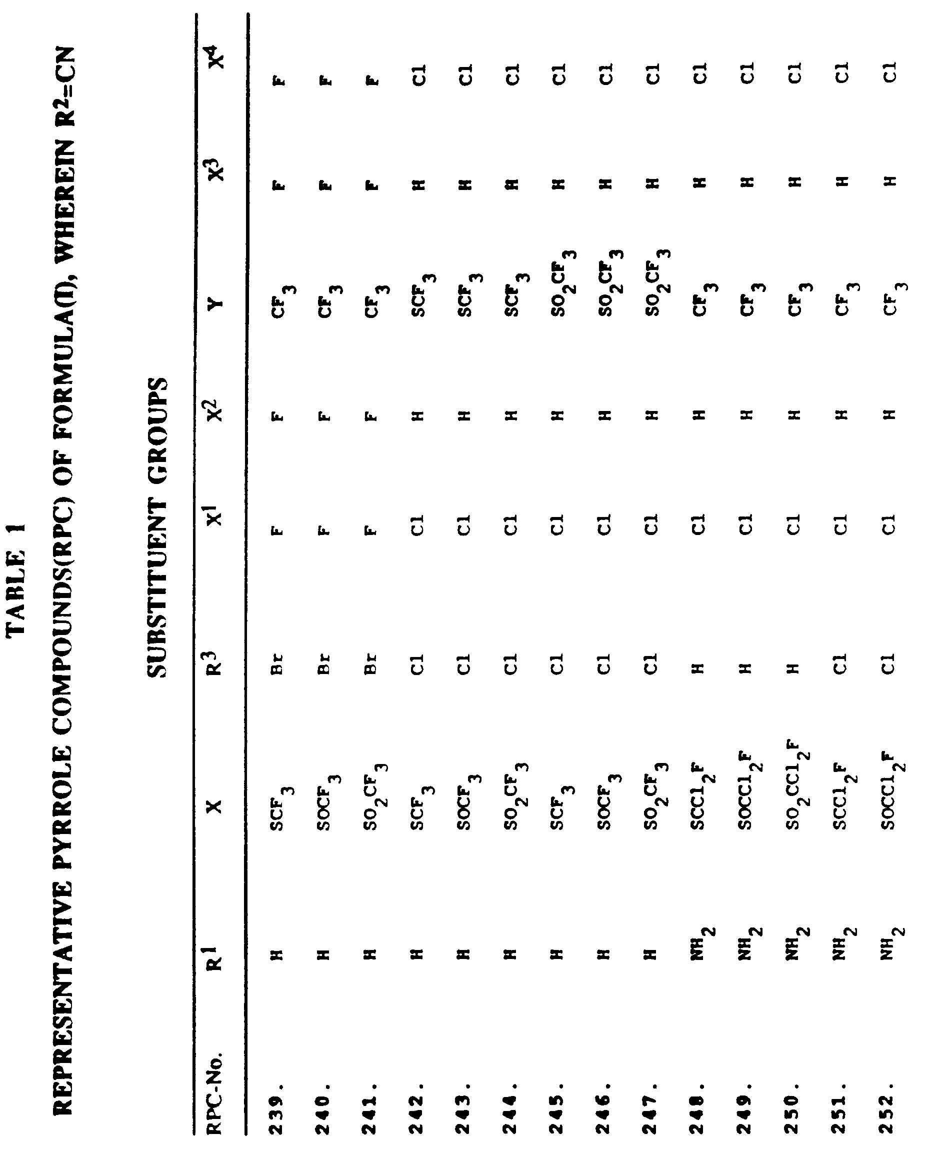

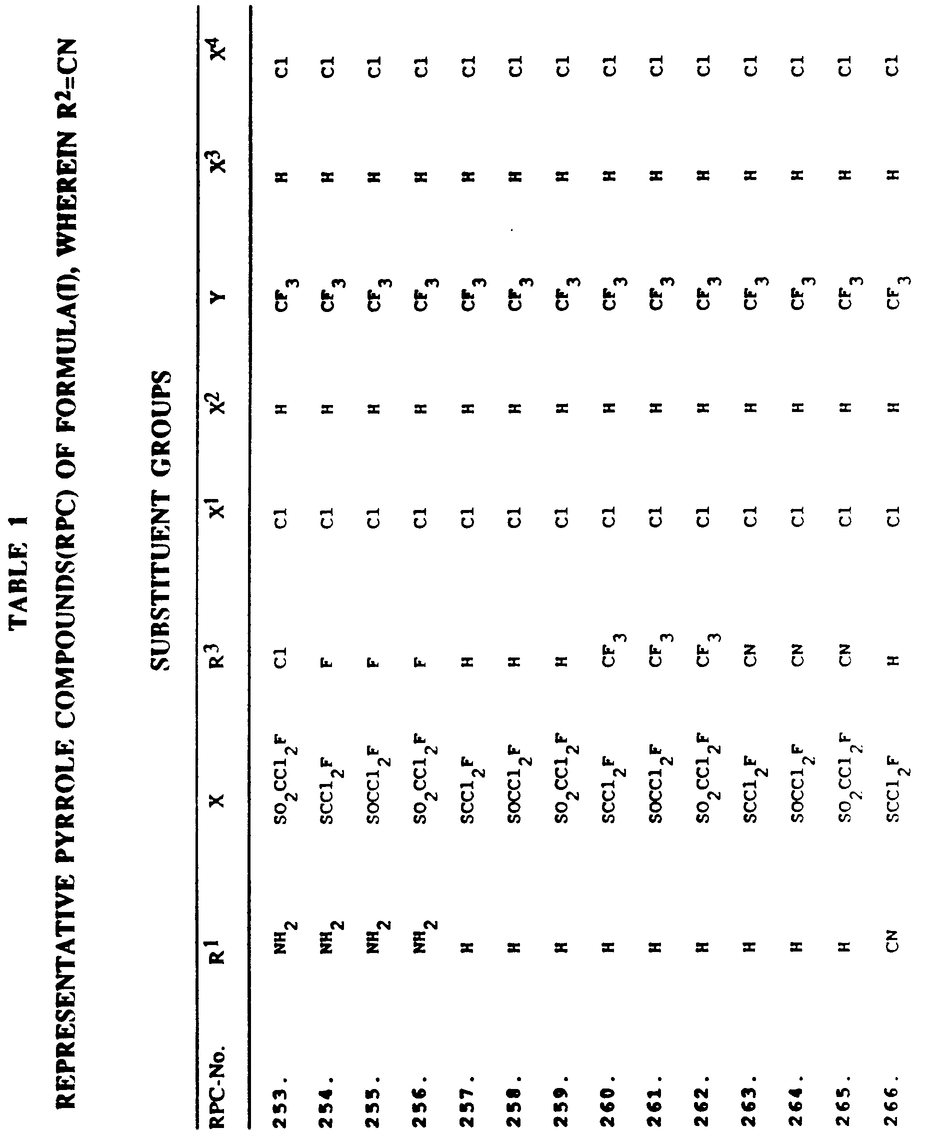

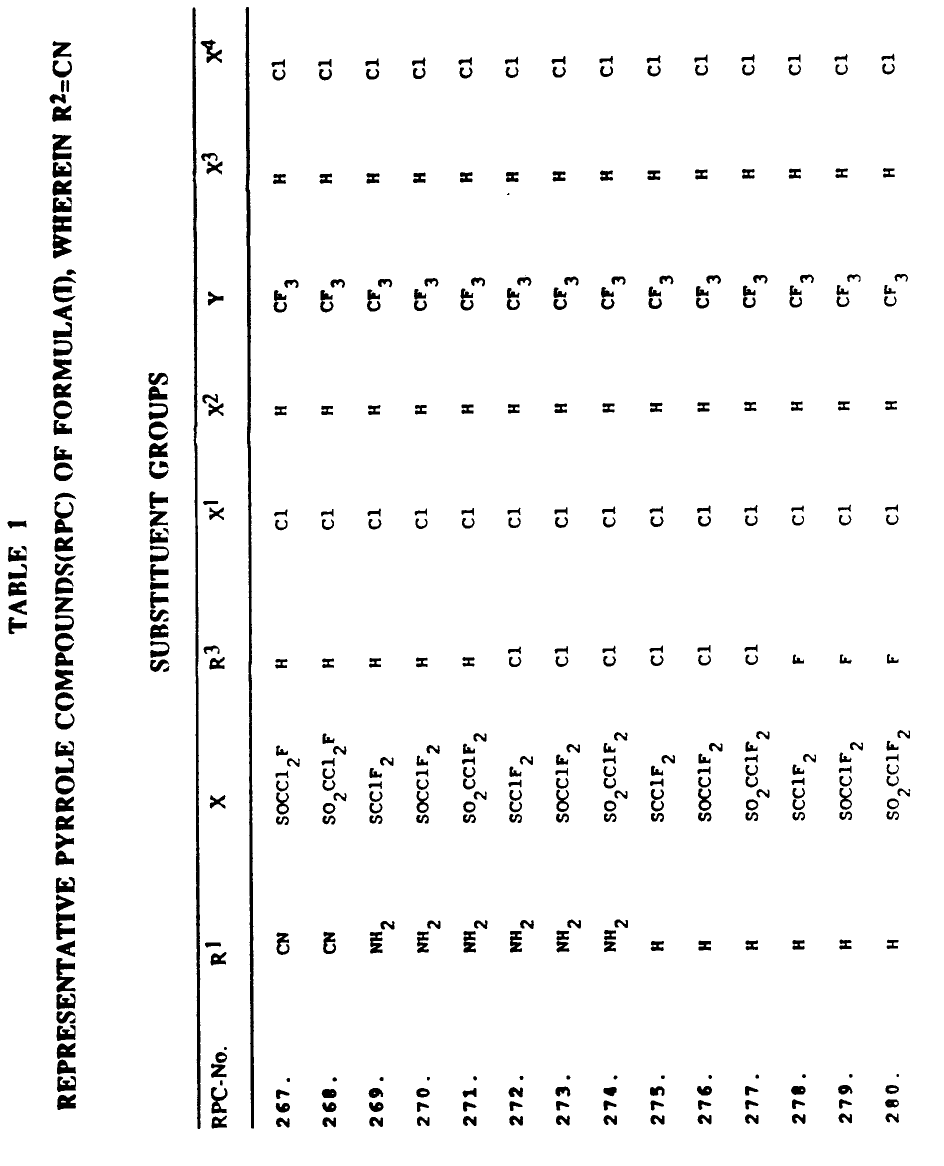

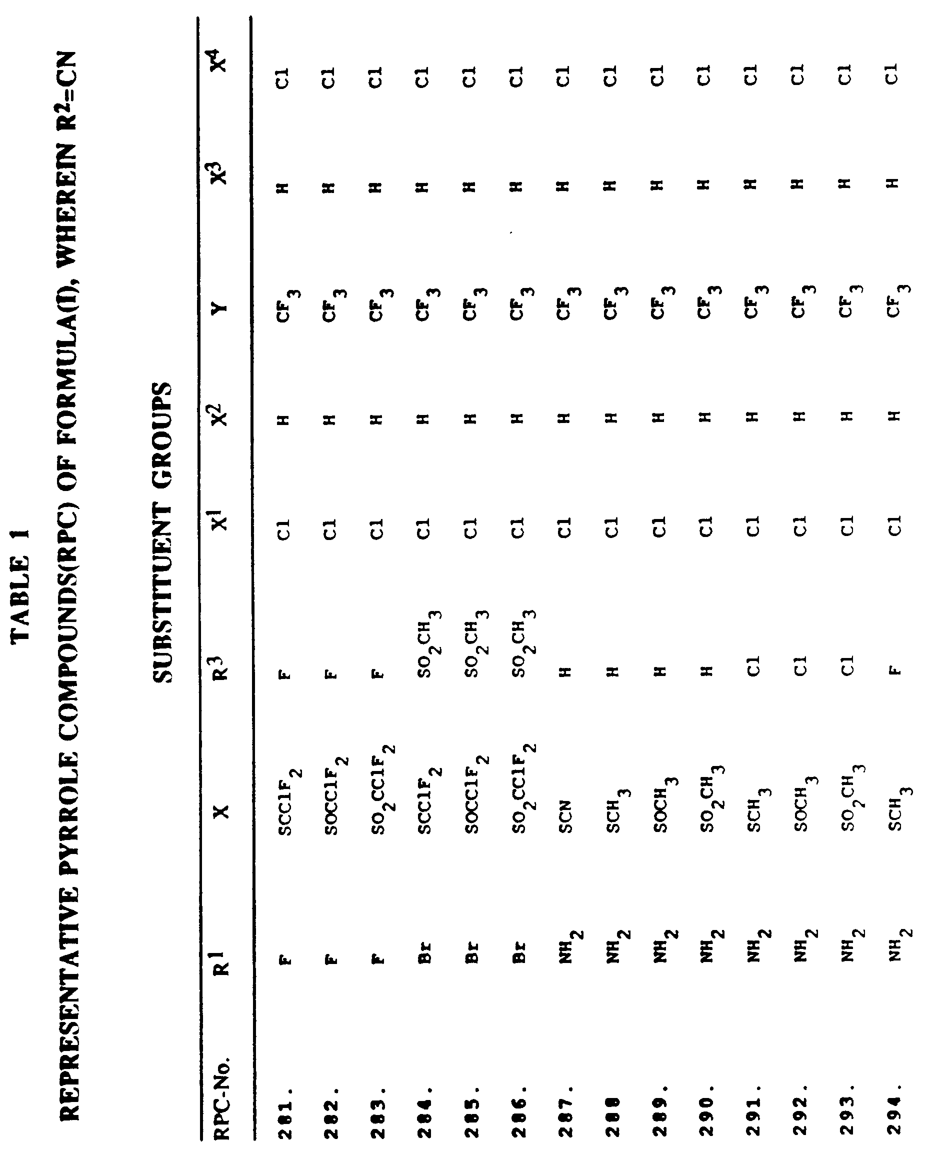

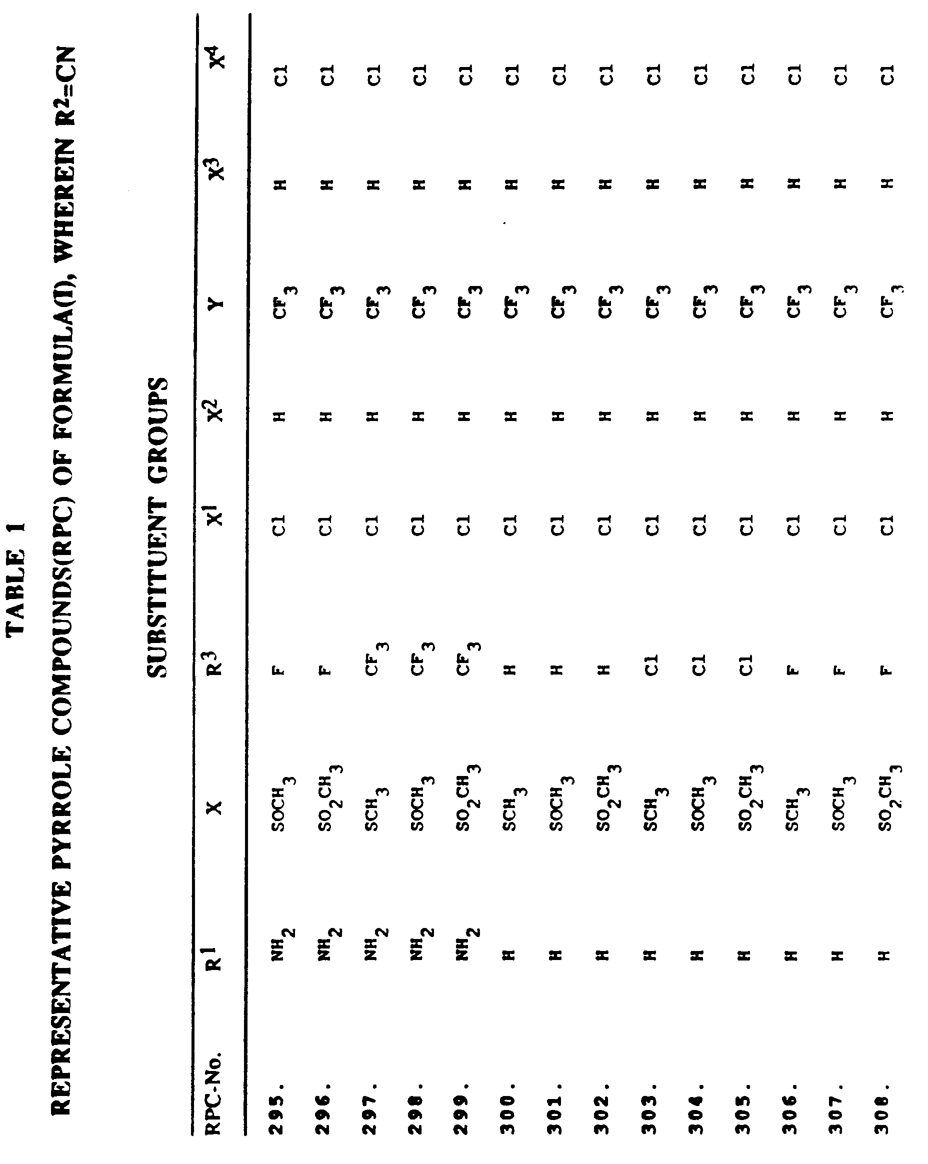

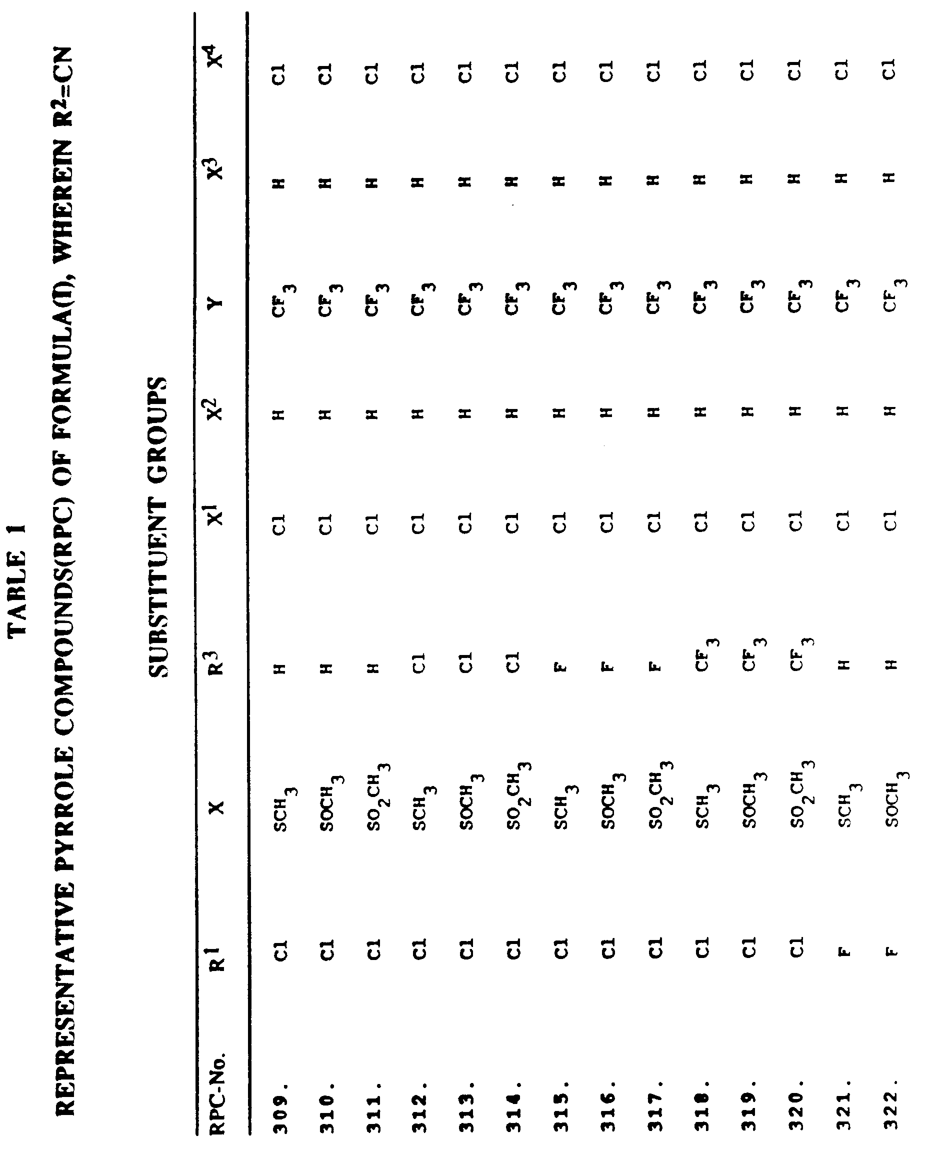

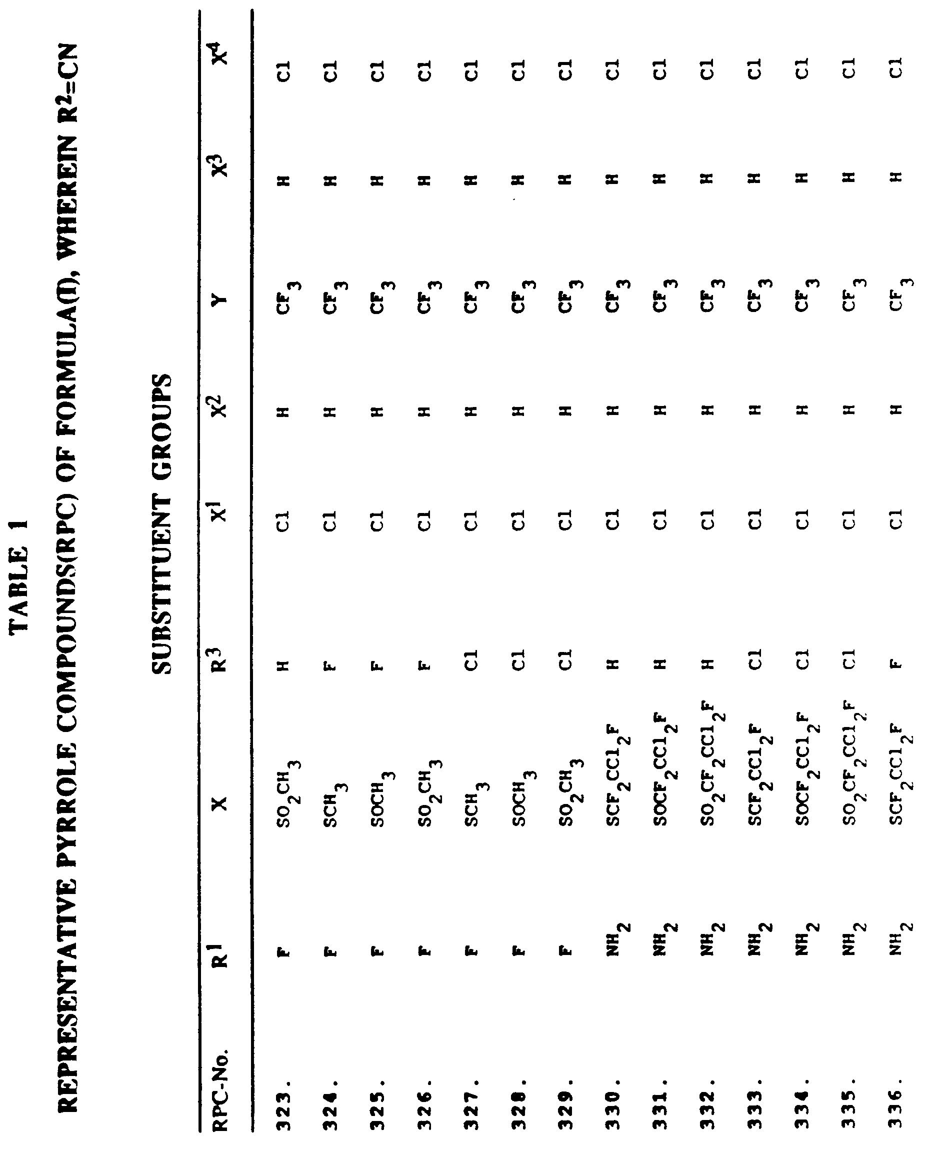

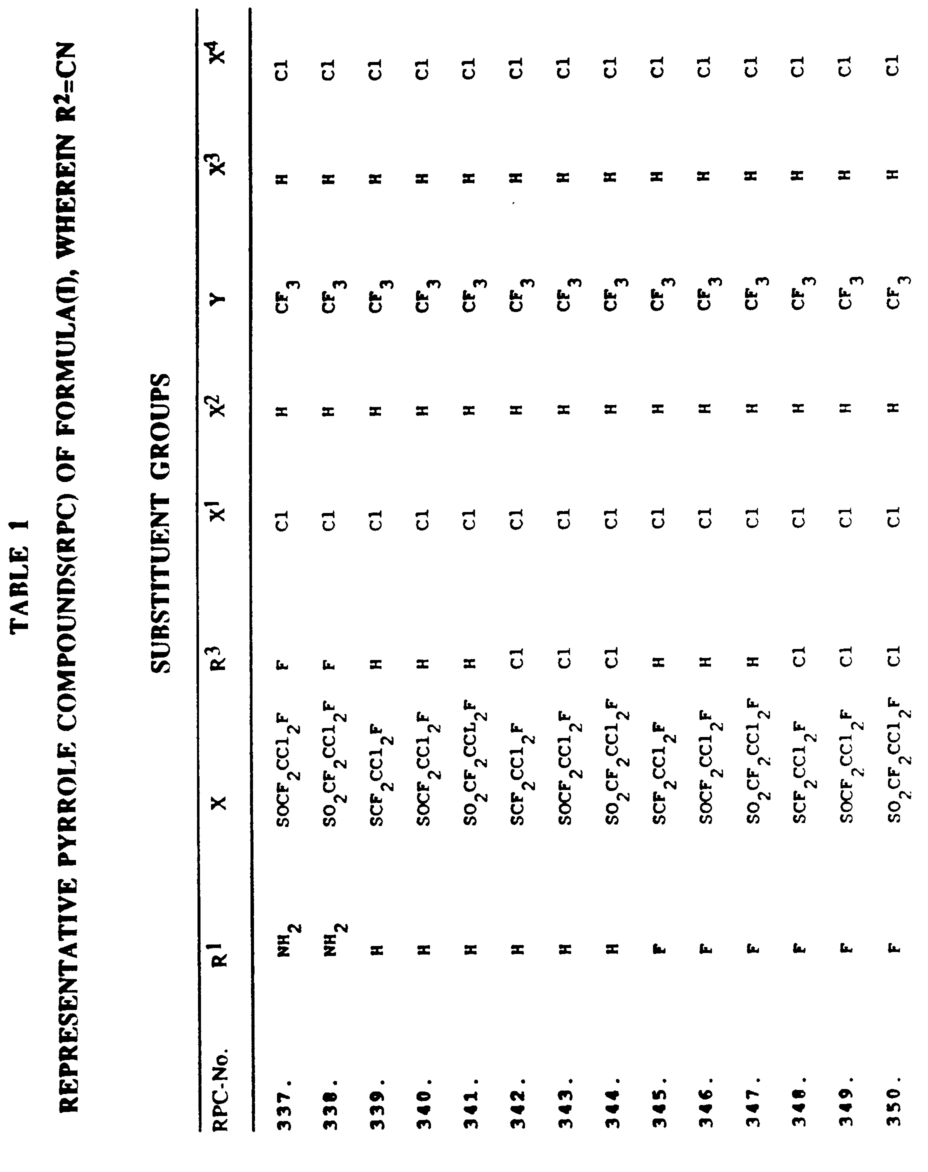

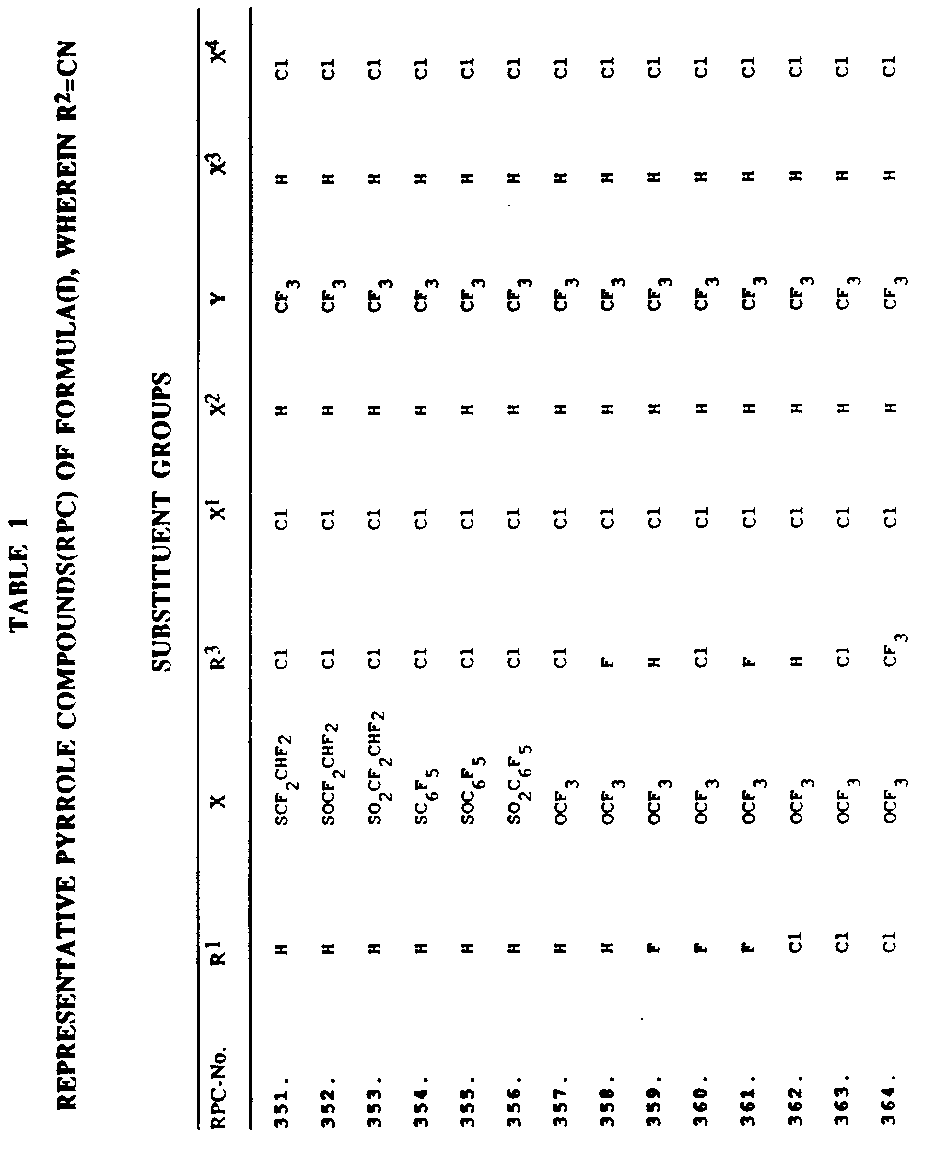

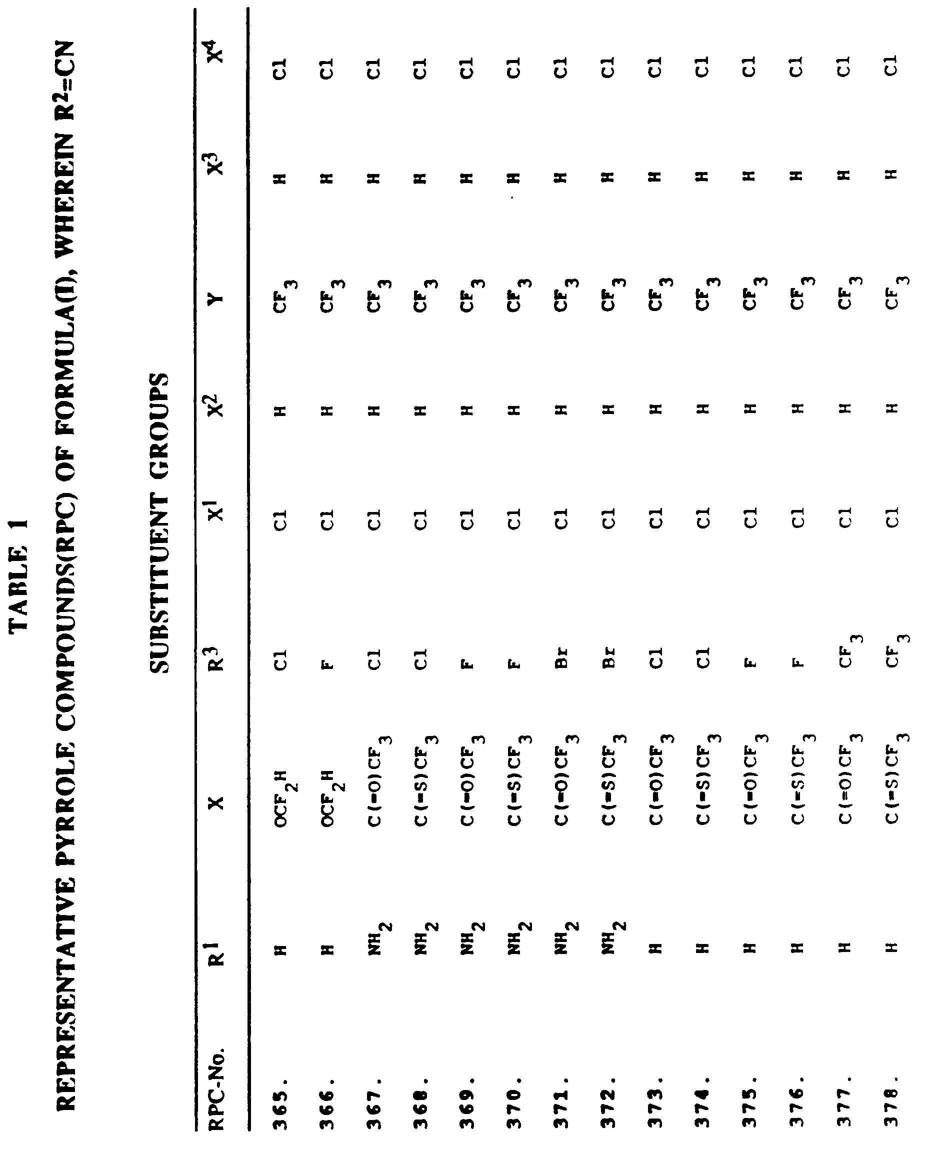

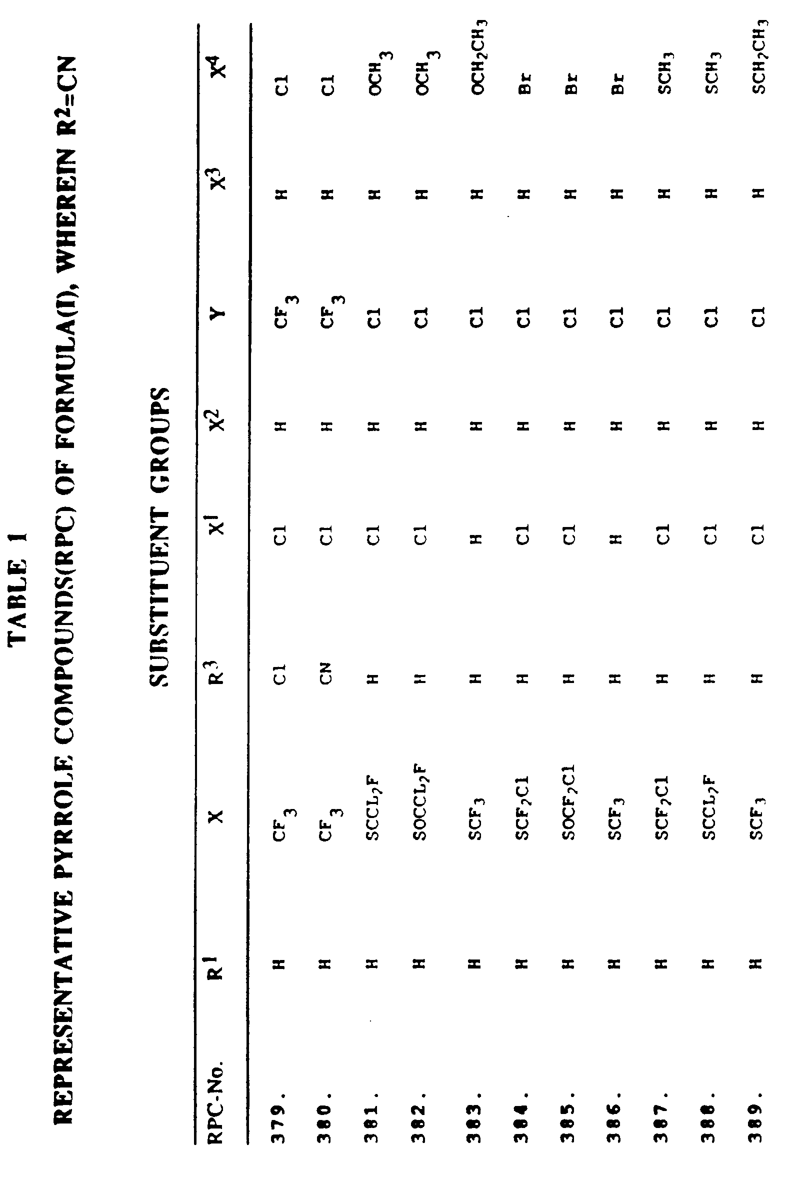

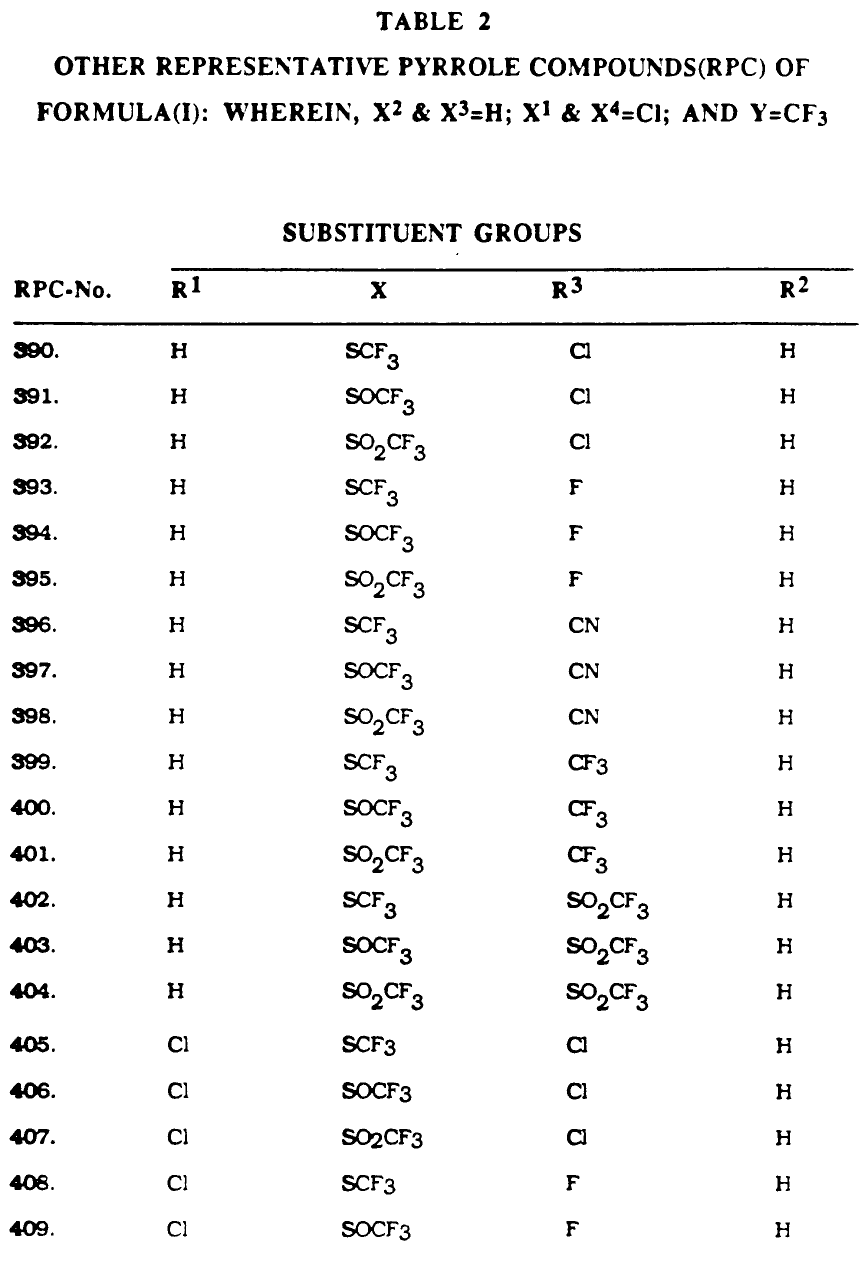

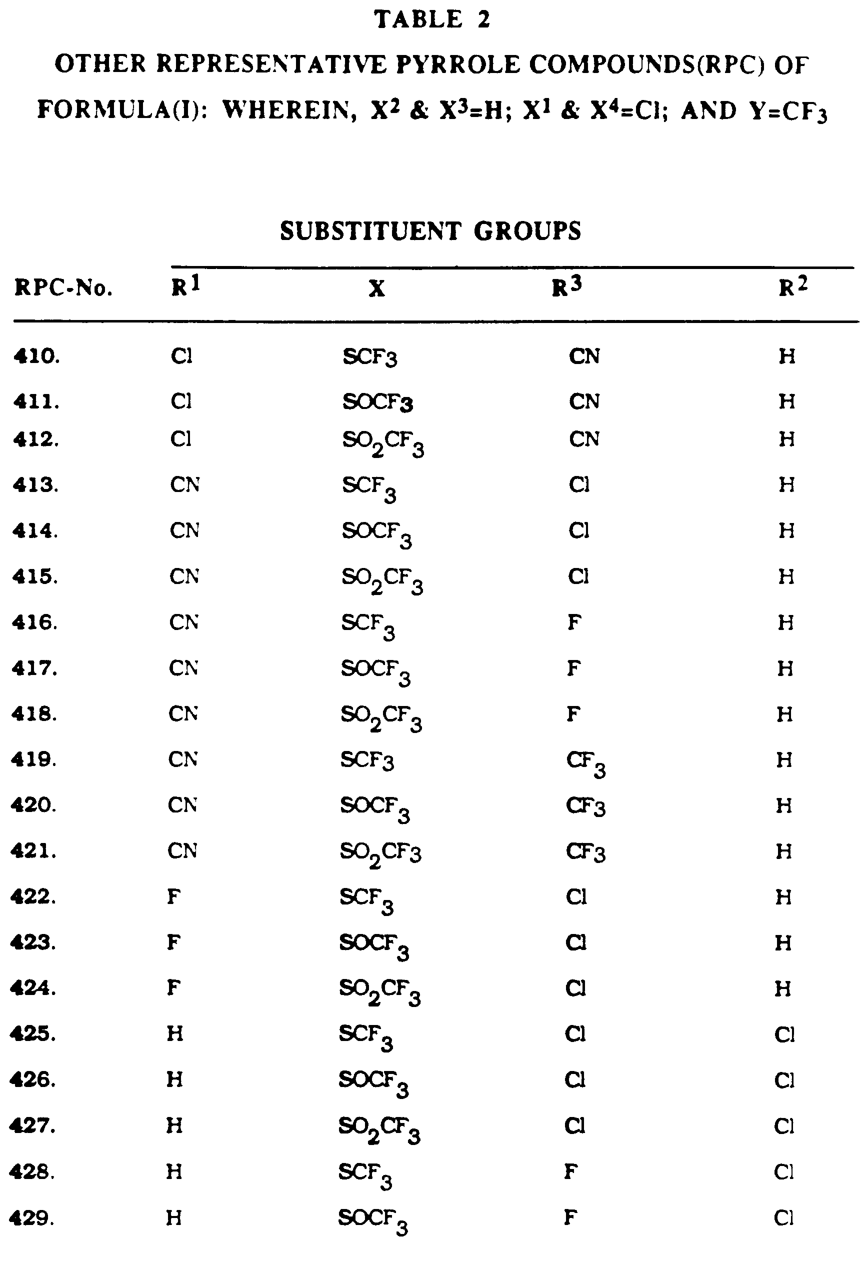

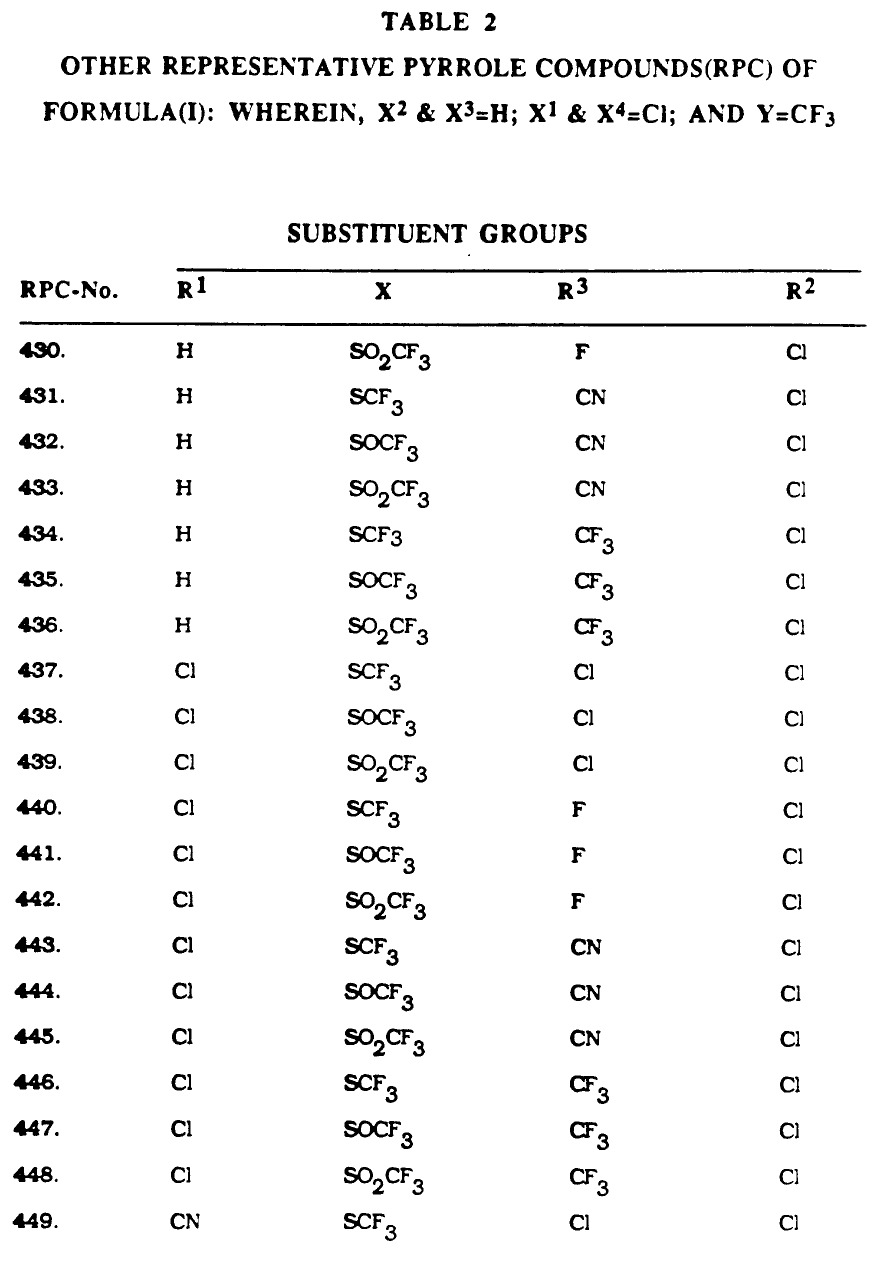

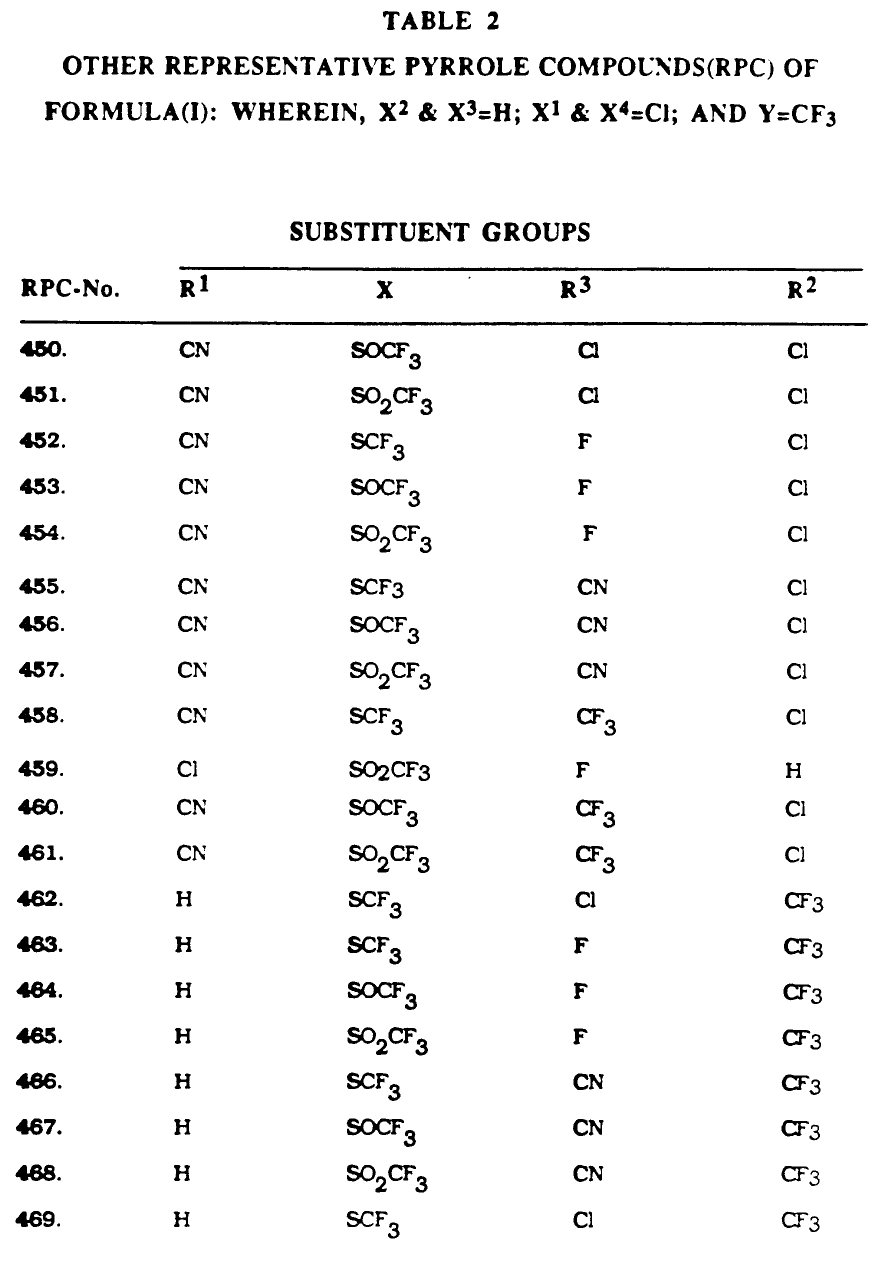

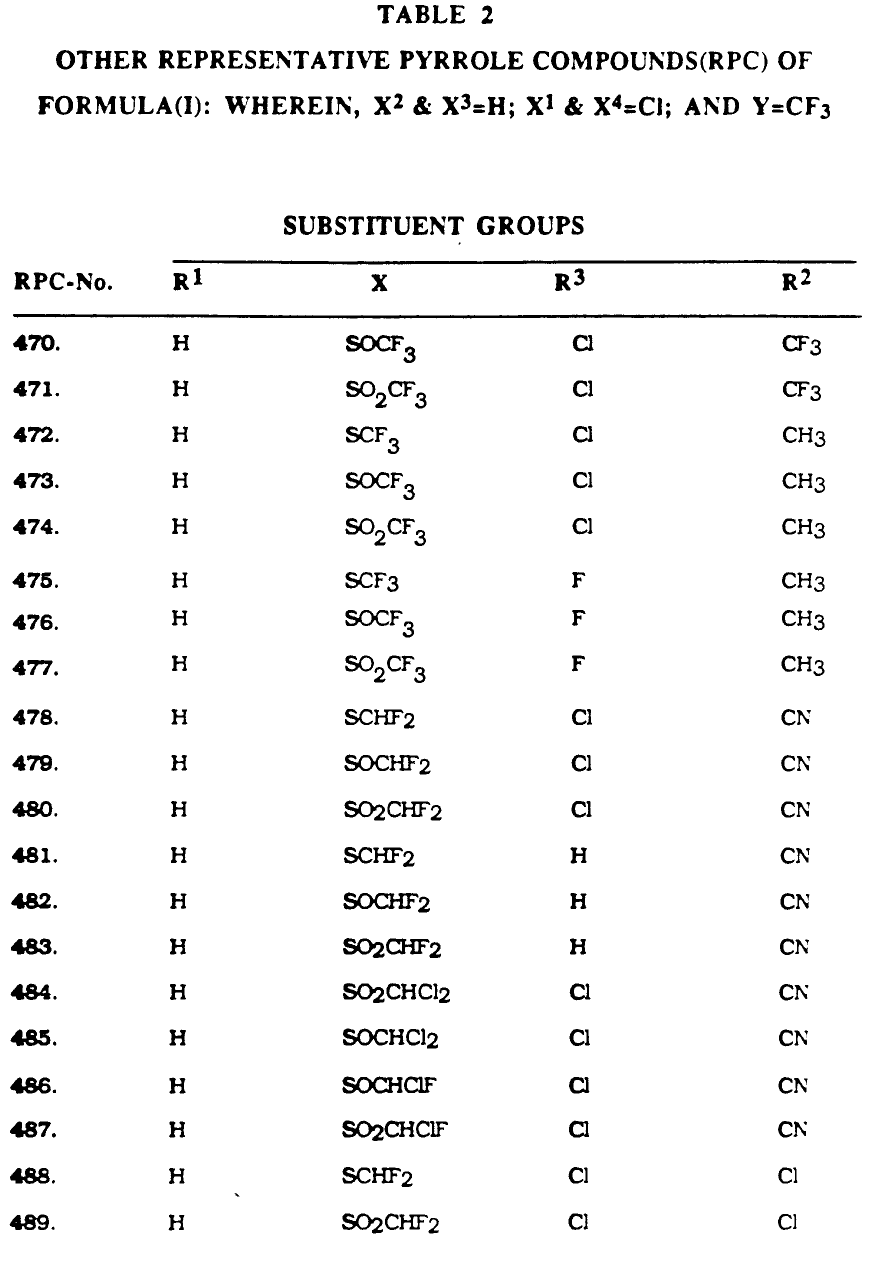

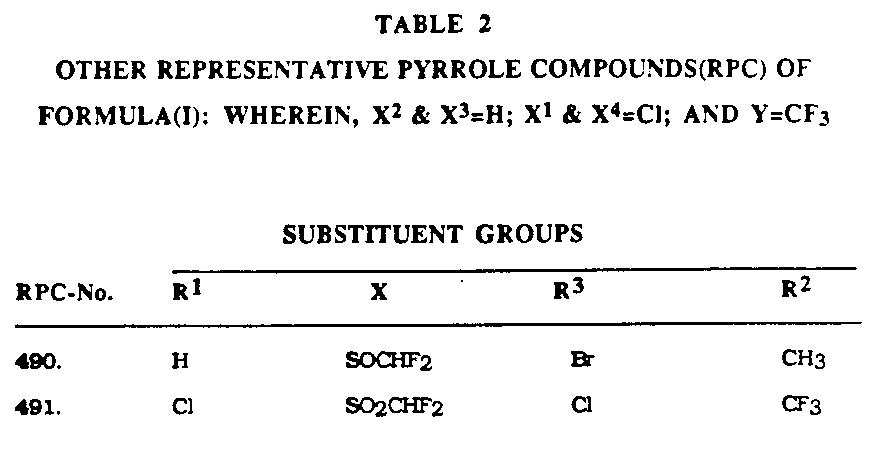

### DETAILED EXAMPLES OF COMPOUND SYNTHESIS

The following **EXAMPLES 1 to 22** further illustrate the methods of synthesis and the physical properties of the insecticidal compounds (and their chemical intermediates) according to the invention.

### EXAMPLE 1

A solution of 910 mg (2.07 mmoles) of 1-(2,6-dichloro-4-trifluoromethylphenyl)-2-chloro-3-cyano-4-trifluoromethylthiopyrrole (made according to the process described in **EXAMPLE 4**) and 492 mg of 80% meta chloroperoxybenzoic acid (394 mg, 2.28 mmoles) in 25 ml of chloroform was stirred at ambient temperature for 1.5 hours and then heated to reflux overnight. An additional 45 mg (0.21 mmoles) of m-chloroperoxybenzoic acid was added and reflux was continued for 1 hour. Heating was then stopped, and the reaction mixture was diluted with dichloromethane and washed with aqueous sodium bicarbonate solution. The organic layer was dried over anhydrous MgSO₄ and concentrated under reduced pressure to give a colorless solid residue. This process was repeated so as to get, all together, 950 mg of product which was chromatographed on silica gel, eluting with 2:1 v/v dichloromethane-hexane. Early fractions contained 310 mg (24%) of 1-(2,6-dichloro-4-trifluoromethylphenyl)-2-chloro-3-cyano-4-trifluoromethylsulfonylpyrrole(**EXAMPLE 1**) as a colorless solid. Recrystallization from hexane-ethyl acetate furnished 240 mg of the sulfone as colorless needles, melting point 198°C.

### EXAMPLE 2

Chromatography at the end of **EXAMPLE 1** was pursued. Later fractions from the chromatography furnished 600 mg (48%) of 1-(2,6-dichloro-4-trifluoromethylphenyl)-2-chloro-3-cyano-4-trifluoromethylsulfinylpyrrole (**EXAMPLE 2**) as a colorless solid. Recrystallization from toluene-hexane furnished 390 mg of the sulfoxide as a colorless powder, mp 152-154.5°C.

### EXAMPLES 3A and 3B

**EXAMPLES 1 and 2** were repeated with 1-(2,6-dichloro-4-trifluoromethylphenyl)-2-chloro-3-cyano-4-trifluoromethylthio-5-bromopyrrole as starting material made according to the process of **EXAMPLE 5**. The compound of **EXAMPLE 3A** is 1-(2,6-dichloro-4-trifluoromethylphenyl)-2-chloro-3-cyano-4-trifluoromethylsulfinyl-5-bromopyrrole. This compound, made using the procedure similar to that of **EXAMPLE 2**, has a melting point of about 123°C. The compound of **EXAMPLE 3B** is 1-(2,6-dichloro-4-trifluoromethylphenyl)-2-chloro-3-cyano-4-trifluoromethylsulfonyl-5-bromopyrrole. This compound, made using the procedure similar to that of **EXAMPLE 1**, has a melting point of about 113°C.

### EXAMPLE 4

A solution of 3 g (6.6 mmoles) of 1-(2,6-dichloro-4-trifluoromethylphenyl)-2-amino-3-trifluoromethylthio-4-cyano-5-chloropyrrole (made according to the process described in **EXAMPLE 8**) in 50 ml of dry tetrahydrofuran was stirred under a nitrogen atmosphere and 3.9 ml (3.4 g, 33 mmoles) of t-butyl nitrite was added. After 30 minutes, the reaction mixture was heated to reflux for about one hour, then concentrated under reduced pressure to give 3.69 g of a solid residue. This process was repeated so as to get all together 4.07 g of solid residue, which was chromatographed on silica gel with a 1:1 v/v dichloromethane-hexane eluent to give 2.9 g (91%) of 1-(2, dichloro-4-trifluoromethylphenyl)-2-chloro-3-cyano-4-trifluoromethylthiopyrrole(**EXAMPLE 4**) as a colorless solid. Recrystallization from hexane-ethyl acetate provided 1.87 g of the product as a colorless powder, melting point at about 137°C.

### EXAMPLE 5

To a heterogeneous mixture of 2.4 g (5.28 mmoles) of 1-(2,6-dichloro-4-trifluoromethylphenyl)-2-amino-3-trifluoromethylthio-4-cyano-5-chloropyrrole (made according to the process described in **EXAMPLE 8**) in 40 ml of bromoform under an inert atmosphere was added 0.94 ml (820 mg, 7.92 mmoles) of t-butyl nitrite. After 15 minutes of stirring at ambient temperature, the reaction mixture was concentrated under reduced pressure to give 3.9 g of residue. This was combined with the product from a previous reaction of 300 mg of the same pyrrole starting material. The crude product was chromatographed on silica gel eluting with 4:1 v/v hexane-dichloromethane. This separated 1.72 g (56%) of 1-(2,6-dichloro-4-trifluoromethylphenyl)-2-bromo-3-trifluoromethylthio-4-cyano-5-chloropyrrole (**EXAMPLE 5**), which was recrystallized from hexane to give 780 mg of the product as a colorless solid, melting point about 92^{o}C.

### EXAMPLE 6

A solution of 1.91 g (4.21 mmoles) of 1-(2,6-dichloro-4-trifluoromethylphenyl)-2-amino-3-trifluoromethylthio-4-cyano-5-chloropyrrole (made according to the process described in **EXAMPLE 8**), 77 mg (0.63) mmoles) of 4-dimethylaminopyridine and 20 ml of pyridine under an inert atmosphere was cooled at 0^{o}C and 1.01 ml (1.50g, 7.14 mmoles) of trifluoroacetic anhydride was added. The reaction mixture was allowed to stir at 0^{o}C for 1 hour and at 20^{o}C for 4 hours, when an additional 0.30 ml (2.1 mmoles) of trifluoroacetic anhydride was added. After a total of 24 hours of reaction time, the reaction mixture was diluted with dichloromethane and concentrated. The residue was washed with aqueous HCl followed by water and recrystallized from hexane-ethyl acetate to give 860 mg (37%) of 1-(2,6-dichloro-4-trifluoromethylphenyl)-2-[(trifluoromethyl)carbonylamino]-3- trifluoromethylthio-4-cyano-5-chloropyrrole (**EXAMPLE 6**) as a slightly green solid, melting point about 190^{o}C.

### EXAMPLE 7

A mixture of 1.50 g (3.3 mmoles) of 1-(2,6-dichloro-4-trifluoromethylphenyl)-2-amino-3-trifluoromethylthio-4-cyano-5-chloropyrrole (made according to the process described in **EXAMPLE 8**), 0.10 g of 4-dimethylaminopyridine, 0.33 ml (0.32 g, 4.1 mmoles) of pyridine, 0.31 ml (0.34 g, 4.3 mmoles) of acetyl chloride and 10 ml of acetonitrile was stirred for four days at 20^{o}C and 1 day at reflux. An additional 0.03 ml of acetyl chloride was then added and reflux was continued for an additional day when the reaction was cooled, diluted with dichloromethane and partitioned successively with aqueous 1N HCl and a saturated aqueous sodium bicarbonate solution. The organic layer was dried over anhydrous magnesium sulfate and evaporated to give 1.42 g of a beige solid. Chromatography on silica gel eluting with 4:1 v/v hexane-ethyl acetate followed by recrystallization from ethanol-water provided 480 mg (29%) of 1-(2,6-dichloro-4-trifluoromethylphenyl)-2-methylcarbonylamino-3-trifluoromethylthio-4-cyano-5-chloropyrrole (**EXAMPLE 7**) as colorless needles, melting point about 216^{o}C.

### EXAMPLE 8

A stirred solution of 1.50 g (3.57 mmoles) of 1-(2,6-dichloro-4-trifluoromethylphenyl)-2-amino-3-trifluoromethylthio-4-cyanopyrrole (made according to the process described in **EXAMPLE 13**) in 15 ml of ethyl ether was cooled to -20°C under an inert atmosphere and a solution of 0.29 ml (0.48 g, 3.6 mmoles) of sulfuryl chloride in 15 ml of anhydrous ethyl ether was added dropwise. The reaction mixture was then allowed to warm to 20°C and stirred for 2.5 days when an additional 0.03 ml (0.4 mmole) of sulfuryl chloride was added and stirring continued for another day. Another 0.03 ml of sulfuryl chloride was added and after an additional day the reaction was quenched with 28 ml of 10% aqueous potassium carbonate solution. The phases were separated and the aqueous layer was extracted with ether. The ethereal layers were then combined, washed with water, dried over anhydrous magnesium sulfate and concentrated to give 1.56 g of a tan solid. The crude product was chromatographed on silica gel with a 2:1 v/v dichloromethane-hexane eluent to provide 1.30 g (80%) of 1-(2,6-dichloro-4-trifluoromethylphenyl)-2-amino-3-trifluoromethylthio-4-cyano-5-chloropyrrole (**EXAMPLE 8**) as a slightly rose colored solid. Recrystallization from cyclohexane furnished 810 mg of the product as off-white needles, melting point about 176°C.

### EXAMPLE 9

A process similar to that of **EXAMPLE 8** was used, except substituting 1-(2-chloro-4-trifluoromethyl-phenyl)-2-amino-3-trifluoromethylthio-4-cyanopyrrole (mp 169°C) as the reactant, which was made by a process according to that described in **EXAMPLE 13**. The final product was 1-(2-chloro-4-trifluoromethylphenyl)-2-amino-3-trifluoromethylthio-4-cyano-5-chloropyrrole (**EXAMPLE 9**), mp about 148°C.

### EXAMPLE 10

A process similar to that of **EXAMPLE 8** was used, except substituting 1-(2,6-dichloro-4-trifluoromethylphenyl)-2-amino-3-dichlorofluoromethylthio-4-cyanopyrrole (mp 202°C) as the reactant, which was made by a process according to that described in **EXAMPLE 13**. The final product was 1-(2,6-dichloro-4-trifluoromethylphenyl)-2-amino-3-dichlorofluoromethylthio-4-cyano-5-chloropyrrole (**EXAMPLE 10**), mp about 207°C.

### EXAMPLE 11

The compound 1-(2,6-dichloro-4-trifluoromethylphenyl)-2,4-bis(trifluoromethylthio)-3-cyano-5-amino pyrrole (**EXAMPLE 11**) has a melting point of 161^{o}C and is made according to the process of **EXAMPLE 13** (first compound) using an excess of trifluoromethanesulfenyl chloride.

### EXAMPLE 12

To a cold (0°C) solution of 1.53 g (3.60 mmoles) of 1-(2,6-dichloro-4-trifluoromethylphenyl)-2-amino-3-trifluoromethylthio-4-cyanopyrrole (made according to the process described in **EXAMPLE 13**, melting point about 182°C) in 15 ml of pyridine was added, under inert atmosphere, a solution of 1.46 g (3.6 mmoles) of 80% pyridinium bromide perbromide in 15 ml of pyridine. After 30 minutes, the reaction mixture was poured into cold (0°C) ethyl ether and a precipitate which formed was removed by filtration. The filtrate was washed with aqueous HCl, aqueous NaOH and water. The organic layer was dried over anhydrous magnesium sulfate and evaporated to yield 1.34 g of a brown solid. This was combined with 230 mg of product from an earlier reaction of 300 mg (0.7 mmole) of 1-(2,6-dichloro-4-trifluoromethylphenyl)-2-amino-3-trifluoromethylthio-4-cyanopyrrole and 0.29 g of 80% pyridinium bromide perbromide. The consolidated products were chromatographed on silica gel eluting with 4:1 v/v hexane-ethyl acetate to give 1.31 g (73%) of 1-(2,6-dichloro-4-trifluoromethylphenyl)-2-amino-3-trifluoromethylthio-4-cyano-5-bromopyrrole (**EXAMPLE 12**) as a white solid. Recrystallization from hexane/ethyl acetate furnished 910 mg of this product as colorless needles, melting point about 160°C.

### EXAMPLE 13

A stirred solution of 2.00 g (6.25 mmoles) of 1-(2,6-dichloro-4-trifluoromethylphenyl)-2-amino-4-cyanopyrrole in 60 ml of dichloromethane, made as indicated hereafter, was cooled with an ice bath and 10 ml of a cold (-78^{o}C) dichloromethane solution containing 0.55 ml (0.85 g, 6.2 mmoles) of trifluoromethanesulfenyl chloride was added in a slow stream. After stirring at 0^{o}C for two hours, a stream of nitrogen was passed through the reaction mixture for one hour. Partitioning with saturated aqueous sodium bicarbonate and water, drying over anhydrous magnesium sulfate and concentrating in vacuo furnished 3.14 g of a light brown solid. This was chromatographed on silica gel with a 3:2 v/v dichloromethane-hexane eluent to provide two colorless solid samples weighing 900 mg and 950 mg. These were recrystallized from chloroform to provide, respectively, 680 mg and 630 mg of 1-(2,6-dichloro-4-trifluoromethylphenyl)-2-amino-3-trifluoromethylthio-4-cyanopyrrole (**EXAMPLE 13**), melting point about 182^{o}C.

The reactant used in this process was made according to the following process: A solution of 4.64 g (14.5 mmoles) of 1-[(2,6-dichloro-4-trifluoromethylphenyl)amino]-2,3-dicyanopropene and 2.02 ml (1.47 g, 14.5 mmoles) of triethylamine in 30 ml of benzene was heated at reflux overnight and then concentrated in vacuo. The residue was partitioned between ethyl ether and water and the ether layer was dried over anhydrous magnesium sulfate and concentrated to give 3.79 g of a light brown solid. Recrystallization from ethanol-water provided 2.79 g (60%) of 1-(2,6-dichloro-4-trifluoromethylphenyl)-2-amino-4-cyanopyrrole, melting point about 176°C.

The starting 1-arylamino-2,3-dicyano propene was made according to the following process: A 20.5 g (0.140 mole) sample of the potassium salt of formylsuccinonitrile was dissolved in approximately 30 ml of water and made acidic with concentrated hydrochloric acid. This was extracted with ethyl ether, the ethereal extract dried over anhydrous magnesium sulfate and evaporated to give 3.87 g of a brown liquid. This was added to a solution containing 5.04 g (22 mmoles) of 2,6-dichloro-4-trifluoromethylaniline and 40 mg of para-toluene sulfonic acid monohydrate in 50 ml of benzene. The heterogeneous reaction mixture was heated to reflux overnight with separation of water. The reaction mixture was then cooled and concentrated to give 7.66 g of a yellow liquid. Trituration with hexane precipitated 6.68 g (95%) of 1-[(2,6-dichloro-4-trifluoromethylphenyl)amino]-2,3-dicyanopropene as a yellow solid. Recrystallization from ethanol/water provided a sample, melting point about 101°C.

### EXAMPLES 14A and 14B

To a suspension of 1.17 g (3.30 mmoles) of 1-(4-trifluoromethylphenyl)-2-amino-3-trifluoromethylthio-4-cyanopyrrole and 0.46 ml (0.34 g, 3.3 mmoles) of triethylamine in 20 ml of chloroform cooled to -20°C was added a solution of 0.19 ml (0.59 g, 3.7 mmoles) of bromine in 5 ml of chloroform. The reaction mixture was stirred at -20°C for 1 hour and then allowed to warm to 0°C. Another 0.04 ml (0.13 g, 0.8 mmole) of bromine was then added and after 15 minutes of additional stirring, the reaction mixture was diluted with dichloromethane and partitioned with water and a saturated aqueous sodium bicarbonate solution. The organic layer was dried over anhydrous magnesium sulfate and concentrated to give 1.11 g of a brown solid. This material was combined with that from a previous reaction of 1.00 g (2.8 mmoles) of 1-(4-trifluoromethylphenyl)-2-amino-3-trifluoromethylthio-4-cyanopyrrole and 0.15 ml of bromine. Chromatography on silica gel eluting with 3:1 v/v dichloromethane-hexane furnished 1.40 g (52%) of 1-(4-trifluoromethylphenyl)-2-amino-3-trifluoromethylthio-4-cyano-5-bromopyrrole(**EXAMPLE 14A**) as a yellow solid. Recrystallization from hexane-ethyl acetate provided the product as light yellow platelets, melting point about 175°C.

The 1-(4-trifluoromethylphenyl)-2-amino-3-trifluoromethylthio-4-cyanopyrrole(**EXAMPLE 14B**), melting point about 152°C, can be made from 1-[(4-trifluoromethylphenyl)amino]-2,3-dicyanopropene according to a process similar to the one described in **EXAMPLE 13**.

### EXAMPLES 15A and 15B

1-[(2-Chloro-4-trifluoromethylphenyl)amino]-2,3-dicyanopropene was made according to a process similar to the one described in **EXAMPLE 13**. This dicyano-propene was used to prepare 1-(2-chloro-4-trifluoromethylphenyl)-2-amino-3-trifluoromethylthio-4-cyanopyrrole(**EXAMPLE 15A**), melting point about 169°C, made according to a process similar to the one described in **EXAMPLE 13**. This pyrrole was used to prepare 1-(2-chloro-4-trifluoromethylphenyl)-2-amino-3-trifluoromethylthio-4-cyano-5-bromopyrrole(**EXAMPLE 15B**), melting point about 157°C, according to the process of **EXAMPLE 14**.

### EXAMPLES 16A and 16B

The 1-(2,6-dichloro-4-trifluoromethylphenyl)-2-amino-4-cyanopyrrole, made according to **EXAMPLE 13**, was treated with CFCl₂-SCl according to the process of **EXAMPLE 13** (which used CF₃SCl) to provide 1-(2,6-dichloro-4-trifluoromethylphenyl)-2-amino-3-dichlorofluoromethylthio-4-cyanopyrrole, (**EXAMPLE 16A**), melting point about 202^{o}C.

This compound was treated with t-butyl nitrite according to the process of **EXAMPLE 4** to provide 1-(2,6-dichloro-4-trifluoromethylphenyl)-2-chloro-3-cyano-4-(dichlorofluoromethylthio)pyrrole (**EXAMPLE 16B**), melting point about 158^{o}C.

### EXAMPLE 17

The last compound of **EXAMPLE 16** was reacted according to a process similar to the process of **EXAMPLES 1 and 2** using hydrogen peroxide in trifluoromethylperacetic acid (instead of m-chloro peroxybenzoic acid) to provide 1-(2,6-dichloro-4-trifluoromethylphenyl)-2-chloro-3-cyano-4-(dichlorofluoromethylsulfinyl)pyrrole (**EXAMPLE 17**), melting point about 119^{o}C.

### EXAMPLE 18

According to the process of **EXAMPLE 17** using a double amount of hydrogen peroxide, the last compound of **EXAMPLE 16** was transformed into 1-(2,6-dichloro-4-trifluoromethylphenyl)-2-chloro-3-cyano-4-(dichlorofluoromethylsulfonyl)pyrrole (**EXAMPLE 18**), melting point about 179^{o}C.

### EXAMPLE 19

The first compound of **EXAMPLE 16** was treated with t-butyl nitrite according to the process of **EXAMPLE 4** to provide 1-(2,6-dichloro-4-trifluoromethylphenyl)-3-cyano-4-(dichlorofluoromethylthio)pyrrole (**EXAMPLE 19**), melting point about 120^{o}C.

### EXAMPLE 20

The compound of **EXAMPLE 19** was oxidized according to the process of **EXAMPLE 17** to provide 1-(2,6-dichloro-4-trifluoromethylphenyl)-3-cyano-4-(dichlorofluoromethylsulfinyl)pyrrole (**EXAMPLE 20**), mp 150-152°C.

### EXAMPLES 21A, 21B and 21C

1-[(2,6-Dichloro-4-trifluoromethoxyphenyl)amino]-2,3-dicyanopropene was prepared according to the process of the last compound of **EXAMPLE 13**, substituting 2,6-dichloro-4-trifluoromethoxyaniline for 2,6-dichloro-4-trifluoromethylaniline.

This compound was converted to 1-(2,6-dichloro-4-trifluoromethoxyphenyl)-2-amino-4-cyanopyrrole according to the process of the second compound of **EXAMPLE 13**.

This compound was converted to 1-(2,6-dichloro-4-trifluoromethoxyphenyl)-2-amino-3-trifluoromethylthio-4-cyanopyrrole according to the process of the first compound of **EXAMPLE 13**.

This compound was converted to 1-(2,6-dichloro-4-trifluoromethoxyphenyl)-2-amino-3-(trifluoromethylthio)-4-cyano-5-chloropyrrole (**EXAMPLE 21A**), mp 196-197°C according to the process of **EXAMPLE 8**.

This compound was converted to 1-(2,6-dichloro-4-trifluoromethoxyphenyl)-2-chloro-3-cyano-4-trifluoromethylthiopyrrole (**EXAMPLE 21B**), melting point 172^{o}C according to the process of **EXAMPLE 4**. This compound was converted to 1-(2,6-dichloro-4-trifluoromethoxyphenyl)-2-chloro-3-cyano-4-trifluoromethylsulfonylpyrrole (**EXAMPLE 21C**), melting point 187^{o}C, according to the process of **EXAMPLE 18**.

### EXAMPLES 22A, 22B, AND 22C

A mixture of 2-chloro-4-chlorosulfenyl-3-cyano-1-(2′,6′-dichloro-4′-trifluoromethyl-phenyl)-pyrrole and 2-chloro-3-cyano-4-dichlorofluoromethylsulfenyl-1-(2′,6′-dichloro-4′-trifluoromethylphenyl)-pyrrole 47.77 g, 0.101 moles, 1.0 eq) was dissolved in trifluoroacetic acid (190 mL) at 0^{o}C. 30% H₂O₂ (10.8 mL, 0.106 moles, 1.05 eq) was added dropwise. The reaction was stirred at 0^{o}C for 7 hrs. 15 min., then placed in the refrigerator (10^{o}C) overnight. More 30% H₂O₂ (10.8 mL, 0.106 moles, 1.05 eq) was added at 0^{o}C the following morning. The reaction was stirred at 0^{o}C for 9 hrs, then placed in the refrigerator overnight. More 30% H₂O₂ (10.8 mL, 0.106 moles, 1.05 eq) was added at 0^{o}C the following morning. After 3.5 hrs., the reaction was poured into 2 liters of ice-water, vigorously stirred, then filtered.

Similarly, a mixture of 2-chloro-4-chlorosulfenyl-3-cyano-1-(2′,6′-dichloro-4′-trifluoromethylphenyl)-pyrrole and 2-chloro-3-cyano-4-dichlorofluoro methylsulfenyl -1-(2′,6′-dichloro-4′-trifluoromethylphenyl)-pyrrole (40.77 g, 0.0848 moles, 1.0 eq) was dissolved in trifluoroacetic acid (188 mL) at 0^{o}C. 30% H₂O₂ (17.7 mL, 0.173 moles, 2.05 eq) was added dropwise. The reaction was stirred at 0^{o}C for 2 hrs. 45 min., then placed in the refrigerator (10^{o}C) overnight. After stirring for 8 hrs. at 0^{o}C, the reaction mixture was again placed in the refrigerator overnight. The reaction was then allowed to warm to room temperature, and stirred overnight at room temperature. More 30% H₂O₂ (9.05 mL, 0.0886 moles, 1.05 eq) was added at 0^{o}C the following morning and the reaction kept at 0^{o}C for 6 hrs. 40 min., then allowed to warm to room temperature and stirred over the weekend. The reaction was poured into 2 liters of ice-water, vigorously stirred, then filtered.

The precipitates from both reactions were combined and dissolved in 500 mL of dichloromethane, washed with 500 mL water, 500 mL 10% aqueous NaHSO₃, and 500 mL sat. NaCl. The organic phase was dried over Na₂SO₄, filtered, and the solvent evaporated to afford 74.96 g (79.9% yield) of a solid. This was recrystallized from 690 mL hexane:dichloro-methane (2:1), to which 20 mL dichloromethane was added, to afford 6.98 g of a solid, identified as 2-chloro-4-chlorosulfonyl-3-cyano-1-(2′,6′-dichloro-4′-trifluoromethylphenyl)-pyrrole (**EXAMPLE 22A**) This was then recrystallized from 103 mL isopropanol to afford 3.97 g . mp 187-188.5 ^{o}C.

2-Chloro-4-chlorosulfonyl-3-cyano-1-(2′,6′-dichloro-4′-trifluoromethylphenyl)-pyrrole (3.97 g, 9.06 mmoles, 1.0 eq) was dissolved in THF (15.8 mL) at 0^{o}C. Triphenylphosphine (2.41 g, 1.0 eq) was added as a solid. The solution turned yellow. After 2.5 hrs., the ice bath was removed and the reaction allowed to stir at room temperature overnight. More triphenylphosphine was added (2.55 g, 9.72 mmoles. 1.06 eq) and the reaction allowed to stir at room temperature overnight. A precipitate formed, 3 mL THF was added, and the reaction mixture washed twice with sat. NaCl, and back-extracted. The organic phase was dried over MgSO₄, filtered, and the solvent evaporated *in vacuo* to afford a waxy-solid, 9.44 g This was chromatographed on silica gel to yield 3.39 g of a waxy solid. This was then recrystallized from 140 mL isopropanol to afford 2.54 g (74.9%) bis-[2-chloro-3-cyano-1-(2′,6′-dichloro-4′-trifluoromethylphenyl)-pyrrol-4-yl]-disulfide (**EXAMPLE 22B**), mp 218.8-220.3^{o}C.

Bis-[2-chloro-3-cyano-1-(2′,6′-dichloro-4′-trifluoromethylphenyl)-pyrrol-4-yl]-disulfide (0.80 g, 1.08 mmoles, 1.0 eq) was dissolved in DMF (10 mL) and cooled to 0^{o}C. Na₂HPO₄ (0.46 g, 3.24 mmoles, 3.0 eq) was dissolved in 5 mL water, then added to the DMF solution. A precipitate formed, so 15 mL DMF and 10 mL water were added. Solid Na₂S₂O₄ (0.564 g, 3.24 mmoles, 3.0 eq) was added. The reaction turned pale yellow in color. Dibromodifluoromethane (0.65 g, 3.1 mmoles, 2.87 eq) was added to a cold tared vial, then transferred to the reaction. The reaction mixture became colorless with a white precipitate. After 1 hr. 50 min., 10 mL DMF was added, followed by an additional 0.93 g of CBr₂F₂, and the reaction vessel sealed and allowed to stir at room temperature overnight. After cooling to 0^{o}C, the reaction mixture was added to 200 mL water and extracted four times with 150 mL ethyl ether. The organic phase was washed twice with 100 mL 5% aqueous HCl, twice with 100 mL sat. NaHCO₃, and with 100 mL sat. NaCl. The organic phase was dried over MgSO₄, filtered, and the solvent evaporated *in vacuo* to afford 80.7 mg of a white solid. The initial aqueous phase was then filtered to collect a white solid which had precipitated overnight. It was dissolved in dichloro-methane, the solvent evaporated *in vacuo*, and dried to afford 0.348 g white solid (total yield, 0.429 g, 40%). This was combined with the 80.7 mg sample and chromatographed on silica gel to afford 0.362 g white solid, identified as 4-bromodifluoromethylsulfenyl-2-chloro-3-cyano-1-(2′,6′-dichloro-4′-trifluoromethylphenyl)-pyrrole (**EXAMPLE 22C**) mp 128.3-133.7^{o}C.

### ADDITIONAL SYNTHESIZED EXAMPLES

Following the procedures detailed above for the synthesis of compounds of **EXAMPLES 1 to 22** or the other methods or processes of synthesis generally described herein, there were prepared a number of additional synthesized examples (ASE) of pyrrole compounds of formula (I). The structures of these compounds and their corresponding melting points are provided in **TABLE 3 (ASE-No's. 1-91**: compounds of formula (I): wherein, X² and X³ are hydrogen and the other substituents are as defined). and **TABLE 4 (ASE-No's. 92-195**: compounds of formula (I): wherein X² and X³ are hydrogen, X¹ and X⁴ are chloro, Y is trifluoromethyl, and the other substituents are as defined).

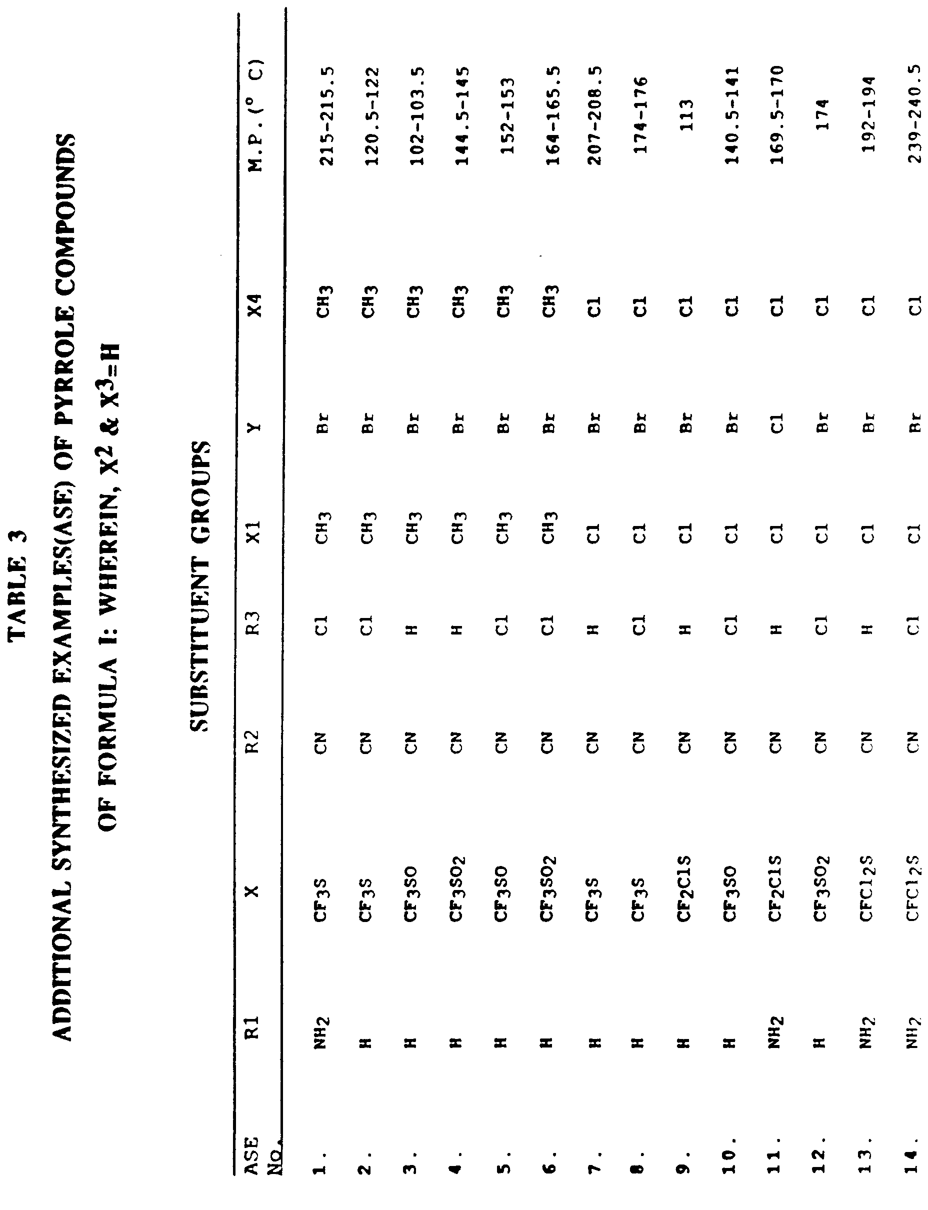

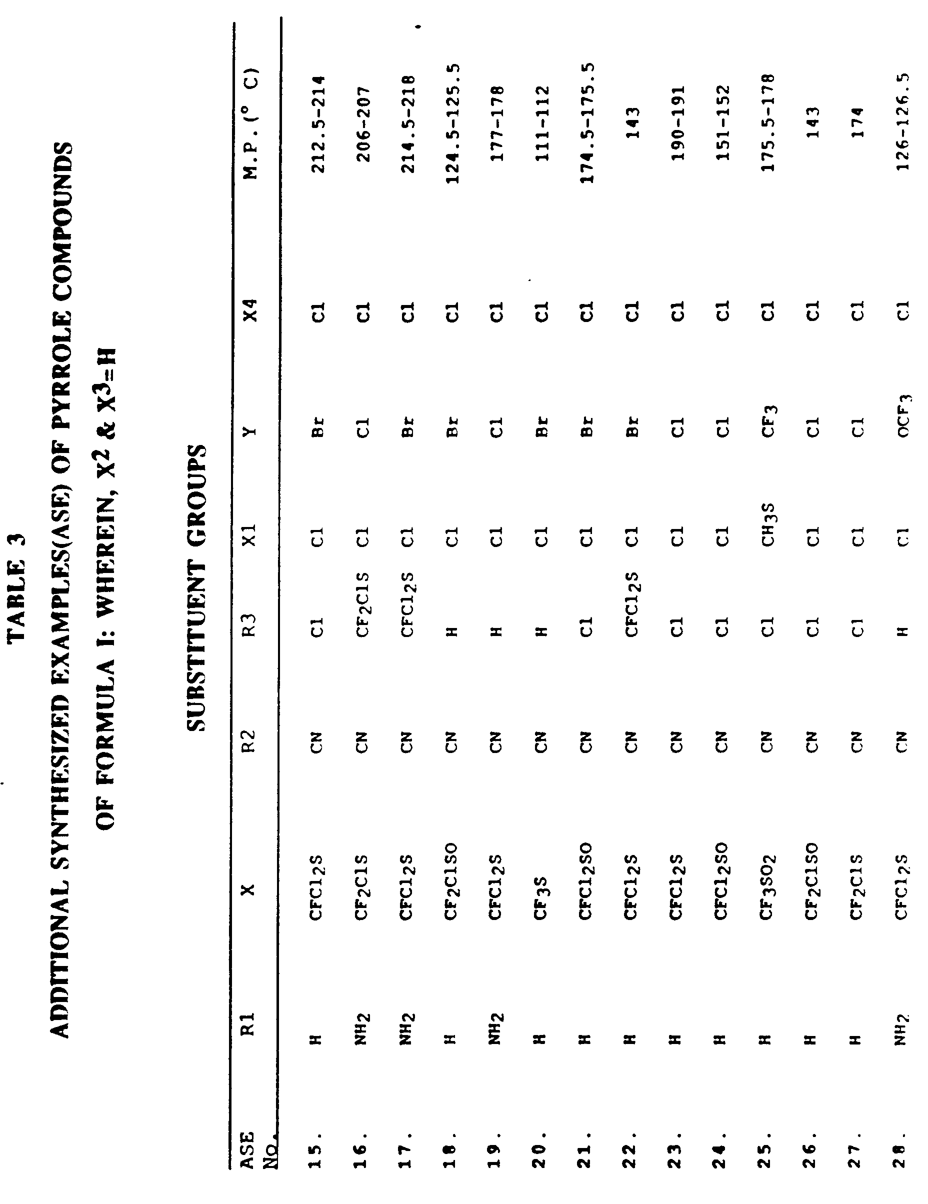

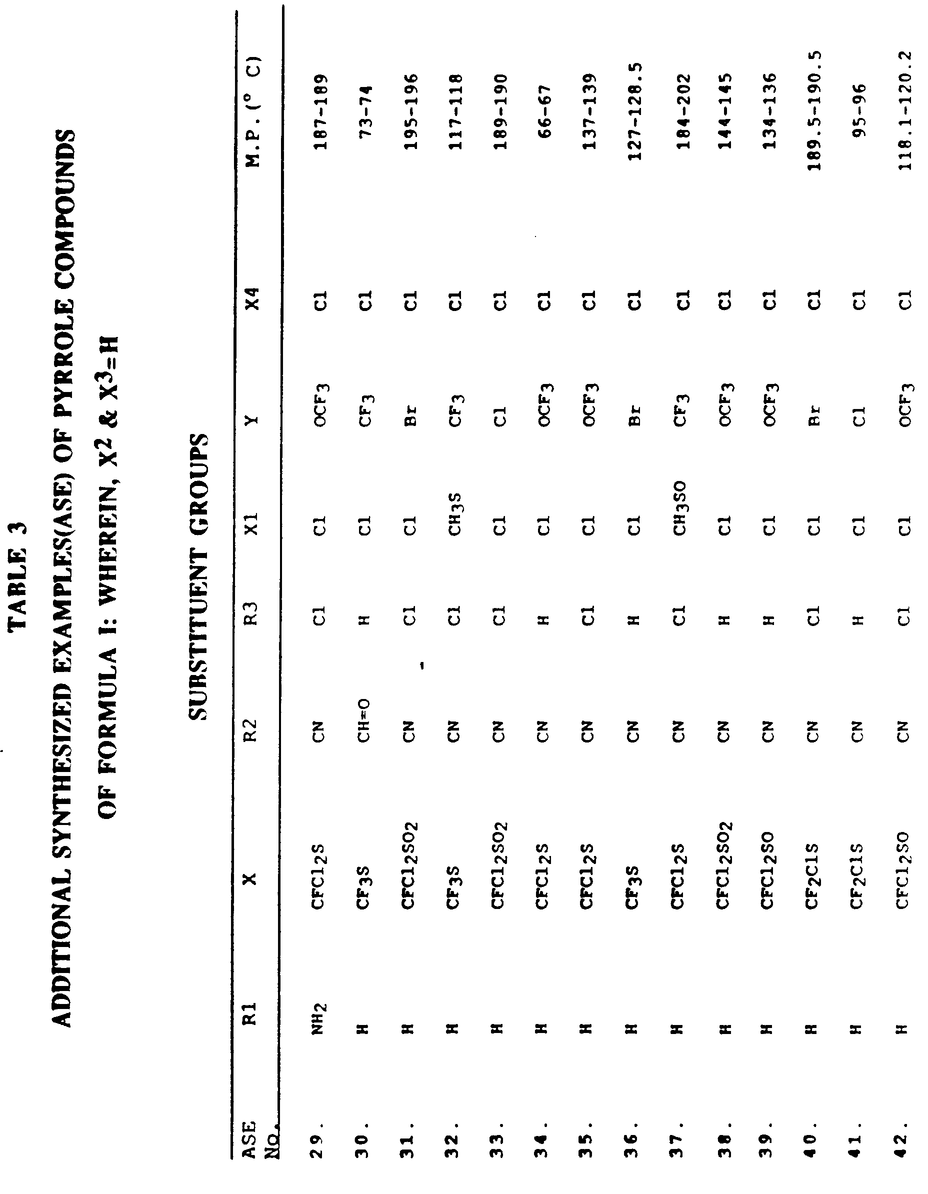

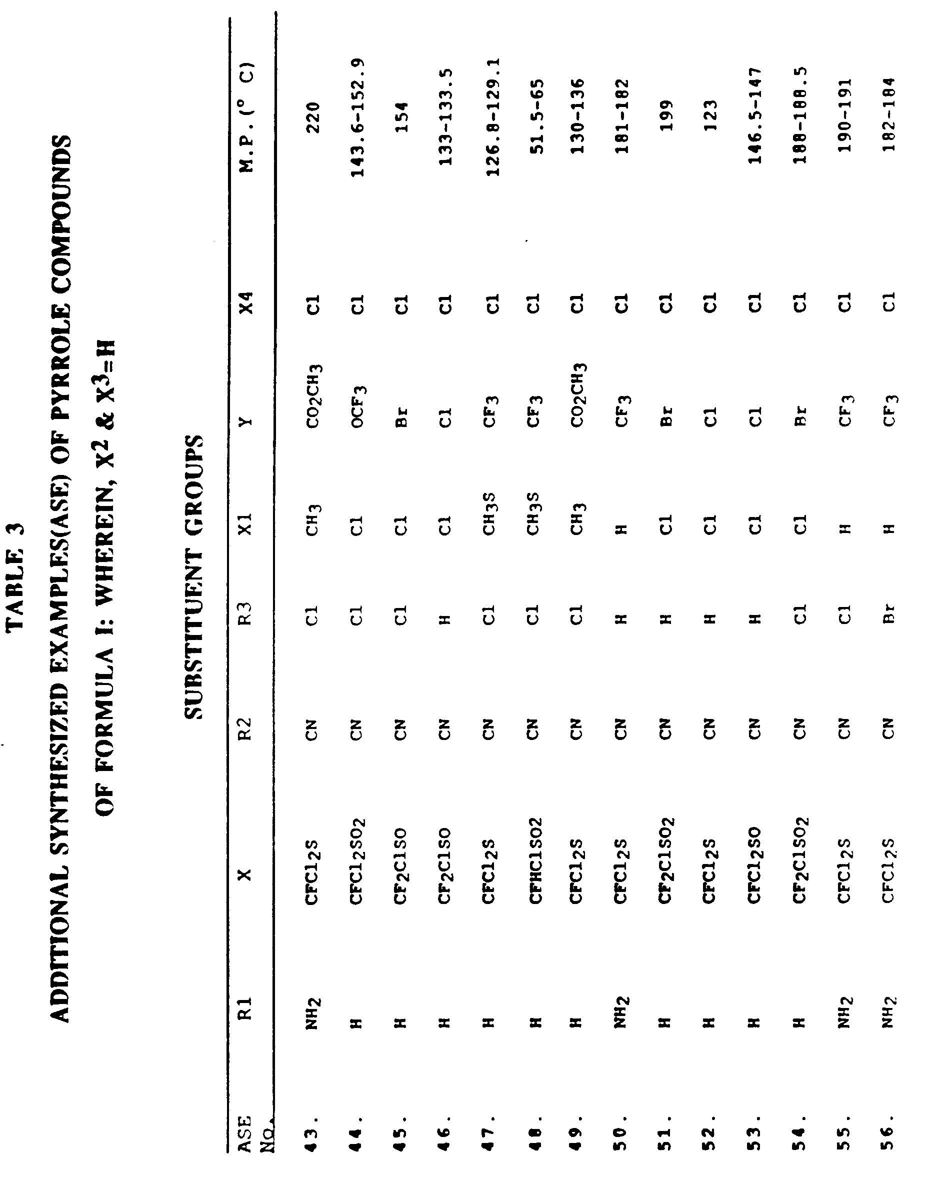

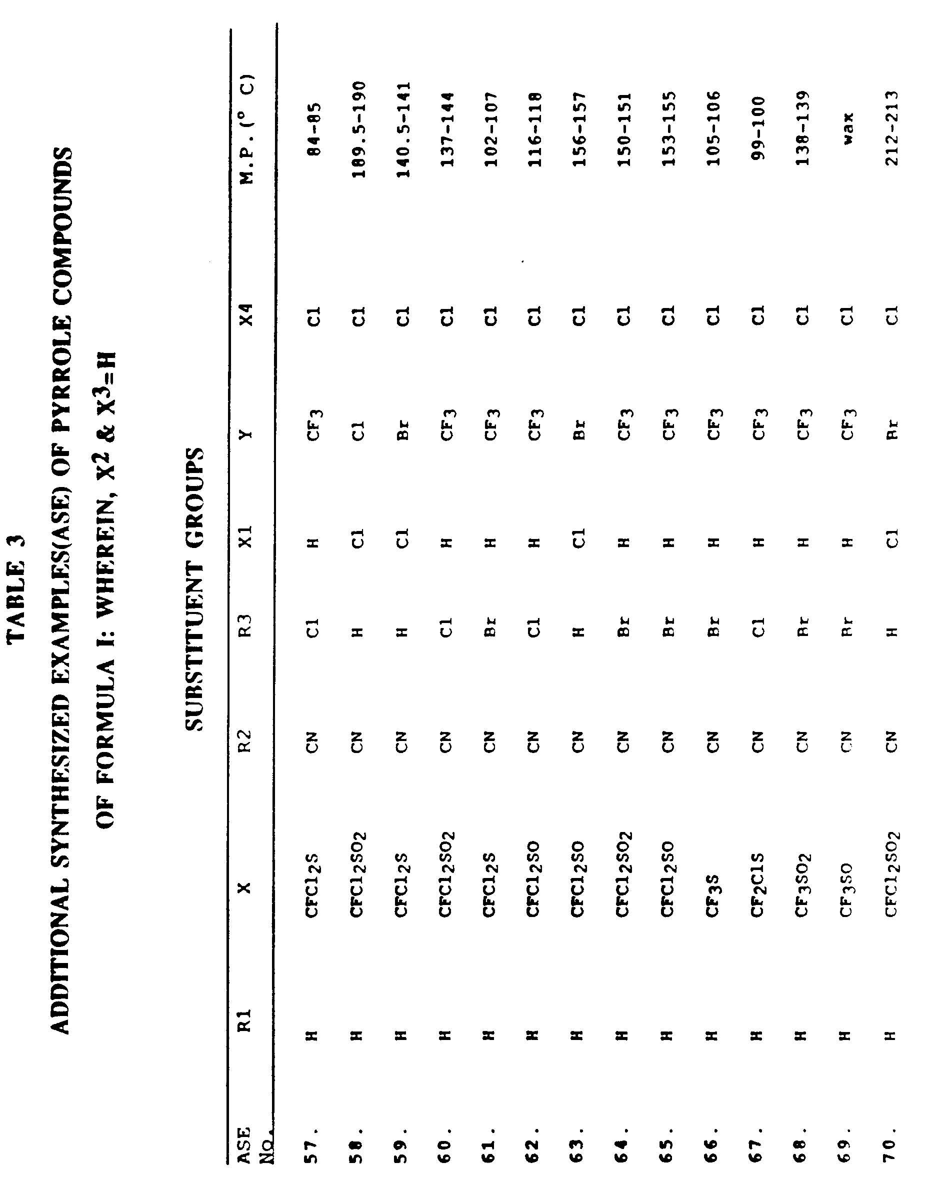

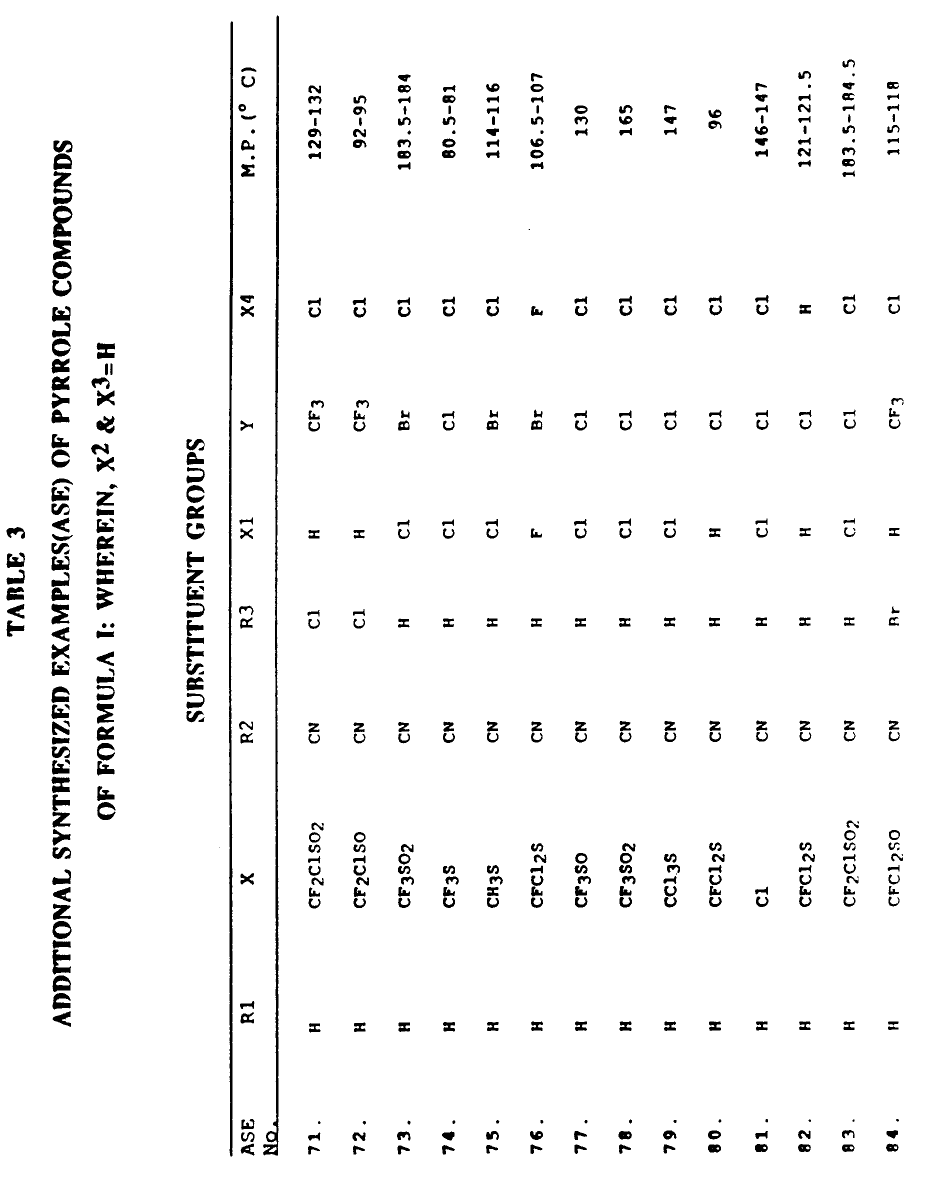

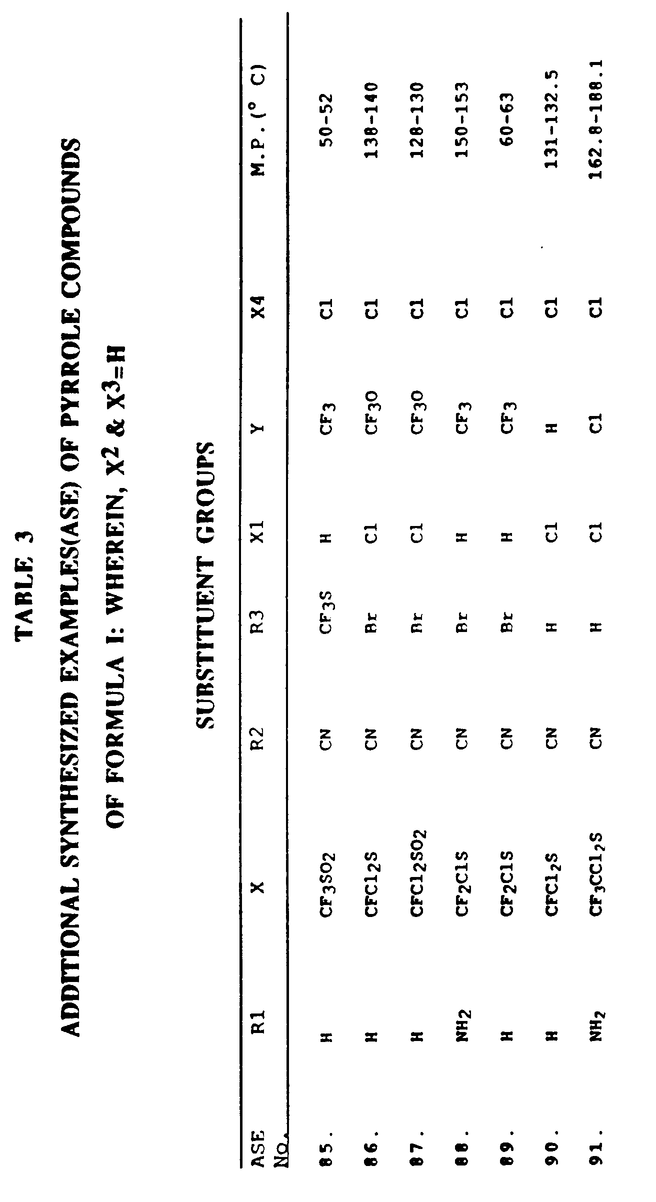

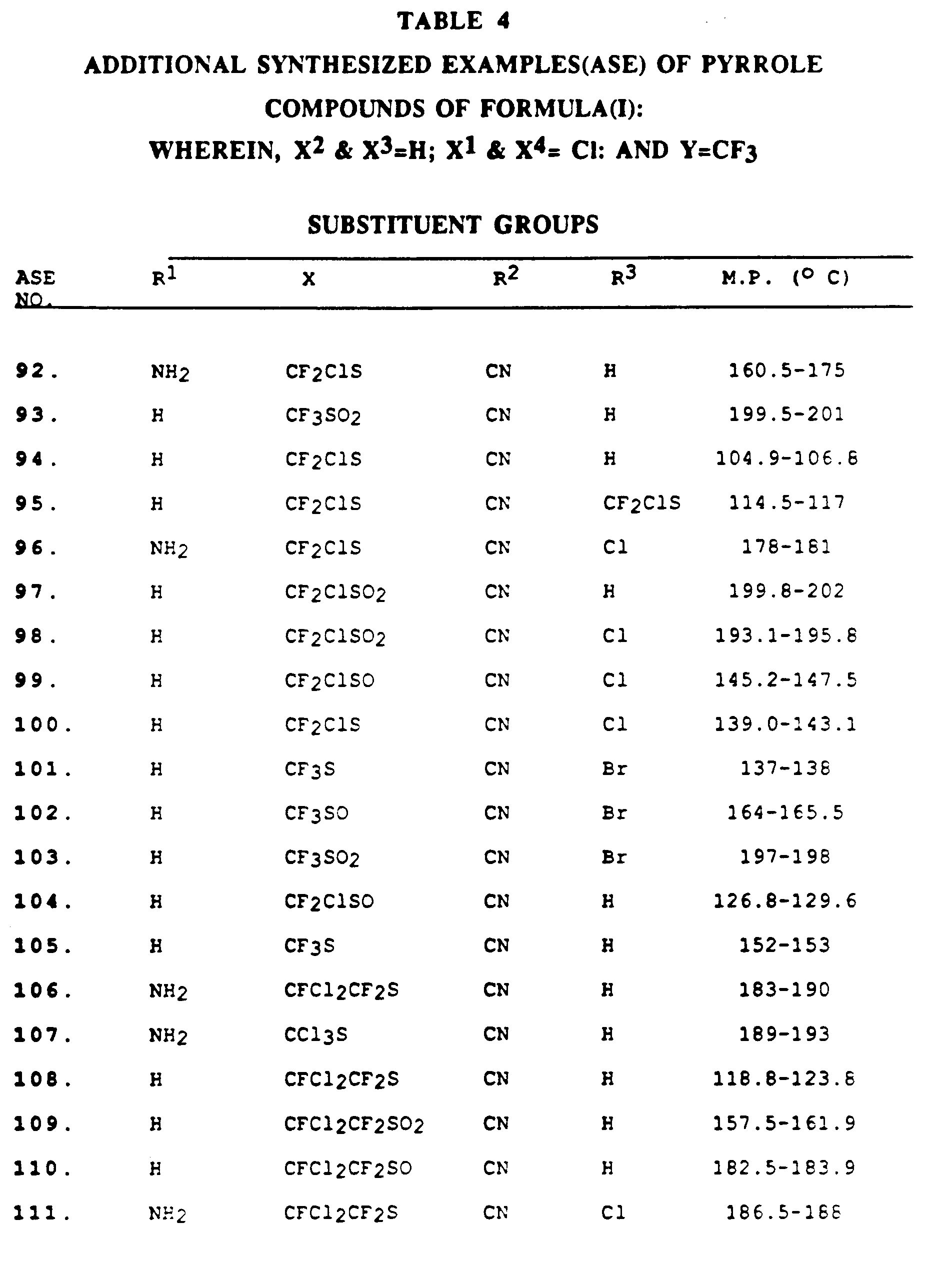

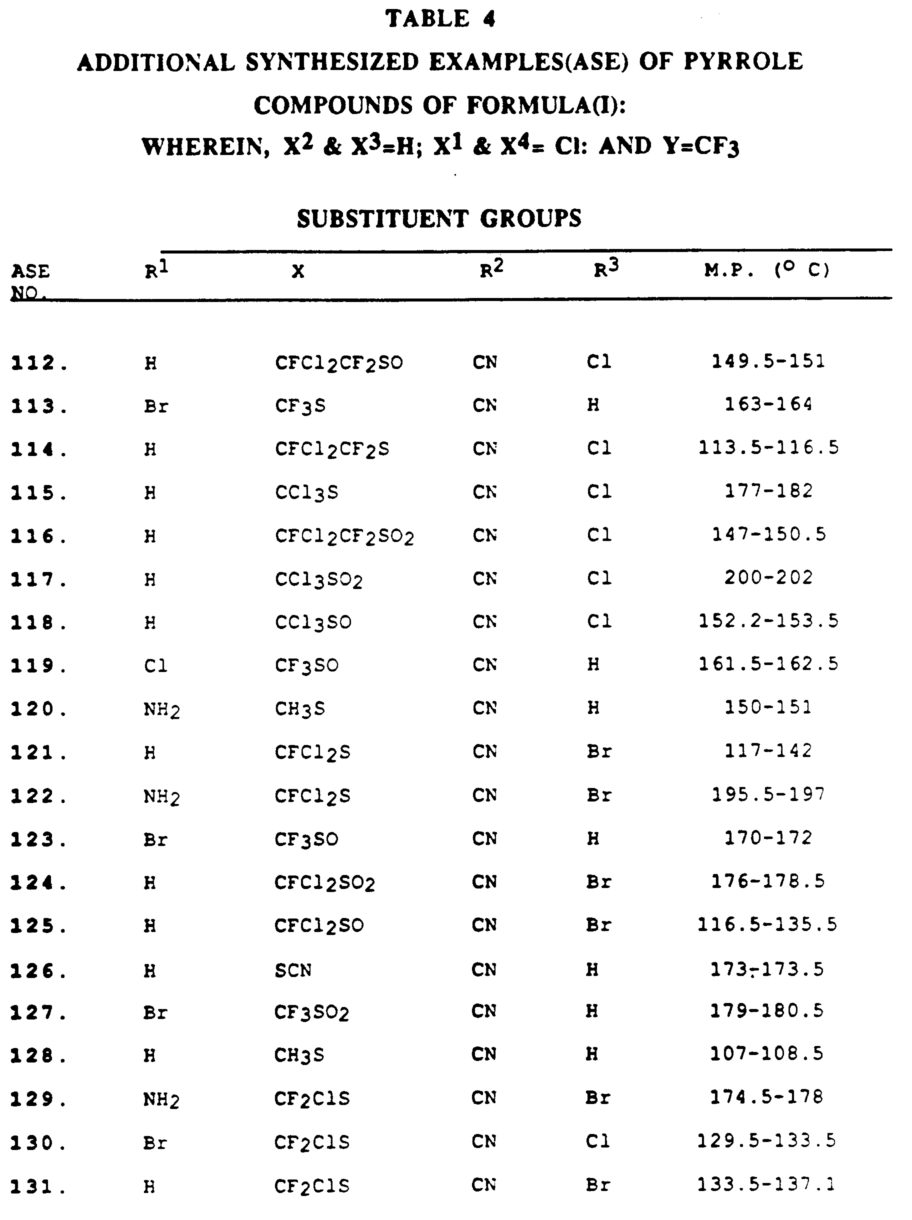

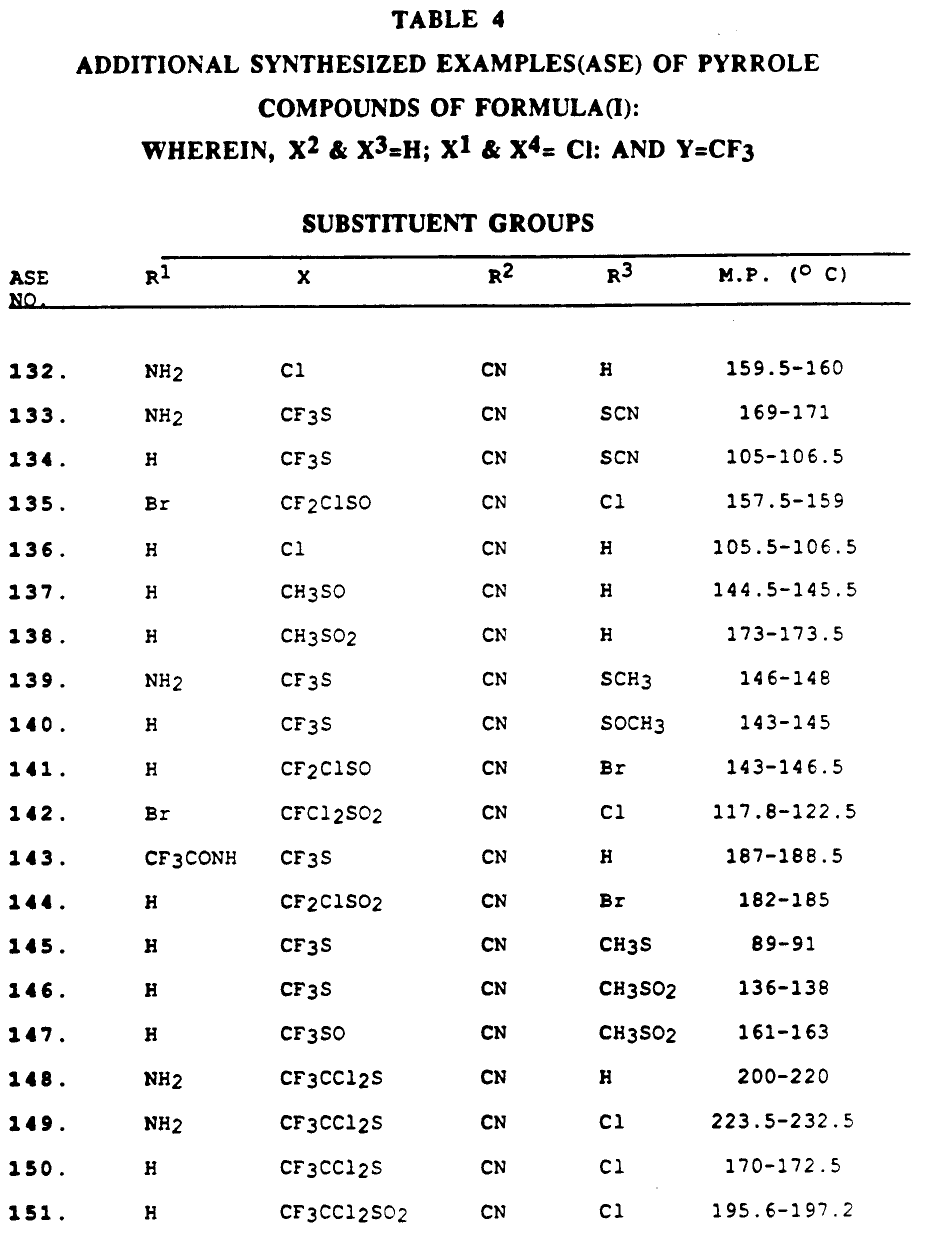

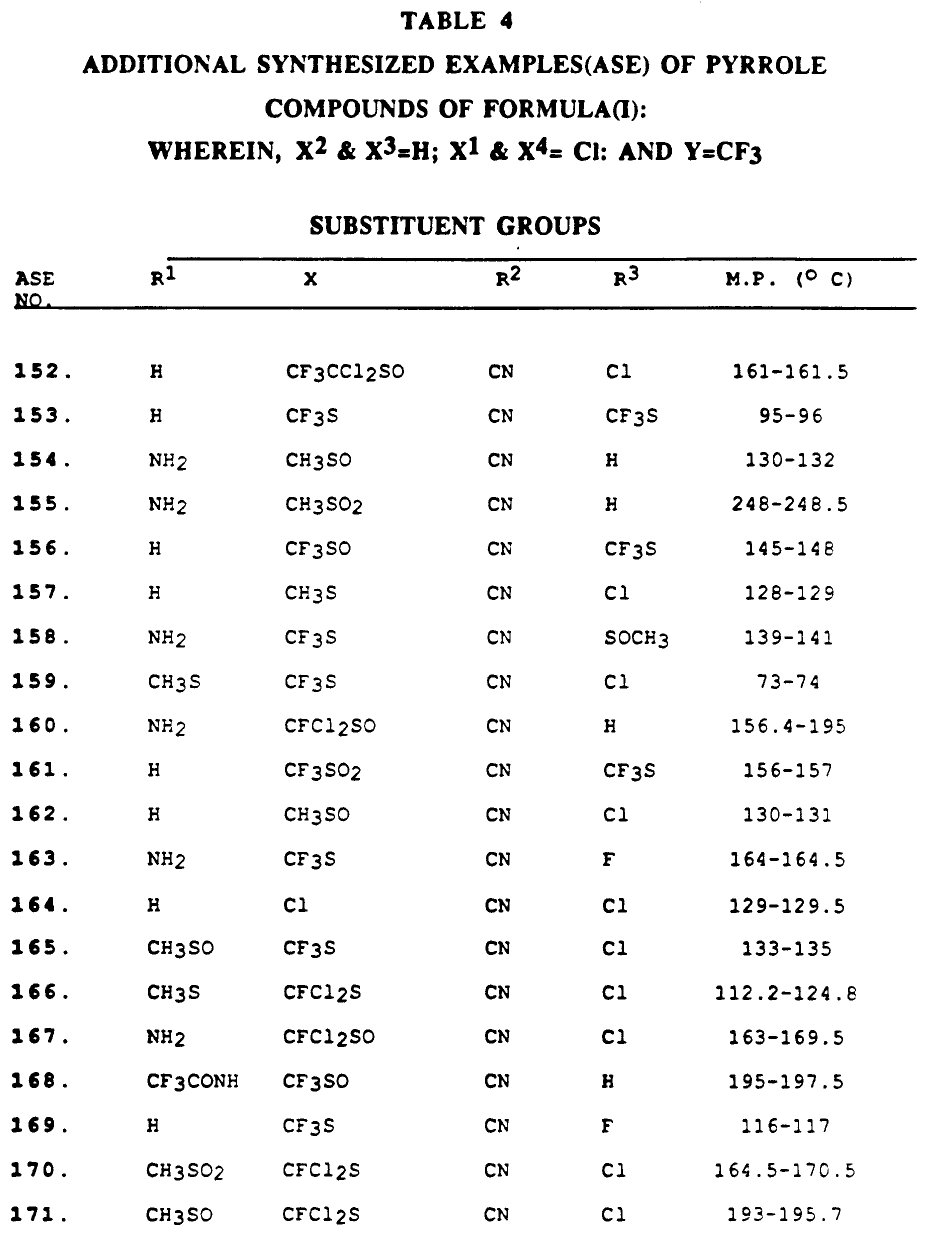

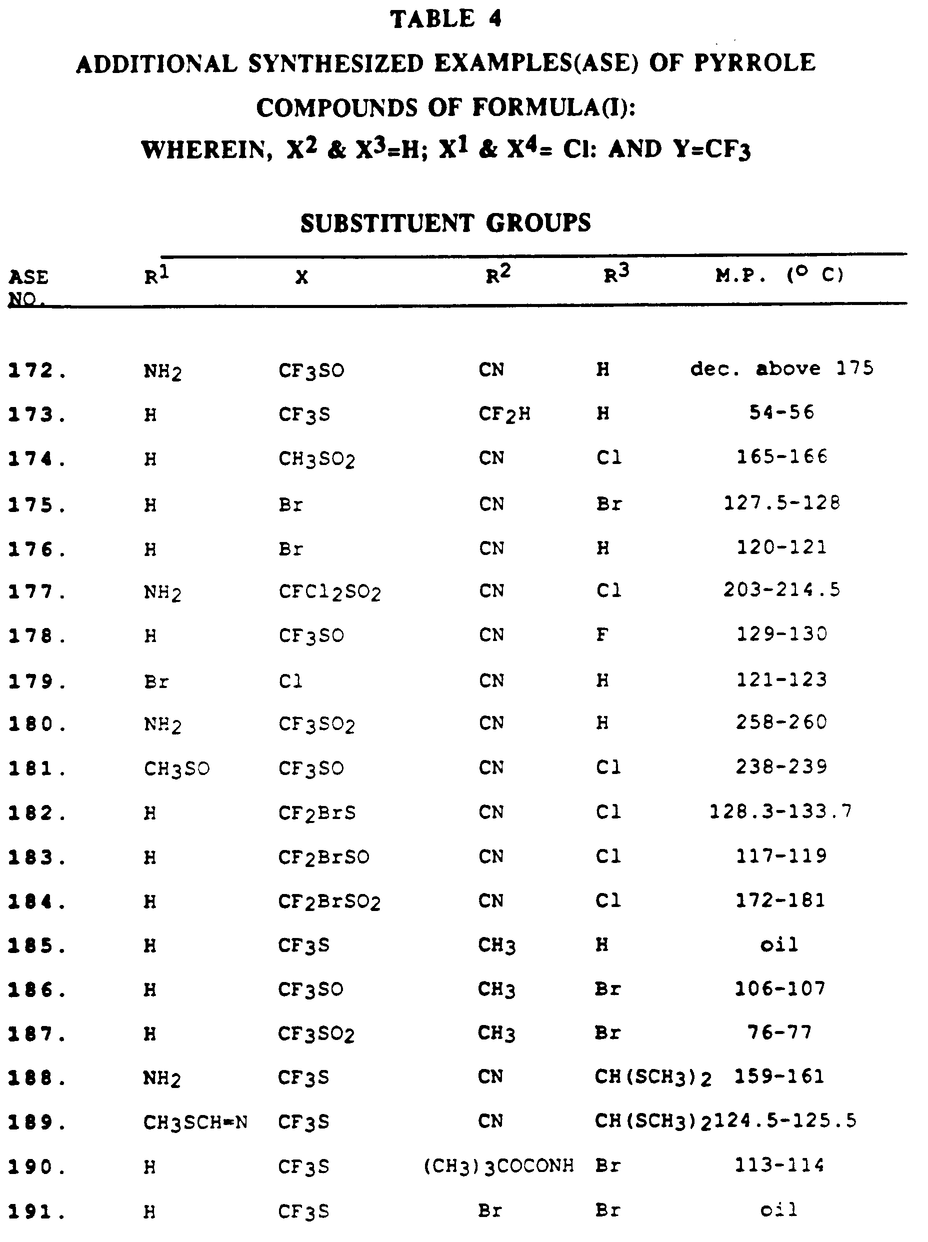

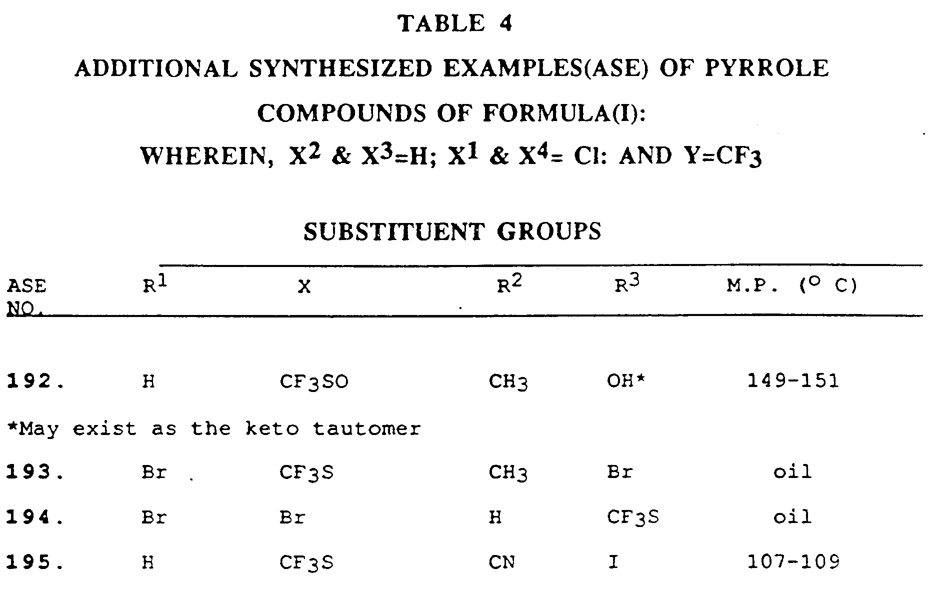

It is to be understood that in the following description of pesticidal methods and compositions references to compounds of formula I are to such compounds wherein the various symbols are as hereinbefore defined but including the compound wherein X⁴ = X¹ = Y = R¹ = X = R² = R³ = Cl and at the same time X³ = X² = H.

### PESTICIDAL METHODS OF USE AND COMPOSITIONS

According to a feature of the present invention, there is provided a method for the control of arthropods, especially insects and arachnids, plant nematodes, and helminth or protozoan pests at a locus which comprises the treatment of the locus (e.g. by application or administration) with an effective amount of a compound of general formula (I), wherein the various symbols are as hereinbefore defined. The compounds of general formula (I) may, in particular, be used in the field of veterinary medicine and livestock husbandry and in the maintenance of public health against arthropods, helminths or protozoa which are parasitic internally or externally upon vertebrates, particularly warm-blooded vertebrates, for example man and domestic animals, e.g. cattle, sheep, goats, equines, swine, poultry, dogs and cats, for example Acarina, including ticks (e.g. Ixodes spp., Boophilus spp. e.g. Boophilus microplus, Amblyomma spp., Hyalomma spp., Rhipicephalus spp. e.g. Rhipicephalus appendiculatus, Haemaphysalis spp., Dermacentor spp., Ornithodorus spp. (e.g. Ornithodorus moubata and mites (e.g. Damalinia spp., Dermahyssus gallinae, Sarcoptes spp. e.g. Sarcoptes scabiei, Psoroptes spp., Chorioptes spp;, Demodex spp., Eutrombicula spp.,); Diptera (e.g. Aedes spp., Anopheles spp., Musca spp., Hypoderma spp., Gasterophilus spp., Simulium spp.); Hemiptera (e.g. Triatoma spp.); Phthirapter (e.g. Damalinia spp., Linognathus spp.); Siphonaptera (e.g. Ctenocephalides spp.); Dictyoptera (e.g. Periplaneta spp., Blatella spp.); Hymenoptera (e.g. Monomorium pharaonis); for example against infections of the gastro-intestinal tract caused by parasitic nematode worms, for example members of the family Trichostrongylidae, Nippostrongylus brasiliensis, Trichinella spiralis, Haemonchus contortus, Trichostrongylus colubriformis, Nematodirus battus, Ostertagia circumcincta, Trichostrongylus axei, Cooperia spp. and Hymenolepis nana; in the control and treatment of protozoal diseases caused by, for example, Eimeria spp. e.g. Eimeria tenella, Eimeria acervulina, Eimeria brunetti, Eimeria maxima and Eimeria necatrix, Trypanosoma cruzi, Leishamania spp., Plasmodium spp., Babesia spp., Trichomonadidae spp., Histomonas spp., Giardia spp., Toxoplasma spp., Entamoeba histolytica and Theileria spp.; in the protection of stored products, for example cereals, including grain and flour, groundnuts, animal feedstuffs, timber and household goods, e.g. carpets and textiles, against attack by arthropods, more especially beetles, including weevils, moths and mites, for example Ephestia spp. (flour moths), Anthrenus spp. (carpet beetles), Tribolium spp. (flour beetles), Sitophilus spp. (grain weevils) and Acarus spp. (mites), in the control of cockroaches, ants and termites and similar arthropod pests in infested domestic and industrial premises and in the control of mosquito larvae in waterways, wells, reservoirs or other running or standing water; for the treatment of foundations, structure and soil in the prevention of the attack on buildings by termites, for example, Reticulitermes spp., Heterotermes spp., Coptotermes spp.; in agriculture, against adults, larvae and eggs of Lepidoptera (butterflies and moths) e.g. Heliothis spp. such as Heliothis virescens (tobacco budworm), Heliothis armigera and Heliothis zea. Spodoptera spp. such as S. exempta, S. littoralis (Egyptian cotton worm), S. eridania (southern army worm), Mamestra configurata (bertha army worm): Earias spp. e.g. E. insulana (Egyptian bollworm), Pectinophora spp. e.g. Pectinophora gossypiella (pink bollworm), Ostrinia spp. such as O. nubilalis (European cornborer), Trichoplusiani (cabbage looper), Pieris spp. (cabbage worms),Laphygma spp. (army worms), Agrotis and Amathes spp.(cutworms), Wiseana spp. (porina moth), Chilo spp. (rice stem borer) Tryporyza spp. and Diatraea spp. (sugar cane borers and rice borers), Sparganothis pilleriana (grape berry moth), Cydia pomonella (codling moth), Archips spp. (fruit tree tortrix moth), Plutella xylostella (diamond back moth); against adult and larvae of Coleoptera (beetles) e.g. Hypothenemus hampei (coffe berry borer), Hylesinus spp. (bark beetles), Anthonomus grandis (cotton boll weevil), Acalymma spp. (cucumber beetles), Lema spp., Psylliodes spp., Leptinotarsa decemlineata (colorado potato beetle), Diabrotica spp. (corn rootworms), Gonocephalum spp. (false wire worms), Agriotes spp. (wireworms), Dermolepida and Heteronychus spp. (white grubs), Phaedon cochleariae (mustard beetle), Lissorhoptrus oryzophilus (rice water weevil), Meligethes spp. (pollen beetles), Ceutorhynchus spp., Rhynchophorus and Cosmopolites spp. (root weevils); against Hemiptera e.g. Psylla spp., Bemisia spp., Trialeurodes spp., Aphis spp., Myzus spp., Megoura viciae, Phylloxera spp., Adelges spp., Phorodon humuli (hop damson aphid), Aeneolamia spp., Nephotettix spp. (rice leaf hoppers), Empoasca spp., Nilaparvata spp., Perkinsiella spp., Pyrilla spp., Aonidiella spp. (red scales), Coccus spp., Pseucoccus spp., Helopeltis spp. (mosquito bugs), Lygus spp., Dysdercus spp., Oxycarenus spp., Nezara spp. Hymenoptera e.g. Athalia spp. and Cephus spp. (saw flies), Atta spp. (leaf cutting ants); Diptera e.g. Hylemyia spp; (root flies), Atherigona spp. and Chlorops spp; (shoot flies), Phytomyza spp. (leaf miners), Ceratitis spp. (fruit flies); Thysanoptera such as Thrips tabaci; Orthoptera such as Locusta and Schistocerca spp., (locusts) and crickets e.g. Gryllus spp., and Acheta spp.; Collembola e.g. Sminthurus spp. and Onychiurus spp. (springtails), Isoptera e.g. Odontotermes spp. (termites), Dermaptera e.g. Forficula spp. (earwigs) and also other arthropods of agricultural significance such as Acari (mites) e.g. Tetranychus spp.; Panonychus spp. and Bryobia spp. (spider mites), Eriophyes spp. (gall mites), Polyphagotarsonemus spp.; Blaniulus spp. (millipedes), Scutigerella spp. (symphilids), Oniscus spp. (woodlice) and Triops spp; (crustacea); nematodes which attack plants and trees of importance to agriculture, forestry and horticulture either directly or by spreading bacterial, viral, mycoplasma or fungal diseases of the plants, root-knot nematodes such as Meloidogyne spp. (e.g. M. incognita); cyst nematodes such as Globodera spp. (e.g. G. rostochiensis); Heterodera spp. (e.g. H. avenae); Radopholus spp. (e.g. R. similis); lesion nematodes such as Pratylenchus spp. (e.g. P. pratensis); Belonolaimus spp. (e.g. B. gracilis); Tylenchulus spp. (e.g. T. semipenetrans);Rotylenchulus spp. (e.g. R. reniformis); Rotylenchus spp. (e.g. R. robustus); Helicotylenchus spp. (e.g. H. multicinctus); Hemicycliophora spp. (e.g. H. gracilis); Criconemoides spp. (e.g. C. similis); Trichodorus spp. (e.g. T. primitivus); dagger nematodes such as Xiphinema spp. (e.g. X. diversicaudatum), Longidorus spp. (e.g. L. elongatus); Hoplolaimus spp. (e.g. H. coronatus); Aphelenchoides spp. (e.g. A. ritzema-bosi, A. besseyi); stem and bulb eelworms such as Ditylenchus spp. (e.g. D. dipsaci).

Further pests which can be controlled with the compounds of the invention include: From the order of Isopoda, for example, Oniseus asellus, Armadillidium vulgare and Porcellio scaber. From the order of Diplopoda, for example, Blaniulus guttulatus. From the order of the Chilopoda, for example Geophilus carpophagus and Scutigera spex. From the order of the Symphyla for example, Scutigerella immaculata. From the order of the Thysanura, for example, Lepisma saccharian. From the order of Collembola, for example, Onychiurus armatus. From the order of the Orthoptera, for example, Blatta orientalis, Periplaneta americana, Leucophaea maderae, Blatella germanica, Acheta domesticus, Gryllotalpa spp., Locusta migratoria migratorioides, Melanoplus differentialis and Schistocerca gregaria. From the order of Dermaptera, for example, Forficula auricularia. From the order of the Isoptera, for example, Reticulitermes spp. From the order of the Anoplura, for example, Phylloxera vastatrix, Pemphigus spp., Pediculus humanus corporis, Haematopinus spp. and Linognathus spp. From the order of the Mallophaga, for example, Trichodectes spp. and Damalinea spp. From the order of the Thysanoptera, for example, Hercinothrips femoralis and Thrips tabaci. From the order of the Heteroptera, for example, Eurygaster spp., Dysdercus intermedius, Piesma quadrata, Cimex lectularius, Rhodnius prolixus and Triatoma spp. From the order of the Coleoptera, for example, Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala Epilachna varivestis Atomaria spp., Oryzaephilus surinamensis, Anthonomus spp., Sitophilus spp., Otiorrhynchus sulcatus, Cosmoplites sordidus, Ceuthorrhynchus assimilis, Hypera postica, Dermestes spp., Trogoderma spp., Anthrenus spp., Attagenus spp., Lyctus spp., Maligethes aeneus, Ptinus spp., Niptus hololeucrus, Gibbium psylloides, Tribolium spp., Tenebrio molitor, Agriotes spp., Conoderus spp., Melolontha melolontha, Amphimallon solstitialis and Costelytra zealandica. From the order of the Hymenoptera, for example, Diprion spp., Hoplocampa spp., Lasius spp., Monomorium pharaonis and Vespa spp. From the order of the Diptera, for example, Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyani, Ceratitis capitata, Dacus oleae and Tipula paludosa. From the order of the Siphonaptera, for example, Xenopsylla cheopis and Ceratophyllus spp. From the order of the Arachnida, for example, Scorpio maurus and Latrodectus mactans. From the order of the Homoptera, for example, Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. and Psylla spp. From the order of the Lepidoptera, of example,Pectinophora gossypiella, Bupalus piniarius, Cheimatobia brumata, Lithocolletis blancardella, Hyponomeuta padella,Plutella maculipennis, Malacosoma neustria, Euproctis chrysorrhoea, Lymantria spp. Bucculatrix thurberiella,Phyllocnistis citrella, Agrotis spp., Euxoa spp., Feltia spp.,Earias insulana, Heliothis spp., Laphygma exigua, Mamestra brassicae, Panolis flammea, Prodenia Litura, Spodoptera spp.,Trichoplusiani, Carpocapsa pomonella. Pieris spp., Chilo spp.,Pyrausta nubilalis, Ephestiakuehniella, Galleria mellonella, Tineola bisselliella, Tinea pellionella, Hofmannophila pseudospretella, Cacoecia podana, Capua reticulana, Choristoneura fumiferana, Clysia ambiguells, Homona magnanime and Tortix viridana.

The invention also provides a method for the control of arthropod or nematode pests of plants which comprises the application to the plants or to the medium in which they grow of an effective amount of a compound of general formula (I).

For the control of arthropods and nematodes, the active compound is generally applied to the locus in which arthropod or nematode infestation is to be controlled at a rate of about 0.005 kg to about 15 kg of active compound per hectare of locus treated, preferably 0.02 kg/ha to 2 kg/ha. Under ideal conditions, depending on the pest to be controlled, the lower rate may offer adequate protection. On the other hand, adverse weather conditions, resistance of the pest and other factors may require that the active ingredient be used in higher proportions. In foliar application, a rate of 0.01 kg to 1 kg/ha may be used.The optimum rate depends usually upon the type of pest being controlled,as well as upon the type and the growth stage of the infested plant, the row spacing and also the method of application.

When the pest is soil-borne, the formulation containing the active compound is distributed evenly over the area to be treated in any convenient manner. Application may be made, if desired, to the field or crop-growing area generally or in close proximity to the seed or plant to be protected from attack. The active component can be washed into the soil by spraying with water over the area or can be left to the natural action of rainfall. During or after application, the formulation can, if desired, be distributed mechanically in the soil, for example by ploughing or disking. Application can be prior to planting, at planting, after planting but before sprouting has taken place or after sprouting.

The compounds of general formula (I) may be applied in solid or liquid compositions to the soil principally to control these nematodes dwelling therein but also to the foliage principally to control these nematodes attacking the aerial parts of the plants (e.g. Aphelenchoides spp. and Ditylenchus spp. listed above).

The compounds of general formula (I) may be of value in controlling pests which feed on parts of the plant remote from the point of application, e.g. leaf feeding insects are killed by the subject compounds applied to roots. In addition the compounds may reduce attacks on the plant by means of antifeeding or repellent effects.

The compounds of general formula (I) are of particular value in the protection of field, forage, plantation, glasshouse, orchard and vineyard crops, of ornamentals and of plantation and forest trees, for example, cereals (such as maize, wheat, rice, sorghum), cotton, tobacco, vegetables and salads (such as beans, cole crops, curcurbits, lettuce, onions, tomatoes and peppers), field crops (such as potato, sugar beet, ground nuts, soyabean, oil seed rape), sugar cane, grassland and forage (such as maize, sorghum, lucerne), plantations (such as of tea, coffe, cocoa, banana, oil palm, cocunut, rubber, spices), orchards and groves (such as of stone and pip fruit, citrus, kiwifruit, avocado, mango,olives and walnuts), vineyards, ornamental plants, flowers and vegetables and shrubs under glass and in gardens and parks, forest trees (both deciduous and evergreen) in forests, plantations and nurseries.

They are also valuable in the protection of timber (standing, felled, converted, stored or structural) from attack by sawflies (e.g. Urocerus) or beetles (e.g. scolytids, platypodids, lyctids, bostrychids, cerambycids, anobiids), or termites, for example, Reticulitermes spp.,Heterotermes spp, Coptotermes spp.

They have applications in the protection of stored products such as grains, fruits, nuts, spices and tobacco, whether whole, milled or compounded into products, from moth, beetle, mite and grain weevil (Sitophilus granarius) attack. Also protected are stored animal products such as skins, hair, wool and leathers in natural or converted form (e.g. as carpets or textiles) from moth and beetle attack; also stored meat and fish from beetle, mite and fly attack.

The compounds of general formula (I) may be of particular value in the control of arthropods, helminths or protozoa which are injurious to, or spread or act as vectors of diseases in man and domestic animal, for example those hereinbefore mentioned, and more especially in the control of ticks, mites, lice, fleas, midges and biting, nuisance and myiasis flies. The compounds of general formula (I) are particularly useful in controlling arthropods, helminths or protozoa which are present inside domestic host animals or which feed in or on the skin or suck the blood of the animal, for which purpose they may be administered orally, parenterally, percutaneously or topically. Coccidiosis, a disease caused by infections by protozoan parasites of the genus Eimeria, is an important potential cause of economic loss in domestic animals and birds, particularly those raised or kept under intensive conditions. For example, cattle, sheep, pigs and rabbits may be affected, but the disease is especially important in poultry, in particular chickens.

The poultry disease is generally spread by the birds picking up the infectious organism in droppings on contaminated litter or ground or by way of food or drinking water. The disease is manifested by hemorrhage, accumulation of blood in the ceca, passage of blood to the droppings, weakness and digestive disturbances. The disease often terminates in the death of the animal, but the fowl which survive severe infections have had their market value substantially reduced as a result of the infection.

Administration of a small amount of a compound of general formula (I) preferably by combination with poultry feed may be effective in preventing or greatly reducing the incidence of coccidiosis. The compounds are effective against both the cecal form (caused by E. tenella) and the intestinal forms (principally caused by E. acervulina, E. brunetti, E. maxima and E. necatrix.

The compounds of general formula (I) may also exert an inhibitory effect on the oocysts by greatly reducing the number and/or the sporulation of those produced.

The compositions hereinafter described for topical application to man and animals and in the protection of stored products, household goods, property and areas of the general environment may, in general, alternatively be employed for application to growing crops and crop growing loci and as a seed dressing. Suitable means of applying the compounds of general formula (I) include:
to persons or animals infested by or exposed to infestation by arthropods, helminths or protozoa, by parenteral, oral or topical application of compositions in which the active ingredient exhibits an immediate and/or prolonged action over a period of time against the arthropods, helminths or protozoa, for example by incorporation in feed or suitable orally-ingestible pharmaceutical formulations, edible baits, salt licks, dietary supplements, pour-on formulations, sprays, baths, dips, showers, jets, dusts, geases, shampoos, creams, wax smears and livestock self-treatment systems; to the environment in general or to specific locations where pests may lurk, including stored products, timber, household goods, and domestic and industrial premises, as sprays, fogs, dusts, smokes, wax-smears, lacquers, granules and baits, and in tricklefeeds to waterways, wells, reservoirs and other running or standing water; to domestic animals in feed to control fly larvae feeding in their faeces; to growing crops as foliar sprays, dusts, granules, fogs and foams; also as suspensions of finely divided and encapsulated compounds of general formula (I) as soil and root treatments by liquid drenches, dusts, granules, smokes and foams; and as seed dressings by liquid slurries and dusts.

The compounds of general formula (I) may be applied to control arthropods, helminths or protozoa in compositions of any type known to the art suitable for internal or external administration to vertebrates or application for the control of arthropods in any premises or indoor or outdoor area, containing as active ingredient at least one compound of general formula (I) in association with one or more compatible diluents or adjuvants appropriate for the intended use. All such compositions may be prepared in any manner known to the art.

Compositions suitable for administration to vertebrates or man include preparations suitable for oral, parenteral, percutaneous, e.g. pour-on, or topical administration.

Compositions for oral administration comprise one or more of the compounds of general formula (I) in association with pharmaceutically acceptable carriers or coatings and include, for example, tablets, pills, capsules, pastes, gels, drenches, medicated feeds, medicated drinking water, medicated dietary supplements, slow-release boluses or other slow-release devices intended to be retained within the gastro-intestinal tract. Any of these may incorporate active ingredient contained within microcapsules or coated with acid-labile or alkali-labile or other pharmaceutically acceptable enteric coatings. Feed premixes and concentrates containing compounds of the present invention for use in preparation of medicated diets, drinking water or other materials for consumption by animals may also be used.

Compositions for parenteral administration include solutions, emulsions or suspensions in any suitable pharmaceutically acceptable vehicle and solid or semisolid subcutaneous implants or pellets designed to release active ingredient over a protracted period and may be prepared and made sterile in any appropriate manner known to the art.

Compositions for percutaneous and topical administration include sprays, dusts, baths, dips, showers, jets, greases, shampoos, creams, wax-smears, or pour-on preparations and devices (e.g. ear tags attached externally to animals in such a way as to provide local or systemic arthropod control.

Solid or liquid baits suitable for controlling arthropods comprise one or more compounds of general formula (I) and a carrier or diluent which may include a food substance or some other substance to induce consumption by the arthropod. When used in agriculture in practice, the compounds according to the invention are seldom employed alone. Most frequently these compounds form part of compositions. These compositions, which can be employed as insecticidal agents, contain a compound according to the invention such as described earlier as the active ingredient in combination with the agriculturally acceptable solid or liquid carriers and surface-active agents which are equally agriculturally acceptable. The inert and usual carriers and the usual surface-active agents can, in particular, be employed.These compositions also form part of the invention.

These compositions may also contain all kinds of other ingredients such as, for example, protective colloids, adhesives, thickeners, thixotropic agents, penetrating agents, spray oils (especially for acaridical uses), stabilisers, preservative agents (especially mold preservatives), sequestering agents, or the like, as well as other known active ingredients with pesticidal properties (particularly insecticides, or fungicides) or with properties regulating the growth of plants. More generally, the compounds employed in the invention may be combined with all the solid or liquid additives corresponding to the usual techniques of formulation.

The use doses of the compounds employed in the invention can vary within wide limits, particularly depending on the nature of the pest to be eliminated and the usual degree of infestation of the crops with these pests.

In general, the compositions according to the invention usually contain approximately 0.05 to 95% (by weight) of one or more active ingredients according to the invention, approximately 1 to 95% of one or more solid or liquid carriers and, optionally, approximately 0.1 to 50% of one or more surface-active agents.

In accordance with what has already been stated the compounds employed in the invention are generally combined with carriers and, optionally, surface-active agents.

In the present account, the term "carrier" denotes an organic or inorganic ingredient, natural or synthetic, with which the active ingredient is combined to facilitate its application to the plant, to seeds or to the soil. This carrier is therefore generally inert and it must be agriculturally acceptable, particularly to the treated plant. The carrier may be solid (clays, natural or synthetic silicates, silica, resins, waxes, solid fertilisers, for example ammonium salts and ground natural minerals, such as kaolins, clays, talc, chalk, quartz, attapulgite, montmorrillonite, bentonite or diatomaceous earth, and ground synthetic minerals, such as silica, alumina, silicates especially aluminium or magnesium silicates. As solid carriers for granules there are suitable: for example crushed and fractionated natural rocks such as calcite, marble, pumice, sepiolite and dolomite, as well as synthetic granules of inorganic and organic meals, and granules of organic material such as sawdust, coconut shells, corn cobs and tobacco stalks; kieselguhr, corn husks, tricalcium phosphate, powdered cork, absorbent carbon black and water soluble polymers, resins, waxes, solid fertilizers, and such solid compositions may, if desired, contain one or more compatible wetting, dispersing, emulsifying or colouring agents which, when solid, may also serve as diluent. The carrier may be also liquid: alcohols, particularly butanol or glycol, as well as their ethers or esters, particularly methylglycol acetate; ketones, particularly acetone, cyclohexanone, methylethyl ketone, methylisobutylketone, and isophorone; petroleum fractions; paraffinic or aromatic hydrocarbons, particularly xylenes or alkyl naphtalenes, petroleum fractions, mineral and vegetable oils; aliphatic chlorinated hydrocarbons, particularly trichloroethane or methylene chloride, or aromatic chlorinated hydrocarbons, particularly chlorobenzenes; water-soluble or strongly polar solvents such as dimethylformamide, dimethyl sulphoxide, or N-methylpyrrolidone as well as water; liquefied gases, and the like, and mixture thereof.

The surface-active agent may be an emulsifying agent, dispersing agent or wetting agent of the ionic or non-ionic type or a mixture of such surface-active agents. There may be mentioned, e.g., salts of polyacrylic acids, salts of lignosulphonic acids, salts of phenolsulphonic or naphthalenesulphonic acids, polycondensates of ethylene oxide with fatty alcohols or fatty acids or fatty esters or fatty amines, substituted phenols (particularly alkylphenols or arylphenols), salts of sulphosuccinic acid esters, taurine derivatives (particularly alkyltaurates), phosphoric esters of alcohols or of polycondensates of ethylene oxide with phenols, esters of fatty acids with polyols, and sulphate, sulphonate and phosphate functional derivatives of the above compounds. The presence of at least one surface-active agent is generally essential when the active ingredient and/or the inert carrier are only slightly water soluble or are not water soluble and the carrier agent of the application is water.

Compositions of the invention may contain further different additives such as adhesives and colorants. Adhesives such as carboxymethylcellulose and natural and synthetic polymers in the form of powders, granules or latices, such as gum arabic, polyvinyl alcohol and polyvinyl acetate, as well as natural phospholipids, such as cephalins and lecithins, and synthetic phospholipids, can be used in the formulations. Further additives can be mineral and vegetable oils. It is possible to use colorants such as inorganic pigments, for example iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

Compositions containing compounds of general formula (I) which may be applied to control arthropod, plant nematode, helminth or protozoan pests, may also contain synergists (e.g. piperonyl butoxide or sesamex), stabilizing substances, other insecticides, acaricides, plant nematocides, anthelmintics or anticoccidials, fungicides (agricultural or veterinary as apropriate e.g. benomyl, iprodione), bactericides, arthropod or vertebrate attractants or repellants or pheromones, reodorants, flavouring agents, dyes and auxiliary therapeutic agents, e.g. trace elements. These may be designed to improve potency, persistence, safety, uptake where desired, spectrum of pests controlled or to enable the composition to perform other useful functions in the same animal or area treated.

Examples of other pesticidally-active compounds which may be included in, or used in conjunction with, the compositions of the present invention are: acephate, chlorpyrifos, demeton-S-methyl, disulfoton, ethoprofos, fenitrothion, malathion, monocrotophos, parathion, phosalone, pirimiphos-methyl, triazophos, cyfluthrin, cypermethrin, deltamethrin, fenpropathrin, fenvalerate, permethrin, aldicarb, carbosulfan, methomyl, oxamyl, pirimicarb, bendiocarb, teflubenzuron, dicofol, endosulfan, lindane, benzoximate, cartap, cyhexatin, tetradifon, avermectins, ivermectin, milbemycins, thiophanate, trichlorfon, dichlorvos, diaveridine and dimetriadazole.

For their agricultural application, the compounds of the formula (I) are therefore generally in the form of compositions, which are in various solid or liquid forms. Liquid compositions, may be used to treat substrates or sites infested or liable to infestation by arthropods including premises, outdoor or indoor storage or processing areas, containers or equipment and standing or running water.

Solid homogeneous or heterogeneous compositions containing one or more compounds of general formula (I) for example granules, pellets, briquettes or capsules, may be used to treat standing or running water over a period of time. A similar effect may be achieved using trickle or intermittent feeds of water dispersible concentrates as described herein.

Compositions in the form of aerosols and aqueous or non-aqueous solutions or dispersions suitable for spraying, fogging and low-or ultra-low volume spraying may also be used.

Solid forms of compositions which can be mentioned are dusting powders (with a content of the compound of formula (I) capable of ranging up to 80%) or wettable powders or granules, particularly those obtained by extrusion, compacting, impregnation of a granular carrier, or granulation starting from a powder (the content of the compound of the formula (I) in these wettable powders or granules being between 0.5 and 80%).

Solutions, in particular emulsifiable concentrates, emulsions, flowables, aerosols, wettable powders (or powder for spraying), dry flowables and pastes, can be mentioned as forms of compositions which are liquid or intended to form liquid compositions when applied.

The emulsifiable or soluble concentrations also comprise most frequently 5 to 80% of active ingredient, while the emulsions or solutions which are ready for application contain, in their case, 0.01 to 20% of active ingredient. Besides the solvent, the emulsifiable concentrates may contain, when required, 2 to 50% of suitable additives, such as stabilisers, surface-active agents, penetrating agents, corrosion inhibitors, colorants or adhesives.

Emulsions of any required concentration, which are particularly suitable for application to plants, may be obtained from these concentrates by dilution with water.

The concentrated suspensions, which can be applied by spraying, are prepared so as to produce a stable fluid product which does not settle (fine grinding) and usually contain from 10 to 75% of active ingredient, from 0.5 to 30% of surface-active agents, from 0.1 to 10% of thixotropic agents, from 0 to 30% of suitable additives such as anti-foaming agents, corrosion inhibitors, stabilisers, penetrating agents, adhesives and, as the carrier, water or an organic liquid in which the active ingredient is poorly soluble or insoluble; some organic solids or inorganic salts may be dissolved in the carrier to help prevent settling or as antifreezes for water.

The wettable powders (or powder for spraying) are usually prepared so that they contain 10 to 80% of active ingredient, and they usually contain, in addition to the solid carrier, from 0 to 5% of a wetting agent, from 3 to 10% of a dispersing agent and, when necessary, from 0 to 80% of one or more stabilisers and/or other additives such as penetrating agents, adhesives, or anti-caking agents, colorants, or the like.

To obtain these wettable powders, the active ingredient or ingredients is, or are, thoroughly mixed in suitable blenders with additional substances which may be impregnated on the porous filler and is, or are, ground using mills or other suitable grinders. This produces wettable powders, the wettability and the suspendability of which are advantageous; they may be suspended in water to give any desired concentration and this suspension can be employed very advantageously in particular for application to plant foliage.

The "water dispersible granules (WG)" (granules which are readily dispersible in water) have a composition which is substantially close to that of the wettable powders. They may be prepared by granulation of formulations described for the wettable powders, either by a wet route (contacting finely divided active ingredient with the inert filler and a little water, e.g. 1 to 20%, or with an aqueous solution of dispersing agent or binder, followed by drying and screening), or by a dry route (compacting followed by grinding and screening).

As already stated, the aqueous dispersions and emulsions, e.g. compositions obtained by diluting with water a wettable powder or an emulsifiable concentrate according to the invention, are included in the general scope of the compositions which may be employed in the present invention. The emulsions may be of the water-in-oil or oil-in-water type and they may have a thick consistency.

All these aqueous dispersions or emulsions or spraying mixtures can be applied to the crops, by any suitable means, chiefly by spraying, at the rates which are generally of the order of 100 to 1,200 liters of spraying mixture per hectare.

The products and compositions according to the invention are conveniently applied to vegetation and in particular to roots or leaves having pests to be eliminated.

Another method of application of the compounds or compositions according to the invention is by chemigation, that is to say the addition of a formulation containing the active ingredient to irrigation water. This irrigation may be sprinkler irrigation for foliar pesticides or it can be ground irrigation or underground irrigation for systemic pesticides. The application dose of active ingredient is generally between 0.1 and 10 kg/ha, preferably between 0.5 and 4 kg/ha. More particularly the rates and concentrations may vary according to the method of application and the nature of the used compositions.

Generally speaking, the compositions for application to control arthropod, plant nematode, helminth or protozoan pests usually contain from 0.00001% to 95%, more particularly from 0.0005% to 50%, by weight of one or more compounds of general formula (I) or of total active ingredients (that is to say the compound(s) of general formula (I) together with other substances toxic to arthropods and plant nematodes, anthelmintics, anticoccidials, synergists, trace elements or stabilisers). The actual compositions employed and their rate of application will be selected to achieve the desired effect(s) by the farmer, livestock producer, medical or veterinary practitioner, pest control operator or other person skilled in the art. Solid and liquid compositions for application topically to animals, timber, stored products or household goods usually contain from 0.00005% to 90%, more particularly from 0.001% to 10%, by weight of one or more compounds of general formula (I). For administration to animals orally or parenterally, including percutaneously solid and liquid compositions normally contain from 0.1% to 90% by weight of one or more compound of general formula (I). Medicated feedstuffs normally contain from 0.001% to 3% by weight of one or more compounds of general formula (I). Concentrates and supplements for mixing with feedstuffs normally contain from 5% to 90% and preferably from 5% to 50%, by weight of one or more compounds of general formula (I). Mineral salt licks normally contain from 0.1% to 10% by weight of one or more compounds of general formula (I).

Dusts and liquid compositions for application to livestock, persons, goods, premises or outdoor areas may contain 0.0001% to 15%, and more especially 0.005% to 2.0%, by weight of one or more compounds of general formula (I). Suitable concentrations in treated waters are between 0.0001 ppm and 20 ppm, and more especially 0.001 ppm to 5.0 ppm. of one or more compounds of general formula (I) and may also be used therapeutically in fish farming with appropriate exposure times. Edible baits may contain from 0.01% to 5% and preferably 0.01% to 1.0% by weight of one or more compounds of general formula (I).

When administered to vertebrates parenterally, orally or by percutaneous or other means, the dosage of compounds of general formula (I) will depend upon the species, age and health of the vertebrate and upon the nature and degree of its actual or potential infestation by arthropod, helminth or protozoan pest. A single dose of 0.1 to 100 mg, preferably 2.0 to 20.0 mg, per kg body weight of the animal or doses of 0.01 to 20.0 mg, preferably 0.1 to 5.0 mg, per kg of body weight of the animal per day for sustained medication are generally suitable by oral or parenteral administration. By use of sustained release formulations or devices, the daily doses required over a period of months may be combined and administered to animals on a single occasion.

The following specific examples illustrate the agrochemical compositions containing the compounds of the invention, and thereafter the insecticidal and acaricidal applications and properties of some compounds.

### COMPOSITION (FORMULATION) USE EXAMPLES

The following composition **EXAMPLES 23 TO 28** illustrate compositions for use against arthropods, especially insects and arachnids, plant nematodes, and helminth or protozoan pests which comprise, as active ingredient, compounds of general formula (I), especially compounds such as those described in the preparative **EXAMPLES 1 to 22 and in Tables 3 and 4**. The compositions described in composition **EXAMPLES 23 to 28** can each be diluted in water to give a sprayable composition at concentrations suitable for use in the field. Generic chemical descriptions of the ingredients (for which all of the following percentages are in weight percent), used in the composition **EXAMPLES 23 TO 28** exemplified below, are as follows:
- Ethylan BCP:: nonylphenol ethylene oxide condensate
- Soprophor BSU:: condensate of tristyrylphenol and ethylene oxide
- Arylan CA:: 70% w/v solution of calcium dodecylbenzenesulphonate
- Solvesso 150: light C10-aromatic solvent
- Arylan S:: sodium dodecylbenesulphonate
- Darvan: sodium lignosulphonate
- Celite PF: synthetic magnesium silicate carrier
- Sopropon T36: sodium salt of polycarboxylic acid
- Rhodigel 23: polysaccharide xanthan gum
- Bentone 38:: organic derivative of magnesium montmorillonite
- Aerosil: silicon dioxide of microfine particle size

### EXAMPLE 23

A water soluble concentrate is prepared from:

| | |
|---|---|
| Active ingredient | 7% |
| ETHYLAN BCP | 10% |
| N-methylpyrrolidone | 83% |

by dissolving the ETHYLAN BCP in a portion of N-methylpyrrolidone, and then adding the active ingredient with heating and stirring until dissolved. The resulting solution is made up to volume by adding the remainder of the solvent.

### EXAMPLE 24

An emulsifiable concentrate is prepared from:

| | |
|---|---|
| Active ingredient | 7% |
| Soprophor BSU | 4% |
| Arylan CA | 4% |
| N-methylpyrrolidone | 50% |
| Solvesso 150 | 35% |

by dissolving Soprophor BSU, Arylan CA and the active ingredient in N-methylpyrrolidone, and then adding Solvesso 150 to volume.

### EXAMPLE 25

A wettable powder is prepared from:

| | |
|---|---|
| Active ingredient | 40% |
| ARYLAN S | 2% |
| Darvan No.2 | 5% |
| Celite PF | 53% |

by mixing the ingredients, and grinding the mixture in a hammer-mill to a particle size less than 50 microns.

### EXAMPLE 26

An aqueous flowable formulation is prepared from:

| | |
|---|---|
| Active ingredient | 40.00% |
| Ethylan BCP | 1.00% |
| Sopropon T36 | 0.20% |
| Ethylene glycol | 5.00% |
| Rhodigel 23 | 0.15% |
| Water | 53.65% |

by intimately mixing the ingredients and grinding in a bead mill until the median particle size is less than 3 microns.

### EXAMPLE 27

An emulsifiable suspension concentrate is prepared from:

| | |
|---|---|
| Active ingredient | 30.0% |
| Ethylan BCP | 10.0% |
| Bentone 38 | 0.5% |
| Solvesso 150 | 59.5% |

by intimately mixing the ingredients and grinding in a bead mill until the median particle size is less than 3 microns.

### EXAMPLE 28

Water dispersible granules are prepared from:

| | |
|---|---|
| Active ingredient | 30% |
| Darvan No.2 | 15% |
| Arylan S | 8% |
| Celite PF | 47% |

by mixing the ingredients, micronising in a fluid-energy mill, and then granulating in a rotating pelletiser by spraying on sufficient water (up to 10%w/v). The resulting granules are dried in a fluid-bed drier to remove excess water.

### EXAMPLE 29

A dusting powder may be prepared by intimately mixing:

| | |
|---|---|
| Active ingredient | 1 to 10% |
| Talc superfine | 99 to 90% |

This powder may be applied to a locus of arthropod infestation, for example refuse tips or dumps, stored products or household goods or animals infested by, or at risk of infestation by, arthropods to control the arthropods by oral ingestion. Suitable means for distributing the dusting powder to the locus of arthropod infestation include mechanical blowers, handshakers and livestock self treatment devices.

### EXAMPLE 30

An edible bait may be prepared by intimately mixing:

| | |
|---|---|
| Active ingredient | 0.1 to 1.0% |
| Wheat flour | 80.0% |
| Molasses | 19.9 to 19.0% |

This edible bait may be distributed at a locus, for example domestic and industrial premises, e.g. kitchens hospitals or stores, or outdoor areas, infested by arthropods, for example ants, locusts, cockroaches and flies, to control arthropods by oral ingestion.

### EXAMPLE 31

A solution may be prepared containing:

| | |
|---|---|
| Active ingredient | 15% |
| Dimethylsulphoxide | 85% |

by dissolving the pyrrole derivative in a portion of the dimethylsulphoxide and then adding more dimethylsulphoxide to the desired volume. This solution may be applied to domestic animals infested by arthropods, percutaneously as a pour-on application or, after sterilisation by filtration through a polytetrafluorothylene membrane (O.22 micrometre pore size), by parenteral injection, at a rate of application of from 1.2 to 12 ml of solution per 100 kg of animal body weight.

### EXAMPLE 32

A wettable powder may be formed from:

| | |
|---|---|
| Active ingredient | 50% |
| Ethylan BCP (9 moles of oxide per mole of phenol) | 5% |
| Aerosil | 5% |
| Celite PF | 40% |

by adsorbing the Ethylan BCP onto the Aerosil, mixing with the other ingredients and grinding the mixture in a hammer-mill to give a wettable powder, which may be diluted with water to a concentration of from 0.001% to 2% w/v of the active compound and applied to a locus of infestation by arthropods, for example dipterous larvae, or plant nematodes by spraying , or to domestic animals infested by, or at risk of infection by, arthropods, helminths or protozoa, by spraying or dipping, or by oral administration in drinking water, to control the arthropods, helminths or protozoa.

### EXAMPLE 33

A slow release bolus may be formed from granules containing a density agent , binder, slow-release agent, and the active ingredient made according to **EXAMPLE 27** at varying percentage compositions. By compressing the mixture, a bolus with a specific gravity of 2 or more can be formed and may be administered orally to ruminant domestic animals for retention within the reticulo-rumen to give a continual slow release of pyrrole compound over an extended period of time to control infestation of the ruminant domestic animals by arthropods, helminths or protozoa.

### EXAMPLE 34

A slow release composition may be prepared from:

| | |
|---|---|
| Active ingredient | 0.5 to 25% |
| Polyvinylchloride base | 75 to 99.5% |

by blending the polyvinylchloride base with the active compound and a suitable plasticiser, e.g. dioctyl phthalate, and melt-extruding or-moulding the homogenous composition into suitable shapes, e.g. granules, pellets, brickettes or strips, suitable, for example, for addition to standing water or, in the case of strips, fabrication into collars or ear-tags for attachment to domestic animals, to control insect pests by slow release of the active compound.

Similar compositions may be prepared by replacing the active ingredient in the composition examples by the approprate quantity of any other compound of general formula (I).

### PESTICIDAL METHODS OF USE EXAMPLES

In the following use **EXAMPLES 35 TO 47,** compounds according to the invention are applied at various concentrations. The use of a 1 ppm (concentration of the compound in parts per million of the test solution applied) foliar solution or suspension or emulsion corresponds approximately to an application of 1 g/ha of active ingredient, based upon an approximate spray volume of 1000 liters/ha (sufficient to run off). Thus in the following applications foliar sprays of from about 6.25 to 500 ppm would correspond to about 6-500 g/ha. For soil applications, a 1 ppm soil concentration, on the basis of about a 7.5 cm soil depth, corresponds to an approximate 1000 g/ha broadcast field application.

### EXAMPLE 35

### Activity on Aphid:

A mixture was made with:
- 0.01 g of active ingredient
- 0.16 g of dimethylformamide
- 0.838 g of acetone
- 0.002 g of a surfactant blend comprising both an alkyl aryl-polyether alcohol and an alkylaryl polyether alcohol having sulfonic groups on the aryl moiety.
- 98.99 g of water

This diluted aqueous mixture was sprayed on potted dwarf nasturtium plants, whereon adults and nymphal stages of the buckthorn aphid (Aphis nasturtii) were reared The number of aphids per pot was 100-150. The volume of sprayed aqueous mixture was sufficient to wet the plants to runoff. After spraying, the pots were stored at 20°C for one day, whereafter the alive aphids were rated. The percentage of mortality given was 100% for compounds of **EXAMPLES 1, 2, 3A, 4, 5, 16C, 17, 18, 19, and 20** and **ASE No's 12, 24, 33, 34, 38, 39, 42, 44, 45, 54, 57, 60, 62, 98-100, 102-104, 125, 128, 130, 131, 135, 137, 141, 142, 144, 157, 158, 162, 165, 166, and 174** at a concentration of 100 ppm.

### EXAMPLE 36

### Activity on Mite:

The same formulation procedure as in **EXAMPLE 35** was used. However, in this case 150-200 two-spotted mites (Tetranychus urticae) were reared on tendergreen beans. After spraying, the plants were kept at 30°C for 5 days. The percentage of mortality of mites was 100% for the compounds of **EXAMPLE 2, 3A, 16C, 17 and 18** and **ASE No's 9, 20, 25, 41, 44, 46, 52, 53, 58, 59, 63, 64, 70, 74, 77-81, 83, 90, 98, 99, 102, 124, and 141** at a concentration of 100 ppm.

### EXAMPLES 37-39

### ACTIVITY ON SOUTHERN ARMYWORM:

**37:** The same formulation as in **EXAMPLE 35** was used. In this case, second instar larvae of southern armyworm (Spodoptera eridania) were reared on Sieva beans of approximately 15 cm in height. The following percent of mortalities were obtained after 5 days: 100% mortality was provided by compounds of **EXAMPLES 3A, 3B, 5, 6, 7, 8, 9, 11, 12, 15B 16C, 17, 18, 20, 21B, 21C,** and **ASE No's 42, 44, 60, 62, 64, 98-100, 102, 103, 121, 124, 125, 131, 141, 142, 144, 162, 166, and 174** at a concentration of 100 ppm and 80% mortality was provided by the compound of **EXAMPLE 13** at 500 ppm.

**38**: The same formulation procedure as in **EXAMPLE 35** was used except in this case it contained the following:
- 2.5 mg of active ingredient
- 0.05 g of dimethyl formamide
- 9.9228 g of acetone
- 0.0247 g of surfactant (as in **EXAMPLE 35**)
- 90 g of water

The compound of **EXAMPLE 4** gave 100% mortality on southern armyworm at 25 ppm.

**39**: The same formulation procedure as in **EXAMPLE 38** was used except in this case it contained the following:
- 0.625 mg of active ingredient
- 12.5 mg of dimethylformamide
- 9.9621 g of acetone
- 0.0247 g of surfactant (as in **EXAMPLE 35**)
- 90 g of water

The compounds of **EXAMPLES 1 and 2** gave 100% mortality on southern armyworm at 6.25 ppm.

### EXAMPLES 40-43

### Activity on Mexican Bean Beetle

**40**: The same formulation procedure as in **EXAMPLE 37** was used except in this case it contained the following:
- 12.5 mg of active ingredient
- 0.25 g of dimethylformamide
- 9.726 g of acetone
- 24.1 mg of surfactant (as in **EXAMPLE 35**)
- 89.988 g of water

Second instar larvae of the mexican bean beetle (Epilachna varivestis, muls) were reared on Sieva beans of approximately 15 cm in height. The following percent mortality was obtained after 5 days: 100% mortality was obtained by the compound of **EXAMPLE 13** at 125 ppm.

**41**: Using the formulation procedure as in **EXAMPLE 38**, but containing the compound of **EXAMPLE 8** as active ingredient there was obtained 100% mortality of Mexican bean beetle at 25 ppm.

**42**: Using the formulation procedure as in **EXAMPLE 35**, but containing the unbrominated compound of **EXAMPLE 15A** as active ingredient there was obtained 100% mortality of Mexican bean beetle at 100 ppm.

**43**: The same formulation procedure as in **EXAMPLE 40** was used except in this case it contained:
- 10 mg of active ingredient
- 0.2 g of dimethylformamide
- 9.7657 g of acetone
- 0.0243 g of surfactant (as in **EXAMPLE 35**)
- 90 g of water

The following percent mortalities on Mexican bean beetle were obtained: 80% mortality by the compounds of **EXAMPLE 15A and 15B** at 100 ppm and 100% mortality by the compounds of **EXAMPLES 1, 2, 9, 17, 18** and **ASE No's 42, 44, 60, 62, 64, 98, 99, 124, 125, 141, 142, and 144** at 100 ppm.

### EXAMPLES 44-46

### Activity on Housefly

The toxicant, in the form of a 10 ml aqueous sugar solution containing 10% w/w of sugar and 100 ppm of the chemical toxicant, was formulated in a similar way as in **EXAMPLE 35**. Further serial dilutions were made as required. The following 3 different formulations were prepared for testing:

| | **EXAMPLE** | | |
|---|---|---|---|
| | **44** | **45** | **46** |
| Active ingredient, mg | 10 | 10 | 1.25 |
| Dimethylformamide, mg | 160 | 200 | 25 |
| Surfactant (as in **EXAMPLE 35**), mg. | 2.15 | 24.3 | 14.25 |
| Acetone, g | 8.42 | 9.766 | 5.73 |
| Water, g | 88.99 | 81 | 84.38 |
| Sugar, g | 10 | 9 | 9.84 |

Twenty five adult flies (Musca domestica) were anesthetized with carbon dioxide and then transferred over to a bait cup containing the toxicant formulation. After one day at 27°C, the percent mortality of flies was measured and was as follows:
- For **EXAMPLE 44**:: 100% mortality by the compounds of **EXAMPLES 1, 2, 3B, 4-6, 8, 9, 16C, 17-20, 21B, and 21C** and **ASE No's 42, 44, 60, 62, 64, 98, 99, 100, 102, 103, 121, 124, 125, 131, 141, 142, 144, 162, 166, and 174** at 100 ppm.
- For **EXAMPLE 45**:: 100% mortality by the compound of **EXAMPLE 12** at 100 ppm.
- For **EXAMPLE 46**:: 100% mortality by the compounds of **EXAMPLES 1, 2 and 5** at 12.5 ppm.

### EXAMPLE 47

### ACTIVITY ON SOUTHERN CORN ROOTWORM:

A formulation was prepared in a similar manner to that used in **EXAMPLE 35** except that in this case, only 48.99g of water was used, providing an initial 200 ppm concentration of the test compound. Aliquots of this formulation were then used directly according to the required test concentration, in ppm (parts per million) by weight, according to the following test procedure.

Into a jar containing 60 g of sandy loam soil was added an aliquot of the 200 ppm test compound formulation (as appropriate for the final soil concentration of the test compound), 3.2 ml of water and five pregerminated corn seedlings. The jar was shaken thoroughly to obtain an even distribution of the test formulation. Following this, twenty southern corn rootworm eggs were placed into a cavity, which was made in the soil. Vermiculite (1 ml) and water (1.7 ml) were then added to this cavity. In a similar manner, an untreated control was prepared by application of the same size aliquot of a water-acetone-DMF emulsifier solution, containing no test compound. Additionally, a treated control with a commercial technical compound, formulated in the same manner, was used as a test standard. After 7 days, the living rootworm larvae were counted using a well known "Berlese" funnel extraction method.

The following compounds all provide 100% control at soil concentrations of 1.45, 0.72 and 0.36 ppm: compounds of **EXAMPLES 3B, 4, and 17-19** and **ASE No's 98, 99, 101, 105, 113, 119, 121, 124, 125, 130 and 173**.

## Claims (Claims for the following Contracting State(s): AT, BE, CH, LI, DE, FR, GB, GR, IT, LU, NL, SE)

1. A compound of formula (I) 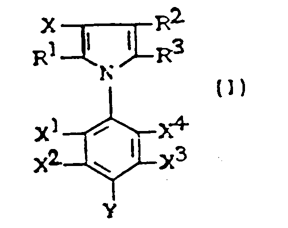 wherein
X is selected from the group consisting of: halogen, cyano, haloalkyl, alkoxy, haloalkoxy, alkylthio, alkylsulfinyl, alkylsulfonyl, haloalkylthio, haloalkylsulfinyl and haloalkylsulfonyl, wherein the alkyl, haloalkyl, alkoxy and haloalkoxy groups herein are linear or branched chains, having less than 10 carbon atoms, and the halosubstitution in all these groups consists of one or more halogen atoms, which are the same or different, from mono-substitution up to complete poly-substitution;
R¹, R² and R³ are individually selected from the group consisting of: one of the same set of substituents as described for X; a hydrogen atom; an alkyl group; and a substituent of which no more than one of R¹, R² and R³ is selected from the group consisting of formyl, amino, alkylamino and dialkylamino, wherein the alkyl groups herein are linear or branched chains, having less than 10 carbon atoms;
Y is selected from the group consisting of: halogen, cyano, alkyl, haloalkyl, alkoxy and haloalkoxy, wherein the alkyl, alkoxy, haloalkyl and haloalkoxy groups herein are linear or branched chains, having less than 10 carbon atoms, and the halo substitution in all these groups herein consists of one or more halogen atoms, which are the same or different, from mono-substitution up to as much as complete poly-substitution;
or Y is a hydrogen atom when:
X is a halogen atom or a group R⁵S(O)n, in which n is 0, 1 or 2 and R⁵ is alkyl or haloalkyl, and the alkyl carbon chains and the halo-substitution are as defined above;
R¹ and R³ are each a hydrogen atom; and
R² is cyano;
X¹, X², X³ and X⁴ are individually selected from the same set of substituents as described for Y or a hydrogen atom, with the following provisos: at least one of R¹, R² and R³ is selected from the same set of substituents as described for X;
if X⁴ and X¹ are H, and X is halogen or cyano then R² is different from X, and
if X⁴ and X¹ are H and Y is methyl, then X is different from bromo;
with the exclusion of the compound wherein X⁴ = X¹ = Y = R¹ = X = R² = R³ = Cl and at the same time X² = X³ = H.

2. The compound of formula (I), according to claim 1, wherein:
X is selected from a halogen atom, or a group selected from cyano, haloalkyl, alkoxy, haloalkoxy, alkylthio, alkylsulfinyl, alkylsulfonyl, haloalkylthio, haloalkylsulfinyl and haloalkylsulfonyl, wherein the alkyl, haloalkyl, alkoxy and haloalkoxy groups herein are linear or branched chains, having less than 10 carbon atoms, and the halosubstitution in all these groups is mono-substitution or up to as much as complete polysubstitution;
R¹, R² and R³ are individually selected from: the same substituents as described for X; a hydrogen atom; an alkyl group; and a substituent of which no more than one of R¹, R² and R³ is selected from the group consisting of formyl, amino, alkylamino and dialkylamino, wherein the alkyl groups herein are linear or branched chains, having less than 10 carbon atoms;
Y is selected from a halogen atom or a group selected from cyano, alkyl, haloalkyl, alkoxy and haloalkoxy, wherein the alkyl, alkoxy, haloalkyl and haloalkoxy groups herein are linear or branched chains, having less than 10 carbon atoms, and the halosubstitution in all these groups herein is mono-substitution or up to as much as complete poly-substitution; and
X¹, X², X³ and X⁴ are as described in claim 1.

3. The compound of formula (I), according to claim 2, wherein:
X is a halogen atom or a group R⁵S(O)n, in which n is 0, 1 or 2 and R⁵ is alkyl or haloalkyl;
R¹ is a hydrogen atom, a halogen atom or alkylthio;
R² is cyano;
R³ is a hydrogen atom or a halogen atom;
Y is a hydrogen atom, a halogen atom, haloalkyl or haloalkoxy, with the proviso that Y is a hydrogen atom as defined in claim 1; and
X¹, X², X³ and X⁴ are individually selected from the group consisting of a hydrogen atom, a halogen atom, C₁₋₃ alkyl, C₁₋₃ alkoxy, and C₁₋₃ alkylthio.

4. The compound according to claim 3, having the formula (II) 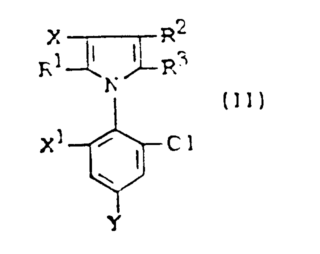 wherein:
X is R⁵S(o)n, in which n is 0, 1 or 2 and R⁵ is CH_{3,} CF₃, CF₂Cl, CFCl₂, CF₂Br, CHF₂, CHCl₂ or CHClF;
R¹ is H, F, Cl or Br;
R³ is H, F, Cl or Br;
X¹ is H or Cl; and
Y is CF₃ or CF₃O.

5. The compound of formula (I) according to claim 1, 2 or 3 wherein:
R¹ is a hydrogen atom or a halogen atom;
R² is cyano;
X is a haloalkyl-S(O)ₙ group, wherein n is 0, 1 or 2;
X¹ and X⁴ are other than a hydrogen atom;
Y is a haloalkyl or haloalkoxy group; and
X² and X³ are a hydrogen atom.

6. The compound according to claim 2, having the formula (II) 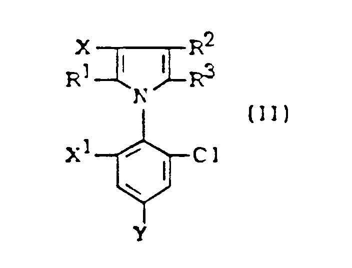 wherein:
X is R⁵S(O)ₙ, in which n is 0, 1 or 2 and R⁵ is CH₃, CF₃, CF₂Cl or CFCl₂;
R² is cyano;
R¹ is H, F, Cl, Br or NH₂;
R³ is H, F, Cl, Br, CF₃ or CN;
X¹ is H or Cl; and
Y is CF₃ or CF₃O.

7. The compound according to claim 1, which is:
1-(2,6-dichloro-4-trifluoromethylphenyl)-2-chloro-3-cyano-4-(trifluoromethylthio)pyrrole;
1-(2,6-dichloro-4-trifluoromethoxyphenyl)-2-chloro-3-cyano-4-(trifluoromethylsulfonyl)pyrrole;
1-(2,6-dichloro-4-trifluoromethylphenyl)-2-chloro-3-cyano-4-(dichlorofluoromethylthio)pyrrole;
1-(2,6-dichloro-4-trifluoromethylphenyl)-2-chloro-3-cyano-4-(dichlorofluoromethylsulfinyl)pyrrole;
1-(2,6-dichloro-4-trifluoromethylphenyl)-2-chloro-3-cyano-4-(dichlorofluoromethylsulfonyl)pyrrole;
1-(2,6-dichloro-4-trifluoromethylphenyl)-2-chloro-3-cyano-4-(chlorodifluoromethylsulfonyl)pyrrole;
1-(2,6-dichloro-4-trifluoromethylphenyl)-2-chloro-3-cyano-4-(chlorodifluoromethylsulfinyl)pyrrole;
1-(2,6-dichloro-4-trifluoromethylphenyl)-2-chloro-3-cyano-4-(chlorodifluoromethylthio)pyrrole;
1-(2,6-dichloro-4-trifluoromethylphenyl)-2-bromo-3-cyano-4-(trifluoromethylsulfinyl)pyrrole;
1-(2,6-dichloro-4-trifluoromethylphenyl)-2-bromo-3-cyano-4-(trifluoromethylsulfonyl)pyrrole;
1-(2,6-dichloro-4-trifluoromethylphenyl)-2-bromo-3-cyano-4-(dichlorofluoromethylthio)pyrrole;
1-(2,6-dichloro-4-trifluoromethylphenyl)-2-bromo-3-cyano-4-(dichlorofluoromethylsulfonyl)pyrrole;
1-(2,6-dichloro-4-trifluoromethylphenyl)-2-bromo-3-cyano-4-(dichlorofluoromethylsulfinyl)pyrrole;
1-(2,6-dichloro-4-trifluoromethylphenyl)-2-bromo-3-cyano-4-(chlorodifluoromethylthio)pyrrole;
1-(2,6-dichloro-4-trifluoromethylphenyl)-2-bromo-3-cyano-4-(chlorodifluoromethylsulfinyl)pyrrole;
1-(2,6-dichloro-4-trifluoromethylphenyl)-2-chloro-3-cyano-4-(dichlorofluoromethylsulfonyl)-5-bromopyrrole;
1-(2,6-dichloro-4-trifluoromethylphenyl)-2-bromo-3-cyano-4-(chlorodifluoromethylsulfonyl)pyrrole;
1-(2,6-dichloro-4-trifluoromethoxyphenyl)-2-chloro-3-cyano-4-(dichlorofluoromethylsulfinyl)pyrrole;
1-(2,6-dichloro-4-trifluoromethoxyphenyl)-2-chloro-3-cyano-4-(dichlorofluoromethylsulfonyl)pyrrole;
1-(2-chloro-4-trifluoromethylphenyl)-2-chloro-3-cyano-4-(dichlorofluoromethylsulfonyl)pyrrole;
1-(2-chloro-4-trifluoromethylphenyl)-2-chloro-3-cyano-4-(dichlorofluoromethylsulfinyl)pyrrole;
1-(2-chloro-4-trifluoromethylphenyl)-2-bromo-3-cyano-4-(dichlorofluoromethylsulfonyl)pyrrole;
1-(2,6-dichloro-4-trifluoromethylphenyl)-2-chloro-3-cyano-4-(dichlorofluoromethylthio)-5-methylthiopyrrole;
1-(2,6-dichloro-4-trifluoromethylphenyl)-2-chloro-3-cyano-4-(bromodifluoromethylthio)pyrrole;
1-(2,6-dichloro-4-trifluoromethylphenyl)-2-chloro-3-cyano-4-(bromodifluoromethylsulfinyl)pyrrole;
1-(2,6-dichloro-4-trifluoromethylphenyl)-2-chloro-3-cyano-4-(bromodifluoromethylsulfonyl)pyrrole;
1-(2,6-dichloro-4-trifluoromethylphenyl)-2-chloro-3-cyano-4-(methylsulfinyl)pyrrole;
1-(2,6-dichloro-4-trifluoromethylphenyl)-2-chloro-3-cyano-4-(methylsulfonyl)pyrrole;
1-(4-bromo-2,6-dichlorophenyl)-3-cyano-4-(chlorodifluoromethylthio)pyrrole;
1-(4-bromo-2,6-dichlorophenyl)-3-cyano-4-(trifluoromethylthio)pyrrole;
1-(2,4,6-trichlorophenyl)-3-cyano-4-(chlorodifluoromethylthio)pyrrole;
1-(2,4,6-trichlorophenyl)-3-cyano-4-(chlorodifluoromethylsulfinyl)pyrrole;
1-(2,4,6-trichlorophenyl)-3-cyano-4-(dichlorofluoromethylthio)pyrrole;
1-(2,4,6-trichlorophenyl)-3-cyano-4-(dichlorofluoromethylsulfinyl)pyrrole;
1-(2,4,6-trichlorophenyl)-3-cyano-4-(dichlorofluoromethylsulfonyl)pyrrole;
1-(4-bromo-2,6-dichlorophenyl)-3-cyano-4-(dichlorofluoromethylthio)pyrrole;
1-(4-bromo-2,6-dichlorophenyl)-3-cyano-4-(dichlorofluoromethylsulfinyl)pyrrole;
1-(4-bromo-2,6-dichlorophenyl)-3-cyano-4-(dichlorofluoromethylsulfonyl)pyrrole;
1-(2,4,6-trichlorophenyl)-3-cyano-4-(trifluoromethylthio)pyrrole;
1-(2,4,6-trichlorophenyl)-3-cyano-4-(trifluoromethylsulfinyl)pyrrole;
1-(2,4,6-trichlorophenyl)-3-cyano-4-(trifluoromethylsulfonyl)pyrrole;
1-(2,4,6-trichlorophenyl)-3-cyano-4-(trichloromethylthio)pyrrole;
1-(2,4-dichlorophenyl)-3-cyano-4-(dichlorofluoromethylthio)pyrrole;
1-(2,4,6-trichlorophenyl)-3-cyano-4-chloropyrrole;
1-(2,4,6-trichlorophenyl)-3-cyano-4-(chlorodifluoromethylsulfonyl)pyrrole;
1-(2,6-dichlorophenyl)-3-cyano-4-(dichlorofluoromethylthio)pyrrole;
1-(4-bromo-2,6-dichlorophenyl)-3-cyano-4-(trifluoromethylsulfinyl)pyrrole;
1-(4-bromo-2,6-dichlorophenyl)-3-cyano-4-(trifluoromethylsulfonyl)pyrrole;
1-(4-bromo-2,6-dichlorophenyl)-3-cyano-4-(chlorodifluoromethylsulfinyl)pyrrole;
1-(4-bromo-2,6-dichlorophenyl)-3-cyano-4-(chlorodifluoromethylsulfonyl)pyrrole;
1-(4-bromo-2,6-dimethylphenyl)-3-cyano-4-(trifluoromethylsulfinyl)pyrrole;
1-(4-bromo-2,6-dimethylphenyl)-3-cyano-4-trifluoromethylsulfonyl)pyrrole; or
1-(4-bromo-2,6-difluorophenyl)-3-cyano-4-(dichlorofluoromethylthio)pyrrole;
1-(2,6-dichloro-4-trifluoromethylphenyl)-2-chloro-3-cyano-4-(trifluoromethylsulfonyl)pyrrole;
1-(2,6-dichloro-4-trifluoromethylphenyl)-2-chloro-3-cyano-4-(trifluoromethylsulfinyl)pyrrole;
1-(2,6-dichloro-4-trifluoromethylphenyl)-2-chloro-3-cyano-4-trifluoromethylsulfinyl-5-bromopyrrole;
1-(2,6-dichloro-4-trifluoromethylphenyl)-2-chloro-3-cyano-4-trifluoromethylsulfonyl-5-bromopyrrole;
1-(2,6-dichloro-4-trifluoromethylphenyl)-2-chloro-3-cyano-4-(trifluoromethylthio)pyrrole;
1-(2,6-dichloro-4-trifluoromethylphenyl)-2-bromo-3-trifluoromethylthio-4-cyano-5-chloropyrrole;
1-(2,6-dichloro-4-trifluoromethylphenyl)-2-[(trifluoromethyl)carbonylamino]-3-trifluoromethylthio-4- cyano-5-chloropyrrole;
1-(2,6-dichloro-4-trifluoromethylphenyl)-2-(methylcarbonylamino)-3-trifluoromethylthio-4-cyano-5-chloropyrrole;
1-(2,6-dichloro-4-trifluoromethylphenyl)-2-amino-3-trifluoromethylthio-4-cyano-5-chloropyrrole;
1-(2-chloro-4-trifluoromethylphenyl)-2-amino-3-trifluoromethylthio-4-cyano-5-chloropyrrole;
1-(2,6-dichloro-4-trifluoromethylphenyl)-2-amino-3-dichlorofluoromethylthio-4-cyano-5-chloropyrrole;
1-(2,6-dichloro-4-trifluoromethylphenyl)-2,4-bis(trifluoromethylthio)-3-cyano-5-aminopyrrole;
1-(2,6-dichloro-4-trifluoromethylphenyl)-2-amino-3-trifluoromethylthio-4-cyano-5-bromopyrrole;
1-(2,6-dichloro-4-trifluoromethylphenyl)-2-amino-3-trifluoromethylthio-4-cyanopyrrole;
1-(4-trifluoromethylphenyl)-2-amino-3-trifluoromethylthio-4-cyano-5-bromopyrrole;
1-(2-chloro-4-trifluoromethylphenyl)-2-amino-3-trifluoromethylthio-4-cyano-5-bromopyrrole;
1-(2,6-dichloro-4-trifluoromethylphenyl)-2-chloro-3-cyano-4-(dichlorofluoromethylthio)pyrrole;
1-(2,6-dichloro-4-trifluoromethylphenyl)-2-chloro-3-cyano-4-(dichlorofluoromethylsulfinyl)pyrrole;
1-(2,6-dichloro-4-trifluoromethylphenyl)-2-chloro-3-cyano-4-(dichlorofluoromethylsulfonyl)pyrrole;
1-(2,6-dichloro-4-trifluoromethylphenyl)-3-cyano-4-(dichlorofluoromethylthio)pyrrole;
1-(2,6-dichloro-4-trifluoromethylphenyl)-3-cyano-4-(dichlorofluoromethylsulfinyl)pyrrole; or
1-(2,6-dichloro-4-trifluoromethoxyphenyl)-2-chloro-3-cyano-4-(trifluoromethylsulfonyl)pyrrole.

8. A process for the preparation of compounds of formula (I) which comprises,
(A) when the compound is of the formula I(a): 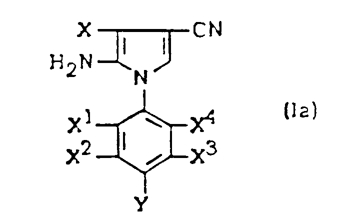 wherein X¹, X², X³, X⁴ and Y have the same meaning as in claim 1 and,
(A-I): X is halogen, trifluoromethyl, cyano, alkylthio, alkylsulfinyl, alkylsulfonyl, haloalkylthio, haloalkylsulfinyl or haloalkylsulfonyl, a process wherein a compound of formula 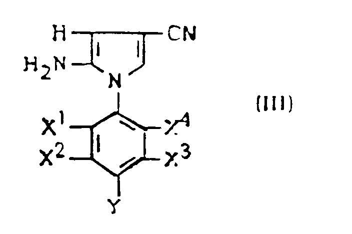 wherein the various symbols are as defined in claim 1 and amino is optionally protected:
(a) is reacted with a halogenating agent, optionally in the presence of a solvent to obtain a compound of formula (Ia) wherein X is halogen, then optionally reacting said compound with trifluoromethyl copper in a known manner to get compounds of formula (Ia) where X is trifluoromethyl;
(b) is reacted with a tris(alkylthio)methane or tris(arylthio)methane in the presence of a Lewis acid, then the obtained compound of formula (XI) 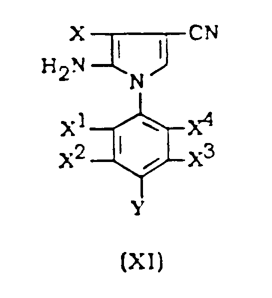 where X is bis(alkylthio)methyl or bis(arylthio)methyl and the other symbols are as defined in claim 1 is optionally reacted with an alkyl nitrite followed by hydrolysis, in order to obtain the compound of formula (XI) where X is formyl, then bringing the said compound into contact with hydroxylamine followed by dehydration in a known manner in order to obtain the compound of formula (Ia) where X is a cyano group;
(c) is reacted with a compound of formula MSCN, M being an alkali metal, in the presence of bromine, in a solvent in order to obtain the compound of formula (Ia) where X is a thiocyanato group and then reacting said compound with an alkyl halide or dialkyl sulfate in the presence of a base in a solvent to get a compound of formula (Ia) where X is alkylthio; or
(d) is reacted with a sulfenyl halide of formula RSHal, in which R is an alkyl, haloalkyl, phenyl or heteroaryl radical and Hal is a halogen atom in an organic liquid reaction medium, optionally in the presence of an acid acceptor in order to get a compound of formula (Ia) where X is alkylthio or haloalkylthio, then optionally oxidizing in a known manner the obtained compound, in order to get a compound of formula (Ia) where X is RS(O)ₙ wherein n equals 1 or 2, or,
(A-II) when X is alkoxy or haloalkoxy, a process wherein a compound of formula 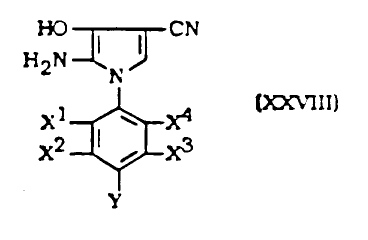 wherein the various symbols are as defined in claim 1 and the amino and cyano groups are suitably protected if necessary:
(a) is reacted with an alkylating agent, optionally in the presence of a base to obtain compounds of formula (Ia) where X is alkoxy; or
(b) is haloalkylated in a known manner to obtain compounds of formula (Ia) where X is haloalkoxy, or,
(A-III) when X is haloalkyl, a process wherein a compound of formula 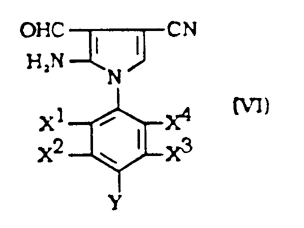 wherein the various symbols are as defined in claim 1 and amino and cyano are suitably protected if needed:
(a) is reacted with a fluorinating agent in a known manner in order to obtain the compound of formula (Ia) wherein X is a difluoromethyl group;
(b) is reacted with an oxidizing agent to convert the group -CHO to a carboxylic acid group, then submitting the compounds obtained to a fluorinating agent in a known manner to get the compound of formula (Ia) where X is trifluoromethyl;
(c) is converted to a compound where the group -CHO is replaced by a methyl group then submitting the compound obtained to a halogenating agent in a solvent in order to obtain a compound of formula (Ia) where X is bromomethyl or chloromethyl; or
(d) is reacted sequentially with a haloalkyl metal derivative or trifluorotrimethylsilane to convert the group -CHO to haloalkylcarbinol then reacting the compound where X is haloalkylcarbinol with a halogenating agent in order to get compounds of formula (Ia) where X is α-haloalkyl-α-halomethyl, all the previous steps having been followed by a deprotection step if needed, or,
(B), when the compound of formula (I) is of the formula I(b): 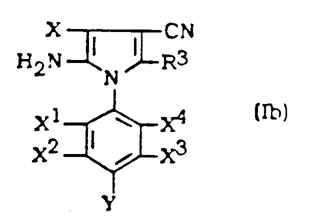 wherein X, X¹, X², X³, X⁴ and Y have the same meaning as in claim 1 and when (B-I) R³ is halogen, formyl, haloalkyl, alkyl, alkylthio, haloalkylthio, alkylsulfinyl, alkylsulfonyl, haloalkylsulfinyl, or haloalkylsulfonyl, a process wherein a compound of formula (Ia) in which X, cyano and amino are optionally protected if required:
(a) is reacted according to A-I(a) above to get compounds of formula (Ib) where R³ is halogen, then optionally said compound is reacted according to A-I(a) above to get compounds of formula (Ib) where R³ is trifluoromethyl;
(b) is reacted according to A-I(b) above to get first a compound of formula (Ib) where R³ is a bis(alkylthio)methyl group or a bis(arylthio)methyl group and then a compound of formula (Ib) wherein R³ is formyl; or
(c) is reacted according to A-I(c,d) above in order to get compounds of formula (Ib) where R³ is alkylthio or haloalkylthio, then optionally oxidized according to A-I(d) above in order to get the compounds of formula (Ib) wherein R³ is alkylsulfinyl, alkylsulfonyl, haloalkylsulfinyl or haloalkylsulfonyl, with the proviso that X is not an RS group which may undergo undesired oxidation; and when X is a halogen, optional treatment with an alkyllithium in a known manner followed by an aqueous quench and sulfenylation according to A-I(c,d) above in order to get compounds of formula (Ib) where X is alkylthio or haloalkylthio, or,
(B-II), when R³ is cyano or alkyl a process wherein a compound of formula 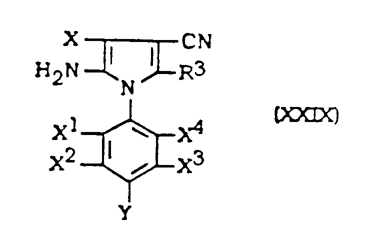 in which R³ is formyl or alkylcarbonyl, the other symbols are as defined in claim 1, and X, cyano and amino are protected if required:
(a) is condensed with hydroxylamine or 0-alkylhydroxylamine or their addition salts in a solvent in order to obtain a compound of formula (Ib) where R³ is hydroxyiminoalkylidenyl or alkoxyiminoalkylidenyl, and converting said group R³ to cyano to obtain a compound of formula (Ib) where R³ is a cyano group;
(b) is reacted, when R³ is formyl, according to A-III(a, b, c or d) above to obtain a compound of formula (Ib) where R³ is methyl or haloalkyl group; or
(c) is reacted, when R³ is formyl, with a Grignard reagent derived from an alkyl halide or an alkyllithium to produce a carbinol, followed by a dehydration step to produce a compound where R³ is alkenyl, followed by reduction in order to get compounds of formula (Ib) where R³ is alkyl; followed optionally by a deprotection step, or,
(B-III) when R³ is alkoxy or haloalkoxy, a process wherein a compound of formula 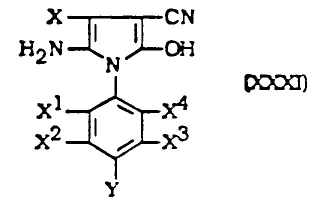 in which amino, cyano and X, are optionally protected is reacted according to A-II(a, b or c) above to obtain a compound of formula (Ib) wherein R³ is alkoxy or haloalkoxy, or,
(C) when the compound of formula I is of the formula (Ic): 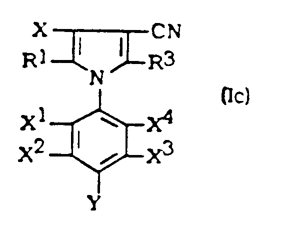 wherein X, R³, X¹, X², X³, X⁴ and Y have the same meaning as in claim 1 and when
(C-I) R¹ is hydrogen, halogen, alkylthio, haloalkylthio, alkylsulfinyl, alkylsulfonyl, haloalkylsulfinyl, haloalkylsulfonyl, alkylamino, dialkylamino, formyl, haloalkyl or alkyl, a process wherein a compound of formula 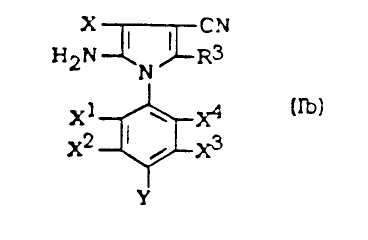 in which the amino group is deprotected after protecting the X, R³ or cyano group if required:
(a) is reacted with a diazotization agent in an inert solvent to obtain a compound of formula (Ic) where R¹ is H;
(b) is reacted with a diazotization agent in the presence of a halogen donor to obtain a compound of formula (Ic) where R¹ is halogen;
(c) is reacted with a diazotization agent in the presence of (SCN)₂ or a disulfide in a solvent to obtain compounds of formula (Ic) where R¹ is alkylthio or haloalkylthio, then optionally oxidized according to A-I(d) above to get a compound of formula (Ic) where R¹ is alkylsulfinyl or alkylsulfonyl;
(d) is reacted according to claim 9(A) below in order to get compounds of formula (Ic) where R¹ is alkylamino or dialkylamino; or
(e) is reacted with sodium nitrite and formaldoxime, copper sulfate and HCl in a known manner in order to get compounds of formula (Ic) where R¹ is formyl, then optionally (i) reacted according to B-II(a) above so as to obtain a compound of formula (Ic) where R¹ is cyano or (ii) is reacted according to A-III(a-d) above to obtain a compound of formula (Ic) where R¹ is haloalkyl or methyl followed if needed by a deprotection step; or
(C-II) R¹ is alkoxy or haloalkoxy, a process wherein a compound of formula 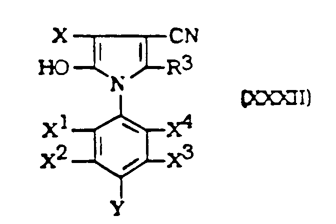 wherein the various symbols are as defined in claim 1 and X, cyano or R³ are optionally protected in a known manner, is reacted according to A-II(a or b) above to obtain a compound of formula (Ic) where R¹ is alkoxy or haloalkoxy followed by an optional deprotection step, or,
(D) when X, R¹, R³, X¹, X², X³, X⁴ and Y have the same meaning as in claim 1 and R² is CHO, a process wherein a compound of formula (Ic) is treated with a reducing agent, in a solvent to convert the group CN to a group CHO and to provide the compound in which R² is CHO, said compound being optionally oxidized in a known manner to get the corresponding compound of formula (XXXV), 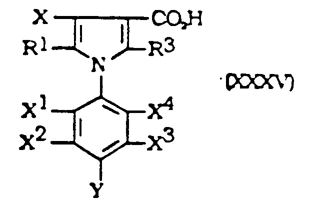 or (E), when X, R¹, R³, X¹, X², X³, X⁴ and Y have the same meaning as in claim 1, and R² is cyano, alkyl or haloalkyl, a process wherein a compound of formula (I) in which R² is CHO, after having optionally protected X, R¹ or R³ if required in a known manner is reacted according to A-III(a, b, c or d), A-II(a or b) or C-I above, followed by a deprotection step if required, or,
(F) when X, R¹, R³, X¹, X², X³, X⁴ and Y have the same meaning as in claim 1 and R² is amino, alkylamino, dialkylamino, hydrogen, halogen, alkylthio, haloalkylthio, alkylsulfinyl, alkylsulfonyl, haloalkylsulfinyl, haloalkylsulfonyl, or trifluoromethyl, a process wherein a compound of formula (XXXV) depicted above after having optionally protected X, R¹ or R³ if required in a known manner is reacted under conditions for the Curtius rearrangement, for example by conversion to an acid chloride followed by reaction with an alkali metal azide, or with diphenyl phosphoryl azide in the presence of an organic base in an alcoholic solvent to produce a carbamate which may then be hydrolyzed to obtain the corresponding compound in which R² is amino which is then optionally reacted according to claim 9(A) below or C-I(a-c) above, then when R² is halogen, optionally reacted according to B-I(a) above, followed by a deprotection step if necessary, or,
(G) when X, R¹, R³, X¹, X², X³, X⁴ and Y have the same meaning as in claim 1 and R² is alkoxy or haloalkoxy, a process wherein a compound of formula 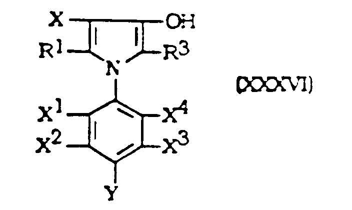 wherein the various symbols are as defined in claim 1 after having optionally protected the X, R¹ or R³ groups if required, is reacted according to B-III above followed by a deprotection step if necessary, or,
(H), when R¹, R², R³, Y, X¹, X², X³ and X⁴ are as defined in claim 1 and X is a perhaloalkylthio group, a process which comprises:
a) reacting a compound of formula (XXXVIII) 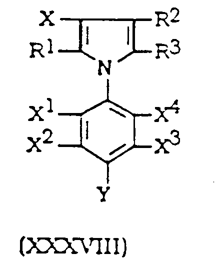 wherein X is hydrogen, and the other symbols are as defined in claim 1, with chlorosulfonic acid (ClSO₃H) at a temperature from 0^{o}C to 150^{o}C and optionally in an organic solvent, to prepare a compound of formula (XXXIX): 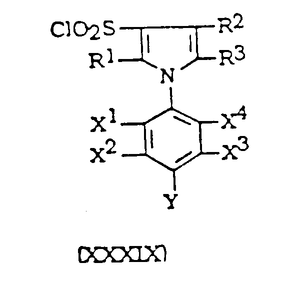 wherein the various symbols are as defined in claim 1;
b) reacting the compound of formula (XXXIX) with a reducing agent at a temperature from 0^{o}C to 110^{o}C in an organic solvent to form a disulfide compound of formula (XLI), 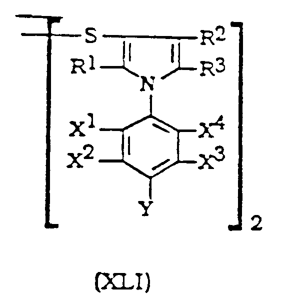 wherein the various symbols are as defined in claim 1; and
c) reacting the disulfide compound of formula (XLI) with a perhaloalkane of formula (XLII), ZCFR⁷R⁸, in which Z is Cl, Br or I, R⁷ is F, Cl or Br, and R⁸ is F, Cl, Br or a perfluoroalkyl group, in the presence of a free radical promoting reducing agent and optionally in the presence of a base, in an organic solvent at a temperature from 20^{o}C to 85^{o}C and optionally under pressure, to prepare a compound of formula (I), wherein X is a perhaloalkylthio group and the other symbols are as defined in claim 1, or,
(I) by the conversion of a compound obtained as described in any one of steps A-H above into a compound of formula (I) as defined in claim 1.

9. A process for the preparation of intermediate compounds useful for the production of compounds of the formula I which comprises:
(A) a process for the preparation of compounds of the formula (XXXIV) 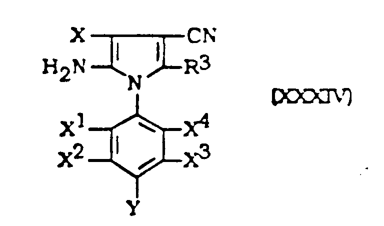 wherein X, X¹, X², X³, X⁴ and Y are as defined in claim 1 and R³ is amino, alkylamino or dialkylamino, wherein a compound of formula (XXX) 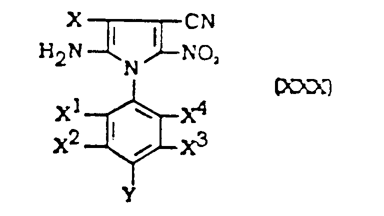 wherein the various symbols are as defined in claim 1 and the amino group is protected in a suitable manner, is reduced in order to get compounds of formula (XXXIV) where R³ is amino, said compounds being reacted:
with an alkylating agent in an organic solvent to obtain a mono or disubstituted amino group or through conversion of the amino group to an alkoxyalkylideneimino followed by reduction in order to obtain compounds of formula (XXXIV) where R³ is alkylamino or dialkylamino; followed by a deprotective step if required, or,
(B) a process for the preparation of compounds of formula (XXVIII), (XXXI), (XXXII) and (XXXV) according to claim 8(A-II), (B-III), (E) or (G), wherein the corresponding compound according to claim 8(A-I)(a), (B-I)(a), (C-I)(b) or (F), after having protected the amino group if present, is converted to a Grignard reagent or lithium derivative, followed by reaction with a trialkyl borate and oxidation in a known manner, followed by a deprotection step if necessary, or,
(C) a process for the preparation of a compound of formula 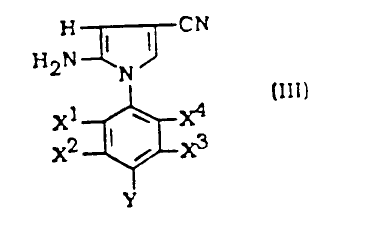 in which X¹, X², X³, X⁴ and Y have the same meaning as in claim 1, wherein a dicyanopropene derivative of formula 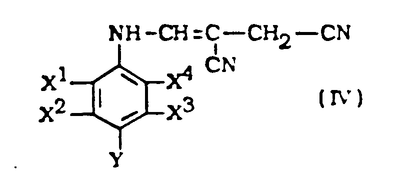 is reacted with a basic agent.

10. An arthropodicidal, plant nematocidal, anthelmintic or antiprotozoal composition which comprises a compound of general formula (I) as defined in any one of claims 1 to 7 or the compound wherein X⁴ = X¹ = Y = R¹ = X = R² = R³ = Cl and at the same time X³ = X² = H in association with one or more compatible diluents or carriers.

11. A composition for use in veterinary medicine and livestock husbandry or in maintenance of public health containing as active ingredient at least one compound according to any of claims 1 to 7 or the compound wherein X⁴ = X¹ = Y = R¹ = X = R² = R³ = Cl and at the same time X³ = X² = H.

12. An insecticidal, acaricidal or nematocidal composition which comprises; an effective amount of a compound of formula (I) according to any one of claims 1 to 7 or the compound wherein X⁴ = X¹ = Y = R¹ = X = R² = R³ = Cl and at the same time X³ = X² = H.

13. A composition according to claim 12 which comprises: between 0.05% and 95%, by weight, of one or more active ingredients: between 1% and 95%, by weight, of one or more agriculturally acceptable carriers: and between 0.1% and 50%, by weight, of one or more agriculturally acceptable surface-active agents.

14. A method for use in veterinary medicine and livestock husbandry or in maintenance of public health for control of arthropod, helminth or protozoan pests which are parasitic internally or externally upon warm-blooded vertebrates which comprises applying to a locus an effective amount of a compound of formula (I), according to any one of claims 1 to 7 or the compound wherein X⁴ = X¹ = Y = R¹ = X = R² = R³ = Cl and at the same time X³ = X² = H.

15. A method for the control of arthropod and nematode pests according to claim 14 wherein the compound is applied to the locus in which the arthropod or the nematode infestation is to be controlled in an amount of from 0.005 kg to 15 kg of the compound per hectare of the locus treated.

16. Compounds of formula (III), (XXVIII), to (XXXII), (XXXIV) to (XXXVI), (XXXVIII), (XXXIX) and (XLI), wherein the various substituents have the same meaning as in the previous claims with the exclusion of compounds wherein R¹=R²=H, R³=amino, X=cyano, X³=X⁴=H and: X¹=methyl, methoxy or chlorine and X²=Y=H; X²=methyl, methoxy or chlorine and X¹=Y=H; Y=methyl, methoxy or chlorine and X¹=X²=H.

## Claims (Claims for the following Contracting State(s): ES)

1. A process for the preparation of a compound of formula (I) 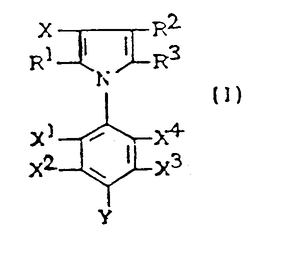 wherein
X is selected from the group consisting of: halogen, cyano, haloalkyl, alkoxy, haloalkoxy, alkylthio, alkylsulfinyl, alkylsulfonyl, haloalkylthio, haloalkylsulfinyl and haloalkylsulfonyl, wherein the alkyl, haloalkyl, alkoxy and haloalkoxy groups herein are linear or branched chains, having less than 10 carbon atoms, and the halosubstitution in all these groups consists of one or more halogen atoms, which are the same or different, from mono-substitution up to complete poly-substitution;
R¹, R² and R³ are individually selected from the group consisting of: one of the same set of substituents as described for X; a hydrogen atom; an alkyl group; and a substituent of which no more than one of R¹, R² and R³ is selected from the group consisting of formyl, amino, alkylamino and dialkylamino, wherein the alkyl groups herein are linear or branched chains, having less than 10 carbon atoms;
Y is selected from the group consisting of: halogen, cyano, alkyl, haloalkyl, alkoxy and haloalkoxy, wherein the alkyl, alkoxy, haloalkyl and haloalkoxy groups herein are linear or branched chains, having less than 10 carbon atoms, and the halo substitution in all these groups herein consists of one or more halogen atoms, which are the same or different, from mono-substitution up to as much as complete poly-substitution;
or Y is a hydrogen atom when:
X is a halogen atom or a group R⁵S(O)n, in which n is 0, 1 or 2 and R⁵ is alkyl or haloalkyl, and the alkyl carbon chains and the halo-substitution are as defined above;
R¹ and R³ are each a hydrogen atom; and
R² is cyano;
X¹, X², X³ and X⁴ are individually selected from the same set of substituents as described for Y or a hydrogen atom, with the following provisos: at least one of R¹, R² and R³ is selected from the same set of substituents as described for X;
if X⁴ and X¹ are H, and X is halogen or cyano then R² is different from X, and
if X⁴ and X¹ are H and Y is methyl, then X is different from bromo;
with the exclusion of the compound wherein X⁴ = X¹ = Y = R¹ = X = R² = R³ = Cl and at the same time X² = X³ = H, which process comprises:
(A) when the compound is of formula I(a): 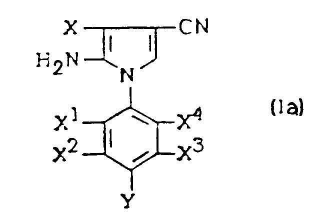 wherein X¹, X², X³, X⁴ and Y are as hereinbefore defined and,
(A-I) : X is halogen, trifluoromethyl, cyano, alkylthio, alkylsulfinyl, alkylsulfonyl, haloalkylthio, haloalkylsulfinyl or haloalkylsulfonyl, a process wherein a compound of formula 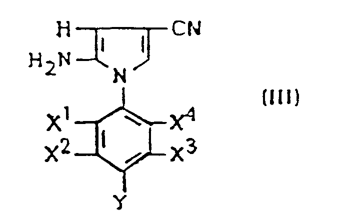 wherein the various symbols are as hereinbefore defined and amino is optionally protected:
(a) is reacted with a halogenating agent, optionally in the presence of a solvent to obtain a compound of formula (Ia) wherein X is halogen, then optionally reacting said compound with trifluoromethyl copper in a known manner to get compounds of formula (Ia) where X is trifluoromethyl;
(b) is reacted with a tris(alkylthio)methane or tris(arylthio)methane in the presence of a Lewis acid, then the obtained compound of formula (XI) 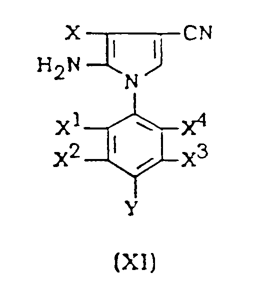 where X is bis(alkylthio)methyl or bis(arylthio)methyl and the other symbols are as hereinbefore defined is optionally reacted with an alkyl nitrite followed by hydrolysis, in order to obtain the compound of formula (XI) where X is formyl, then bringing the said compound into contact with hydroxylamine followed by dehydration in a known manner in order to obtain the compound of formula (Ia) where X is a cyano group;
(c) is reacted with a compound of formula MSCN, M being an alkali metal, in the presence of bromine, in a solvent in order to obtain the compound of formula (Ia) where X is a thiocyanato group and then reacting said compound with an alkyl halide or dialkyl sulfate in the presence of a base in a solvent to get a compound of formula (Ia) where X is alkylthio; or
(d) is reacted with a sulfenyl halide of formula RSHal, in which R is an alkyl, haloalkyl, phenyl or heteroaryl radical and Hal is a halogen atom in an organic liquid reaction medium, optionally in the presence of an acid acceptor in order to get a compound of formula (Ia) where X is alkylthio or haloalkylthio, then optionally oxidizing in a known manner the obtained compound, in order to get a compound of formula (Ia) where X is RS(O)ₙ wherein n equals 1 or 2, or,
(A-II) when X is alkoxy or haloalkoxy, a process wherein a compound of formula 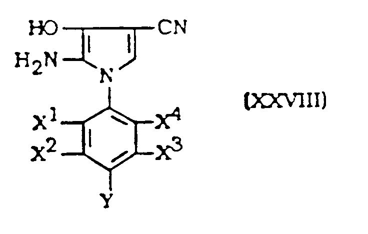 wherein the various symbols are as hereinbefore defined and the amino and cyano groups are suitably protected if necessary:
(a) is reacted with an alkylating agent, optionally in the presence of a base to obtain compounds of formula (Ia) where X is alkoxy; or
(b) is haloalkylated in a known manner to obtain compounds of formula (Ia) where X is haloalkoxy, or
(A-III) when X is haloalkyl, a process wherein a compound of formula 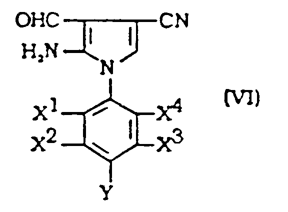 wherein the various symbols are as hereinbefore defined and amino and cyano are suitably protected if needed:
(a) is reacted with a fluorinating agent in a known manner in order to obtain the compound of formula (Ia) wherein X is a difluoromethyl group;
(b) is reacted with an oxidizing agent to convert the group -CHO to a carboxylic acid group, then submitting the compounds obtained to a fluorinating agent in a known manner to get the compound of formula (Ia) where X is trifluoromethyl;
(c) is converted to a compound where the group -CHO is replaced by a methyl group than submitting the compound obtained to a halogenating agent in a solvent in order to obtain a compound of formula (Ia) where X is bromomethyl or chloromethyl; or
(d) is reacted sequentially with a haloalkyl metal derivative or trifluorotrimethylsilane to convert the group -CHO to haloalkylcarbinol then reacting the compound where X is haloalkylcarbinol with a halogenating agent in order to get compounds of formula (Ia) where X is α-haloalkyl-α-halomethyl, all the previous steps having been followed by a deprotection step if needed, or,
(B), when the compound of formula (I) is of the formula I(b): 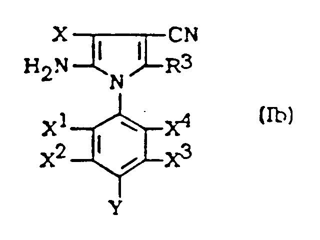 wherein X, X¹, X², X³, X⁴ and Y are as hereinbefore defined and when (B-I) R³ is halogen, formyl, haloalkyl, alkyl, alkylthio, haloalkylthio, alkylsulfinyl, alkylsulfonyl, haloalkylsulfinyl or haloalkylsulfonyl, a process wherein a compound of formula (Ia) in which X, cyano and amino are optionally protected if required:
(a) is reacted according to A-I(a) above to get compounds of formula (Ib) where R³ is halogen, then optionally said compound is reacted according to A-I(a) above to get compounds of formula (Ib) where R³ is trifluoromethyl;
(b) is reacted according to A-I(b) above to get first a compound of formula (Ib) where R³ is a bis(alkylthio)methyl group or a bis(arylthio)methyl group and then a compound of formula (Ib) wherein R³ is formyl; or
(c) is reacted according to A-I(c,d) above in order to get compounds of formula (Ib) where R³ is alkylthio or haloalkylthio, then optionally oxidized according to A-I(d) above in order to get the compounds of formula (Ib) wherein R³ is alkylsulfinyl, alkylsulfonyl, haloalkylsulfinyl or haloalkylsulfonyl with the proviso that X is not an RS group which may undergo undesired oxidation; and when X is a halogen, optional treatment with an alkyllithium in a known manner followed by an aqueous quench and sulfenylation according to A-I(c,d) above in order to get compounds of formula (Ib) where X is alkylthio or haloalkylthio, or,
(B-II), when R³ is cyano or alkyl a process wherein a compound of formula 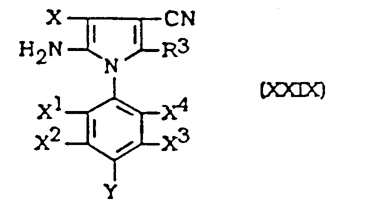 in which R³ is formyl or alkylcarbonyl, the other symbols are as hereinbefore defined, and X, cyano and amino are protected if required:
(a) is condensed with hydroxylamine or O-alkylhydroxylamine or their addition salts in a solvent in order to obtain a compound of formula (Ib) where R³ is hydroxyiminoalkylidenyl or alkoxyiminoalkylidenyl, and converting said group R³ to cyano to obtain a compound of formula (Ib) where R³ is a cyano group;
(b) is reacted, when R³ is formyl, according to A-III(a, b, c or d) above to obtain a compound of formula (Ib) where R³ is methyl or haloalkyl group; or
(c) is reacted, when R³ is formyl, with a Grignard reagent derived frog an alkyl halide or an alkyllithium to produce a carbinol, followed by a dehydration step to produce a compound where R³ is alkenyl, followed by reduction in order to get compounds of formula (Ib) where R³ is alkyl; followed optionally by a deprotection step, or,
(B-III) when R³ is alkoxy or haloalkoxy, a process wherein a compound of formula 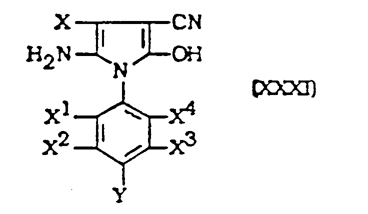 in which amino, cyano and X, are optionally protected is reacted according to A-II(a, b or c) above to obtain a compound of formula (Ib) wherein R³ is alkoxy or,
(C) when the compound of formula I is of the formula (Ic): 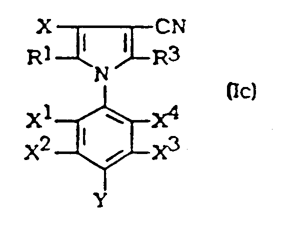 wherein X, R³, X¹, X², X³, X⁴ and Y are as hereinbefore defined and when
(C-I) R¹ is hydrogen, halogen, alkylthio, haloalkylthio, alkylsulfinyl, alkylsulfonyl, haloalkylsulfinyl, haloalkylsulfonyl, alkylamino, dialkylamino, formyl, haloalkyl or alkyl, a process wherein a compound of formula 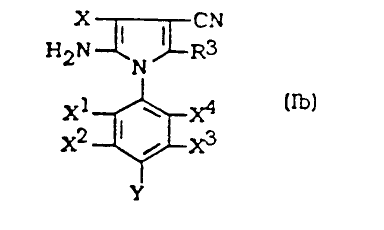 in which the amino group is deprotected after protecting the X, R³ or cyano group if required:
(a) is reacted with a diazotization agent in an inert solvent to obtain a compound of formula (Ic) where R¹ is H;
(b) is reacted with a diazotization agent in the presence of a halogen donor to obtain a compound of formula (Ic) where R¹ is halogen;
(c) is reacted with a diazotization agent in the presence of (SCN)₂ or a disulfide in a solvent to obtain compounds of formula (Ic) where R¹ is alkylthio or haloalkylthio, then optionally oxidized according to A-I(d) above to get a compound of formula (Ic) where R¹ is alkylsulfinyl or alkylsulfonyl;
(d) is reacted according to claim 8(A) below in order to get compounds of formula (Ic) where R¹ is alkylamino or dialkylamino; or
(e) is reacted with sodium nitrite and formaldoxime, copper sulfate and HCl in a known manner in order to get compounds of formula (Ic) where R¹ is formyl, then optionally (i) reacted according to B-II(a) above so as to obtain a compound of formula (Ic) where R¹ is cyano or (ii) is reacted according to A-III(a-d) above to obtain a compound of formula (Ic) where R¹ is haloalkyl or methyl followed if needed by a deprotection step;
(C-II) R¹ is alkoxy or haloalkoxy, a process wherein a compound of formula 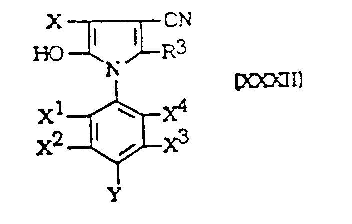 wherein the various symbols are as hereinbefore defined and X, cyano or R³ are optionally protected in a known manner, is reacted according to A-III(a or b) above to obtain a compound of formula (Ic) where R¹ is alkoxy or haloalkoxy following by an optional deprotection step, or,
(D) when X, R¹, R³, X¹, X², X³, X⁴ and Y are as hereinbefore defined and R² is CHO, a process wherein a compound of formula (Ic) is treated with a reducing agent, in a solvent to convert the group CN to a group CHO and to provide the compound in which R² is CHO, said compound being optionally oxidized in a known manner to get the corresponding compound of formula (XXXV), 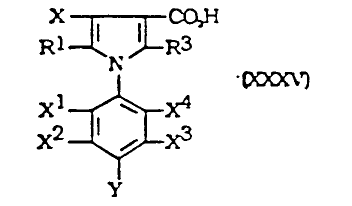
or (E), when X, R¹, R³, X¹, X², X³, X⁴ and Y are as hereinbefore defined and R² is
cyano, alkyl or haloalkyl, a process wherein a compound of formula (I) in which R² is CHO, after having optionally protected X, R¹ or R³ if required in a known manner is reacted according to A-III(a, b, c or d), A-II(a or b) or C-I above, followed by a deprotection step if required, or,
(F) when X, R¹, R³, X¹, X², X³, X⁴ and Y are as hereinbefore defined and R² is amino, alkylamino, dialkylamino, hydrogen, halogen, alkylthio, haloalkylthio, alkylsulfinyl, alkylsulfonyl, haloalkylsulfinyl, haloalkylsulfonyl or trifluoromethyl, a process wherein a compound of formula (XXXV) depicted above after having optionally protected X, R¹ or R³ if required in a known manner is reacted under conditions for the Curtius rearrangement, for example by conversion to an acid chloride followed by reaction with an alkali metal azide, or with diphenyl phosphoryl azide in the presence of an organic base in an alcoholic solvent to produce a carbamate which may then be hydrolyzed to obtain the corresponding compound in which R² is amino which is then optionally reacted according to claim 8(A) below or C-I(a-c) above, then when R² is halogen, optionally reacted according to B-I(a) above, followed by a deprotection step if necessary, or,
(G) when X, R¹, R³, X¹, X², X³, X⁴ and Y are as hereinbefore defined and R² is alkoxy or haloalkoxy, a process wherein a compound of formula 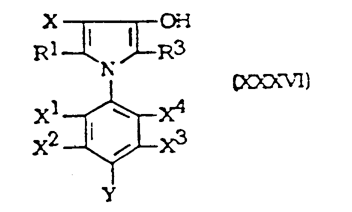 wherein the various symbols are as hereinbefore defined after having optionally protected the X, R¹ or R³ groups if required, is reacted according B-III above followed by a deprotection step if necessary, or,
(H), when R¹, R², R³, Y, X¹, X², X³ and X⁴ are as hereinbefore defined and X is a perhaloalkylthio group, a process which comprises:
a) reacting a compound of formula (XXXVIII) 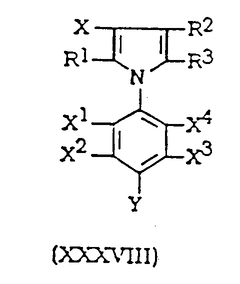 wherein X is hydrogen, and the other symbols are as hereinbefore defined, with chlorosulfonic acid (ClSO₃H) at a temperature from 0°C to 150°C and optionally in an organic solvent, to prepare a compound of formula (XXXIX): 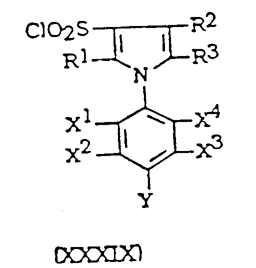 wherein the various symbols are as hereinbefore defined;
b) reacting the compound of formula (XXXIX) with a reducing agent at a temperature from 0^{o}C to 110^{o}C in an organic solvent to form a disulfide compound of formula (XLI), 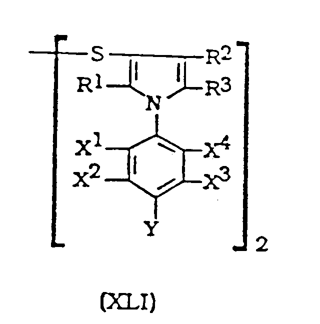 wherein the various symbols are as hereinbefore defined; and
c) reacting the disulfide compound of formula (XLI) with a perhaloalkane of formula (XLII), ZCFR⁷R⁸, in which Z is Cl, Br or I, R⁷ is F, Cl or Br, and R⁸ is F, Cl, Br or a perfluoroalkyl group, in the presence of a free radical promoting reducing agent and optionally in the presence of a base, in an organic solvent at a temperature from 20^{o}C to 85^{o}C and optionally under pressure, to prepare a compound of formula (I), wherein X is a perhaloalkylthio group and the other symbols are as hereinbefore defined, or,
(I) by the conversion of a compound obtained as described in any one of steps A-H above into a compound of formula (I) as hereinbefore defined.

2. A process according to claim 1, wherein:
X is selected from a halogen atom, or a group selected from cyano, haloalkyl, alkoxy, haloalkoxy, alkylthio, alkylsulfinyl, alkylsulfonyl, haloalkylthio, haloalkylsulfinyl and haloalkylsulfonyl, wherein the alkyl, haloalkyl, alkoxy and haloalkoxy groups herein are linear or branched chains, having less than 10 carbon atoms, and the halosubstitution in all these groups is mono-substitution or up to as much as complete poly-substitution;
R¹, R² and R³ are individually selected from: the same substituents as described for X; a hydrogen atom; an alkyl group; and a substituent of which no more than one of R¹, R² and R³ is selected from the group consisting of formyl, amino, alkylamino and dialkylamino, wherein the alkyl groups herein are linear or branched chains, having less than 10 carbon atoms;
Y is selected from a halogen atom or a group selected from cyano, alkyl, haloalkyl, alkoxy and haloalkoxy, wherein the alkyl, alkoxy, haloalkyl and haloalkoxy groups herein are linear or branched chains, having less than 10 carbon atoms, and the halosubstitution in all these groups herein is mono-substitution or up to as much as complete poly-substitution; and
X¹, X², X³ and X⁴ are as described in claim 1.

3. A process according to claim 2 wherein:
X is a halogen atom or a group R⁵S(O)n, in which n is 0, 1 or 2 and R⁵ is alkyl or haloalkyl;
R¹ is a hydrogen atom, a halogen atom or alkylthio;
R² is cyano;
R³ is a hydrogen atom or a halogen atom;
Y is a hydrogen atom, a halogen atom, haloalkyl or haloalkoxy, with the proviso that Y is a hydrogen atom as defined in claim 1; and
X¹, X², X³ and X⁴ are individually selected from the group consisting of a hydrogen atom, a halogen atom, C₁₋₃ alkyl, C₁₋₃ alkoxy, and C₁₋₃ alkylthio.

4. A process according to claim 3, wherein the compound of formula (I) has the formula (II) 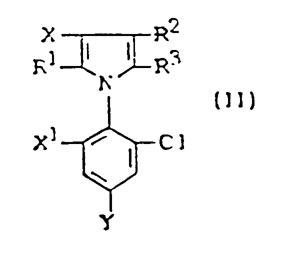 wherein:
X is R⁵S(O)n, in which n is 0, 1 or 2 and R⁵ is CH_{3,} CF₃, CF₂Cl, CFCl₂, CF₂Br, CHF₂, CHCl₂ or CHClF;
R¹ is H, F, Cl or Br;
R³ is H, F, Cl or Br;
X¹ is H or Cl; and
Y is CF₃ or CF₃O.

5. A process according to claim 1, 2 or 3 wherein:
R¹ is a hydrogen atom or a halogen atom;
R² is cyano;
X is a haloalkyl-S(O)n group, wherein n is 0, 1 or 2;
X¹ and X⁴ are other than a hydrogen atom;
Y is a haloalkyl or haloalkoxy group; and
X² and X³ are a hydrogen atom.

6. A process according to claim 2, wherein the compound of formula (I) has the formula (II) 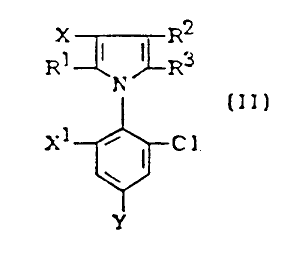 wherein:
X is R⁵S(O)ₙ, in which n is 0, 1 or 2 and R⁵ is CH₃, CF₃, CF₂Cl or CFCl₂;
R² is cyano;
R¹ is H, F, Cl, Br or NH₂;
R³ is H, F, Cl, Br, CF₃ or CN;
X¹ is H or Cl; and
Y is CF₃ or CF₃O.

7. A process according to claim 1, wherein the compound of formula (I) is:
1-(2,6-dichloro-4-trifluoromethylphenyl)-2-chloro-3-cyano-4-(trifluoromethylthio)pyrrole;
1-(2,6-dichloro-4-trifluoromethoxyphenyl)-2-chloro-3-cyano-4-(trifluoromethylsulfonyl)pyrrole;
1-(2,6-dichloro-4-trifluoromethylphenyl)-2-chloro-3-cyano-4-(dichlorofluoromethylthio)pyrrole;
1-(2,6-dichloro-4-trifluoromethylphenyl)-2-chloro-3-cyano-4-(dichlorofluoromethylsulfinyl)pyrrole;
1-(2,6-dichloro-4-trifluoromethylphenyl)-2-chloro-3-cyano-4-(dichlorofluoromethylsulfonyl)pyrrole;
1-(2,6-dichloro-4-trifluoromethylphenyl)-2-chloro-3-cyano-4-(chlorodifluoromethylsulfonyl)pyrrole;
1-(2,6-dichloro-4-trifluoromethylphenyl)-2-chloro-3-cyano-4-(chlorodifluoromethylsulfinyl)pyrrole;
1-(2,6-dichloro-4-trifluoromethylphenyl)-2-chloro-3-cyano-4-(chlorodifluoromethylthio)pyrrole;
1-(2,6-dichloro-4-trifluoromethylphenyl)-2-bromo-3-cyano-4-(trifluoromethylsulfinyl)pyrrole;
1-(2,6-dichloro-4-trifluoromethylphenyl)-2-bromo-3-cyano-4-(trifluoromethylsulfonyl)pyrrole;
1-(2,6-dichloro-4-trifluoromethylphenyl)-2-bromo-3-cyano-4-(dichlorofluoromethylthio)pyrrole;
1-(2,6-dichloro-4-trifluoromethylphenyl)-2-bromo-3-cyano-4-(dichlorofluoromethylsulfonyl)pyrrole;
1-(2,6-dichloro-4-trifluoromethylphenyl)-2-bromo-3-cyano-4-(dichlorofluoromethylsulfinyl)pyrrole;
1-(2,6-dichloro-4-trifluoromethylphenyl)-2-bromo-3-cyano-4-(chlorodifluoromethylthio)pyrrole;
1-(2,6-dichloro-4-trifluoromethylphenyl)-2-bromo-3-cyano-4-(chlorodifluoromethylsulfinyl)pyrrole;
1-(2,6-dichloro-4-trifluoromethylphenyl)-2-chloro-3-cyano-4-(dichlorofluoromethylsulfonyl)-5-bromopyrrole;
1-(2,6-dichloro-4-trifluoromethylphenyl)-2-bromo-3-cyano-4-(chlorodifluoromethylsulfonyl)pyrrole;
1-(2,6-dichloro-4-trifluoromethoxyphenyl)-2-chloro-3-cyano-4-(dichlorofluoromethylsulfinyl)pyrrole;
1-(2,6-dichloro-4-trifluoromethoxyphenyl)-2-chloro-3-cyano-4-(dichlorofluoromethylsulfonyl)pyrrole;
1-(2-chloro-4-trifluoromethylphenyl)-2-chloro-3-cyano-4-(dichlorofluoromethylsulfonyl)pyrrole;
1-(2-chloro-4-trifluoromethylphenyl)-2-chloro-3-cyano-4-(dichlorofluoromethylsulfinyl)pyrrole;
1-(2-chloro-4-trifluoromethylphenyl)-2-bromo-3-cyano-4-(dichlorofluoromethylsulfonyl)pyrrole;
1-(2,6-dichloro-4-trifluoromethylphenyl)-2-chloro-3-cyano-4-(dichlorofluoromethylthio)-5-methylthiopyrrole;
1-(2,6-dichloro-4-trifluoromethylphenyl)-2-chloro-3-cyano-4-(bromodifluoromethylthio)pyrrole;
1-(2,6-dichloro-4-trifluoromethylphenyl)-2-chloro-3-cyano-4-(bromodifluoromethylsulfinyl)pyrrole;
1-(2,6-dichloro-4-trifluoromethylphenyl)-2-chloro-3-cyano-4-(bromodifluoromethylsulfonyl)pyrrole;
1-(2,6-dichloro-4-trifluoromethylphenyl)-2-chloro-3-cyano-4-(methylsulfinyl)pyrrole;
1-(2,6-dichloro-4-trifluoromethylphenyl)-2-chloro-3-cyano-4-(methylsulfonyl)pyrrole;
1-(4-bromo-2,6-dichlorophenyl)-3-cyano-4-(chlorodifluoromethylthio)pyrrole;
1-(4-bromo-2,6-dichlorophenyl)-3-cyano-4-(trifluoromethylthio)pyrrole;
1-(2,4,6-trichlorophenyl)-3-cyano-4-(chlorodifluoromethylthio)pyrrole;
1-(2,4,6-trichlorophenyl)-3-cyano-4-(chlorodifluoromethylsulfinyl)pyrrole;
1-(2,4,6-trichlorophenyl)-3-cyano-4-(dichlorofluoromethylthio)pyrrole;
1-(2,4,6-trichlorophenyl)-3-cyano-4-(dichlorofluoromethylsulfinyl)pyrrole;
1-(2,4,6-trichlorophenyl)-3-cyano-4-(dichlorofluoromethylsulfonyl)pyrrole;
1-(4-bromo-2,6-dichlorophenyl)-3-cyano-4-(dichlorofluoromethylthio)pyrrole;
1-(4-bromo-2,6-dichlorophenyl)-3-cyano-4-(dichlorofluoromethylsulfinyl)pyrrole;
1-(4-bromo-2,6-dichlorophenyl)-3-cyano-4-(dichlorofluoromethylsulfonyl)pyrrole;
1-(2,4,6-trichlorophenyl)-3-cyano-4-(trifluoromethylthio)pyrrole;
1-(2,4,6-trichlorophenyl)-3-cyano-4-(trifluoromethylsulfinyl)pyrrole;
1-(2,4,6-trichlorophenyl)-3-cyano-4-(trifluoromethylsulfonyl)pyrrole;
1-(2,4,6-trichlorophenyl)-3-cyano-4-(trichloromethylthio)pyrrole;
1-(2,4-dichlorophenyl)-3-cyano-4-(dichlorofluoromethylthio)pyrrole;
1-(2,4,6-trichlorophenyl)-3-cyano-4-chloropyrrole;
1-(2,4,6-trichlorophenyl)-3-cyano-4-(chlorodifluoromethylsulfonyl)pyrrole;
1-(2,6-dichlorophenyl)-3-cyano-4-(dichlorofluoromethylthio)pyrrole;
1-(4-bromo-2,6-dichlorophenyl)-3-cyano-4-(trifluoromethylsulfinyl)pyrrole;
1-(4-bromo-2,6-dichlorophenyl)-3-cyano-4-(trifluoromethylsulfonyl)pyrrole;
1-(4-bromo-2,6-dichlorophenyl)-3-cyano-4-(chlorodifluoromethylsulfinyl)pyrrole;
1-(4-bromo-2,6-dichlorophenyl)-3-cyano-4-(chlorodifluoromethylsulfonyl)pyrrole;
1-(4-bromo-2,6-dimethylphenyl)-3-cyano-4-(trifluoromethylsulfinyl)pyrrole;
1-(4-bromo-2,6-dimethylphenyl)-3-cyano-4-trifluoromethylsulfonyl)pyrrole; or
1-(4-bromo-2,6-difluorophenyl)-3-cyano-4-(dichlorofluoromethylthio)pyrrole:
1-(2,6-dichloro-4-trifluoromethylphenyl)-2-chloro-3-cyano-4-(trifluoromethylsulfonyl)pyrrole;
1-(2,6-dichloro-4-trifluoromethylphenyl)-2-chloro-3-cyano-4-(trifluoromethylsulfinyl)pyrrole;
1-(2,6-dichloro-4-trifluoromethylphenyl)-2-chloro-3-cyano-4-trifluoromethylsulfinyl-5-bromopyrrole;
1-(2,6-dichloro-4-trifluoromethylphenyl)-2-chloro-3-cyano-4-trifluoromethylsulfonyl-5-bromopyrrole;
1-(2,6-dichloro-4-trifluoromethylphenyl)-2-chloro-3-cyano-4-(trifluoromethylthio)pyrrole;
1-(2,6-dichloro-4-trifluoromethylphenyl)-2-bromo-3-trifluoromethylthio-4-cyano-5-chloropyrrole;
1-(2,6-dichloro-4-trifluoromethylphenyl)-2-[(trifluoromethyl)carbonylamino]-3-trifluoromethylthio-4- cyano-5-chloropyrrole;
1-(2,6-dichloro-4-trifluoromethylphenyl)-2-(methylcarbonylamino)-3-trifluoromethylthio-4-cyano-5-chloropyrrole;
1-(2,6-dichloro-4-trifluoromethylphenyl)-2-amino-3-trifluoromethylthio-4-cyano-5-chloropyrrole;
1-(2-chloro-4-trifluoromethylphenyl)-2-amino-3-trifluoromethylthio-4-cyano-5-chloropyrrole;
1-(2,6-dichloro-4-trifluoromethylphenyl)-2-amino-3-dichlorofluoromethylthio-4-cyano-5-chloropyrrole;
1-(2,6-dichloro-4-trifluoromethylphenyl)-2,4-bis(trifluoromethylthio)-3-cyano-5-aminopyrrole;
1-(2,6-dichloro-4-trifluoromethylphenyl)-2-amino-3-trifluoromethylthio-4-cyano-5-bromopyrrole;
1-(2,6-dichloro-4-trifluoromethylphenyl)-2-amino-3-trifluoromethylthio-4-cyanopyrrole;
1-(4-trifluoromethylphenyl)-2-amino-3-trifluoromethylthio-4-cyano-5-bromopyrrole;
1-(2-chloro-4-trifluoromethylphenyl)-2-amino-3-trifluoromethylthio-4-cyano-5-bromopyrrole;
1-(2,6-dichloro-4-trifluoromethylphenyl)-2-chloro-3-cyano-4-(dichlorofluoromethylthio)pyrrole;
1-(2,6-dichloro-4-trifluoromethylphenyl)-2-chloro-3-cyano-4-(dichlorofluoromethylsulfinyl)pyrrole;
1-(2,6-dichloro-4-trifluoromethylphenyl)-2-chloro-3-cyano-4-(dichlorofluoromethylsulfonyl)pyrrole;
1-(2,6-dichloro-4-trifluoromethylphenyl)-3-cyano-4-(dichlorofluoromethylthio)pyrrole;
1-(2,6-dichloro-4-trifluoromethylphenyl)-3-cyano-4-(dichlorofluoromethylsulfinyl)pyrrole; or
1-(2,6-dichloro-4-trifluoromethoxyphenyl)-2-chloro-3-cyano-4-(trifluoromethylsulfonyl)pyrrole.

8. A process for the preparation of intermediate compounds useful for the production of compounds of the formula (I) which comprises:
(A) a process for the preparation of compounds of the formula (XXXIV) 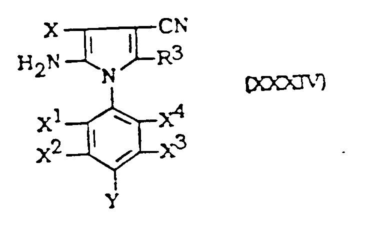 wherein X, X¹, X², X³, X⁴ and Y are as defined in claim 1 and R³ is amino, alkylamino or dialkylamino, wherein a compound of formula (XXX) 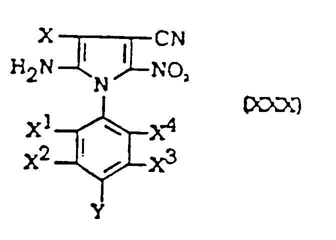 wherein the various symbols are as defined in claim 1 and the amino group is protected in a suitable manner, is reduced in order to get compounds of formula (XXXIV) where R³ is amino, said compounds being reacted:
with an alkylating agent in an organic solvent to obtain a mono or disubstituted amino group or through conversion of the amino group to an alkoxyalkylideneimino followed by reduction in order to obtain compounds of formula (XXXIV) where R³ is alkylamino or dialkylamino; followed by a deprotective step if required, or,
(B) a process for the preparation of compounds of formula (XXVIII), (XXXI), (XXXII) and (XXXV) as defined in claim 1(A-II), (B-III), (E) or (G), wherein the corresponding compound defined in claim 1(A-I)(a), (B-I)(a), (C-I)(b) or (F), after having protected the amino group if present, is converted to a Grignard reagent or lithium derivative, followed by reaction with a trialkyl borate and oxidation in a known manner, followed by a deprotection step if necessary, or,
(C) a process for the preparation of a compound of formula 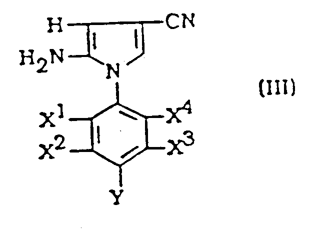 in which X¹, X², X³, X⁴ and Y have the same meaning as in claim 1, wherein a dicyanopropene derivative of formula 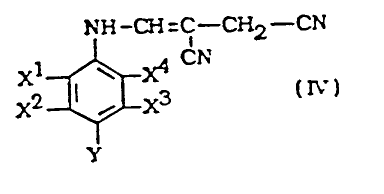 is reacted with a basic agent.

9. A process for the preparation of an arthropodicidal, plant nematocidal, anthelmintic or antiprotozoal composition which comprises a compound of general formula (I) as defined in any one of claims 1 to 7 or the compound wherein X⁴=X¹=Y=R¹=X=R²=R³=Cl and at the same time X³=X²=H in association with one or more compatible diluents or carriers, which process comprises formulating the compound of formula (I) or the compound wherein X⁴=X¹=Y=R¹=X=R²=R³=Cl and at the same time X³=X²=H with the one or more compatible diluents or carriers.

10. A process for the preparation of a composition for use in veterinary medicine and livestock husbandry or in maintenance of public health containing as active ingredient at least one compound of formula (I) as defined in any one of claims 1 to 7 or the compound wherein X⁴=X¹=Y=R¹=X=R²=R³=Cl and at the same time X³=X²=H, which process comprises formulating the compound of formula (I) or the compound wherein X⁴=X¹=Y=R¹=X=R²=R³=Cl and at the same time X³=X²=H.

11. A process for the preparation of an insecticidal, acaricidal or nematocidal composition which comprises an effective amount of a compound of formula (I) as defined in any one of claims 1 to 7 or the compound wherein X⁴=X¹=Y=R¹=X=R²=R³=Cl and at the same time X³=X²=H, which process comprises formulating the compound of formula (I) or the compound wherein X⁴=X¹=Y=R¹=X=R²=R³=Cl and at the same time X³=X²=H.

12. A process according to claim 11 wherein the composition comprises:
between 0.05% and 95%, by weight, of one or more active ingredients: between 1% and 95%, by weight, of one or more agriculturally acceptable carriers: between 0.1% and 50%, by weight, of one or more agriculturally acceptable surface-active agents.

13. A method for use in veterinary medicine and livestock husbandry or in maintenance of public health for control of arthropod, helminth or protozoan pests which are parasitic internally or externally upon warm-blooded vertebrates which comprises applying to a locus an effective amount of a compound of formula (I), as defined in any one of claims 1 to 7 or the compound wherein X⁴=X¹=Y=R¹=X=R²=R³=Cl and at the same time X³=X²=H.

14. A method for the control of arthropod and nematode pests according to claim 13 wherein the compound is applied to the locus in which the arthropod or the nematode infestation is to be controlled in an amount of from 0.005 kg to 15 kg of the compound per hectare of the locus treated.

15. A compound of formula (I) as defined in any one of claims 1 to 7.

16. Compounds of formula (III), (XXVIII) to (XXXII), (XXXIV) to (XXXVI), (XXXVIII), (XXXIX) and (XLI), wherein the various substituents have the same meaning as in the previous claims with the exclusion of compounds wherein R¹=R²=H, R³=amino, X=cyano, X³=X⁴=H and: X¹=methyl, methoxy or chlorine and X²=Y=H; X²=methyl, methoxy or chlorine and X¹=Y=H; Y=methyl, methoxy or chlorine and X¹=X²=H.

17. A composition as defined in any one of claims 9 to 12.

## Patentansprüche (Patentansprüche für folgende(n) Vertragsstaat(en): AT, BE, CH, LI, DE, FR, GB, GR, IT, LU, NL, SE)

1. Verbindung der Formel (I): 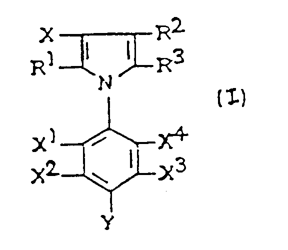 in der:
X unter Halogen, Cyano, Halogenalkyl, Alkoxy, Halogenalkoxy, Alkylthio, Alkylsulfinyl, Alkylsulfonyl, Halogenalkylthio, Halogenalkylsulfinyl und Halogenalkylsulfonyl ausgewählt ist, wobei die Alkyl-, Halogenalkyl-, Alkoxy- und Halogenalkoxygruppen hier lineare oder verzweigte Ketten mit weniger als 10 Kohlenstoffatomen sind und die Halogensubstitution sämtlicher dieser Gruppen aus einem oder mehreren Halogenatomen, die gleich oder voneinander verschieden sind, besteht und von der Monosubstitution bis zur vollständigen Polysubstitution reicht,
R¹, R² und R³ individuell unter den für X beschriebenen Substituenten, einem Wasserstoffatom und einer Alkylgruppe ausgewählt sind und nicht mehr als einer der Substituenten R¹, R² und R³ unter Formyl, Amino, Alkylamino und Dialkylamino ausgewählt ist, wobei die Alkylgruppen hier lineare oder verzweigte Ketten mit weniger als 10 Kohlenstoffatomen sind,
Y unter Halogen, Cyano, Alkyl, Halogenalkyl, Alkoxy und Halogenalkoxy ausgewählt ist, wobei die Alkyl-, Alkoxy-, Halogenalkyl- und Halogenalkoxygruppen hier lineare oder verzweigte Ketten mit weniger als 10 Kohlenstoffatomen sind und die Halogensubstitution sämtlicher dieser Gruppen hier aus einem oder mehreren Halogenatomen, die gleich oder voneinander verschieden sind, besteht und von der Monosubstitution bis zur vollständigen Polysubstitution reicht,
oder Y ein Wasserstoffatom ist, wenn
X ein Halogenatom oder eine Gruppe R⁵S(O)ₙ ist, wobei n 0, 1 oder 2 und R⁵ Alkyl oder Halogenalkyl ist und die Alkylkohlenstoffketten und die Halogensubstitution wie oben definiert sind,
R¹ und R³ jeweils ein Wasserstoffatom sind und
R² Cyano ist,
X¹, X², X³ und X⁴ individuell unter den für Y beschriebenen Substituenten oder einem Wasserstoffatom ausgewählt sind, mit den folgenden Maßgaben: mindestens einer der Substituenten R¹, R² und R³ ist unter den für X beschriebenen Substituenten ausgewählt,
wenn X⁴ und X¹ H sind und X Halogen oder Cyano ist, unterscheidet sich R² von X und
wenn X⁴ und X¹ H sind und Y Methyl ist, ist X nicht Brom, wobei die Verbindung ausgenommen ist, bei der X⁴ = X¹ = Y = R¹ = X = R² = R³ = Cl und gleichzeitig X² = X³ = H ist.

2. Verbindung der Formel (I) nach Anspruch 1, wobei:
X unter einem Halogenatom oder einer unter Cyano, Halogenalkyl, Alkoxy, Halogenalkoxy, Alkylthio, Alkylsulfinyl, Alkylsulfonyl, Halogenalkylthio, Halogenalkylsulfinyl und Halogenalkylsulfonyl ausgewählten Gruppe ausgewählt ist, wobei die Alkyl-, Halogenalkyl-, Alkoxy- und Halogenalkoxygruppen hier lineare oder verzweigte Ketten mit weniger als 10 Kohlenstoffatomen sind und die Halogensubstitution sämtlicher dieser Gruppen von der Monosubstitution bis zur vollständigen Polysubstitution reicht,
R¹, R² und R³ individuell unter den für X beschriebenen Substituenten, einem Wasserstoffatom und einer Alkylgruppe ausgewählt sind und nicht mehr als einer der Substituenten R¹, R² und R³ unter Formyl, Amino, Alkylamino und Dialkylamino ausgewählt ist, wobei die Alkylgruppen hier lineare oder verzweigte Ketten mit weniger als 10 Kohlenstoffatomen sind,
Y unter einem Halogenatom oder einer unter Cyano, Alkyl, Halogenalkyl, Alkoxy und Halogenalkoxy ausgewählten Gruppe ausgewählt ist, wobei die Alkyl-, Alkoxy-, Halogenalkyl- und Halogenalkoxygruppen hier lineare oder verzweigte Ketten mit weniger als 10 Kohlenstoffatomen sind und die Halogensubstitution sämtlicher dieser Gruppen hier die Monosubstitution ist oder bis zur vollständigen Polysubstitution reicht und
X¹, X², X³ und X⁴ das in Anspruch 1 beschriebene sind.

3. Verbindung der Formel (I) nach Anspruch 2, wobei bedeuten:
X ein Halogenatom oder eine Gruppe R⁵S(O)ₙ, wobei n 0, 1 oder 2 und R⁵ Alkyl oder Halogenalkyl ist,
R¹ ein Wasserstoffatom, ein Halogenatom oder Alkylthio,
R² Cyano,
R³ ein Wasserstoffatom oder ein Halogenatom,
Y ein Wasserstoffatom, ein Halogenatom, Halogenalkyl oder Halogenalkoxy, mit der Maßgabe, daß Y ein wie in Anspruch 1 definiertes Wasserstoffatom ist und wobei
X¹, X², X³ und X⁴ individuell unter einem Wasserstoffatom, einem Halogenatom, C₁₋₃-Alkyl, C₁₋₃-Alkoxy und C₁₋₃-Alkylthio ausgewählt sind.

4. Verbindung nach Anspruch 3 mit der Formel (II): 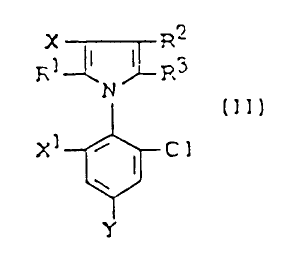 wobei bedeuten:
X R⁵S(O)ₙ, wobei n 0, 1 oder 2 und R⁵ CH₃, CF₃, CF₂Cl, CFCl₂, CF₂Br, CHF₂, CHCl₂ oder CHClF ist,
R¹ H, F, Cl oder Br,
R³ H, F, Cl oder Br,
X¹ H oder Cl, und
Y CF₃ oder CF₃O.

5. Verbindung der Formel (I) nach Anspruch 1, 2 oder 3, wobei bedeuten:
R¹ ein Wasserstoffatom oder ein Halogenatom,
R² Cyano,
X eine Halogenalkyl-S(O)ₙ-Gruppe, wobei n 0, 1 oder 2 ist,
X¹ und X⁴ kein Wasserstoffatom,
Y eine Halogenalkyl- oder Halogenalkoxygruppe und
X² und X³ ein Wasserstoffatom.

6. Verbindung nach Anspruch 2 der Formel (II): 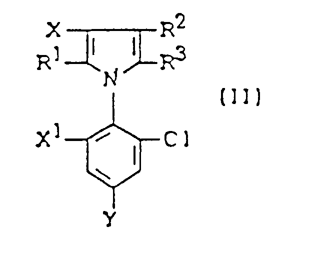 wobei bedeuten:
X R⁵S(O)ₙ, wobei n 0, 1 oder 2 und R⁵ CH₃, CF₃, CF₂Cl oder CFCl₂ ist,
R² Cyano,
R¹ H, F, Cl, Br oder NH₂,
R³ H, F, Cl, Br, CF₃ oder CN,
X¹ H oder Cl, und
Y CF₃ oder CF₃O.

7. Verbindung nach Anspruch 1, nämlich:
1-(2,6-Dichlor-4-trifluormethylphenyl)-2-chlor-3-cyano-4-(trifluormethylthio)pyrrol,
1-(2,6-Dichlor-4-trifluormethoxyphenyl)-2-chlor-3-cyano-4-(trifluormethylsulfonyl)pyrrol,
1-(2,6-Dichlor-4-trifluormethylphenyl)-2-chlor-3-cyano-4-(dichlorfluormethylthio)pyrrol,
1-(2,6-Dichlor-4-trifluormethylphenyl)-2-chlor-3-cyano-4-(dichlorfluormethylsulfinyl)pyrrol,
1-(2,6-Dichlor-4-trifluormethylphenyl)-2-chlor-3-cyano-4-(dichlorfluormethylsulfonyl)pyrrol,
1-(2,6-Dichlor-4-trifluormethylphenyl)-2-chlor-3-cyano-4-(chlordifluormethylsulfonyl)pyrrol,
1-(2,6-Dichlor-4-trifluormethylphenyl)-2-chlor-3-cyano-4-(chlordifluormethylsulfinyl)pyrrol,
1-(2,6-Dichlor-4-trifluormethylphenyl)-2-chlor-3-cyano-4-(chlordifluornethylthio)pyrrol,
1-(2,6-Dichlor-4-trifluormethylphenyl)-2-brom-3-cyano-4-(trifluormethylsulfinyl)pyrrol,
1-(2,6-Dichlor-4-trifluormethylphenyl)-2-brom-3-cyano-4-(trifluormethylsulfonyl)pyrrol,
1-(2,6-Dichlor-4-trifluormethylphenyl)-2-brom-3-cyano-4-(dichlorfluormetbylthio)pyrrol,
1-(2,6-Dichlor-4-trifluormethylphenyl)-2-brom-3-cyano-4-(dichlorfluormethylsulfonyl)pyrrol,
1-(2,6-Dichlor-4-trifluormethylphenyl)-2-brom-3-cyano-4-(dichlorfluormethylsulfinyl)pyrrol,
1-(2,6-Dichlor-4-trifluormethylphenyl)-2-brom-3-cyano-4-(chlordifluormethylthio)pyrrol,
1-(2,6-Dichlor-4-trifluormethylphenyl)-2-brom-3-cyano-4-(chlordifluormethylsulfinyl)pyrrol,
1-(2,6-Dichlor-4-trifluormethylphenyl)-2-chlor-3-cyano-4-(dichlorfluormethylsulfonyl)-5-brompyrrol,
1-(2,6-Dichlor-4-trifluormethylphenyl)-2-brom-3-cyano-4-(chlordifluormethylsulfonyl)pyrrol,
1-(2,6-Dichlor-4-trifluormethoxyphenyl)-2-chlor-3-cyano-4-(dichlorfluormethylsulfinyl)pyrrol,
1-(2,6-Dichlor-4-trifluormethoxyphenyl)-2-chlor-3-cyano-4-(dichlorfluormethylsulfonyl)pyrrol,
1-(2-Chlor-4-trifluormethylphenyl)-2-chlor-3-cyano-4-(dichlorfluormethylsulfonyl)pyrrol,
1-(2-Chlor-4-trifluormethylphenyl)-2-chlor-3-cyano-4-(dichlorfluormethylsulfinyl)pyrrol,
1-(2-Chlor-4-trifluormethylphenyl)-2-brom-3-cyano-4-(dichlorfluormethylsulfonyl)pyrrol,
1-(2,6-Dichlor-4-trifluormethylphenyl)-2-chlor-3-cyano-4-(dichlorfluormethylthio)-5-methylthiopyrrol,
1-(2,6-Dichlor-4-trifluormethylphenyl)-2-chlor-3-cyano-4-(bromdifluormethylthio)pyrrol,
1-(2,6-Dichlor-4-trifluormethylphenyl)-2-chlor-3-cyano-4-(bromdifluormethylsulfinyl)pyrrol,
1-(2,6-Dichlor-4-trifluormethylphenyl)-2-chlor-3-cyano-4-(bromdifluormethylsulfonyl)pyrrol,
1-(2,6-Dichlor-4-trifluormethylphenyl)-2-chlor-3-cyano-4-(methylsulfinyl)pyrrol,
1-(2,6-Dichlor-4-trifluormethylphenyl)-2-chlor-3-cyano-4-(methylsulfonyl)pyrrol,
1-(4-Brom-2,6-dichlorphenyl)-3-cyano-4-(chlordifluormethylthio)pyrrol,
1-(4-Brom-2,6-dichlorphenyl)-3-cyano-4-(trifluormethylthio)pyrrol,
1-(2,4,6-Trichlorphenyl)-3-cyano-4-(chlordifluormethylthio)pyrrol,
1-(2,4,6-Trichlorphenyl)-3-cyano-4-(chlordifluormethylsulfinyl)pyrrol,
1-(2,4,6-Trichlorphenyl)-3-cyano-4-(dichlorfluormethylthio)pyrrol,
1-(2,4,6-Trichlorphenyl)-3-cyano-4-(dichlorfluormethylsulfinyl)pyrrol,
1-(2,4,6-Trichlorphenyl)-3-cyano-4-(dichlorfluormethylsulfonyl)pyrrol,
1-(4-Brom-2,6-dichlorphenyl)-3-cyano-4-(dichlorfluormethylthio)pyrrol,
1-(4-Brom-2,6-dichlorphenyl)-3-cyano-4-(dichlorfluormethylsulfinyl)pyrrol,
1-(4-Brom-2,6-dichlorphenyl)-3-cyano-4-(dichlorfluormethylsulfonyl)pyrrol,
1-(2,4,6-Trichlorphenyl)-3-cyano-4-(trifluormethylthio)pyrrol,
1-(2,4,6-Trichlorphenyl)-3-cyano-4-(trifluormethylsulfinyl)pyrrol,
1-(2,4,6-Trichlorphenyl)-3-cyano-4-(trifluormethylsulfonyl)pyrrol,
1-(2,4,6-Trichlorphenyl)-3-cyano-4-(trichlormethylthio)pyrrol,
1-(2,4-Dichlorphenyl)-3-cyano-4-(dichlorfluormethylthio)pyrrol,
1-(2,4,6-Trichlorphenyl)-3-cyano-4-chlorpyrrol,
1-(2,4,6-Trichlorphenyl)-3-cyano-4-(chlordifluormethylsulfonyl)pyrrol,
1-(2,6-Dichlorphenyl)-3-cyano-4-(dichlorfluormethylthio)pyrrol,
1-(4-Brom-2,6-dichlorphenyl)-3-cyano-4-(trifluormethylsulfinyl)pyrrol,
1-(4-Brom-2,6-dichlorphenyl)-3-cyano-4-(trifluormethylsulfonyl)pyrrol,
1-(4-Brom-2,6-dichlorphenyl)-3-cyano-4-(chlordifluormethylsulfinyl)pyrrol,
1-(4-Brom-2,6-dichlorphenyl)-3-cyano-4-(chlordifluormethylsulfonyl)pyrrol,
1-(4-Brom-2,6-dimethylphenyl)-3-cyano-4-(trifluormethylsulfinyl)pyrrol,
1-(4-Brom-2,6-dimethylphenyl)-3-cyano-4-(trifluormethylsulfonyl)pyrrol oder
1-(4-Brom-2,6-difluorphenyl)-3-cyano-4-(dichlorfluormethylthio)pyrrol,
1-(2,6-Dichlor-4-trifluormethylphenyl)-2-chlor-3-cyano-4-(trifluormethylsulfonyl)pyrrol,
1-(2,6-Dichlor-4-trifluormethylphenyl)-2-chlor-3-cyano-4-(trifluormethylsulfinyl)pyrrol,
1-(2,6-Dichlor-4-trifluormethylphenyl)-2-chlor-3-cyano-4-trifluormethylsulfinyl-5-brompyrrol,
1-(2,6-Dichlor-4-trifluormethylphenyl)-2-chlor-3-cyano-4-trifluormethylsulfonyl-5-brompyrrol,
1-(2,6-Dichlor-4-trifluormethylphenyl)-2-chlor-3-cyano-4-(trifluormethylthio)pyrrol,
1-(2,6-Dichlor-4-trifluormethylphenyl)-2-brom-3-trifluormethylthio-4-cyano-5-chlorpyrrol,
1-(2,6-Dichlor-4-trifluormethylphenyl)-2-[(trifluormethyl)carbonylamino]-3-trifluormethylthio-4-cyano-5-chlorpyrrol,
1-(2,6-Dichlor-4-trifluormethylphenyl)-2-(methylcarbonylamino)-3-trifluormethylthio-4-cyano-5-chlorpyrrol,
1-(2,6-Dichlor-4-trifluormethylphenyl)-2-amino-3-trifluormethylthio-4-cyano-5-chlorpyrrol,
1-(2-Chlor-4-trifluormethylphenyl)-2-amino-3-trifluormethylthio-4-cyano-5-chlorpyrrol,
1-(2,6-Dichlor-4-trifluormethylphenyl)-2-amino-3-dichlorfluormethylthio-4-cyano-5-chlorpyrrol,
1-(2,6-Dichlor-4-trifluormethylphenyl)-2,4-bis(trifluormethylthio)-3-cyano-5-aminopyrrol,
1-(2,6-Dichlor-4-trifluormethylphenyl)-2-amino-3-trifluormethylthio-4-cyano-5-brompyrrol,
1-(2,6-Dichlor-4-trifluormethylphenyl)-2-amino-3-trifluormethylthio-4-cyanopyrrol,
1-(4-Trifluormethylphenyl)-2-amino-3-trifluormethylthio-4-cyano-5-brompyrrol,
1-(2-Chlor-trifluormethylphenyl)-2-amino-3-trifluormethylthio-4-cyano-5-brompyrrol,
1-(2,6-Dichlor-4-trifluormethylphenyl)-2-chlor-3-cyano-4-(dichlorfluormethylthio)pyrrol,
1-(2,6-Dichlor-4-trifluormethylphenyl)-2-chlor-3-cyano-4-(dichlorfluormethylsulfinyl)pyrrol,
1-(2,6-Dichlor-4-trifluormethylphenyl)-2-chlor-3-cyano-4-(dichlorfluormethylsulfonyl)pyrrol,
1-(2,6-Dichlor-4-trifluormethylphenyl)-3-cyano-4-(dichlorfluormethylthio)pyrrol,
1-(2,6-Dichlor-4-trifluormethylphenyl)-3-cyano-4-(dichlorfluormethylsulfinyl)pyrrol oder
1-(2,6-Dichlor-4-trifluormethoxyphenyl)-2-chlor-3-cyano-4-(trifluormethylsulfonyl)pyrrol.

8. Verfahren zur Herstellung von Verbindungen der Formel (I), welches umfaßt:
(A) wenn die Verbindung die Formel I(a) hat: 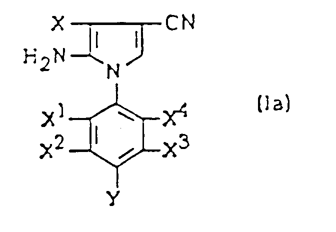 wobei X¹, X², X³, X⁴ und Y die gleiche Bedeutung wie in Anspruch 1 haben und
(A-I): X Halogen, Trifluormethyl, Cyano, Alkylthio, Alkylsulfinyl, Alkylsulfonyl, Halogenalkylthio, Halogenalkylsulfinyl oder Halogenalkylsulfonyl ist, ein Verfahren, bei dem eine Verbindung der Formel 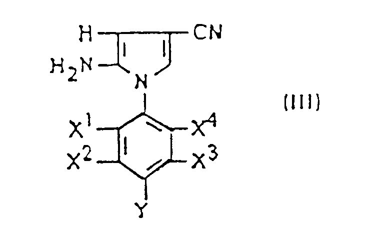 in der die verschiedenen Symbole wie in Anspruch 1 definiert sind und das Amino gegebenenfalls geschützt ist:
(a) mit einem Halogenierungsmittel, gegebenenfalls in Gegenwart eines Lösungsmittels, unter Erhalt einer Verbindung der Formel (Ia), bei der X Halogen ist, umgesetzt wird, und dann gegebenenfalls die Verbindung mit Trifluormethylkupfer auf bekannte Weise unter Erhalt von Verbindungen der Formel (Ia), bei denen X Trifluormethyl ist, umgesetzt wird,
(b) mit einem Tris(alkylthio)methan oder Tris(arylthio)methan in Gegenwart einer Lewis-Säure umgesetzt wird und dann die erhaltene Verbindung der Formel (XI): 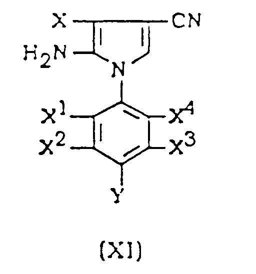 in der X Bis(alkylthio)methyl oder Bis(arylthiomethyl) ist und die anderen Symbole wie in Anspruch 1 definiert sind, gegebenenfalls mit einem Alkylnitrit umgesetzt und anschließend hydrolysiert wird, um die Verbindung der Formel (XI) zu erhalten, bei der X Formyl ist, dann diese Verbindung mit Hydroxylamin in Kontakt gebracht und anschließend auf bekannte Weise dehydratisiert wird, um die Verbindung der Formel (Ia) zu erhalten, bei der X eine Cyanogruppe ist,
(c) mit einer Verbindung der Formel MSCN, wobei M ein Alkalimetall ist, in Gegenwart von Brom in einem Lösungsmittel unter Erhalt der Verbindung der Formel (Ia), bei der X eine Thiocyanatogruppe ist, umgesetzt wird, und dann diese Verbindung mit einem Alkylhalogenid oder Dialkylsulfat in Gegenwart einer Base in einem Lösungsmittel unter Erhalt einer Verbindung der Formel (Ia), bei der X Alkylthio ist, umgesetzt wird, oder
(d) mit einem Sulfenylhalogenid der Formel RSHal, wobei R ein Alkyl-, Halogenalkyl-, Phenyl- oder Heteroaryl-Rest und Hal ein Halogenatom ist, in einem organischen flüssigen Reaktionsmedium, gegebenenfalls in Gegenwart eines Säureakzeptors, unter Erhalt einer Verbindung der Formel (Ia), bei der X Alkylthio oder Halogenalkylthio ist, umgesetzt wird und dann die erhaltene Verbindung gegebenenfalls auf bekannte Weise unter Erhalt einer Verbindung der Formel (Ia), bei der X RS(O)ₙ ist, wobei n 1 oder 2 ist, oxidiert wird, oder
(A-II) wenn X Alkoxy oder Halogenalkoxy ist, ein Verfahren, bei dem eine Verbindung der Formel: 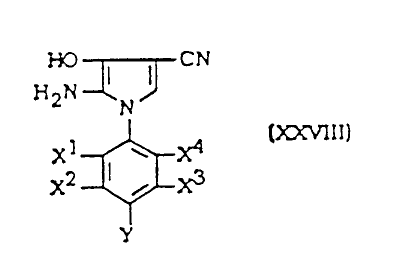 wobei die verschiedenen Symbole wie in Anspruch 1 definiert sind und die Amino- und Cyano-Gruppen geeignet geschützt sind, falls dies erforderlich ist,
(a) mit einem Alkylierungsmittel, gegebenenfalls in Gegenwart einer Base, unter Erhalt von Verbindungen der Formel (Ia), bei denen X Alkoxy ist, umgesetzt wird, oder
(b) auf bekannte Weise unter Erhalt von Verbindungen der Formel (Ia), bei denen X Halogenalkoxy ist, halogenalkyliert wird, oder
(A-III) wenn X Halogenalkyl ist, ein Verfahren, bei dem eine Verbindung der Formel: 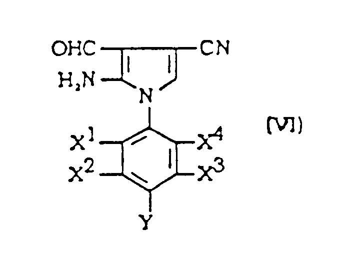 wobei die verschiedenen Symbole wie in Anspruch 1 definiert sind und das Amino und Cyano geeignet geschützt sind, falls dies erforderlich ist,
(a) mit einem Fluorierungsmittel auf bekannte Weise unter Erhalt der Verbindung der Formel (Ia), bei der X eine Difluormethylgruppe ist, umgesetzt wird,
(b) mit einem Oxidationsmittel zur Umwandlung der Gruppe -CHO in eine Carbonsäuregruppe umgesetzt wird und dann die erhaltenen Verbindungen auf bekannte Weise einem Fluorierungsmittel unter Erhalt der Verbindung der Formel (I), bei der X Trifluormethyl ist, ausgesetzt werden,
(c) in eine Verbindung umgewandelt wird, bei der die Gruppe -CHO durch eine Methylgruppe ersetzt ist, worauf die erhaltene Verbindung einem Halogenierungsmittel in einem Lösungsmittel unter Erhalt einer Verbindung der Formel (Ia), bei der X Brommethyl oder Chlormethyl ist, ausgesetzt wird, oder
(d) nacheinander mit einem Halogenalkylmetall-Derivat oder Trifluortrimethylsilan zur Umwandlung der Gruppe -CHO in Halogenalkylcarbinol umgesetzt wird und dann die Verbindung, bei der X Halogenalkylcarbinol ist, mit einem Halogenierungsmittel unter Erhalt von Verbindungen der Formel (Ia), bei denen X α-Halogenalkyl-α-halogenmethyl ist, umgesetzt wird, worauf sich sämtlichen vorgenannten Schritten ein Schritt zur Anspaltung der Schutzgruppen anschließt, wenn dies erforderlich ist, oder
(B) wenn die Verbindung der Formel (I) die Formel I(b) hat: 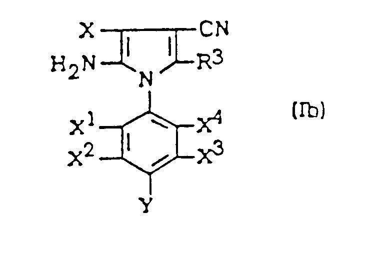 wobei X, X¹, X², X³, X⁴ und Y die gleiche Bedeutung wie in Anspruch 1 haben, und wenn (B-I) R³ Halogen, Formyl, Halogenalkyl, Alkyl, Alkylthio, Halogenalkylthio, Alkylsulfinyl, Alkylsulfonyl, Halogenalkylsulfinyl oder Halogenalkylsulfonyl ist, ein Verfahren, bei dem eine Verbindung der Formel (Ia), bei der X, Cyano und Amino gegebenenfalls geschützt sind, wenn dies erforderlich ist,
(a) gemäß dem obigen Anschnitt A-I(a) unter Erhalt von Verbindungen der Formel (Ib), bei denen R³ Halogen ist, umgesetzt wird, worauf die Verbindung dann gegebenenfalls gemäß dem obigen Abschnitt A-I(a) unter Erhalt von Verbindungen der Formel (Ib), bei denen R³ Trifluormethyl ist, umgesetzt wird,
(b) gemäß dem obigen Anschnitt A-I(b) umgesetzt wird, so daß sich zuerst eine Verbindung der Formel (Ib) ergibt, bei der R³ eine Bis(alkylthio)methylgruppe oder eine Bis(arylthio)methylgruppe ist, und dann eine Verbindung der Formel (Ib), bei der R³ Formyl ist, oder
(c) gemäß dem obigen Abschnitt A-I(c,d) unter Erhalt von Verbindungen der Formel (Ib), bei denen R³ Alkylthio der Halogenalkylthio ist, umgesetzt wird und dann gegebenenfalls gemäß dem obigen Abschnitt A-I(d) unter Erhalt von Verbindungen der Formel (Ib), bei denen R³ Alkylsulfinyl, Alkylsulfonyl, Halogenalkylsulfinyl oder Halogenalkylsulfonyl ist, oxidiert wird, mit der Maßgabe, daß X keine RS-Gruppe ist, die unerwünscht oxidiert werden kann, worauf, wenn X ein Halogen ist, gegebenenfalls mit einem Alkyllithium auf bekannte Weise behandelt und anschließend gemäß dem obigen Abschnitt A-I(c,d) wäßrig gelöscht und sulfenyliert wird, so daß sich Verbindungen der Formel (Ib) ergeben, bei denen X Alkylthio oder Halogenalkylthio ist, oder
(B-II) wenn R³ Cyano oder Alkyl ist, ein Verfahren, bei dem eine Verbindung der Formel: 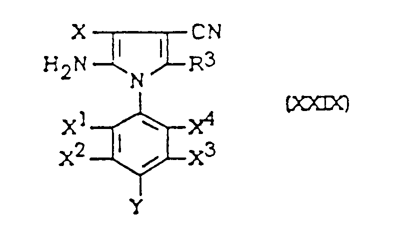 in der R³ Formyl oder Alkylcarbonyl ist, die anderen Symbole wie in Anspruch 1 definiert sind und X, Cyano und Amino geschützt sind, falls dies erforderlich ist,
(a) mit Hydroxylamin oder O-Alkylhydroxylamin oder deren Additionssalzen in einem Lösungsmittel unter Erhalt einer Verbindung der Formel (Ib), bei der R³ Hydroxyiminoalkylidenyl oder Alkoxyiminoalkylidenyl ist, kondensiert wird und die Gruppe R³ unter Erhalt einer Verbindung der Formel (Ib), bei der R³ eine Cyanogruppe ist, in Cyano umgewandelt wird,
(b) wenn R³ Formyl ist, gemäß dem obigen Abschnitt A-III(a, b, c oder d) unter Erhalt einer Verbindung der Formel (Ib), bei der R³ eine Methyl- oder Halogenalkylgruppe ist, umgesetzt wird, oder
(c) wenn R³ Formyl ist, mit einem Grignard Reagens, das von einem Alkylhalogenid stammt, oder einem Alkyllithium umgesetzt wird, so daß ein Carbinol gebildet wird, worauf sich ein Dehydratisierungsschritt zur Herstellung einer Verbindung anschließt, bei der R³ Alkenyl ist, wonach unter Erhalt von Verbindungen der Formel (Ib), bei denen R³ Alkyl ist, reduziert wird und sich gegebenenfalls ein Schritt zur Schutzgruppenabspaltung anschließt, oder
(B-III) wenn R³ Alkoxy oder Halogenalkoxy ist, ein Verfahren, bei dem eine Verbindung der Formel 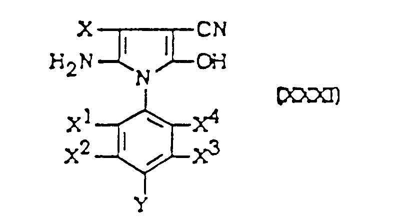 in der Amino, Cyano und X gegebenenfalls geschützt sind, gemäß dem obigen Abschnitt A-II(a, b oder c) unter Erhalt einer Verbindung der Formel (Ib), bei der R³ Alkoxy oder Halogenalkoxy ist, umgesetzt wird, oder
(C) wenn die Verbindung der Formel I die Formel (Ic) hat: 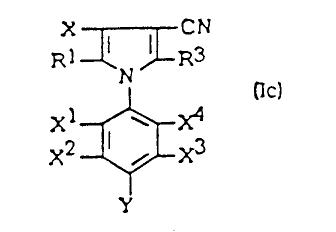 in der X, R³, X¹, X², X³, X⁴ und Y die gleichen Bedeutungen wie in Anspruch 1 haben, und wenn
(C-I) R¹ Wasserstoff, Halogen, Alkylthio, Halogenalkylthio, Alkylsulfinyl, Alkylsulfonyl, Halogenalkylsulfinyl, Halogenalkylsulfonyl, Alkylamino, Dialkylamino, Formyl, Halogenalkyl oder Alkyl ist, ein Verfahren, bei dem eine Verbindung der Formel: 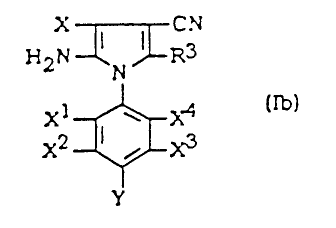 in der die Aminogruppe von der Schutzgruppe befreit wird, nachdem die X-, R³- oder Cyanogruppe geschützt worden ist, falls dies erforderlich ist,
(a) mit einem Diazotierungsmittel in einem inerten Lösungsmittel unter Erhalt einer Verbindung der Formel (Ic), bei der R¹ H ist, umgesetzt wird,
(b) mit einem Diazotierungsmittel in Gegenwart eines Halogendonors unter Erhalt einer Verbindung der Formel (Ic), bei der R¹ Halogen ist, umgesetzt wird,
(c) mit einem Diazotierungsmittel in Gegenwart von (SCN)₂ oder eines Disulfids in einem Lösungsmittel unter Erhalt von Verbindungen der Formel (Ic), bei denen R¹ Alkylthio oder Halogenalkylthio ist, umgesetzt wird, worauf dann gegebenenfalls gemäß dem obigen Abschnitt A-I(d) unter Erhalt einer Verbindung der Formel (Ic), bei der R¹ Alkylsulfinyl oder Alkylsulfonyl ist, oxidiert wird,
(d) gemäß dem nachstehenden Anspruch 9 (A) unter Erhalt von Verbindungen der Formel (Ic), bei denen R¹ Alkylamino oder Dialkylamino ist, umgesetzt wird, oder
(e) mit Natriumnitrit und Formaldoxim, Kupfersulfat und HCl auf bekannte Weise unter Erhalt von Verbindungen der Formel (Ic), bei denen R¹ Formyl ist, umgesetzt wird, dann gegebenenfalls (i) gemäß dem obigen Abschnitt B-II(a) unter Erhalt einer Verbindung der Formel (Ic), bei der R¹ Cyano ist, umgesetzt wird, oder (ii) gemäß dem obigen Abschnitt A-III(a-d) unter Erhalt einer Verbindung der Formel (Ic), bei der R¹ Halogenalkyl oder Methyl ist, umgesetzt wird, worauf sich, falls erforderlich, ein Schritt zur Schutzgruppenabspaltung anschließt, oder
(C-II) wenn R¹ Alkoxy oder Halogenalkoxy ist, ein Verfahren, bei dem eine Verbindung der Formel 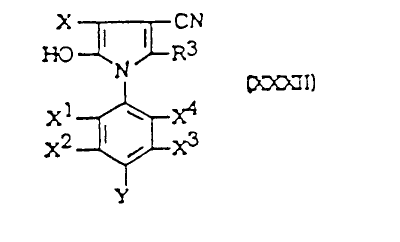 in der die verschiedenen Symbole wie in Anspruch 1 definiert sind und X, Cyano oder R³ auf bekannte Weise gegebenenfalls geschützt sind, gemäß dem obigen Abschnitt A-II(a oder b) unter Erhalt einer Verbindung der Formel (Ic), bei der R¹ Alkoxy oder Halogenalkoxy ist, umgesetzt wird, worauf sich gegebenenfalls ein Schritt zur Schutzgruppenabspaltung anschließt, oder
(D) wenn X, R¹, R³, X¹, X², X³, X⁴ und Y die gleichen Bedeutungen wie in Anspruch 1 haben und R² CHO ist, ein Verfahren, bei dem eine Verbindung der Formel (Ic) mit einem Reduktionsmittel in einem Lösungsmittel zur Umwandlung der Gruppe CN in eine Gruppe CHO und zur Bereitstellung der Verbindung, bei der R² CHO ist, behandelt wird, worauf die Verbindung gegebenenfalls auf bekannte Weise unter Erhalt der entsprechenden Verbindung der Formel (XXXV) oxidiert wird 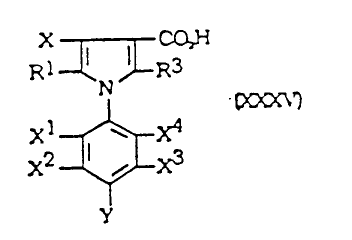 oder
(E) wenn X, R¹, R³, X¹, X², X³, X⁴ und Y die gleichen Bedeutungen wie in Anspruch 1 haben und R² Cyano, Alkyl oder Halogenalkyl ist, ein Verfahren, bei dem eine Verbindung der Formel (I), bei der R² CHO ist, nachdem X, R¹ oder R³ gegebenenfalls, wenn dies erforderlich ist, auf bekannte Weise geschützt worden sind, gemäß den obigen Abschnitten A-III(a, b, c oder d), A-II(a oder b) oder C-I umgesetzt wird, worauf sich ein Schritt zur Schutzgruppenabspaltung anschließt, wenn dies erforderlich ist, oder
(F) wenn X, R¹, R³, X¹, X², X³, X⁴ und Y die gleichen Bedeutungen wie in Anspruch 1 haben und R² Amino, Alkylamino, Dialkylamino, Wasserstoff, Halogen, Alkylthio, Halogenalkylthio, Alkylsulfinyl, Alkylsulfonyl, Halogenalkylsulfinyl, Halogenalkylsulfonyl oder Trifluormethyl ist, ein Verfahren, bei dem eine Verbindung der oben angegebenen Formel (XXXV) nachdem X, R¹ oder R³ gegebenenfalls geschützt worden sind, falls dies erforderlich ist, auf bekannte Weise unter Bedingungen für die Curtius-Umlagerung umgesetzt wird, beispielsweise durch Umwandlung in ein Säurechlorid und sich anschließende Umsetzung mit einem Alkalimetallazid, oder mit Diphenylphosphorylazid in Gegenwart einer organischen Base in einem alkoholischen Lösungsmittel zur Bildung eines Carbamats, das dann unter Erhalt der entsprechenden Verbindung, bei der R² Amino ist, hydrolysiert werden kann, welche dann gegebenenfalls gemäß dem nachstehenden Anspruch 9(A) oder dem obigen Abschnitt C-I(a-c) umgesetzt wird, worauf, wenn R² Halogen ist, gegebenenfalls gemäß dem obigen Abschnitt B-I(a) umgesetzt wird und sich ein Schritt zur Schutzgruppenabspaltung anschließt, wenn dies erforderlich ist, oder
(G) wenn X, R¹, R³, X¹, X², X³, X⁴ und Y die gleichen Bedeutungen wie in Anspruch 1 haben und R² Alkoxy oder Halogenalkoxy ist, ein Verfahren, bei dem eine Verbindung der Formel 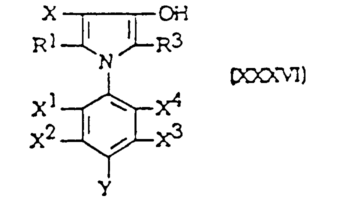 in der die verschiedenen Symbole wie in Anspruch 1 definiert sind, nachdem die Gruppen X, R¹ oder R³ gegebenenfalls geschützt worden sind, falls dies erforderlich ist, gemäß dem obigen Abschnitt B-III umgesetzt wird, worauf sich ein Schritt zur Schutzgruppenabspaltung anschließt, wenn dies erforderlich ist, oder
(H) wenn R¹, R², R³, Y, X¹, X², X³ und X⁴ wie in Anspruch 1 definiert sind und X eine Perhalogenalkylthiogruppe ist, ein Verfahren, bei dem
a) eine Verbindung der Formel (XXXVIII) 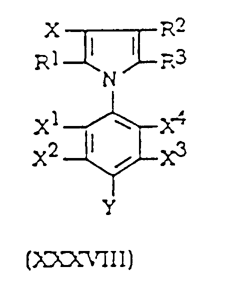 in der X Wasserstoff ist und die anderen Symbole wie in Anspruch 1 definiert sind, mit Chlorsulfonsäure (ClSO₃H) bei einer Temperatur von 0°C bis 150°C und gegebenenfalls in einem organischen Lösungsmittel zur Herstellung einer Verbindung der Formel (XXXIX): 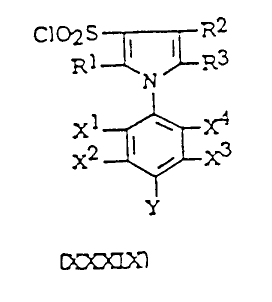 in der die verschiedenen Symbole wie in Anspruch 1 definiert sind, umgesetzt wird,
b) die Verbindung der Formel (XXXIX) mit einem Reduktionsmittel bei einer Temperatur von 0°C bis 110°C in einem organischen Lösungsmittel zu Bildung einer Disulfid-Verbindung der Formel (XLI): 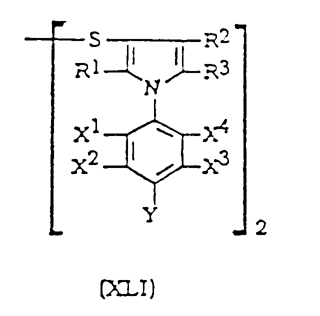 in der die verschiedenen Symbole wie in Anspruch 1 definiert sind, umgesetzt wird, und
c) die Disulfid-Verbindung der Formel (XLI) mit einem Perhalogenalkan der Formel (XLII), ZCFR⁷R⁸, wobei Z Cl, Br oder J, R⁷ F, Cl oder Br und R⁸ F, Cl, Br oder eine Perfluoralkylgruppe ist, in Gegenwart eines die Bildung von freien Radikalen fördernden Reduktionsmittel und gegebenenfalls in Gegenwart einer Base in einem organischen Lösungsmittel bei einer Temperatur von 20°C bis 85°C und gegebenenfalls unter Druck zur Herstellung einer Verbindung der Formel (I), bei der X eine Perhalogenalkylthiogruppe ist und die anderen Symbole wie in Anspruch 1 definiert sind, umgesetzt wird, oder
(I) die Umwandlung einer nach einem der obigen Schritte A-H erhaltenen Verbindung in eine Verbindung der in Anspruch 1 definierten Formel (I).

9. Verfahren zur Herstellung von Zwischenproduktverbindungen, die zur Herstellung von Verbindungen der Formel I verwendbar sind, welches umfaßt:
(A) ein Verfahren zur Herstellung von Verbindungen der Formel (XXXIV): 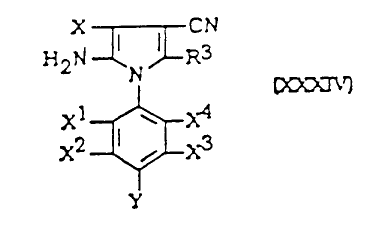 in der X, X¹, X², X³, X⁴ und Y wie in Anspruch 1 definiert sind und R³ Amino, Alkylamino oder Dialkylamino ist, bei dem eine Verbindung der Formel (XXX): 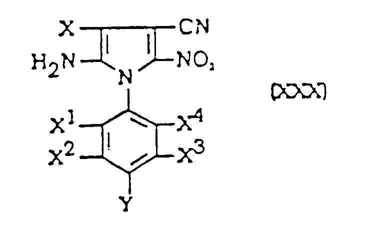 in der die verschiedenen Symbole wie in Anspruch 1 definiert sind und die Aminogruppe auf geeignete Weise geschützt ist, zum Erhalt von Verbindungen der Formel (XXXIV), bei denen R³ Amino ist, reduziert wird, worauf die Verbindungen mit einem Alkylierungsmittel in einem organischen Lösungsmittel zum Erhalt einer mono- oder disubstituierten Aminogruppe umgesetzt werden oder die Aminogruppe in Alkoxyalkylidenimino umgewandelt wird, worauf zum Erhalt von Verbindungen der Formel (XXXIV), bei denen R³ Alkylamino oder Dialkylamino ist, reduziert wird und sich ein Schritt zur Schutzgruppenabspaltung anschließt, wenn dies erforderlich ist, oder
(B) ein Verfahren zur Herstellung von Verbindungen der Formel (XXVIII), (XXXI), (XXXII) und (XXXV) gemaß Anspruch 8(A-II), (B-III), (E) oder (G), bei dem die entsprechende Verbindung gemäß Anspruch 8(A-I)(a), (B-I)(a), (C-I)(b) oder (F), nachdem die Aminogruppe, sofern sie vorhanden ist, geschützt worden ist, in ein Grignard-Reagens oder ein Lithium-Derivat umgewandelt wird, worauf mit einem Trialkylborat umgesetzt und auf bekannte Weise oxidiert wird und sich ein Schritt zur Abspaltung der Schutzgruppe anschließt, wenn dies erforderlich ist, oder
(C) ein Verfahren zur Herstellung einer Verbindung der Formel: 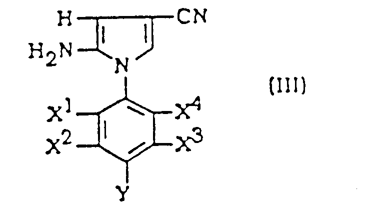 in der X¹, X², X³, X⁴ und Y die gleichen Bedeutungen haben wie in Anspruch 1, bei dem ein Dicyanopropen-Derivat der Formel: 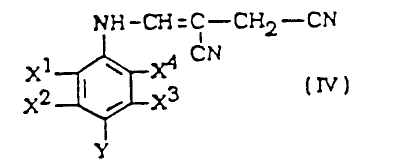 mit einem basischen Mittel umgesetzt wird.

10. Arthropodicide, pflanzennematocide, antihelmintische oder antiprotozoäre Zusammensetzung, die eine Verbindung der allgemeinen Formel (I) nach einem der Ansprüche 1 bis 7 oder die Verbindung, bei der X⁴=X¹=Y=R¹=X=R²=R³=Cl und gleichzeitig X³=X²=H ist, in Verbindung mit einem oder mehren verträglichen Verdünnungsmitteln oder Trägermitteln enthält.

11. Zusammensetzung zur Verwendung in der Veterinärmedizin und der Nutztierhaltung oder zur Aufrechterhaltung der Volksgesundheit, die als Wirkstoff mindestens eine Verbindung nach einem der Ansprüche 1 bis 7 oder die Verbindung, bei der X⁴=X¹=Y=R¹=X=R²=R³=Cl und gleichzeitig X³=X²=H ist, enthält.

12. Insecticide, acaricide oder nematocide Zusammensetzung, die eine wirksame Menge einer Verbindung der Formel (I) nach einem der Ansprüche 1 bis 7 oder der Verbindung, bei der X⁴=X¹=Y=R¹=X=R²=R³=Cl und gleichzeitig X³=X²=H ist, enthält.

13. Zusammensetzung nach Anspruch 12, die zwischen 0,05 Gew.-% und 95 Gew.-% eines oder mehrerer Wirkstoffe, zwischen 1 Gew.-% und 95 Gew.-% eines oder mehrerer landwirtschaftlich akzeptabler Träger und zwischen 0,1 Gew.-% und 50 Gew.-% eines oder mehrerer landwirtschaftlich akzeptabler grenzflächenaktiver Mittel enthält.

14. Verfahren zur Anwendung in der Veterinärmedizin und der Nutztierhaltung oder zur Aufrechterhaltung der Volksgesundheit zur Bekämpfung von Arthropoden-, Helminthen- oder Protozoenschädlingen, die bei warmblütigen Wirbeltieren innere oder äußere Parasiten sind, bei dem auf eine Örtlichkeit eine wirksame Menge einer Verbindung der Formel (I) nach einem der Ansprüche 1 bis 7 oder der Verbindung, bei X⁴=X¹=Y=R¹=X=R²=R³=Cl und gleichzeitig X³=X²=H ist, ausgebracht wird.

15. Verfahren zur Bekämpfung von Arthropoden- und Nematodenschädlingen nach Anspruch 14, wobei die Verbindung auf die Örtlichkeit, auf welcher der Arthropoden- oder der Nematodenbefall bekämpft werden soll, in einer Menge von 0,005 kg bis 15 kg der Verbindung pro ha der behandelten Örtlichkeit ausgebracht wird.

16. Verbindungen der Formeln (III), (XXVIII) bis (XXXII), (XXXIV) bis (XXXVI), (XXXVIII), (XXXIX) und (XLI), in denen die verschiedenen Substituenten die gleichen Bedeutungen wie in den vorhergehenden Ansprüchen haben, wobei Verbindungen ausgenommen sind, bei denen R¹=R²=H, R³=Amino, X=Cyano, X³=X⁴=H und: X¹=Methyl, Methoxy oder Chlor und X²=Y=H; X²=Methyl, Methoxy oder Chlor und X¹=Y=H; Y=Methyl, Methoxy oder Chlor und X¹=X²=H ist.

## Patentansprüche (Patentansprüche für folgende(n) Vertragsstaat(en): ES)

1. Verfahren zur Herstellung einer Verbindung der Formel (I): 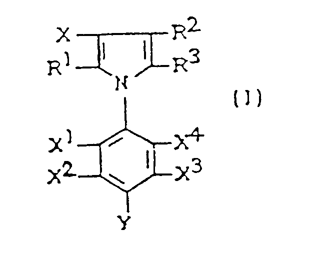 in der:
X unter Halogen, Cyano, Halogenalkyl, Alkoxy, Halogenalkoxy, Alkylthio, Alkylsulfinyl, Alkylsulfonyl, Halogenalkylthio, Halogenalkylsulfinyl und Halogenalkylsulfonyl ausgewählt ist, wobei die Alkyl-, Halogenalkyl-, Alkoxy- und Halogenalkoxygruppen hier lineare oder verzweigte Ketten mit weniger als 10 Kohlenstoffatomen sind und die Halogensubstitution sämtlicher dieser Gruppen aus einem oder mehreren Halogenatomen, die gleich oder voneinander verschieden sind, besteht und von der Monosubstitution bis zur vollständigen Polysubstitution reicht,
R¹, R² und R³ individuell unter den für X beschriebenen Substituenten, einem Wasserstoffatom und einer Alkylgruppe ausgewählt sind und nicht mehr als einer der Substituenten R¹, R² und R³ unter Formyl, Amino, Alkylamino und Dialkylamino ausgewählt ist, wobei die Alkylgruppen hier lineare oder verzweigte Ketten mit weniger als 10 Kohlenstoffatomen sind,
Y unter Halogen, Cyano, Alkyl, Halogenalkyl, Alkoxy und Halogenalkoxy ausgewählt ist, wobei die Alkyl-, Alkoxy-, Halogenalkyl- und Halogenalkoxygruppen hier lineare oder verzweigte Ketten mit weniger als 10 Kohlenstoffatomen sind und die Halogensubstitution sämtlicher dieser Gruppen hier aus einem oder mehreren Halogenatomen, die gleich oder voneinander verschieden sind, besteht und von der Monosubstitution bis zur vollständigen Polysubstitution reicht,
oder Y ein Wasserstoffatom ist, wenn
X ein Halogenatom oder eine Gruppe R⁵S(O)ₙ ist, wobei n 0, 1 oder 2 und R⁵ Alkyl oder Halogenalkyl ist und die Alkylkohlenstoffketten und die Halogensubstitution wie oben definiert sind,
R¹ und R³ jeweils ein Wasserstoffatom sind und
R² Cyano ist,
X¹, X², X³ und X⁴ individuell unter den für Y beschriebenen Substituenten oder einem Wasserstoffatom ausgewählt sind, mit den folgenden Maßgaben: mindestens einer der Substituenten R¹, R² und R³ ist unter den für X beschriebenen Substituenten ausgewählt,
wenn X⁴ und X¹ H sind und X Halogen oder Cyano ist, unterscheidet sich R² von X und
wenn X⁴ und X¹ H sind und Y Methyl ist, ist X nicht Brom, wobei die Verbindung ausgenommen ist, bei der X⁴ = X¹ = Y = R¹ = X = R² = R³ = Cl und gleichzeitig X² = X³ = H ist, welches umfaßt:
(A) wenn die Verbindung die Formel I(a) hat: 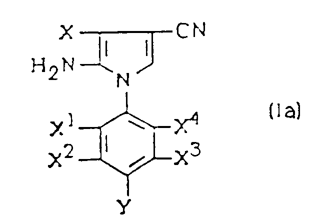 wobei X¹, X², X³, X⁴ und Y wie oben definiert sind und
(A-I): X Halogen, Trifluormethyl, Cyano, Alkylthio, Alkylsulfinyl, Alkylsulfonyl, Halogenalkylthio, Halogenalkylsulfinyl oder Halogenalkylsulfonyl ist, ein Verfahren, bei dem eine Verbindung der Formel 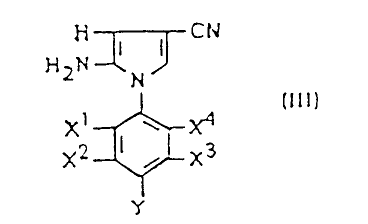 in der die verschiedenen Symbole wie oben definiert sind und das Amino gegebenenfalls geschützt ist:
(a) mit einem Halogenierungsmittel, gegebenenfalls in Gegenwart eines Lösungsmittels, unter Erhalt einer Verbindung der Formel (Ia), bei der X Halogen ist, umgesetzt wird, und dann gegebenenfalls die Verbindung mit Trifluormethylkupfer auf bekannte Weise unter Erhalt von Verbindungen der Formel (Ia), bei denen X Trifluormethyl ist, umgesetzt wird,
(b) mit einem Tris(alkylthio)methan oder Tris(arylthio)methan in Gegenwart einer Lewis-Säure umgesetzt wird und dann die erhaltene Verbindung der Formel (XI): 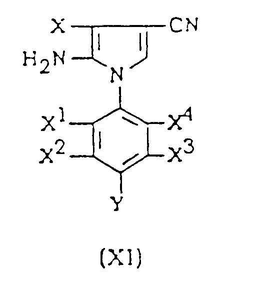 in der X Bis(alkylthio)methyl oder Bis(arylthiomethyl) ist und die anderen Symbole wie oben definiert sind, gegebenenfalls mit einem Alkylnitrit umgesetzt und anschließend hydrolysiert wird, um die Verbindung der Formel (XI) zu erhalten, bei der X Formyl ist, dann diese Verbindung mit Hydroxylamin in Kontakt gebracht und anschließend auf bekannte Weise dehydratisiert wird, um die Verbindung der Formel (Ia) zu erhalten, bei der X eine Cyanogruppe ist,
(c) mit einer Verbindung der Formel MSCN, wobei M ein Alkalimetall ist, in Gegenwart von Brom in einem Lösungsmittel unter Erhalt der Verbindung der Formel (Ia), bei der X eine Thiocyanatogruppe ist, umgesetzt wird, und dann diese Verbindung mit einem Alkylhalogenid oder Dialkylsulfat in Gegenwart einer Base in einem Lösungsmittel unter Erhalt einer Verbindung der Formel (Ia), bei der X Alkylthio ist, umgesetzt wird, oder
(d) mit einem Sulfenylhalogenid der Formel RSHal, wobei R ein Alkyl-, Halogenalkyl-, Phenyl- oder Heteroaryl-Rest und Hal ein Halogenatom ist, in einem organischen flüssigen Reaktionsmedium, gegebenenfalls in Gegenwart eines Säureakzeptors, unter Erhalt einer Verbindung der Formel (Ia), bei der X Alkylthio oder Halogenalkylthio ist, umgesetzt wird und dann die erhaltene Verbindung gegebenenfalls auf bekannte Weise unter Erhalt einer Verbindung der Formel (Ia), bei der X RS(O)ₙ ist, wobei n 1 oder 2 ist, oxidiert wird, oder
(A-II) wenn X Alkoxy oder Halogenalkoxy ist, ein Verfahren, bei dem eine Verbindung der Formel: 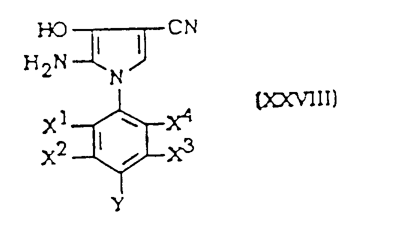 wobei die verschiedenen Symbole wie oben definiert sind und die Amino- und Cyano-Gruppen geeignet geschützt sind, falls dies erforderlich ist,
(a) mit einem Alkylierungsmittel, gegebenenfalls in Gegenwart einer Base, unter Erhalt von Verbindungen der Formel (Ia), bei denen X Alkoxy ist, umgesetzt wird, oder
(b) auf bekannte Weise unter Erhalt von Verbindungen der Formel (Ia), bei denen X Halogenalkoxy ist, halogenalkyliert wird, oder
(A-III) wenn X Halogenalkyl ist, ein Verfahren, bei dem eine Verbindung der Formel: 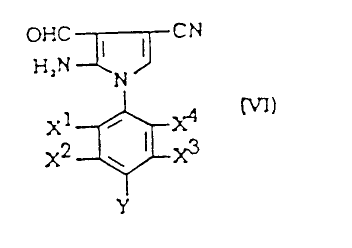 wobei die verschiedenen Symbole wie oben definiert sind und das Amino und Cyano geeignet geschützt sind, falls dies erforderlich ist,
(a) mit einem Fluorierungsmittel auf bekannte Weise unter Erhalt der Verbindung der Formel (Ia), bei der X eine Difluormethylgruppe ist, umgesetzt wird,
(b) mit einem Oxidationsmittel zur Umwandlung der Gruppe -CHO in eine Carbonsäuregruppe umgesetzt wird und dann die erhaltenen Verbindungen auf bekannte Weise einem Fluorierungsmittel unter Erhalt der Verbindung der Formel (I), bei der X Trifluormethyl ist, ausgesetzt werden,
(c) in eine Verbindung umgewandelt wird, bei der die Gruppe -CHO durch eine Methylgruppe ersetzt ist, worauf die erhaltene Verbindung einem Halogenierungsmittel in einem Lösungsmittel unter Erhalt einer Verbindung der Formel (Ia), bei der X Brommethyl oder Chlormethyl ist, ausgesetzt wird, oder
(d) nacheinander mit einem Halogenalkylmetall-Derivat oder Trifluortrimethylsilan zur Umwandlung der Gruppe -CHO in Halogenalkylcarbinol umgesetzt wird und dann die Verbindung, bei der X Halogenalkylcarbinol ist, mit einem Halogenierungsmittel unter Erhalt von Verbindungen der Formel (Ia), bei denen X α-Halogenalkyl-α-halogenmethyl ist, umgesetzt wird, worauf sich sämtlichen vorgenannten Schritten ein Schritt zur Abspaltung der Schutzgruppen anschließt, wenn dies erforderlich ist, oder
(B) wenn die Verbindung der Formel (I) die Formel I(b) hat: 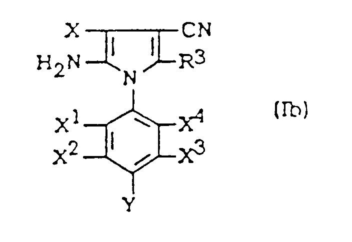 wobei X, X¹, X², X³, X⁴ und Y wie oben definiert sind, und wenn (B-I) R³ Halogen, Formyl, Halogenalkyl, Alkyl, Alkylthio, Halogenalkylthio, Alkylsulfinyl, Alkylsulfonyl, Halogenalkylsulfinyl oder Halogenalkylsulfonyl ist, ein Verfahren, bei dem eine Verbindung der Formel (Ia), bei der X, Cyano und Amino gegebenenfalls geschützt sind, wenn dies erforderlich ist,
(a) gemäß dem obigen Abschnitt A-I(a) unter Erhalt von Verbindungen der Formel (Ib), bei denen R³ Halogen ist, umgesetzt wird, worauf die Verbindung dann gegebenenfalls gemäß dem obigen Abschnitt A-I(a) unter Erhalt von Verbindungen der Formel (Ib), bei denen R³ Trifluormethyl ist, umgesetzt wird,
(b) gemäß dem obigen Abschnitt A-I(b) umgesetzt wird, so daß sich zuerst eine Verbindung der Formel (Ib) ergibt, bei der R³ eine Bis(alkylthio)methylgruppe oder eine Bis(arylthio)methylgruppe ist, und dann eine Verbindung der Formel (Ib), bei der R³ Formyl ist, oder
(c) gemäß dem obigen Abschnitt A-I(c,d) unter Erhalt von Verbindungen der Formel (Ib), bei denen R³ Alkylthio der Halogenalkylthio ist, umgesetzt wird und dann gegebenenfalls gemäß dem obigen Abschnitt A-I(d) unter Erhalt von Verbindungen der Formel (Ib), bei denen R³ Alkylsulfinyl, Alkylsulfonyl, Halogenalkylsulfinyl oder Halogenalkylsulfonyl ist, oxidiert wird, mit der Maßgabe, daß X keine RS-Gruppe ist, die unerwünscht oxidiert werden kann, worauf, wenn X ein Halogen ist, gegebenenfalls mit einem Alkyllithium auf bekannte Weise behandelt und anschließend gemäß dem obigen Abschnitt A-I(c,d) wäßrig gelöscht und sulfenyliert wird, so daß sich Verbindungen der Formel (Ib) ergeben, bei denen X Alkylthio oder Halogenalkylthio ist, oder
(B-II) wenn R³ Cyano oder Alkyl ist, ein Verfahren, bei dem eine Verbindung der Formel: 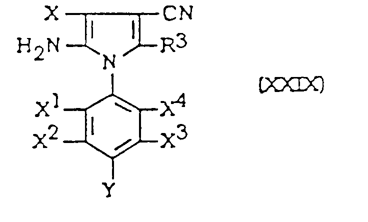 in der R³ Formyl oder Alkylcarbonyl ist, die anderen Symbole wie oben definiert sind und X, Cyano und Amino geschützt sind, falls dies erforderlich ist,
(a) mit Hydroxylamin oder O-Alkylhydroxylamin oder deren Additionssalzen in einem Lösungsmittel unter Erhalt einer Verbindung der Formel (Ib), bei der R³ Hydroxyiminoalkylidenyl oder Alkoxyiminoalkylidenyl ist, kondensiert wird und die Gruppe R³ unter Erhalt einer Verbindung der Formel (Ib), bei der R³ eine Cyanogruppe ist, in Cyano umgewandelt wird,
(b) wenn R³ Formyl ist, gemäß dem obigen Abschnitt A-III(a, b, c oder d) unter Erhalt einer Verbindung der Formel (Ib), bei der R³ eine Methyl- oder Halogenalkylgruppe ist, umgesetzt wird, oder
(c) wenn R³ Formyl ist, mit einem Grignard Reagens, das von einem Alkylhalogenid stammt, oder einem Alkyllithium umgesetzt wird, so daß ein Carbinol gebildet wird, worauf sich ein Dehydratisierungsschritt zur Herstellung einer Verbindung anschließt, bei der R³ Alkenyl ist, wonach unter Erhalt von Verbindungen der Formel (Ib), bei denen R³ Alkyl ist, reduziert wird und sich gegebenenfalls ein Schritt zur Schutzgruppenabspaltung anschließt, oder
(B-III) wenn R³ Alkoxy oder Halogenalkoxy ist, ein Verfahren, bei dem eine Verbindung der Formel 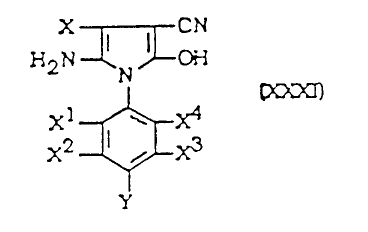 in der Amino, Cyano und X gegebenenfalls geschützt sind, gemäß dem obigen Abschnitt A-II(a, b oder c) unter Erhalt einer Verbindung der Formel (Ib), bei der R³ Alkoxy oder Halogenalkoxy ist, umgesetzt wird, oder
(C) wenn die Verbindung der Formel I die Formel (Ic) hat: 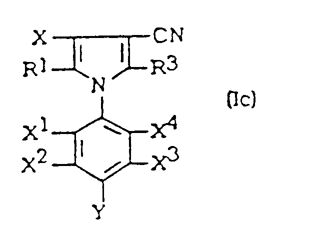 in der X, R³, X¹, X², X³, X⁴ und Y wie oben definiert sind, und wenn
(C-I) R¹ Wasserstoff, Halogen, Alkylthio, Halogenalkylthio, Alkylsulfinyl, Alkylsulfonyl, Halogenalkylsulfinyl, Halogenalkylsulfonyl, Alkylamino, Dialkylamino, Formyl, Halogenalkyl oder Alkyl ist, ein Verfahren, bei dem eine Verbindung der Formel: 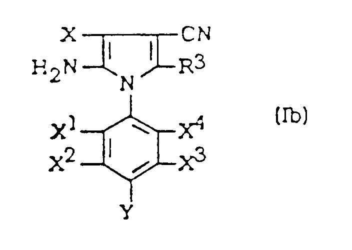 in der die Aminogruppe von der Schutzgruppe befreit wird, nachdem die X-, R³- oder Cyanogruppe geschützt worden ist, falls dies erforderlich ist,
(a) mit einem Diazotierungsmittel in einem inerten Lösungsmittel unter Erhalt einer Verbindung der Formel (Ic), bei der R¹ H ist, umgesetzt wird,
(b) mit einem Diazotierungsmittel in Gegenwart eines Halogendonors unter Erhalt einer Verbindung der Formel (Ic), bei der R¹ Halogen ist, umgesetzt wird,
(c) mit einem Diazotierungsmittel in Gegenwart von (SCN)₂ oder eines Disulfids in einem Lösungsmittel unter Erhalt von Verbindungen der Formel (Ic), bei denen R¹ Alkylthio oder Halogenalkylthio ist, umgesetzt wird, worauf dann gegebenenfalls gemäß dem obigen Abschnitt A-I(d) unter Erhalt einer Verbindung der Formel (Ic), bei der R¹ Alkylsulfinyl oder Alkylsulfonyl ist, oxidiert wird,
(d) gemäß dem nachstehenden Anspruch 8 (A) unter Erhalt von Verbindungen der Formel (Ic), bei denen R¹ Alkylamino oder Dialkylamino ist, umgesetzt wird, oder
(e) mit Natriumnitrit und Formaldoxim, Kupfersulfat und HCl auf bekannte Weise unter Erhalt von Verbindungen der Formel (Ic), bei denen R¹ Formyl ist, umgesetzt wird, dann gegebenenfalls (i) gemäß dem obigen Abschnitt B-II(a) unter Erhalt einer Verbindung der Formel (Ic), bei der R¹ Cyano ist, umgesetzt wird, oder (ii) gemäß dem obigen Abschnitt A-III(a-d) unter Erhalt einer Verbindung der Formel (Ic), bei der R¹ Halogenalkyl oder Methyl ist, umgesetzt wird, worauf sich, falls erforderlich, ein Schritt zur Schutzgruppenabspaltung anschließt, oder
(C-II) wenn R¹ Alkoxy oder Halogenalkoxy ist, ein Verfahren, bei dem eine Verbindung der Formel 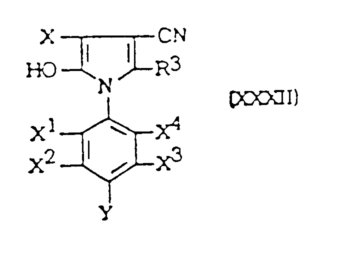 in der die verschiedenen Symbole wie oben definiert sind und X, Cyano oder R³ auf bekannte Weise gegebenenfalls geschützt sind, gemäß dem obigen Abschnitt A-III(a oder b) unter Erhalt einer Verbindung der Formel (Ic), bei der R¹ Alkoxy oder Halogenalkoxy ist, umgesetzt wird, worauf sich gegebenenfalls ein Schritt zur Schutzgruppenabspaltung anschließt, oder
(D) wenn X, R¹, R³, X¹, X², X³, X⁴ und Y wie oben definiert sind und R² CHO ist, ein Verfahren, bei dem eine Verbindung der Formel (Ic) mit einem Reduktionsmittel in einem Lösungsmittel zur Umwandlung der Gruppe CN in eine Gruppe CHO und zur Bereitstellung der Verbindung, bei der R² CHO ist, behandelt wird, worauf die Verbindung gegebenenfalls auf bekannte Weise unter Erhalt der entsprechenden Verbindung der Formel (XXXV) oxidiert wird 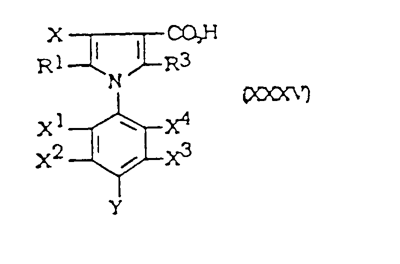 oder
(E) wenn X, R¹, R³, X¹, X², X³, X⁴ und Y wie oben definiert sind und R² Cyano, Alkyl oder Halogenalkyl ist, ein Verfahren, bei dem eine Verbindung der Formel (I), bei der R² CHO ist, nachdem X, R¹ oder R³ gegebenenfalls, wenn dies erforderlich ist, auf bekannte Weise geschützt worden sind, gemäß den obigen Abschnitten A-III(a, b, c oder d), A-II(a oder b) oder C-I umgesetzt wird, worauf sich ein Schritt zur Schutzgruppenabspaltung anschließt, wenn dies erforderlich ist, oder
(F) wenn X, R¹, R³, X¹, X², X³, X⁴ und Y wie oben definiert sind und R² Amino, Alkylamino, Dialkylamino, Wasserstoff, Halogen, Alkylthio, Halogenalkylthio, Alkylsulfinyl, Alkylsulfonyl, Halogenalkylsulfinyl, Halogenalkylsulfonyl oder Trifluormethyl ist, ein Verfahren, bei dem eine Verbindung der oben angegebenen Formel (XXXV) nachdem X, R¹ oder R³ gegebenenfalls geschützt worden sind, falls dies erforderlich ist, auf bekannte Weise unter Bedingungen für die Curtius-Umlagerung umgesetzt wird, beispielsweise durch Umwandlung in ein Säurechlorid und sich anschließende Umsetzung mit einem Alkalimetallazid, oder mit Diphenylphosphorylazid in Gegenwart einer organischen Base in einem alkoholischen Lösungsmittel zur Bildung eines Carbamats, das dann unter Erhalt der entsprechenden Verbindung, bei der R² Amino ist, hydrolysiert werden kann, welche dann gegebenenfalls gemäß dem nachstehenden Anspruch 8(A) oder dem obigen Abschnitt C-I(a-c) umgesetzt wird, worauf, wenn R² Halogen ist, gegebenenfalls gemäß dem obigen Abschnitt B-I(a) umgesetzt wird und sich ein Schritt zur Schutzgruppenabspaltung anschließt, wenn dies erforderlich ist, oder
(G) wenn X, R¹, R³, X¹, X², X³, X⁴ und Y wie oben definiert sind und R² Alkoxy oder Halogenalkoxy ist, ein Verfahren, bei dem eine Verbindung der Formel 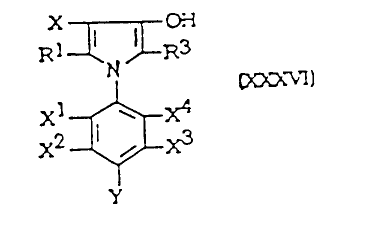 in der die verschiedenen Symbole wie oben definiert sind, nachdem die Gruppen X, R¹ oder R³ gegebenenfalls geschützt worden sind, falls dies erforderlich ist, gemäß dem obigen Abschnitt B-III umgesetzt wird, worauf sich ein Schritt zur Schutzgruppenabspaltung anschließt, wenn dies erforderlich ist, oder
(H) wenn R¹, R², R³, Y, X¹, X², X³ und X⁴ wie oben definiert sind und X eine Perhalogenalkylthiogruppe ist, ein Verfahren, bei dem
a) eine Verbindung der Formel (XXXVIII) 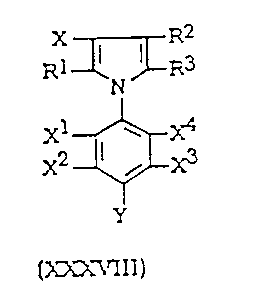 in der X Wasserstoff ist und die anderen Symbole wie oben definiert sind, mit Chlorsulfonsäure (ClSO₃H) bei einer Temperatur von 0°C bis 150°C und gegebenenfalls in einem organischen Lösungsmittel zur Herstellung einer Verbindung der Formel (XXXIX): 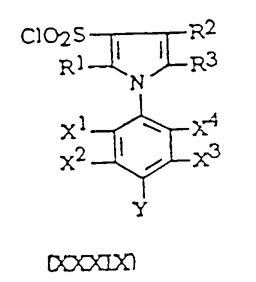 in der die verschiedenen Symbole wie oben definiert sind, umgesetzt wird,
b) die Verbindung der Formel (XXXIX) mit einem Reduktionsmittel bei einer Temperatur von 0°C bis 110°C in einem organischen Lösungsmittel zu Bildung einer Disulfid-Verbindung der Formel (XLI): 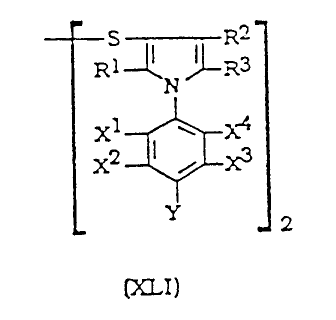 in der die verschiedenen Symbole wie oben definiert sind, umgesetzt wird, und
c) die Disulfid-Verbindung der Formel (XLI) mit einem Perhalogenalkan der Formel (XLII), ZCFR⁷R⁸, wobei Z Cl, Br oder J, R⁷ F, Cl oder Br und R⁸ F, Cl, Br oder eine Perfluoralkylgruppe ist, in Gegenwart eines die Bildung von freien Radikalen fördernden Reduktionsmittel und gegebenenfalls in Gegenwart einer Base in einem organischen Lösungsmittel bei einer Temperatur von 20°C bis 85°C und gegebenenfalls unter Druck zur Herstellung einer Verbindung der Formel (I), bei der X eine Perhalogenalkylthiogruppe ist und die anderen Symbole wie oben definiert sind, umgesetzt wird, oder
(I) die Umwandlung einer nach einem der obigen Schritte A-H erhaltenen Verbindung in eine Verbindung der oben definierten Formel (I).

2. Verfahren nach Anspruch 1, wobei:
X unter einem Halogenatom oder einer unter Cyano, Halogenalkyl, Alkoxy, Halogenalkoxy, Alkylthio, Alkylsulfinyl, Alkylsulfonyl, Halogenalkylthio, Halogenalkylsulfinyl und Halogenalkylsulfonyl ausgewählten Gruppe ausgewählt ist, wobei die Alkyl-, Halogenalkyl-, Alkoxy- und Halogenalkoxygruppen hier lineare oder verzweigte Ketten mit weniger als 10 Kohlenstoffatomen sind und die Halogensubstitution sämtlicher dieser Gruppen von der Monosubstitution bis zur vollständigen Polysubstitution reicht,
R¹, R² und R³ individuell unter den für X beschriebenen Substituenten, einem Wasserstoffatom und einer Alkylgruppe ausgewählt sind und nicht mehr als einer der Substituenten R¹, R² und R³ unter Formyl, Amino, Alkylamino und Dialkylamino ausgewählt ist, wobei die Alkylgruppen hier lineare oder verzweigte Ketten mit weniger als 10 Kohlenstoffatomen sind,
Y unter einem Halogenatom oder einer unter Cyano, Alkyl, Halogenalkyl, Alkoxy und Halogenalkoxy ausgewählten Gruppe ausgewählt ist, wobei die Alkyl-, Alkoxy-, Halogenalkyl- und Halogenalkoxygruppen hier lineare oder verzweigte Ketten mit weniger als 10 Kohlenstoffatomen sind und die Halogensubstitution sämtlicher dieser Gruppen hier die Monosubstitution ist oder bis zur vollständigen Polysubstitution reicht und
X¹, X², X³ und X⁴ das in Anspruch 1 beschriebene sind.

3. Verfahren nach Anspruch 2, wobei bedeuten:
X ein Halogenatom oder eine Gruppe R⁵S(O)ₙ, wobei n 0, 1 oder 2 und R⁵ Alkyl oder Halogenalkyl ist,
R¹ ein Wasserstoffatom, ein Halogenatom oder Alkylthio,
R² Cyano,
R³ ein Wasserstoffatom oder ein Halogenatom,
Y ein Wasserstoffatom, ein Halogenatom, Halogenalkyl oder Halogenalkoxy, mit der Maßgabe, daß Y ein wie in Anspruch 1 definiertes Wasserstoffatom ist und wobei
X¹, X², X³ und X⁴ individuell unter einem Wasserstoffatom, einem Halogenatom, C₁₋₃-Alkyl, C₁₋₃-Alkoxy und C₁₋₃-Alkylthio ausgewählt sind.

4. Verfahren nach Anspruch 3, wobei die Verbindung der Formel (I) die Formel (II) hat: 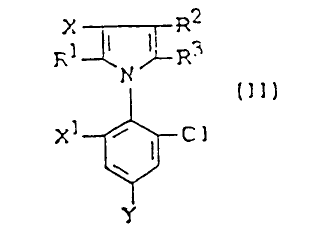 wobei bedeuten:
X R⁵S(O)ₙ, wobei n 0, 1 oder 2 und R⁵ CH₃, CF₃, CF₂Cl, CFCl₂, CF₂Br, CHF₂, CHCl₂ oder CHClF ist,
R¹ H, F, Cl oder Br,
R³ H, F, Cl oder Br,
X¹ H oder Cl, und
Y CF₃ oder CF₃O.

5. Verfahren nach Anspruch 1, 2 oder 3, wobei bedeuten:
R¹ ein Wasserstoffatom oder ein Halogenatom,
R² Cyano,
X eine Halogenalkyl-S(O)ₙ-Gruppe, wobei n 0, 1 oder 2 ist,
X¹ und X⁴ kein Wasserstoffatom,
Y eine Halogenalkyl- oder Halogenalkoxygruppe und
X² und X³ ein Wasserstoffatom.

6. Verfahren nach Anspruch 2, wobei die Verbindung der Formel (I) die Formel (II) hat: 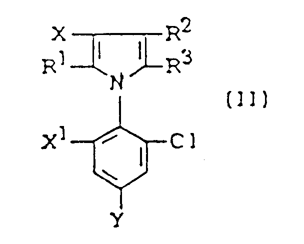 wobei bedeuten:
X R⁵S(O)ₙ, wobei n 0, 1 oder 2 und R⁵ CH₃, CF₃, CF₂Cl oder CFCl₂ ist,
R² Cyano,
R¹ H, F, Cl, Br oder NH₂,
R³ H, F, Cl, Br, CF₃ oder CN,
X¹ H oder Cl, und
Y CF₃ oder CF₃O.

7. Verfahren nach Anspruch 1, wobei die Verbindung der Formel (I) ist:
1-(2,6-Dichlor-4-trifluormethylphenyl)-2-chlor-3-cyano-4-(trifluormethylthio)pyrrol,
1-(2,6-Dichlor-4-trifluormethoxyphenyl)-2-chlor-3-cyano-4-(trifluormethylsulfonyl)pyrrol,
1-(2,6-Dichlor-4-trifluormethylphenyl)-2-chlor-3-cyano-4-(dichlorfluormethylthio)pyrrol,
1-(2,6-Dichlor-4-trifluormethylphenyl)-2-chlor-3-cyano-4-(dichlorfluormethylsulfinyl)pyrrol,
1-(2,6-Dichlor-4-trifluormethylphenyl)-2-chlor-3-cyano-4-(dichlorfluormethylsulfonyl)pyrrol,
1-(2,6-Dichlor-4-trifluormethylphenyl)-2-chlor-3-cyano-4-(chlordifluormethylsulfonyl)pyrrol,
1-(2,6-Dichlor-4-trifluormethylphenyl)-2-chlor-3-cyano-4-(chlordifluormethylsulfinyl)pyrrol,
1-(2,6-Dichlor-4-trifluormethylphenyl)-2-chlor-3-cyano-4-(chlordifluormethylthio)pyrrol,
1-(2,6-Dichlor-4-trifluormethylphenyl)-2-brom-3-cyano-4-(trifluormethylsulfinyl)pyrrol,
1-(2,6-Dichlor-4-trifluormethylphenyl)-2-brom-3-cyano-4-(trifluormethylsulfonyl)pyrrol,
1-(2,6-Dichlor-4-trifluormethylphenyl)-2-brom-3-cyano-4-(dichlorfluormethylthio)pyrrol,
1-(2,6-Dichlor-4-trifluormethylphenyl)-2-brom-3-cyano-4-(dichlorfluormethylsulfonyl)pyrrol,
1-(2,6-Dichlor-4-trifluormethylphenyl)-2-brom-3-cyano-4-(dichlorfluormethylsulfinyl)pyrrol,
1-(2,6-Dichlor-4-trifluormethylphenyl)-2-brom-3-cyano-4-(chlordifluormethylthio)pyrrol,
1-(2,6-Dichlor-4-trifluormethylphenyl)-2-brom-3-cyano-4-(chlordifluormethylsulfinyl)pyrrol,
1-(2,6-Dichlor-4-trifluormethylphenyl)-2-chlor-3-cyano-4-(dichlorfluormethylsulfonyl)-5-brompyrrol,
1-(2,6-Dichlor-4-trifluormethylphenyl)-2-brom-3-cyano-4-(chlordifluormethylsulfonyl)pyrrol,
1-(2,6-Dichlor-4-trifluormethoxyphenyl)-2-chlor-3-cyano-4-(dichlorfluormethylsulfinyl)pyrrol,
1-(2,6-Dichlor-4-trifluormethoxyphenyl)-2-chlor-3-cyano-4-(dichlorfluormethylsulfonyl)pyrrol,
1-(2-Chlor-4-trifluormethylphenyl)-2-chlor-3-cyano-4-(dichlorfluormethylsulfonyl)pyrrol,
1-(2-Chlor-4-trifluormethylphenyl)-2-chlor-3-cyano-4-(dichlorfluormethylsulfinyl)pyrrol,
1-(2-Chlor-4-trifluormethylphenyl)-2-brom-3-cyano-4-(dichlorfluormethylsulfonyl)pyrrol,
1-(2,6-Dichlor-4-trifluormethylphenyl)-2-chlor-3-cyano-4-(dichlorfluormethylthio)-5-methylthiopyrrol,
1-(2,6-Dichlor-4-trifluormethylphenyl)-2-chlor-3-cyano-4-(bromdifluormethylthio)pyrrol,
1-(2,6-Dichlor-4-trifluormethylphenyl)-2-chlor-3-cyano-4-(bromdifluormethylsulfinyl)pyrrol,
1-(2,6-Dichlor-4-trifluormethylphenyl)-2-chlor-3-cyano-4-(bromdifluormethylsulfonyl)pyrrol,
1-(2,6-Dichlor-4-trifluormethylphenyl)-2-chlor-3-cyano-4-(methylsulfinyl)pyrrol,
1-(2,6-Dichlor-4-trifluormethylphenyl)-2-chlor-3-cyano-4-(methylsulfonyl)pyrrol,
1-(4-Brom-2,6-dichlorphenyl)-3-cyano-4-(chlordifluormethylthio)pyrrol,
1-(4-Brom-2,6-dichlorphenyl)-3-cyano-4-(trifluormethylthio)pyrrol,
1-(2,4,6-Trichlorphenyl)-3-cyano-4-(chlordifluormethylthio)pyrrol,
1-(2,4,6-Trichlorphenyl)-3-cyano-4-(chlordifluormethylsulfinyl)pyrrol,
1-(2,4,6-Trichlorphenyl)-3-cyano-4-(dichlorfluormethylthio)pyrrol,
1-(2,4,6-Trichlorphenyl)-3-cyano-4-(dichlorfluormethylsulfinyl)pyrrol,
1-(2,4,6-Trichlorphenyl)-3-cyano-4-(dichlorfluormethylsulfonyl)pyrrol,
1-(4-Brom-2,6-dichlorphenyl)-3-cyano-4-(dichlorfluormethylthio)pyrrol,
1-(4-Brom-2,6-dichlorphenyl)-3-cyano-4-(dichlorfluormethylsulfinyl)pyrrol,
1-(4-Brom-2,6-dichlorphenyl)-3-cyano-4-(dichlorfluormethylsulfonyl)pyrrol,
1-(2,4,6-Trichlorphenyl)-3-cyano-4-(trifluormethylthio)pyrrol,
1-(2,4,6-Trichlorphenyl)-3-cyano-4-(trifluormethylsulfinyl)pyrrol,
1-(2,4,6-Trichlorphenyl)-3-cyano-4-(trifluormethylsulfonyl)pyrrol,
1-(2,4,6-Trichlorphenyl)-3-cyano-4-(trichlormethylthio)pyrrol,
1-(2,4-Dichlorphenyl)-3-cyano-4-(dichlorfluormethylthio)pyrrol,
1-(2,4,6-Trichlorphenyl)-3-cyano-4-chlorpyrrol,
1-(2,4,6-Trichlorphenyl)-3-cyano-4-(chlordifluormethylsulfonyl)pyrrol,
1-(2,6-Dichlorphenyl)-3-cyano-4-(dichlorfluormethylthio)pyrrol,
1-(4-Brom-2,6-dichlorphenyl)-3-cyano-4-(trifluormethylsulfinyl)pyrrol,
1-(4-Brom-2,6-dichlorphenyl)-3-cyano-4-(trifluormethylsulfonyl)pyrrol,
1-(4-Brom-2,6-dichlorphenyl)-3-cyano-4-(chlordifluormethylsulfinyl)pyrrol,
1-(4-Brom-2,6-dichlorphenyl)-3-cyano-4-(chlordifluormethylsulfonyl)pyrrol,
1-(4-Brom-2,6-dimethylphenyl)-3-cyano-4-(trifluormethylsulfinyl)pyrrol,
1-(4-Brom-2,6-dimethylphenyl)-3-cyano-4-(trifluormethylsulfonyl)pyrrol oder
1-(4-Brom-2,6-difluorphenyl)-3-cyano-4-(dichlorfluormethylthio)pyrrol,
1-(2,6-Dichlor-4-trifluormethylphenyl)-2-chlor-3-cyano-4-(trifluormethylsulfonyl)pyrrol,
1-(2,6-Dichlor-4-trifluormethylphenyl)-2-chlor-3-cyano-4-(trifluormethylsulfinyl)pyrrol,
1-(2,6-Dichlor-4-trifluormethylphenyl)-2-chlor-3-cyano-4-trifluormethylsulfinyl-5-brompyrrol,
1-(2,6-Dichlor-4-trifluormethylphenyl)-2-chlor-3-cyano-4-trifluormethylsulfonyl-5-brompyrrol,
1-(2,6-Dichlor-4-trifluormethylphenyl)-2-chlor-3-cyano-4-(trifluormethylthio)pyrrol,
1-(2,6-Dichlor-4-trifluormethylphenyl)-2-brom-3-trifluormethylthio-4-cyano-5-chlorpyrrol,
1-(2,6-Dichlor-4-trifluormethylphenyl)-2-[(trifluormethyl)carbonylamino]-3-trifluormethylthio-4-cyano-5-chlorpyrrol,
1-(2,6-Dichlor-4-trifluormethylphenyl)-2-(methylcarbonylamino)-3-trifluormethylthio-4-cyano-5-chlorpyrrol,
1-(2,6-Dichlor-4-trifluormethylphenyl)-2-amino-3-trifluormethylthio-4-cyano-5-chlorpyrrol,
1-(2-Chlor-4-trifluormethylphenyl)-2-amino-3-trifluormethylthio-4-cyano-5-chlorpyrrol,
1-(2,6-Dichlor-4-trifluormethylphenyl)-2-amino-3-dichlorfluormethylthio-4-cyano-5-chlorpyrrol,
1-(2,6-Dichlor-4-trifluormethylphenyl)-2,4-bis(trifluormethylthio)-3-cyano-5-aminopyrrol,
1-(2,6-Dichlor-4-trifluormethylphenyl)-2-amino-3-trifluormethylthio-4-cyano-5-brompyrrol,
1-(2,6-Dichlor-4-trifluormethylphenyl)-2-amino-3-trifluormethylthio-4-cyanopyrrol,
1-(4-Trifluormethylphenyl)-2-amino-3-trifluormethylthio-4-cyano-5-brompyrrol,
1-(2-Chlor-trifluormethylphenyl)-2-amino-3-trifluormethylthio-4-cyano-5-brompyrrol,
1-(2,6-Dichlor-4-trifluormethylphenyl)-2-chlor-3-cyano-4-(dichlorfluormethylthio)pyrrol,
1-(2,6-Dichlor-4-trifluormethylphenyl)-2-chlor-3-cyano-4-(dichlorfluormethylsulfinyl)pyrrol,
1-(2,6-Dichlor-4-trifluormethylphenyl)-2-chlor-3-cyano-4-(dichlorfluormethylsulfonyl)pyrrol,
1-(2,6-Dichlor-4-trifluormethylphenyl)-3-cyano-4-(dichlorfluormethylthio)pyrrol,
1-(2,6-Dichlor-4-trifluormethylphenyl)-3-cyano-4-(dichlorfluormethylsulfinyl)pyrrol oder
1-(2,6-Dichlor-4-trifluormethoxyphenyl)-2-chlor-3-cyano-4-(trifluormethylsulfonyl)pyrrol.

8. Verfahren zur Herstellung von Zwischenproduktverbindungen, die zur Herstellung von Verbindungen der Formel I verwendbar sind, welches umfaßt:
(A) ein Verfahren zur Herstellung von Verbindungen der Formel (XXXIV): 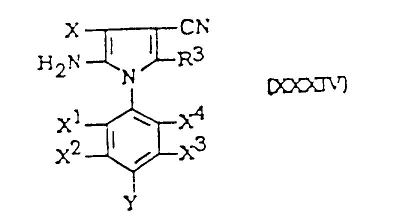 in der X, X¹, X², X³, X⁴ und Y wie in Anspruch 1 definiert sind und R³ Amino, Alkylamino oder Dialkylamino ist, bei dem eine Verbindung der Formel (XXX): 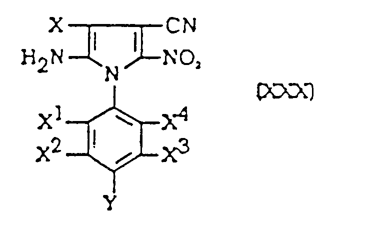 in der die verschiedenen Symbole wie in Anspruch 1 definiert sind und die Aminogruppe auf geeignete Weise geschützt ist, zum Erhalt von Verbindungen der Formel (XXXIV), bei denen R³ Amino ist, reduziert wird, worauf die Verbindungen mit einem Alkylierungsmittel in einem organischen Lösungsmittel zum Erhalt einer mono- oder disubstituierten Aminogruppe umgesetzt werden oder die Aminogruppe in Alkoxyalkylidenimino umgewandelt wird, worauf zum Erhalt von Verbindungen der Formel (XXXIV), bei denen R³ Alkylamino oder Dialkylamino ist, reduziert wird und sich ein Schritt zur Schutzgruppenabspaltung anschließt, wenn dies erforderlich ist, oder
(B) ein Verfahren zur Herstellung von Verbindungen der Formel (XXVIII), (XXXI), (XXXII) und (XXXV) gemäß Anspruch 1(A-II), (B-III), (E) oder (G), bei dem die entsprechende Verbindung gemäß Anspruch 1(A-I)(a), (B-I)(a), (C-I)(b) oder (F), nachdem die Aminogruppe, sofern sie vorhanden ist, geschützt worden ist, in ein Grignard-Reagens oder ein Lithium-Derivat umgewandelt wird, worauf mit einem Trialkylborat umgesetzt und auf bekannte Weise oxidiert wird und sich ein Schritt zur Abspaltung der Schutzgruppe anschließt, wenn dies erforderlich ist, oder
(C) ein Verfahren zur Herstellung einer Verbindung der Formel: 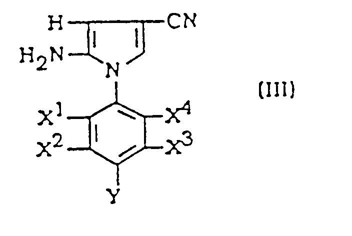 in der X¹, X², X³, X⁴ und Y die gleichen Bedeutungen haben wie in Anspruch 1, bei dem ein Dicyanopropen-Derivat der Formel: 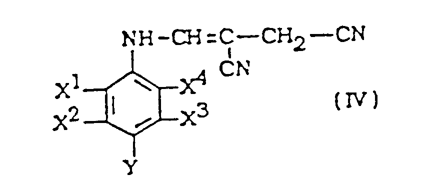 mit einem basischen Mittel umgesetzt wird.

9. Verfahren zur Herstellung einer arthropodiciden, pflanzennematociden, antihelmintischen oder antiprotozoären Zusammensetzung, die eine Verbindung der allgemeinen Formel (I) nach einem der Ansprüche 1 bis 7 oder die Verbindung, bei X⁴=X¹=Y=R¹=X=R²=R³=Cl und gleichzeitig X³=X²=H ist, in Verbindung mit einem oder mehren verträglichen Verdünnungsmitteln oder Trägermitteln enthält, bei dem die Verbindung der Formel (I) oder die Verbindung, bei der X⁴=X¹=Y=R¹=X=R²=R³=Cl und gleichzeitig X³=X²=H ist, mit einem oder mehreren verträglichen Verdünnungsmitteln oder Trägern formuliert wird.

10. Verfahren zur Herstellung einer Zusammensetzung zur Verwendung in der Veterinärmedizin und der Nutztierhaltung oder zur Aufrechterhaltung der Volksgesundheit, die als Wirkstoff mindestens eine Verbindung der Formel (I) nach einem der Ansprüche 1 bis 7 oder die Verbindung, bei der X⁴=X¹=Y=R¹=X=R²=R³=Cl und gleichzeitig X³=X²=H ist, enthält, bei dem die Verbindung der Formel (I) oder die Verbindung, bei der X⁴=X¹=Y=R¹=X=R²=R³=Cl und gleichzeitig X³=X²=H ist, formuliert wird.

11. Verfahren zur Herstellung einer insecticiden, acariciden oder nematociden Zusammensetzung, die eine wirksame Menge einer Verbindung der Formel (I) nach einem der Ansprüche 1 bis 7 oder der Verbindung, bei der X⁴=X¹=Y=R¹=X=R²=R³=Cl und gleichzeitig X³=X²=H ist, enthält, bei dem die Verbindung der Formel (I) oder die Verbindung, bei der X⁴=X¹=Y=R¹=X=R²=R³=Cl und gleichzeitig X³=X²=H ist, formuliert wird.

12. Verfahren nach Anspruch 11, wobei die Zusammensetzung enthält:
zwischen 0,05 Gew.-% und 95 Gew.-% eines oder mehrerer Wirkstoffe, zwischen 1 Gew.-% und 95 Gew.-% eines oder mehrerer landwirtschaftlich akzeptabler Träger, zwischen 0,1 Gew.-% und 50 Gew.-% eines oder mehrerer landwirtschaftlich akzeptabler grenzflächenaktiver Mittel.

13. Verfahren zur Anwendung in der Veterinärmedizin und der Nutztierhaltung oder zur Aufrechterhaltung der Volksgesundheit zur Bekämpfung von Arthropoden-, Helminthen- oder Protozoenschädlingen, die bei warmblütigen Wirbeltieren innere oder äußere Parasiten sind, bei dem auf eine Örtlichkeit eine wirksame Menge einer Verbindung der Formel (I) nach einem der Ansprüche 1 bis 7 oder der Verbindung, bei X⁴=X¹=Y=R¹=X=R²=R³=Cl und gleichzeitig X³=X²=H ist, ausgebracht wird.

14. Verfahren zur Bekämpfung von Arthropoden- und Nematodenschädlingen nach Anspruch 13, wobei die Verbindung auf die Örtlichkeit, auf welcher der Arthropoden- oder der Nematodenbefall bekämpft werden soll, in einer Menge von 0,005 kg bis 15 kg der Verbindung pro ha der behandelten Örtlichkeit ausgebracht wird.

15. Verbindung der Formel (I) nach einem der Ansprüche 1 bis 7.

16. Verbindungen der Formeln (III), (XXVIII) bis (XXXII), (XXXIV) bis (XXXVI), (XXXVIII), (XXXIX) und (XLI), in denen die verschiedenen Substituenten die gleichen Bedeutungen wie in den vorhergehenden Ansprüchen haben, wobei Verbindungen ausgenommen sind, bei denen R¹=R²=H, R³=Amino, X=Cyano, X³=X⁴=H und: X¹=Methyl, Methoxy oder Chlor und X²=Y=H; X²=Methyl, Methoxy oder Chlor und X¹=Y=H; Y=Methyl, Methoxy oder Chlor und X¹=X²=H ist.

17. Zusammensetzung, wie in einem der Ansprüche 9 bis 12 definiert.

## Revendications (Revendications pour l'(les) Etat(s) contractant(s) suivant(s): AT, BE, CH, LI, DE, FR, GB, GR, IT, LU, NL, SE)

1. Composé de formule (I) 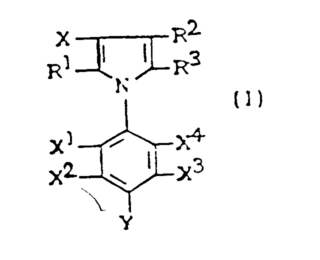 dans laquelle
X est choisi dans le groupe consistant en : un halogène, des groupes cyano, halogénalkyle, alkoxy, halogénalkoxy, alkylthio, alkylsulfinyle, alkylsulfonyle, halogénalkylthio, halogénalkylsulfinyle et halogénalkylsulfonyle dont les groupes alkyle, halogénalkyle, alkoxy et halogénalkoxy sont des chaînes linéaires ou ramifiées ayant moins de 10 atomes de carbone, et la substitution halogéno dans tous ces groupes consiste en un ou plusieurs atomes d'halogènes, qui sont identiques ou différents, depuis une mono-substitution jusqu'à une polysubstitution complète ;
R¹, R² et R³ sont choisis, individuellement, dans le groupe consistant en : l'un des substituants du même ensemble que ceux qui sont indiqués pour X ; un atome d'hydrogène ; un groupe alkyle ; et pas plus de l'un des substituants R¹, R² et R³ n'étant choisi dans le groupe consistant en formyle, amino, alkylamino et dialkylamino dont les groupes alkyle sont des chaînes linéaires ou ramifiées ayant moins de 10 atomes de carbone ;
Y est choisi dans le groupe consistant en : un halogène, des groupes cyano, alkyle, halogénalkyle, alkoxy et halogénalkoxy, dont les groupes alkyle, alkoxy, halogénalkyle et halogénalkoxy sont des chaînes linéaires ou ramifiées, ayant moins de 10 atomes de carbone, et la substitution halogéno dans tous ces groupes consistant en un ou plusieurs atomes d'halogènes, qui sont identiques ou différents, allant de la monosubstitution jusqu'à une polysubstitution complète ;
ou bien Y est un atome d'hydrogène lorsque :
X est un atome d'halogène ou un groupe R⁵S(O)n, dans lequel n a la valeur 0, 1 ou 2 et R⁵ est un groupe alkyle ou halogénalkyle, et les chaînes alkyliques carbonées et la substitution halogéno sont telles que définies ci-dessus ;
R¹ et R³ représentent chacun un atome d'hydrogène ; et
R² est un groupe cyano ;
X¹, X², X³ et X⁴ sont choisis, individuellement, dans le même ensemble de substituants que celui qui a été indiqué pour Y ou représentent un atome d'hydrogène, sous les réserves suivantes : qu'au moins l'un de R¹, R² et R³ soit choisi dans le même ensemble de substituants que celui qui a été indiqué pour X ;
que si X⁴ et X¹ sont de l'hydrogène et X représente un halogène ou un groupe cyano, R² soit alors différent de X, et
que si X⁴ et X¹ représentent de l'hydrogène et Y est un groupe méthyle, X soit alors différent d'un radical bromo ;
à l'exclusion du composé dans lequel X⁴ = X¹ = Y = R¹ = R² = R³ = Cl en même temps que X² = X³ = H.

2. Composé de formule (I) suivant la revendication 1, dans lequel :
X est choisi entre un atome d'halogène ou un groupe choisi entre des groupes cyano, halogénalkyle, alkoxy, halogénalkoxy, alkylthio, alkylsulfinyle, alkylsulfonyle, halogénalkylthio, halogénalkylsulfinyle et halogénalkylsulfonyle, dont les groupes alkyle, halogénalkyle, alkoxy et halogénalkoxy sont des chaînes linéaires ou ramifiées, ayant moins de 10 atomes de carbone, et la substitution halogéno dans tous ces groupes est une monosubstitution ou va jusqu'à une polysubstitution totale ;
R¹, R² et R³ sont choisis, individuellement, entre : les mêmes substituants ceux qui sont décrits pour X ; un atome d'hydrogène ; un groupe alkyle ; et un substituant tel que pas plus de l'un des substituants R¹, R² et R³ ne soit choisi dans le groupe consistant en formyle, amino, alkylamino et dialkylamino dont les groupes alkyle sont des chaînes linéaires ou ramifiés ayant moins de 10 atomes de carbone ;
Y est choisi entre un atome d'halogène et un groupe choisi entre cyano, alkyle, halogénalkyle, alkoxy et halogénalkoxy, dont les groupes alkyle, alkoxy, halogénalkyle et halogénalkoxy sont des chaînes linéaires ou ramifiées, ayant moins de 10 atomes de carbone, et la substitution halogéno dans tous ces groupes est une monosubstitution ou va jusqu'à une polysubstitution totale ; et
X¹, X², X³ et X⁴ sont tels que définis dans la revendication 1.

3. Composé de formule (I) suivant la revendication 2, dans laquelle :
X est un atome d'halogène ou un groupe R⁵S(O)n, dans lequel n a la valeur 0, 1 ou 2 et R⁵ est un groupe alkyle ou halogénalkyle ;
R¹ est un atome d'hydrogène, un atome d'halogène ou un groupe alkylthio ;
R² est un groupe cyano ;
R³ est un atome d'hydrogène ou un atome d'halogène ;
Y est un atome d'hydrogène, un atome d'halogène, un groupe halogénalkyle ou halogénalkoxy, sous réserve que Y soit un atome d'hydrogène de la manière définie dans la revendication 1 ; et
X¹, X², X³ et X⁴ sont choisis, individuellement, dans le groupe consistant en un atome d'hydrogène, un atome d'halogène, un groupe alkyle en C₁ à C₃, alkoxy en C₁ à C₃ et alkylthio en C₁ à C₃.

4. Composé suivant la revendication 3, répondant à la formule (II) 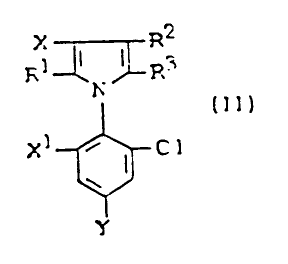 dans laquelle :
X est un groupe R⁵S(O)n, dans lequel n a la valeur 0, 1 ou 2 et R⁵ est un groupe CH₃, CF₃, CF₂Cl, CFCl₂, CF₂Br, CHF₂, CHCl₂ ou CHClF ;
R¹ représente H, F, Cl ou Br ;
R³ représente H, F, Cl ou Br ;
X¹ représente H ou Cl ; et
Y est un groupe CF₃ ou CF₃O.

5. Composé de formule (I) suivant la revendication 1, 2 ou 3, dans lequel :
R¹ est un atome d'hydrogène ou un atome d'halogène ;
R² est un groupe cyano ;
X est un groupe halogénalkyle-S(O)ₙ dans lequel n a la valeur 0, 1 ou 2 ;
X¹ et X⁴ représentent autre chose qu'un atome d'hydrogène ;
Y est un groupe halogénalkyle ou halogénalkoxy ; et
X² et X³ représentent un atome d'hydrogène.

6. Composé suivant la revendication 2, répondant à la formule (II) 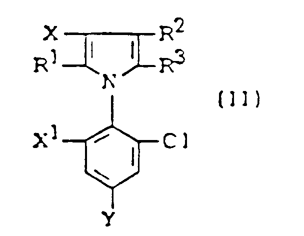 dans laquelle :
X est un groupe R⁵S(O)ₙ, dans lequel n a la valeur 0, 1 ou 2 et R⁵ est un groupe CH₃, CF₃, CF₂Cl ou CFCl₂ ;
R² est un groupe cyano ;
R¹ représente H, F, Cl, Br ou NH₂ ;
R³ représente H, F, Cl, Br, CF₃ ou CN ;
X¹ représente H ou Cl ; et
Y est un groupe CF₃ ou CF₃O.

7. Composé suivant la revendication 1, qui est :
le 1-(2,6-dichloro-4-trifluorométhylphényl)-2-chloro-3-cyano-4-(trifluorométhylthio)pyrrole ;
le 1-(2,6-dichloro-4-trifluorométhoxyphényl)-2-chloro-3-cyano-4-(trifluométhylsulfonyl)pyrrole ;
le 1-(2,6-dichloro-4-trifluorométhylphényl)-2-chloro-3-cyano-4-(dichlorofluorométhylthio)pyrrole ;
le 1-(2,6-dichloro-4-trifluorométhylphényl)-2-chloro-3-cyano-4-(dichlorofluorométhylsulfinyl)pyrrole ;
le 1-(2,6-dichloro-4-trifluorométhylphényl)-2-chloro-3-cyano-4-(dichlorofluorométhylsulfonyl)pyrrole ;
le 1-(2,6-dichloro-4-trifluorométhylphényl)-2-chloro-3-cyano-4-(chlorodifluorométhylsulfonyl)pyrrole ;
le 1-(2,6-dichloro-4-trifluorométhylphényl)-2-chloro-3-cyano-4-(chlorodifluorométhylsulfinyl)pyrrole ;
le 1-(2,6-dichloro-4-trifluorométhylphényl)-2-chloro-3-cyano-4-(chlorodifluorométhylthio)pyrrole ;
le 1-(2,6-dichloro-4-trifluorométhylphényl)-2-bromo-3-cyano-4-(trifluorométhylsulfinyl)pyrrole ;
le 1-(2,6-dichloro-4-trifluorométhylphényl)-2-bromo-3-cyano-4-(trifluorométhylsulfonyl)pyrrole ;
le 1-(2,6-dichloro-4-trifluorométhylphényl)-2-bromo-3-cyano-4-(dichlorofluorométhylthio)pyrrole ;
le 1-(2,6-dichloro-4-trifluorométhylphényl)-2-bromo-3-cyano-4-(dichlorofluorométhylsulfonyl)pyrrole ;
le 1-(2,6-dichloro-4-trifluorométhylphényl)-2-bromo-3-cyano-4-(dichlorofluorométhylsulfinyl)pyrrole ;
le 1-(2,6-dichloro-4-trifluorométhylphényl)-2-bromo-3-cyano-4-(chlorodifluorométhylthio)pyrrole ;
le 1-(2,6-dichloro-4-trifluorométhylphényl)-2-bromo-3-cyano-4-(chlorodifluorométhylsulfinyl)pyrrole ;
le 1-(2,6-dichloro-4-trifluorométhylphényl)-2-chloro-3-cyano-4-(dichlorofluorométhylsulfonyl)-5-bromopyrrole ;
le 1-(2,6-dichloro-4-trifluorométhylphényl)-2-bromo-3-cyano-4-(chlorodifluorométhylsulfonyl)pyrrole ;
le 1-(2,6-dichloro-4-trifluorométhoxyphényl)-2-chloro-3-cyano-4-(dichlorofluorométhylsulfinyl)pyrrole ;
le 1-(2,6-dichloro-4-trifluorométhoxyphényl)-2-chloro-3-cyano-4-(dichlorofluorométhylsulfonyl)pyrrole ;
le 1-(2-chloro-4-trifluorométhylphényl)-2-chloro-3-cyano-4-(dichlorofluorométhylsulfonyl)pyrrole ;
le 1-(2-chloro-4-trifluorométhylphényl)-2-chloro-3-cyano-4-(dichlorofluorométhylsulfinyl)pyrrole ;
le 1-(2-chloro-4-trifluorométhylphényl)-2-bromo-3-cyano-4-(dichlorofluorométhylsulfonyl)pyrrole ;
le 1-(2,6-dichloro-4-trifluorométhylphényl)-2-chloro-3-cyano-4-(dichlorofluorométhylthio)-5-méthylthiopyrrole ;
le 1-(2,6-dichloro-4-trifluorométhylphényl)-2-chloro-3-cyano-4-(bromodifluorométhylthio)pyrrole ;
le 1-(2,6-dichloro-4-trifluorométhylphényl)-2-chloro-3-cyano-4-(bromodifluorométhylsulfinyl)pyrrole ;
le 1-(2,6-dichloro-4-trifluorométhylphényl)-2-chloro-3-cyano-4-(bromodifluorométhylsulfonyl)pyrrole ;
le 1-(2,6-dichloro-4-trifluorométhylphényl)-2-chloro-3-cyano-4-(méthylsulfinyl)pyrrole ;
le 1-(2,6-dichloro-4-trifluorométhylphényl)-2-chloro-3-cyano-4-(méthylsulfonyl)pyrrole ;
le 1-(4-bromo-2,6-dichlorophényl)-3-cyano-4-(chlorodifluorométhylthio)pyrrole ;
le 1-(4-bromo-2,6-dichlorophényl)-3-cyano-4-(trifluorométhylthio)pyrrole ;
le 1-(2,4,6-trichlorophényl)-3-cyano-4-(chlorodifluorométhylthio)pyrrole ;
le 1-(2,4,6-trichlorophényl)-3-cyano-4-(chlorodifluorométhylsulfinyl)pyrrole ;
le 1-(2,4,6-trichlorophényl)-3-cyano-4-(dichlorofluorométhylthio)pyrrole ;
le 1-(2,4,6-trichlorophényl)-3-cyano-4-(dichlorofluorométhylsulfinyl)pyrrole ;
le 1-(2,4,6-trichlorophényl)-3-cyano-4-(dichlorofluorométhylsulfonyl)pyrrole ;
le 1-(4-bromo-2,6-dichlorophényl)-3-cyano-4-(dichlorofluorométhylthio)pyrrole ;
le 1-(4-bromo-2,6-dichlorophényl)-3-cyano-4-(dichlorofluorométhylsulfinyl)pyrrole ;
le 1-(4-bromo-2,6-dichlorophényl)-3-cyano-4-(dichlorofluorométhylsulfonyl)pyrrole ;
le 1-(2,4,6-trichlorophényl)-3-cyano-4-(trifluorométhylthio)pyrrole ;
le 1-(2,4,6-trichlorophényl)-3-cyano-4-(trifluorométhylsulfinyl)pyrrole ;
le 1-(2,4,6-trichlorophényl)-3-cyano-4-(trifluorométhylsulfonyl)pyrrole ;
le 1-(2,4,6-trichlorophényl)-3-cyano-4-(trichlorométhylthio)pyrrole ;
le 1-(2,4-dichlorophényl)-3-cyano-4-(dichlorofluorométhylthio)pyrrole ;
le 1-(2,4,6-trichlorophényl)-3-cyano-4-chloropyrrole ;
le 1-(2,4,6-trichlorophényl)-3-cyano-4-(chlorodifluorométhylsulfonyl)pyrrole ;
le 1-(2,6-dichlorophényl)-3-cyano-4-(dichlorofluorométhylthio)pyrrole ;
le 1-(4-bromo-2,6-dichlorophényl)-3-cyano-4-(trifluorométhylsulfinyl)pyrrole ;
le 1-(4-bromo-2,6-dichlorophényl)-3-cyano-4-(trifluorométhylsulfonyl)pyrrole ;
le 1-(4-bromo-2,6-dichlorophényl)-3-cyano-4-(chlorodifluorométhylsulfinyl)pyrrole ;
le 1-(4-bromo-2,6-dichlorophényl)-3-cyano-4-(chlorodifluorométhylsulfonyl)pyrrole ;
le 1-(4-bromo-2,6-diméthylphényl)-3-cyano-4-(trifluorométhylsulfinyl)pyrrole ;
le 1-(4-bromo-2,6-dimethylphényl)-3-cyano-4-(trifluorométhylsulfonyl)pyrrole ; ou
le 1-(4-bromo-2,6-difluorophényl)-3-cyano-4-(dichlorofluorométhylthio)pyrrole ;
le 1-(2,6-dichloro-4-trifluorométhylphényl)-2-chloro-3-cyano-4-(trifluorométhylsulfonyl)pyrrole ;
le 1-(2,6-dichloro-4-trifluorométhylphényl)-2-chloro-3-cyano-4-(trifluorométhylsulfinyl)pyrrole ;
le 1-(2,6-dichloro-4-trifluorométhylphényl)-2-chloro-3-cyano-4-trifluorométhylsulfinyl-5-bromopyrrole ;
le 1-(2,6-dichloro-4-trifluorométhylphényl)-2-chloro-3-cyano-4-trifluorométhylsulfonyl-5-bromopyrrole ;
le 1-(2,6-dichloro-4-trifluorométhylphényl)-2-chloro-3-cyano-4-(trifluorométhylthio)pyrrole ;
le 1-(2,6-dichloro-4-trifluorométhylphényl)-2-bromo-3-trifluorométhylthio-4-cyano-5-chloropyrrole ;
le 1-(2,6-dichloro-4-trifluorométhylphényl)-2-[(trifluorométhyl)carbonylamino]-3-trifluorométhylthio-4-cyano-5-chloropyrrole ;
le 1-(2,6-dichloro-4-trifluorométhylphényl)-2-(méthylcarbonylamino)-3-trifluorométhylthio-4-cyano-5-chloropyrrole ;
le 1-(2,6-dichloro-4-trifluorométhylphényl)-2-amino-3-trifluorométhylthio-4-cyano-5-chloropyrrole ;
le 1-(2-chloro-4-trifluorométhylphényl)-2-amino-3-trifluorométhylthio-4-cyano-5-chloropyrrole ;
le 1-(2,6-dichloro-4-trifluorométhylphényl)-2-amino-3-dichlorofluorométhylthio-4-cyano-5-chloropyrrole ;
le 1-(2,6-dichloro-4-trifluorométhylphényl)-2,4-bis(trifluorométhylthio)-3-cyano-5-aminopyrrole ;
le 1-(2,6-dichloro-4-trifluorométhylphényl)-2-amino-3-trifluorométhylthio-4-cyano-5-bromopyrrole ;
le 1-(2,6-dichloro-4-trifluorométhylphényl)-2-amino-3-trifluorométhylthio-4-cyanopyrrole ;
le 1-(4-trifluorométhylphényl)-2-amino-3-trifluorométhylthio-4-cyano-5-bromopyrrole ;
le 1-(2-chloro-4-trifluorométhylphényl)-2-amino-3-trifluorométhylthio-4-cyano-5-bromopyrrole ;
le 1-(2,6-dichloro-4-trifluorométhylphényl)-2-chloro-3-cyano-4-(dichlorofluorométhylthio)pyrrole ;
le 1-(2,6-dichloro-4-trifluorométhylphényl)-2-chloro-3-cyano-4-(dichlorofluorométhylsulfinyl)pyrrole ;
le 1-(2,6-dichloro-4-trifluorométhylphényl)-2-chloro-3-cyano-4-(dichlorofluorométhylsulfonyl)pyrrole ;
le 1-(2,6-dichloro-4-trifluorométhylphényl)-3-cyano-4-(dichlorofluorométhylthio)pyrrole ;
le 1-(2,6-dichloro-4-trifluorométhylphényl)-3-cyano-4-(dichlorofluorométhylsulfinyl)pyrrole ; ou
le 1-(2,6-dichloro-4-trifluorométhylphényl)-2-chloro-3-cyano-4-(trifluorométhylsulfonyl)pyrrole.

8. Procédé de production de composés de formule (I), qui comprend :
(A) lorsque le composé répond à la formule I(a) : 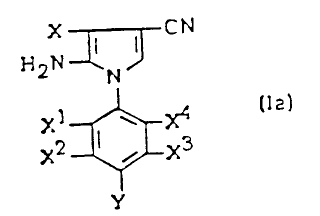 dans laquelle X¹, X², X³, X⁴ et Y ont la même définition que dans la revendication 1, et
(A-I) : X est un halogène, un groupe trifluorométhyle, cyano, alkylthio, alkylsulfinyle, alkylsulfonyle, halogénalkylthio, halogénalkylsulfinyle ou halogénalkylsulfonyle, un procédé dans lequel un composé de formule 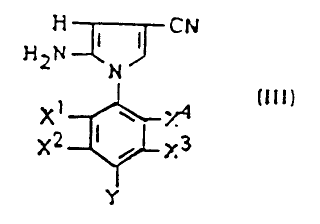 dans laquelle les divers symboles sont tels que définis dans la revendication 1 et le groupe amino est facultativement protégé :
(a) est amené à réagir avec un agent d'halogénation, facultativement en présence d'un solvant pour obtenir un composé de formule (Ia) dans laquelle X est un halogène, puis on fait réagir facultativement ce composé avec le trifluorométhyl-cuivre d'une manière connue pour obtenir des composés de formule (Ia) dans laquelle X est un groupe trifluorométhyle ;
(b) est amené à réagir avec un tris(alkylthio)méthane ou un tris(arylthio)méthane en présence d'un acide de Lewis, puis le composé obtenu de formule (XI) 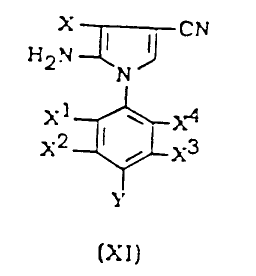 dans laquelle X est un groupe bis(alkylthio)méthyle ou bis(arylthio)méthyle et les autres symboles sont tels que définis dans la revendication 1, est amené à réagir facultativement avec un nitrite d'alkyle, la réaction étant suivie d'une hydrolyse, en vue d'obtenir le composé de formule (XI) dans laquelle X est un groupe formyle, puis le composé en question est mis en contact avec l'hydroxylamine et ce contact est suivi d'une déshydratation d'une manière connue en vue d'obtenir le composé de formule (Ia) dans laquelle X est un groupe cyano ;
(c) est amené à réagir avec un composé de formule MSCN, M étant un métal alcalin, en présence de brome, dans un solvant en vue d'obtenir le composé de formule (Ia) dans laquelle X est un groupe thiocyanato, puis ce composé est amené à réagir avec un halogénure d'alkyle ou un sulfate de dialkyle en présence d'une base dans un solvant pour former un composé de formule (Ia) dans laquelle X est un groupe alkylthio ; ou bien
(d) est amené à réagir avec un halogénure de sulfényle de formule RSHal, dans laquelle R est un radical alkyle, halogénalkyle, phényle ou hétéroaryle et Hal est un atome d'halogène, dans un milieu réactionnel liquide organique, facultativement en présence d'un accepteur d'acide en vue d'obtenir un composé de formule (Ia) dans laquelle X est un groupe alkylthio ou halogénalkylthio, après quoi on effectue facultativement l'oxydation, d'une manière connue, du composé obtenu en vue de produire un composé de formule (Ia) dans laquelle X est un groupe RS(O)ₙ dans laquelle n est égal à 1 ou 2, ou bien
(A-II) lorsque X est un groupe alkoxy ou halogénalkoxy, un procédé dans lequel un composé de formule 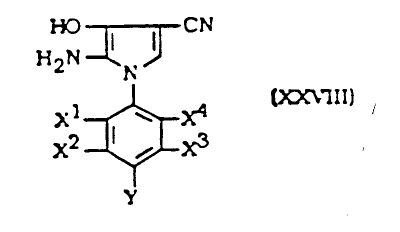 dans laquelle les divers symboles sont tels que définis dans la revendication 1 et des groupes amino et cyano sont convenablement protégés si nécessaire :
(a) est amené à réagir avec un agent d'alkylation, facultativement en présence d'une base, pour obtenir des composés de formule (Ia) dans laquelle X est un groupe alkoxy ; ou bien
(b) est halogénalkylé d'une manière connue pour obtenir des composés de formule (Ia) dans laquelle X est un groupe halogénalkoxy, ou bien
(A-III) lorsque X est un groupe halogénalkyle, un procédé dans lequel un composé de formule 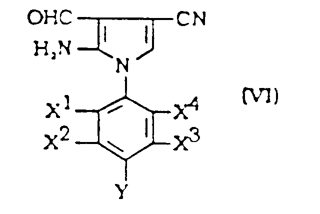 dans laquelle les divers symboles sont tels que définis dans la revendication 1 et les groupes amino et cyano sont convenablement protégés si nécessaire :
(a) est amené à réagir avec un agent de fluoration d'une manière connue en vue d'obtenir le composé de formule (Ia) dans laquelle X est un groupe difluorométhyle ;
(b) est amené à réagir avec un agent oxydant pour convertir le groupe -CHO en un groupe acide carboxylique, puis les composés obtenus sont soumis à l'action d'un agent de fluoration d'une manière connue pour obtenir le composé de formule (Ia) dans laquelle X est un groupe trifluorométhyle ;
(c) est converti en un composé dans lequel le groupe -CHO est remplacé par un groupe méthyle, puis le composé obtenu est soumis à l'action d'un agent d'halogénation dans un solvant en vue d'obtenir un composé de formule (Ia) dans laquelle X est un groupe bromométhyle ou chlorométhyle ; ou bien
(d) est amené à réagir successivement avec un dérivé halogénalkylique de métal ou le trifluorotriméthylsilane pour convertir le groupe -CHO en un groupe halogénalkylcarbinol, puis le composé dans lequel X est un groupe halogénalkylcarbinol est amené à réagir avec un agent d'halogénation en vue d'obtenir des composés de formule (Ia) dans laquelle X est un groupe α-halogénalkyl-α-halogénométhyle, toutes les étapes précédentes ayant été suivies d'une étape d'élimination du groupe protecteur, si nécessaire, ou bien
(B) lorsque le composé de formule (I) répond à la formule I(b) : 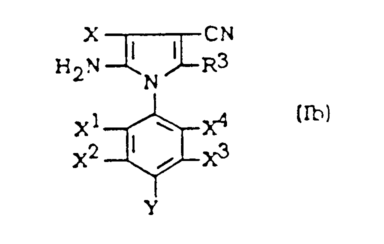 dans laquelle X, X¹, X², X³, X⁴ et Y ont la même définition que dans la revendication 1 et lorsque (B-I) R³ est un halogène, un groupe formyle, halogénalkyle, alkyle, alkylthio, halogénalkylthio, alkylsulfinyle, alkylsulfonyle, halogénalkylsulfinyle ou halogénalkylsulfonyle, un procédé dans lequel un composé de formule (Ia) dans laquelle X, le groupe cyano et le groupe amino sont facultativement protégés si nécessaire :
(a) est amené à réagir conformément à A-I(a) ci-dessus afin d'obtenir des composés de formule (Ib) dans laquelle R³ est un halogène, puis facultativement, ce composé est amené à réagir conformément à A-I(a) ci-dessus pour obtenir des composés de formule (Ib) dans laquelle R³ est un groupe trifluorométhyle ;
(b) est amené à réagir conformément à A-I(b) ci-dessus pour obtenir tout d'abord un composé de formule (Ib) dans laquelle R³ est un groupe bis(alkylthio)méthyle ou un groupe bis(arylthio)méthyle, puis un composé de formule (Ib) dans laquelle R³ est un groupe formyle ; ou bien
(c) est amené à réagir conformément à A-I(c,d) ci-dessus en vue d'obtenir des composés de formule (Ib) dans laquelle R³ est un groupe alkylthio ou halogénalkylthio, puis facultativement oxydé conformément à A-I(d) ci-dessus en vue d'obtenir les composés de formule (Ib) dans laquelle R³ est un groupe alkylsulfinyle, alkylsulfonyle, halogénalkylsulfinyle ou halogénalkylsulfonyle, sous réserve que X ne soit pas un groupe RS qui puisse subir une oxydation indésirable ; et lorsque X est un halogène, on conduit un traitement facultatif avec un alkyl-lithium d'une manière connue, puis une désactivation aqueuse et une sulfénylation conformément à A-I(c,d) ci-dessus en vue d'obtenir des composés de formule (Ib) dans laquelle X est un groupe alkylthio ou halogénalkylthio, ou bien
(B-II) lorsque R³ est un groupe cyano ou un groupe alkyle, un procédé dans lequel un composé de formule 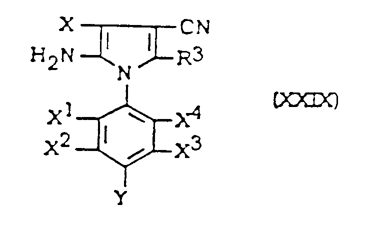 dans laquelle R³ est un groupe formyle ou alkylcarbonyle, les autres symboles étant tels que définis dans la revendication 1, et X, le groupe cyano et le groupe amino sont protégés si nécessaire :
(a) est condensé avec l'hydroxylamine ou une O-alkylhydroxylamine ou leurs sels d'addition dans un solvant en vue d'obtenir un composé de formule (Ib) dans laquelle R³ est un groupe hydroxyiminoalkylidényle ou alkoxyiminoalkylidényle, et ce groupe R³ est converti en un groupe cyano afin d'obtenir un composé de formule (Ib) dans laquelle R³ est un groupe cyano ;
(b) est amené à réagir, lorsque R³ est un groupe formyle, conformément à A-III(a, b, c ou d) ci-dessus afin d'obtenir un composé de formule (Ib) dans laquelle R³ est un groupe méthyle ou halogénalkyle ; ou bien
(c) est amené à réagir, lorsque R³ est un groupe formyle, avec un réactif de Grignard dérivé d'un halogénure d'alkyle ou d'un alkyl-lithium pour produire un carbinol, la réaction étant suivie d'une étape de déshydratation afin de produire un composé dans lequel R³ est un groupe alcényle, puis d'une réduction en vue d'obtenir des composés de formule (Ib) dans laquelle R³ est un groupe alkyle ; la réduction étant facultativement suivie d'une étape d'élimination du groupe protecteur, ou bien
(B-III) lorsque R³ est un groupe alkoxy ou halogénalkoxy, un procédé dans lequel un composé de formule 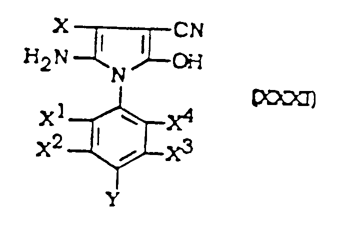 dans laquelle les groupes amino, cyano et X sont facultativement protégés, est amené à réagir conformément à A-II(a, b ou c) ci-dessus afin d'obtenir un composé de formule (Ib) dans laquelle R³ est un groupe alkoxy ou halogénalkoxy, ou bien
(c) lorsque le composé de formule I répond à la formule (Ic) : 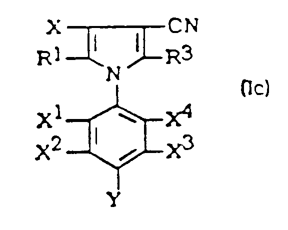 dans laquelle X, R³, X¹, X², X³, X⁴ et Y ont les mêmes définitions que dans la revendication 1 et lorsque
(C-I) R¹ est l'hydrogène, un halogène, un groupe alkylthio, halogénalkylthio, alkylsulfinyle, alkylsulfonyle, halogénalkylsulfinyle, halogénalkylsulfonyle, alkylamino, dialkylamino, formyle, halogénalkyle ou alkyle, un procédé dans lequel un composé de formule 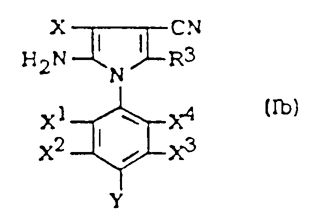 dans laquelle le groupe amino est débarrassé du groupe protecteur après protection des groupes X, R³ ou cyano si nécessaire :
(a) est amené à réagir avec un agent de diazotation dans un solvant inerte afin d'obtenir un composé de formule (Ic) dans laquelle R¹ représente H ;
(b) est amené à réagir avec un agent de diazotation en présence d'un donneur d'halogène pour obtenir un composé de formule (Ic) dans laquelle R¹ est un halogène ;
(c) est amené à réagir avec un agent de diazotation en présence de (SCN)₂ ou d'un disulfure dans un solvant pour obtenir des composés de formule (Ic) dans laquelle R¹ est un groupe alkylthio ou halogénalkylthio, après quoi on effectue facultativement une oxydation conformément à A-I(d) ci-dessus pour obtenir un composé de formule (Ic) dans laquelle R¹ est un groupe alkylsulfinyle ou alkylsulfonyle ;
(d) est amené à réagir conformément à la revendication 9(A) ci-dessous en vue d'obtenir des composés de formule (Ic) dans laquelle R¹ est un groupe alkylamino ou dialkylamino ; ou bien
(e) est amené à réagir avec du nitrite de sodium et de la formaldoxime, du sulfate de cuivre et du HCl d'une manière connue en vue d'obtenir des composés de formule (Ic) dans laquelle R¹ est un groupe formyle, puis, à titre facultatif, (i) amené à réagir conformément à B-II(a) ci-dessus afin d'obtenir un composé de formule (Ic) dans laquelle R¹ est un groupe cyano ou (ii) amené à réagir conformément à A-III(a-d) ci-dessus pour obtenir un composé de formule (Ic) dans laquelle R¹ est un groupe halogénalkyle ou méthyle, la réaction étant suivie si nécessaire d'une étape d'élimination du groupe protecteur ; ou bien
(C-II) R¹ est un groupe alkoxy ou halogénalkoxy, un procédé dans lequel un composé de formule 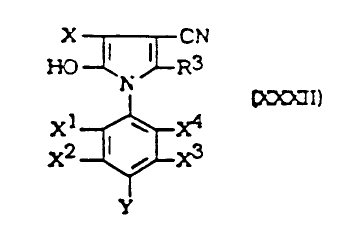 dans laquelle les divers symboles sont tels que définis dans la revendication 1 et X, le groupe cyano ou R³ sont facultativement protégés d'une manière connue, est amené à réagir conformément à A-II(a ou b) ci-dessus pour obtenir un composé de formule (Ic) dans laquelle R¹ est un groupe alkoxy ou halogénalkoxy, la réaction étant suivie d'une étape facultative d'élimination du groupe protecteur, ou bien
(D) lorsque X, R¹, R³, X¹, X², X³, X⁴ et Y ont la même définition que dans la revendication 1 et R² est un groupe CHO, un procédé dans lequel un composé de formule (Ic) est traité avec un agent réducteur dans un solvant pour convertir le groupe CN en un groupe CHO et pour former le composé dans lequel R² est un groupe CHO, ce composé étant facultativement oxydé d'une manière connue pour produire le composé correspondant de formule (XXXV) 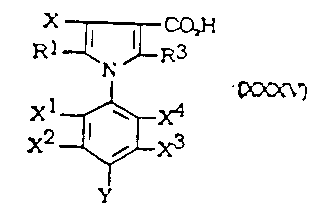 ou bien (E) lorsque X, R¹, R³, X¹, X², X³, X⁴ et Y ont la même définition que dans la revendication 1, et R² est un groupe cyano, alkyle ou halogénalkyle, un procédé dans lequel un composé de formule (I), dans laquelle R² est un groupe CHO, après protection facultative de X, R¹ ou R³ si nécessaire, d'une manière connue, est amené à réagir conformément à A-III(a, b, c, ou d), A-II(a ou b) ou C-I ci-dessus, la réaction étant suivie, si nécessaire, d'une étape d'élimination du groupe protecteur, ou bien
(F) lorsque X, R¹, R³, X¹, X², X³, X⁴ et Y ont la même définition que dans la revendication 1, et R² est un groupe amino, alkylamino, dialkylamino, hydrogène, halogène, alkylthio, halogénalkylthio, alkylsulfinyle, alkylsulfonyle, halogénalkylsulfinyle, halogénalkylsulfonyle ou trifluorométhyle, un procédé dans lequel un composé de formule (XXXV) représenté ci-dessus, après protection facultative de X, R¹ ou R³ si nécessaire d'une manière connue, est amené à réagir dans des conditions de transposition de Curtius, par exemple par conversion en un chlorure d'acide, suivie d'une réaction avec un azoture de métal alcalin ou avec le diphénylphosphorylazide en présence d'une base organique dans un solvant alcoolique pour produire un carbamate qui peut ensuite être hydrolysé afin d'obtenir le composé correspondant dans lequel R² est un groupe amino que l'on fait ensuite facultativement réagir conformément à la revendication 9(A) ci-dessous ou C-I(a-c) ci-dessus, puis, lorsque R² est un halogène, on conduit facultativement la réaction conformément à B-I(a) ci-dessus, suivie, si nécessaire, d'une étape d'élimination du groupe protecteur, ou bien
(G) lorsque X, R¹, R³, X¹, X², X³, X⁴ et Y ont la même définition que dans la revendication 1, et R² est un groupe alkoxy ou halogénalkoxy, un procédé dans lequel un composé de formule 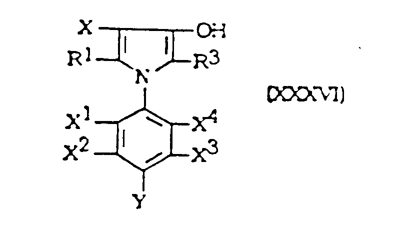 dans laquelle les divers symboles sont tels que définis dans la revendication 1, après protection facultative des groupes X, R¹ ou R³, si nécessaire, est amené à réagir conformément à B-III ci-dessus, la réaction étant suivie, si nécessaire, d'une étape d'élimination du groupe protecteur, ou bien
(H) lorsque R¹, R², R³, Y, X¹, X², X³ et X⁴ sont tels que définis dans la revendication 1 et X est un groupe perhalogénalkylthio, un procédé qui comprend :
a) la réaction d'un composé de formule (XXXVIII) 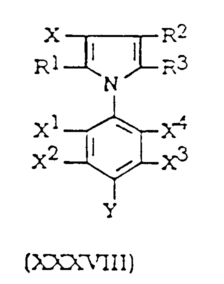 dans laquelle X est de l'hydrogène, et les autres symboles sont tels que définis dans la revendication 1, avec l'acide chlorosulfonique (ClSO₃H) à une température de 0°C à 150°C et, facultativement, dans un solvant organique, pour préparer un composé de formule (XXXIX) : 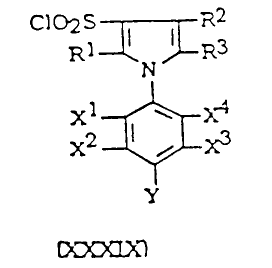 dans laquelle les divers symboles sont tels que définis dans la revendication 1 ;
b) la réaction du composé de formule (XXXIX) avec un agent réducteur à une température de 0°C à 110°C dans un solvant organique pour former un disulfure de formule (XLI) 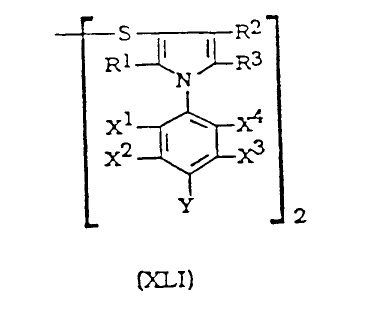 dans laquelle les divers symboles sont tels que définis dans la revendication 1 ; et
c) la réaction du disulfure de formule (XLI) avec un perhalogénalcane de formule (XLII), ZCFR⁷R⁸, dans lequel Z représente Cl, Br ou I, R⁷ représente F, Cl ou Br, et R⁸ représente F, Cl, Br ou un groupe perfluoralkyle, en présence d'un agent réducteur promoteur de radicaux libres et à titre facultatif en présence d'une base, dans un solvant organique à une température de 20°C à 85°C et à titre facultatif sous pression, pour préparer un composé de formule (I) dans laquelle X est un groupe perhalogénalkylthio et les autres symboles sont tels que définis dans la revendication 1, ou bien
(I) par transformation d'un composé obtenu comme décrit dans l'une quelconque des étapes A à H ci-dessus en un composé de formule (I) tel que défini dans la revendication 1.

9. Procédé de production de composés intermédiaires utiles pour l'obtention de composés de formule (I), qui comprend :
(A) un procédé de production de composés de formule (XXXIV) 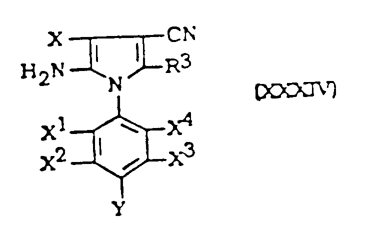 dans laquelle X, X¹, X², X³, X⁴ et Y sont tels que définis dans la revendication 1 et R³ est un groupe amino, alkylamino ou dialkylamino, dans lequel un composé de formule (XXX) 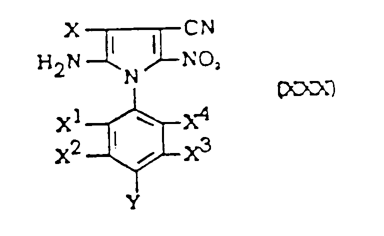 dans laquelle les divers symboles sont tels que définis dans la revendication 1 et le groupe amino est protégé d'une manière convenable, est réduit en vue d'obtenir des composés de formule (XXXIV) dans laquelle R³ est un groupe amino, ces composés étant amenés à réagir :
avec un agent d'alkylation dans un solvant organique pour obtenir un groupe amino monosubstitué ou disubstitué ou par l'intermédiaire de la transformation du groupe amino en un groupe alkoxyalkylidène-imino, suivie d'une réduction en vue d'obtenir des composés de formule (XXXIV) dans laquelle R³ est un groupe alkylamino ou dialkylamino ; l'opération étant suivie, si nécessaire, dans l'étape d'élimination du groupe protecteur, ou bien
(B) un procédé de préparation de composés de formules (XXVIII), (XXXI), (XXXII) et (XXXV) suivant la revendication 8(A-II), (B-III), (E) ou (G), dans lequel le composé correspondant suivant la revendication 8(A-I) (a), (B-I)(a), (C-I)(b) ou (F), après que le groupe amino éventuellement présent a été protégé, est converti en un réactif de Grignard ou en un dérivé de lithium, la conversion étant suivie d'une réaction avec un borate de trialkyle et d'une oxydation d'une manière connue, puis, si nécessaire, d'une étape d'élimination du groupe protecteur, ou bien
(C) un procédé de production d'un composé de formule 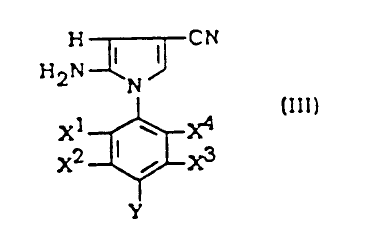 dans laquelle X¹, X², X³, X⁴ et Y ont la même définition que dans la revendication 1, dans lequel un dérivé de dicyanopropène de formule 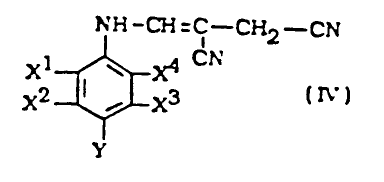 est amené à réagir avec un agent basique.

10. Composition pour combattre des arthropodes, des nématodes des plantes, des helminthes ou des protozoaires, qui comprend un composé de formule générale (I) tel que défini dans l'une quelconque des revendications 1 à 7 ou le composé dans lequel X⁴=X¹=Y=R¹=X=R²=R³=Cl en même temps que X³=X²=H, en association avec un ou plusieurs diluants ou véhicules compatibles.

11. Composition destinée à être utilisée en médecine vétérinaire et dans l'exploitation du bétail ou dans le maintien de l'hygiène publique, contenant comme ingrédient actif au moins un composé suivant l'une quelconque des revendications 1 à 7 ou le composé dans lequel X⁴=X¹=Y=R¹=X=R²=R³=Cl en même temps que X³=X²=H.

12. Composition insecticide, acaricide ou nématocide, qui comprend une quantité efficace d'un composé de formule (I) suivant l'une quelconque des revendications 1 à 7 ou le composé dans lequel X⁴=X¹=Y=R¹=X=R²=R³=Cl en même temps que X³=X²=H.

13. Composition suivant la revendication 12, qui comprend entre 0,05 % et 95 %, en poids, d'un ou plusieurs ingrédients actifs ; entre 1 % et 95 %, en poids, d'un ou plusieurs véhicules acceptables en agriculture ; et entre 0,1 % et 50 %, en poids, d'un ou plusieurs agents tensio-actifs acceptables en agriculture.

14. Procédé destiné à être utilisé en médecine vétérinaire et dans l'exploitation du bétail ou dans le maintien de l'hygiène publique pour combattre des arthropodes, des helminthes ou des protozoaires qui sont des endoparasites ou des ectoparasites de vertébrés à sang chaud, qui comprend l'application à un site d'une quantité efficace d'un composé de formule (I) suivant l'une quelconque des revendications 1 à 7 ou du composé dans lequel X⁴=X¹=Y=R¹=X=R²=R³=Cl en même temps que X³=X²=H.

15. Procédé pour combattre des arthropodes et nématodes parasites suivant la revendication 14, dans lequel le composé est appliqué au site dans lequel l'infestation par l'arthropode ou le nématode doit être combattue, en une quantité de 0,005 kg à 15 kg du composé par hectare du site traité.

16. Composés de formules (III), (XXVIII), (XXXII), (XXXIV) à (XXXVI), (XXXVIII), (XXXIX) et (XLI), dans lesquels les divers substituants ont la même définition que dans les revendications précédentes, à l'exclusion des composés dans lesquels R¹=R²=H, R³=amino, X=cyano, X³=X⁴=H et : X¹=méthyle, méthoxy ou chlore et X²=Y=H ; X²=méthyle, méthoxy ou chlore et X¹=Y=H ; Y=méthyle, méthoxy ou chlore et X¹=X²=H.

## Revendications (Revendications pour l'(les) Etat(s) contractant(s) suivant(s): ES)

1. Procédé de production d'un composé de formule (I) 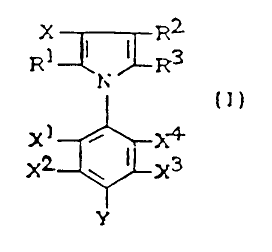 dans laquelle
X est choisi dans le groupe consistant en : un halogène, des groupes cyano, halogénalkyle, alkoxy, halogénalkoxy, alkylthio, alkylsulfinyle, alkylsulfonyle, halogénalkylthio, halogénalkylsulfinyle et halogénalkylsulfonyle dont les groupes alkyle, halogénalkyle, alkoxy et halogénalkoxy sont des chaînes linéaires ou ramifiées ayant moins de 10 atomes de carbone, et la substitution halogéno dans tous ces groupes consiste en un ou plusieurs atomes d'halogènes, qui sont identiques ou différents, depuis une monosubstitution jusqu'à une polysubstitution complète ;
R¹, R² et R³ sont choisis, individuellement, dans le groupe consistant en : l'un des substituants du même ensemble que ceux qui sont indiqués pour X ; un atome d'hydrogène ; un groupe alkyle ; et pas plus de l'un des substituants R¹, R² et R³ n'étant choisi dans le groupe consistant en formyle, amino, alkylamino et dialkylamino dont les groupes alkyle sont des chaînes linéaires ou ramifiées ayant moins de 10 atomes de carbone ;
Y est choisi dans le groupe consistant en : un halogène, des groupes cyano, alkyle, halogénalkyle, alkoxy et halogénalkoxy, dont les groupes alkyle, alkoxy, halogénalkyle et halogénalkoxy sont des chaînes linéaires ou ramifiées, ayant moins de 10 atomes de carbone, et la substitution halogéno dans tous ces groupes consistant en un ou plusieurs atomes d'halogènes, qui sont identiques ou différents, allant de la monosubstitution jusqu'à une polysubstitution complète ;
ou bien Y est un atome d'hydrogène lorsque :
X est un atome d'halogène ou un groupe R⁵S(O)n, dans lequel n a la valeur 0, 1 ou 2 et R⁵ est un groupe alkyle ou halogénalkyle, et les chaînes alkyliques carbonées et la substitution halogéno sont telles que définies ci-dessus ;
R¹ et R³ représentent chacun un atome d'hydrogène ; et
R² est un groupe cyano ;
X¹, X², X³ et X⁴ sont choisis, individuellement, dans le même ensemble de substituants que celui qui a été indiqué pour Y ou représentent un atome d'hydrogène, sous les réserves suivantes : qu'au moins l'un de R¹, R² et R³ soit choisi dans le même ensemble de substituants que celui qui a été indiqué pour X ;
que si X⁴ et X¹ sont de l'hydrogène et X représente un halogène ou un groupe cyano, R² soit alors différent de X, et
que si X⁴ et X¹ représentent de l'hydrogène et Y est un groupe méthyle, X soit alors différent d'un radical bromo ;
à l'exclusion du composé dans lequel X⁴ = X¹ = Y = R¹ = R² = R³ = Cl en même temps que X² = X³ = H,
procédé qui comprend :
(A) lorsque le composé répond à la formule I(a) : 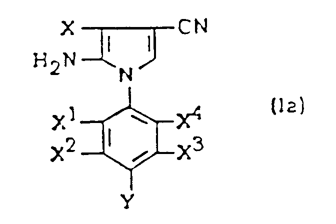 dans laquelle X¹, X², X³, X⁴ et Y ont la même définition que ci-dessus et
(A-I) : X est un halogène, un groupe trifluorométhyle, cyano, alkylthio, alkylsulfinyle, alkylsulfonyle, halogénalkylthio, halogénalkylsulfinyle ou halogénalkylsulfonyle, un procédé dans lequel un composé de formule 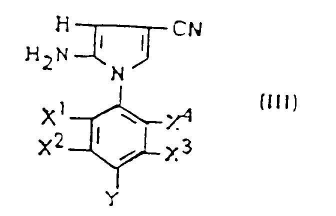 dans laquelle les divers symboles sont tels que définis ci-dessus et le groupe amino est facultativement protégé :
(a) est amené à réagir avec un agent d'halogénation, facultativement en présence d'un solvant pour obtenir un composé de formule (Ia) dans laquelle X est un halogène, puis on fait réagir facultativement ce composé avec le trifluorométhyl-cuivre d'une manière connue pour obtenir des composés de formule (Ia) dans laquelle X est un groupe trifluorométhyle ;
(b) est amené à réagir avec un tris(alkylthio)méthane ou un tris(arylthio)méthane en présence d'un acide de Lewis, puis le composé obtenu de formule (XI) 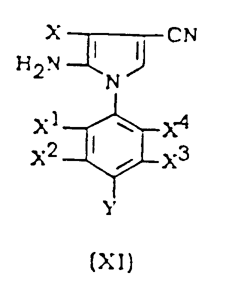 dans laquelle X est un groupe bis(alkylthio)méthyle ou bis(arylthio)méthyle et les autres symboles sont tels que définis ci-dessus, est amené à réagir facultativement avec un nitrite d'alkyle, la réaction étant suivie d'une hydrolyse, en vue d'obtenir le composé de formule (XI) dans laquelle X est un groupe formyle, puis le composé en question est mis en contact avec l'hydroxylamine et ce contact est suivi d'une déshydratation d'une manière connue en vue d'obtenir le composé de formule (Ia) dans laquelle X est un groupe cyano ;
(c) est amené à réagir avec un composé de formule MSCN, M étant un métal alcalin, en présence de brome, dans un solvant en vue d'obtenir le composé de formule (Ia) dans laquelle X est un groupe thiocyanato, puis ce composé est amené à réagir avec un halogénure d'alkyle ou un sulfate de dialkyle en présence d'une base dans un solvant pour former un composé de formule (Ia) dans laquelle X est un groupe alkylthio ; ou bien
(d) est amené à réagir avec un halogénure de sulfényle de formule RSHal, dans laquelle R est un radical alkyle, halogénalkyle, phényle ou hétéroaryle et Hal est un atome d'halogène, dans un milieu réactionnel liquide organique, facultativement en présence d'un accepteur d'acide en vue d'obtenir un composé de formule (Ia) dans laquelle X est un groupe alkylthio ou halogénalkylthio, après quoi on effectue facultativement l'oxydation, d'une manière connue, du composé obtenu en vue de produire un composé de formule (Ia) dans laquelle X est un groupe RS(O)ₙ dans laquelle n est égal à 1 ou 2, ou bien
(A-II) lorsque X est un groupe alkoxy ou halogénalkoxy, un procédé dans lequel un composé de formule 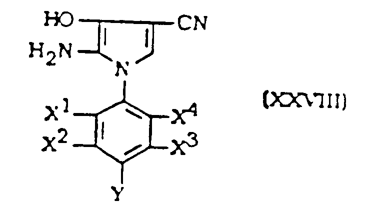 dans laquelle les divers symboles sont tels que définis ci-dessus et des groupes amino et cyano sont convenablement protégés si nécessaire :
(a) est amené à réagir avec un agent d'alkylation, facultativement en présence d'une base, pour obtenir des composés de formule (Ia) dans laquelle X est un groupe alkoxy ; ou bien
(b) est halogénalkylé d'une manière connue pour obtenir des composés de formule (Ia) dans laquelle X est un groupe halogénalkoxy, ou bien
(A-III) lorsque X est un groupe halogénalkyle, un procédé dans lequel un composé de formule 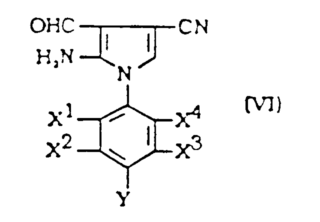 dans laquelle les divers symboles sont tels que définis ci-dessus et les groupes amino et cyano sont convenablement protégés si nécessaire :
(a) est amené à réagir avec un agent de fluoration d'une manière connue en vue d'obtenir le composé de formule (Ia) dans laquelle X est un groupe difluorométhyle ;
(b) est amené à réagir avec un agent oxydant pour convertir le groupe -CHO en un groupe acide carboxylique, puis les composés obtenus sont soumis à l'action d'un agent de fluoration d'une manière connue pour obtenir le composé de formule (Ia) dans laquelle X est un groupe trifluorométhyle ;
(c) est converti en un composé dans lequel le groupe -CHO est remplacé par un groupe méthyle, puis le composé obtenu est soumis à l'action d'un agent d'halogénation dans un solvant en vue d'obtenir un composé de formule (Ia) dans laquelle X est un groupe bromométhyle ou chlorométhyle ; ou bien
(d) est amené à réagir successivement avec un dérivé halogénalkylique de métal ou le trifluorotriméthylsilane pour convertir le groupe -CHO en un groupe halogénalkylcarbinol, puis le composé dans lequel X est un groupe halogénalkylcarbinol est amené à réagir avec un agent d'halogénation en vue d'obtenir des composés de formule (Ia) dans laquelle X est un groupe α-halogénalkyl-α-halogénométhyle, toutes les étapes précédentes ayant été suivies d'une étape d'élimination du groupe protecteur, si nécessaire, ou bien
(B) lorsque le composé de formule (I) répond à la formule I(b) : 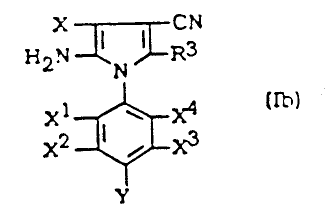 dans laquelle X, X¹, X², X³, X⁴ et Y ont la même définition comme défini ci-dessus et lorsque (B-I) R³ est un halogène, un groupe formyle, halogénalkyle, alkyle, alkylthio, halogénalkylthio, alkylsulfinyle, alkylsulfonyle, halogénalkylsulfinyle ou halogénalkylsulfonyle, un procédé dans lequel un composé de formule (Ia) dans laquelle X, le groupe cyano et le groupe amino sont facultativement protégés si nécessaire :
(a) est amené à réagir conformément à A-I(a) ci-dessus afin d'obtenir des composés de formule (Ib) dans laquelle R³ est un halogène, puis facultativement, ce composé est amené à réagir conformément à A-I(a) ci-dessus pour obtenir des composés de formule (Ib) dans laquelle R³ est un groupe trifluorométhyle ;
(b) est amené à réagir conformément à A-I(b) ci-dessus pour obtenir tout d'abord un composé de formule (Ib) dans laquelle R³ est un groupe bis(alkylthio)méthyle ou un groupe bis(arylthio)méthyle, puis un composé de formule (Ib) dans laquelle R³ est un groupe formyle ; ou bien
(c) est amené à réagir conformément à A-I(c,d) ci-dessus en vue d'obtenir des composés de formule (Ib) dans laquelle R³ est un groupe alkylthio ou halogénalkylthio, puis facultativement oxydé conformément à A-I(d) ci-dessus en vue d'obtenir les composés de formule (Ib) dans laquelle R³ est un groupe alkylsulfinyle, alkylsulfonyle, halogénalkylsulfinyle ou halogénalkylsulfonyle, sous réserve que X ne soit pas un groupe RS qui puisse subir une oxydation indésirable ; et lorsque X est un halogène, on conduit un traitement facultatif avec un alkyl-lithium d'une manière connue, puis une désactivation aqueuse et une sulfénylation conformément à A-I(c,d) ci-dessus en vue d'obtenir des composés de formule (Ib) dans laquelle X est un groupe alkylthio ou halogénalkylthio, ou bien
(B-II) lorsque R³ est un groupe cyano ou un groupe alkyle, un procédé dans lequel un composé de formule 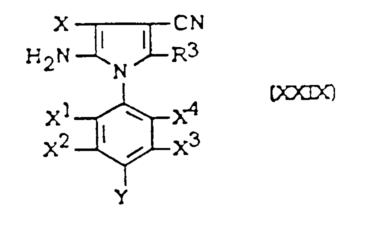 dans laquelle R³ est un groupe formyle ou alkylcarbonyle, les autres symboles étant tels que définis dans la revendication 1, et X, le groupe cyano et le groupe amino sont protégés si nécessaire :
(a) est condensé avec l'hydroxylamine ou une O-alkylhydroxylamine ou leurs sels d'addition dans un solvant en vue d'obtenir un composé de formule (Ib) dans laquelle R³ est un groupe hydroxyiminoalkylidényle ou alkoxyiminoalkylidényle, et ce groupe R³ est converti en un groupe cyano afin d'obtenir un composé de formule (Ib) dans laquelle R³ est un groupe cyano ;
(b) est amené à réagir, lorsque R³ est un groupe formyle, conformément à A-III(a, b, c ou d) ci-dessus afin d'obtenir un composé de formule (Ib) dans laquelle R³ est un groupe méthyle ou halogénalkyle ; ou bien
(c) est amené à réagir, lorsque R³ est un groupe formyle, avec un réactif de Grignard dérivé d'un halogénure d'alkyle ou d'un alkyl-lithium pour produire un carbinol, la réaction étant suivie d'une étape de déshydratation afin de produire un composé dans lequel R³ est un groupe alcényle, puis d'une réduction en vue d'obtenir des composés de formule (Ib) dans laquelle R³ est un groupe alkyle ; la réduction étant facultativement suivie d'une étape d'élimination du groupe protecteur, ou bien
(B-III) lorsque R³ est un groupe alkoxy ou halogénalkoxy, un procédé dans lequel un composé de formule 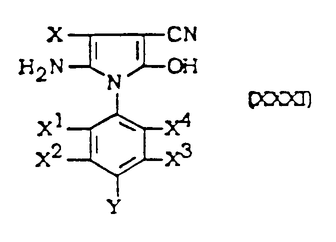 dans laquelle les groupes amino, cyano et X sont facultativement protégés, est amené à réagir conformément à A-II(a, b ou c) ci-dessus afin d'obtenir un composé de formule (Ib) dans laquelle R³ est un groupe alkoxy ou halogénalkoxy, ou bien
(c) lorsque le composé de formule I répond à la formule (Ic) : 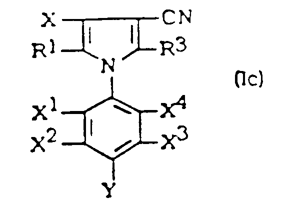 dans laquelle X, R³, X¹, X², X³, X⁴ et Y ont les mêmes définitions comme défini ci-dessus et lorsque
(C-I) R¹ est l'hydrogène, un halogène, un groupe alkylthio, halogénalkylthio, alkylsulfinyle, alkylsulfonyle, halogénalkylsulfinyle, halogénalkylsulfonyle, alkylamino, dialkylamino, formyle, halogénalkyle ou alkyle, un procédé dans lequel un composé de formule 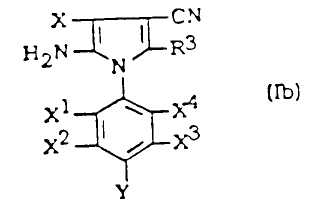 dans laquelle le groupe amino est débarrassé du groupe protecteur après protection des groupes X, R³ ou cyano si nécessaire :
(a) est amené à réagir avec un agent de diazotation dans un solvant inerte afin d'obtenir un composé de formule (Ic) dans laquelle R¹ représente H ;
(b) est amené à réagir avec un agent de diazotation en présence d'un donneur d'halogène pour obtenir un composé de formule (Ic) dans laquelle R¹ est un halogène ;
(c) est amené à réagir avec un agent de diazotation en présence de (SCN)₂ ou d'un disulfure dans un solvant pour obtenir des composés de formule (Ic) dans laquelle R¹ est un groupe alkylthio ou halogénalkylthio, après quoi on effectue facultativement une oxydation conformément à A-I(d) ci-dessus pour obtenir un composé de formule (Ic) dans laquelle R¹ est un groupe alkylsulfinyle ou alkylsulfonyle ;
(d) est amené à réagir conformément à la revendication 9(A) ci-dessous en vue d'obtenir des composés de formule (Ic) dans laquelle R¹ est un groupe alkylamino ou dialkylamino ; ou bien
(e) est amené à réagir avec du nitrite de sodium et de la formaldoxime, du sulfate de cuivre et du HCl d'une manière connue en vue d'obtenir des composés de formule (Ic) dans laquelle R¹ est un groupe formyle, puis, à titre facultatif, (i) amené à réagir conformément à B-II (a) ci-dessus afin d'obtenir un composé de formule (Ic) dans laquelle R¹ est un groupe cyano ou (ii) amené à réagir conformément à A-III(a-d) ci-dessus pour obtenir un composé de formule (Ic) dans laquelle R¹ est un groupe halogénalkyle ou méthyle, la réaction étant suivie si nécessaire d'une étape d'élimination du groupe protecteur ; ou bien
(C-II) R¹ est un groupe alkoxy ou halogénalkoxy, un procédé dans lequel un composé de formule 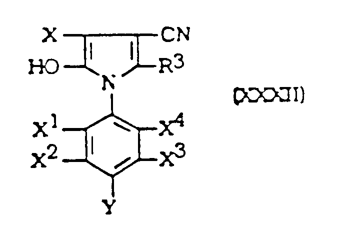 dans laquelle les divers symboles sont tels que définis ci-dessus et X, le groupe cyano ou R³ sont facultativement protégés d'une manière connue, est amené à réagir conformément à A-II(a ou b) ci-dessus pour obtenir un composé de formule (Ic) dans laquelle R¹ est un groupe alkoxy ou halogénalkoxy, la réaction étant suivie d'une étape facultative d'élimination du groupe protecteur, ou bien
(D) lorsque X, R¹, R³, X¹, X², X³, X⁴ et Y ont la même définition que ci-dessus et R² est un groupe CHO, un procédé dans lequel un composé de formule (Ic) est traité avec un agent réducteur dans un solvant pour convertir le groupe CN en un groupe CHO et pour former le composé dans lequel R² est un groupe CHO, ce composé étant facultativement oxydé d'une manière connue pour produire le composé correspondant de formule (XXXV) 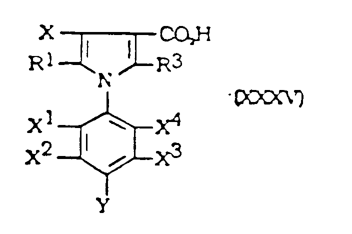 ou bien (E) lorsque X, R¹, R³, X¹, X², X³, X⁴ et Y ont la même définition que ci-dessus, et R² est un groupe cyano, alkyle ou halogénalkyle, un procédé dans lequel un composé de formule (I), dans laquelle R² est un groupe CHO, après protection facultative de X, R¹ ou R³ si nécessaire, d'une manière connue, est amené à réagir conformément à A-III(a, b, c, ou d), A-II(a ou b) ou C-I ci-dessus, la réaction étant suivie, si nécessaire, d'une étape d'élimination du groupe protecteur, ou bien
(F) lorsque X, R¹, R³, X¹, X², X³, X⁴ et Y ont la même définition que ci-dessus, et R² est un groupe amino, alkylamino, dialkylamino, hydrogène, halogène, alkylthio, halogénalkylthio, alkylsulfinyle, alkylsulfonyle, halogénalkylsulfinyle, halogénalkylsulfonyle ou trifluorométhyle, un procédé dans lequel un composé de formule (XXXV) représenté ci-dessus, après protection facultative de X, R¹ ou R³ si nécessaire d'une manière connue, est amené à réagir dans des conditions de transposition de Curtius, par exemple par conversion en un chlorure d'acide, suivie d'une réaction avec un azoture de métal alcalin ou avec le diphénylphosphorylazide en présence d'une base organique dans un solvant alcoolique pour produire un carbamate qui peut ensuite être hydrolysé afin d'obtenir le composé correspondant dans lequel R² est un groupe amino que l'on fait ensuite facultativement réagir conformément à la revendication 9(A) ci-dessous ou C-I(a-c) ci-dessus, puis, lorsque R² est un halogène, on conduit facultativement la réaction conformément à B-I(a) ci-dessus, suivie, si nécessaire, d'une étape d'élimination du groupe protecteur, ou bien
(G) lorsque X, R¹, R³, X¹, X², X³, X⁴ et Y ont la même définition que dans la revendication 1, et R² est un groupe alkoxy ou halogénalkoxy, un procédé dans lequel un composé de formule 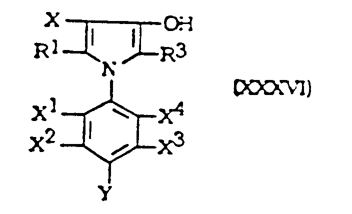 dans laquelle les divers symboles sont tels que définis ci-dessus, après protection facultative des groupes X, R¹ ou R³, si nécessaire, est amené à réagir conformément à B-III ci-dessus, la réaction étant suivie, si nécessaire, d'une étape d'élimination du groupe protecteur, ou bien
(H) lorsque R¹, R², R³, Y, X¹, X², X³ et X⁴ sont tels que définis dans la revendication 1 et X est un groupe perhalogénalkylthio, un procédé qui comprend :
a) la réaction d'un composé de formule (XXXVIII) 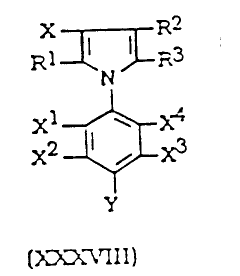 dans laquelle X est de l'hydrogène, et les autres symboles sont tels que définis comme défini ci-dessus, avec l'acide chlorosulfonique (ClSO₃H) à une température de 0°C à 150°C et, facultativement, dans un solvant organique, pour préparer un composé de formule (XXXIX) : 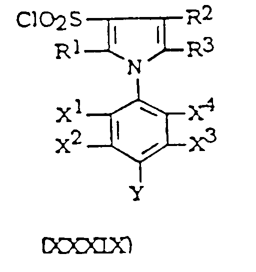 dans laquelle les divers symboles sont tels que définis ci-dessus ;
b) la réaction du composé de formule (XXXIX) avec un agent réducteur à une température de 0°C à 110°C dans un solvant organique pour former un disulfure de formule (XLI) 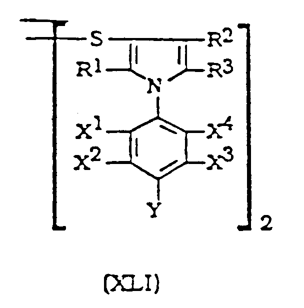 dans laquelle les divers symboles sont tels que définis ci-dessus ; et
c) la réaction du disulfure de formule (XLI) avec un perhalogénalcane de formule (XLII), ZCFR⁷R⁸, dans lequel Z représente Cl, Br ou I, R⁷ représente F, Cl ou Br, et R⁸ représente F, Cl, Br ou un groupe perfluoralkyle, en présence d'un agent réducteur promoteur de radicaux libres et à titre facultatif en présence d'une base, dans un solvant organique à une température de 20°C à 85°C et à titre facultatif sous pression, pour préparer un composé de formule (I) dans laquelle X est un groupe perhalogénalkylthio et les autres symboles sont tels que définis ci-dessus, ou bien
(I) par transformation d'un composé obtenu comme décrit dans l'une quelconque des étapes A à H ci-dessus en un composé de formule (I) tel que défini ci-dessus.

2. Procédé suivant la revendication 1, dans lequel :
X est choisi entre un atome d'halogène ou un groupe choisi entre des groupes cyano, halogénalkyle, alkoxy, halogénalkoxy, alkylthio, alkylsulfinyle, alkylsulfonyle, halogénalkylthio, halogénalkylsulfinyle et halogénalkylsulfonyle, dont les groupes alkyle, halogénalkyle, alkoxy et halogénalkoxy sont des chaînes linéaires ou ramifiées, ayant moins de 10 atomes de carbone, et la substitution halogéno dans tous ces groupes est une monosubstitution ou va jusqu'à une polysubstitution totale ;
R¹, R² et R³ sont choisis, individuellement, entre : les mêmes substituants ceux qui sont décrits pour X ; un atome d'hydrogène ; un groupe alkyle ; et un substituant tel que pas plus de l'un des substituants R¹, R² et R³ ne soit choisi dans le groupe consistant en formyle, amino, alkylamino et dialkylamino dont les groupes alkyle sont des chaînes linéaires ou ramifiés ayant moins de 10 atomes de carbone ;
Y est choisi entre un atome d'halogène et un groupe choisi entre cyano, alkyle, halogénalkyle, alkoxy et halogénalkoxy, dont les groupes alkyle, alkoxy, halogénalkyle et halogénalkoxy sont des chaînes linéaires ou ramifiées, ayant moins de 10 atomes de carbone, et la substitution halogéno dans tous ces groupes est une monosubstitution ou va jusqu'à une polysubstitution totale ; et
X¹, X², X³ et X⁴ sont tels que définis dans la revendication 1.

3. Procédé suivant la revendication 2, dans lequel :
X est un atome d'halogène ou un groupe R⁵S(O)n, dans lequel n a la valeur 0, 1 ou 2 et R⁵ est un groupe alkyle ou halogénalkyle ;
R¹ est un atome d'hydrogène, un atome d'halogène ou un groupe alkylthio ;
R² est un groupe cyano ;
R³ est un atome d'hydrogène ou un atome d'halogène ;
Y est un atome d'hydrogène, un atome d'halogène, un groupe halogénalkyle ou halogénalkoxy, sous réserve que Y soit un atome d'hydrogène de la manière définie dans la revendication 1 ; et
X¹, X², X³ et X⁴ sont choisis, individuellement, dans le groupe consistant en un atome d'hydrogène, un atome d'halogène, un groupe alkyle en C₁ à C₃, alkoxy en C₁ à C₃ et alkylthio en C₁ à C₃.

4. Procédé suivant la revendication 3, dans lequel le composé de formule (I) répond à la formule (II) 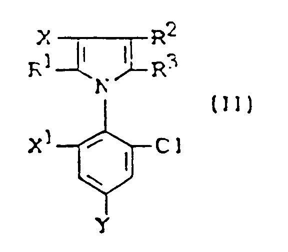 dans laquelle :
X est un groupe R⁵S(O)n, dans lequel n a la valeur 0, 1 ou 2 et R⁵ est un groupe CH₃, CF₃, CF₂Cl, CFCl₂, CF₂Br, CHF₂, CHCl₂ ou CHClF ;
R¹ représente H, F, Cl ou Br ;
R³ représente H, F, Cl ou Br ;
X¹ représente H ou Cl ; et
Y est un groupe CF₃ ou CF₃O.

5. Procédé suivant la revendication 1, 2 ou 3, dans lequel :
R¹ est un atome d'hydrogène ou un atome d'halogène ;
R² est un groupe cyano ;
X est un groupe halogénalkyle-S(O)ₙ dans lequel n a la valeur 0, 1 ou 2 ;
X¹ et X⁴ représentent autre chose qu'un atome d'hydrogène ;
Y est un groupe halogénalkyle ou halogénalkoxy ; et
X² et X³ représentent un atome d'hydrogène.

6. Procédé suivant la revendication 2, dans lequel le composé de formule (I) répond à la formule (II) 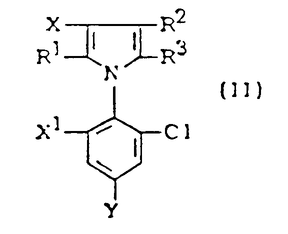 dans laquelle :
X est un groupe de formule R⁵S(O)ₙ, dans laquelle n a la valeur 0, 1 ou 2 et R⁵ est un groupe CH₃, CF₃, CF₂Cl ou CFCl₂ ;
R² est un groupe cyano ;
R¹ représente H, F, Cl, Br ou NH₂ ;
R³ représente H, F, Cl, Br, CF₃ ou CN ;
X¹ représente H ou Cl ; et
Y est un groupe CF₃ ou CF₃O.

7. Procédé suivant la revendication 1, dans lequel le composé de formule (I) est :
le 1-(2,6-dichloro-4-trifluorométhylphényl)-2-chloro-3-cyano-4-(trifluorométhylthio)pyrrole ;
le 1-(2,6-dichloro-4-trifluorométhoxyphényl)-2-chloro-3-cyano-4-(trifluométhylsulfonyl)pyrrole ;
le 1-(2,6-dichloro-4-trifluorométhylphényl)-2-chloro-3-cyano-4-(dichlorofluorométhylthio)pyrrole ;
le 1-(2,6-dichloro-4-trifluorométhylphényl)-2-chloro-3-cyano-4-(dichlorofluorométhylsulfinyl)pyrrole ;
le 1-(2,6-dichloro-4-trifluorométhylphényl)-2-chloro-3-cyano-4-(dichlorofluorométhylsulfonyl)pyrrole ;
le 1-(2,6-dichloro-4-trifluorométhylphényl)-2-chloro-3-cyano-4-(chlorodifluorométhylsulfonyl)pyrrole ;
le 1-(2,6-dichloro-4-trifluorométhylphényl)-2-chloro-3-cyano-4-(chlorodifluorométhylsulfinyl)pyrrole ;
le 1-(2,6-dichloro-4-trifluorométhylphényl)-2-chloro-3-cyano-4-(chlorodifluorométhylthio)pyrrole ;
le 1-(2,6-dichloro-4-trifluorométhylphényl)-2-bromo-3-cyano-4-(trifluorométhylsulfinyl)pyrrole ;
le 1-(2,6-dichloro-4-trifluorométhylphényl)-2-bromo-3-cyano-4-(trifluorométhylsulfonyl)pyrrole ;
le 1-(2,6-dichloro-4-trifluorométhylphényl)-2-bromo-3-cyano-4-(dichlorofluorométhylthio)pyrrole ;
le 1-(2,6-dichloro-4-trifluorométhylphényl)-2-bromo-3-cyano-4-(dichlorofluorométhylsulfonyl)pyrrole ;
le 1-(2,6-dichloro-4-trifluorométhylphényl)-2-bromo-3-cyano-4-(dichlorofluorométhylsulfinyl)pyrrole ;
le 1-(2,6-dichloro-4-trifluorométhylphényl)-2-bromo-3-cyano-4-(chlorodifluorométhylthio)pyrrole ;
le 1-(2,6-dichloro-4-trifluorométhylphényl)-2-bromo-3-cyano-4-(chlorodifluorométhylsulfinyl)pyrrole ;
le 1-(2,6-dichloro-4-trifluorométhylphényl)-2-chloro-3-cyano-4-(dichlorofluorométhylsulfonyl)-5-bromopyrrole ;
le 1-(2,6-dichloro-4-trifluorométhylphényl)-2-bromo-3-cyano-4-(chlorodifluorométhylsulfonyl)pyrrole ;
le 1-(2,6-dichloro-4-trifluorométhoxyphényl)-2-chloro-3-cyano-4-(dichlorofluorométhylsulfinyl)pyrrole ;
le 1-(2,6-dichloro-4-trifluorométhoxyphényl)-2-chloro-3-cyano-4-(dichlorofluorométhylsulfonyl)pyrrole ;
le 1-(2-chloro-4-trifluorométhylphényl)-2-chloro-3-cyano-4-(dichlorofluorométhylsulfonyl)pyrrole ;
le 1-(2-chloro-4-trifluorométhylphényl)-2-chloro-3-cyano-4-(dichlorofluorométhylsulfinyl)pyrrole ;
le 1-(2-chloro-4-trifluorométhylphényl)-2-bromo-3-cyano-4-(dichlorofluorométhylsulfonyl)pyrrole ;
le 1-(2,6-dichloro-4-trifluorométhylphényl)-2-chloro-3-cyano-4-(dichlorofluorométhylthio)-5-méthylthiopyrrole ;
le 1-(2,6-dichloro-4-trifluorométhylphényl)-2-chloro-3-cyano-4-(bromodifluorométhylthio)pyrrole ;
le 1-(2,6-dichloro-4-trifluorométhylphényl)-2-chloro-3-cyano-4-(bromodifluorométhylsulfinyl)pyrrole ;
le 1-(2,6-dichloro-4-trifluorométhylphényl)-2-chloro-3-cyano-4-(bromodifluorométhylsulfonyl)pyrrole ;
le 1-(2,6-dichloro-4-trifluorométhylphényl)-2-chloro-3-cyano-4-(méthylsulfinyl)pyrrole ;
le 1-(2,6-dichloro-4-trifluorométhylphényl)-2-chloro-3-cyano-4-(méthylsulfonyl)pyrrole ;
le 1-(4-bromo-2,6-dichlorophényl)-3-cyano-4-(chlorodifluorométhylthio)pyrrole ;
le 1-(4-bromo-2,6-dichlorophényl)-3-cyano-4-(trifluorométhylthio)pyrrole ;
le 1-(2,4,6-trichlorophényl)-3-cyano-4-(chlorodifluorométhylthio)pyrrole ;
le 1-(2,4,6-trichlorophényl)-3-cyano-4-(chlorodifluorométhylsulfinyl)pyrrole ;
le 1-(2,4,6-trichlorophényl)-3-cyano-4-(dichlorofluorométhylthio)pyrrole ;
le 1-(2,4,6-trichlorophényl)-3-cyano-4-(dichlorofluorométhylsulfinyl)pyrrole ;
le 1-(2,4,6-trichlorophényl)-3-cyano-4-(dichlorofluorométhylsulfonyl)pyrrole ;
le 1-(4-bromo-2,6-dichlorophényl)-3-cyano-4-(dichlorofluorométhylthio)pyrrole ;
le 1-(4-bromo-2,6-dichlorophényl)-3-cyano-4-(dichlorofluorométhylsulfinyl)pyrrole ;
le 1-(4-bromo-2,6-dichlorophényl)-3-cyano-4-(dichlorofluorométhylsulfonyl)pyrrole ;
le 1-(2,4,6-trichlorophényl)-3-cyano-4-(trifluorométhylthio)pyrrole ;
le 1-(2,4,6-trichlorophényl)-3-cyano-4-(trifluorométhylsulfinyl)pyrrole ;
le 1-(2,4,6-trichlorophényl)-3-cyano-4-(trifluorométhylsulfonyl)pyrrole ;
le 1-(2,4,6-trichlorophényl)-3-cyano-4-(trichlorométhylthio)pyrrole ;
le 1-(2,4-dichlorophényl)-3-cyano-4-(dichlorofluorométhylthio)pyrrole ;
le 1-(2,4,6-trichlorophényl)-3-cyano-4-chloropyrrole ;
le 1-(2,4,6-trichlorophényl)-3-cyano-4-(chlorodifluorométhylsulfonyl)pyrrole ;
le 1-(2,6-dichlorophényl)-3-cyano-4-(dichlorofluorométhylthio)pyrrole ;
le 1-(4-bromo-2,6-dichlorophényl)-3-cyano-4-(trifluorométhylsulfinyl)pyrrole ;
le 1-(4-bromo-2,6-dichlorophényl)-3-cyano-4-(trifluorométhylsulfonyl)pyrrole ; ou
le 1-(4-bromo-2,6-dichlorophényl)-3-cyano-4-(chlorodifluorométhylsulfinyl)pyrrole ;
le 1-(4-bromo-2,6-dichlorophényl)-3-cyano-4-(chlorodifluorométhylsulfonyl)pyrrole ;
le 1-(4-bromo-2,6-diméthylphényl)-3-cyano-4-(tridifluorométhylsulfinyl)pyrrole ;
le 1-(4-bromo-2,6-diméthylphényl)-3-cyano-4-(tridifluorométhylsulfonyl)pyrrole ;
le 1-(4-bromo-2,6-difluorophényl)-3-cyano-4-(dichlorofluorométhylthio)pyrrole ;
le 1-(2,6-dichloro-4-trifluorométhylphényl)-2-chloro-3-cyano-4-(trifluorométhylsulfonyl)pyrrole ;
le 1-(2,6-dichloro-4-trifluorométhylphényl)-2-chloro-3-cyano-4-(trifluorométhylsulfinyl)pyrrole ;
le 1-(2,6-dichloro-4-trifluorométhylphényl)-2-chloro-3-cyano-4-trifluorométhylsulfinyl-5-brom-pyrrole ;
le 1-(2,6-dichloro-4-trifluorométhylphényl)-2-chloro-3-cyano-4-trifluorométhylsulfonyl-5-bromopyrrole ;
le 1-(2,6-dichloro-4-trifluorométhylphényl)-2-chloro-3-cyano-4-(trifluorométhylthio)pyrrole ;
le 1-(2,6-dichloro-4-trifluorométhylphényl)-2-bromo-3-trifluorométhylthio-4-cyano-5-chloropyrrole ;
le 1-(2,6-dichloro-4-trifluorométhylphényl)-2-[(trifluorométhyl)carbonylamino]-3-trifluorométhylthio-4-cyano-5-chloropyrrole ;
le 1-(2,6-dichloro-4-trifluorométhylphényl)-2-(méthylcarbonylamino)-3-trifluorométhylthio-4-cyano-5-chloropyrrole ;
le 1-(2,6-dichloro-4-trifluorométhylphényl)-2-amino-3-trifluorométhylthio-4-cyano-5-chloropyrrole ;
le 1-(2-chloro-4-trifluorométhylphényl)-2-amino-3-trifluorométhylthio-4-cyano-5-chloropyrrole ;
le 1-(2,6-dichloro-4-trifluorométhylphényl)-2-amino-3-dichlorofluorométhylthio-4-cyano-5-chloropyrrole ;
le 1-(2,6-dichloro-4-trifluorométhylphényl)-2,4-bis(trifluorométhylthio)-3-cyano-5-aminopyrrole ;
le 1-(2,6-dichloro-4-trifluorométhylphényl)-2-amino-3-trifluorométhylthio-4-cyano-5-bromopyrrole ;
le 1-(2,6-dichloro-4-trifluorométhylphényl)-2-amino-3-trifluorométhylthio-4-cyanopyrrole ;
le 1-(4-trifluorométhylphényl)-2-amino-3-trifluorométhylthio-4-cyano-5-bromopyrrole ;
le 1-(2-chloro-4-trifluorométhylphényl)-2-amino-3-trifluorométhylthio-4-cyano-5-bromopyrrole ;
le 1-(2,6-dichloro-4-trifluorométhylphényl)-2-chloro-3-cyano-4-(dichlorofluorométhylthio)pyrrole ;
le 1-(2,6-dichloro-4-trifluorométhylphényl)-2-chloro-3-cyano-4-(dichlorofluorométhylsulfinyl)pyrrole ;
le 1-(2,6-dichloro-4-trifluorométhylphényl)-2-chloro-3-cyano-4-(dichlorofluorométhylsulfonyl)pyrrole ;
le 1-(2,6-dichloro-4-trifluorométhylphényl)-3-cyano-4-(dichlorofluorométhylthio)pyrrole ;
le 1-(2,6-dichloro-4-trifluorométhylphényl)-3-cyano-4-(dichlorofluorométhylsulfinyl)pyrrole ; ou
le 1-(2,6-dichloro-4-trifluorométhylphényl)-2-chloro-3-cyano-4-(trifluorométhylsulfonyl)pyrrole.

8. Procédé de production de composés intermédiaires utiles pour l'obtention de composés de formule (I), qui comprend :
(A) un procédé de production de composés de formule (XXXIV) 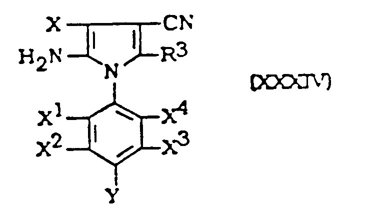 dans laquelle X, X¹, X², X³, X⁴ et Y sont tels que définis dans la revendication 1 et R³ est un groupe amino, alkylamino ou dialkylamino, dans lequel un composé de formule (XXX) 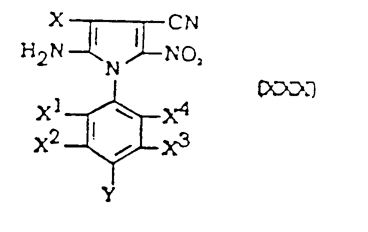 dans laquelle les divers symboles sont tels que définis dans la revendication 1 et le groupe amino est protégé d'une manière convenable, est réduit en vue d'obtenir des composés de formule (XXXIV) dans laquelle R³ est un groupe amino, ces composés étant amenés à réagir :
avec un agent d'alkylation dans un solvant organique pour obtenir un groupe amino monosubstitué ou disubstitué ou par l'intermédiaire de la transformation du groupe amino en un groupe alkoxyalkylidène-imino, suivie d'une réduction en vue d'obtenir des composés de formule (XXXIV) dans laquelle R³ est un groupe alkylamino ou dialkylamino ; l'opération étant suivie, si nécessaire, dans l'étape d'élimination du groupe protecteur, ou bien
(B) un procédé de préparation de composés de formules (XXVIII), (XXXI), (XXXII) et (XXXV) suivant la revendication 1(A-II), (B-III), (E) ou (G), dans lequel le composé correspondant suivant la revendication 1(A-I) (a), (B-I)(a), (C-I)(b) ou (F), après que le groupe amino éventuellement présent a été protégé, est converti en un réactif de Grignard ou en un dérivé de lithium, la conversion étant suivie d'une réaction avec un borate de trialkyle et d'une oxydation d'une manière connue, puis, si nécessaire, d'une étape d'élimination du groupe protecteur, ou bien
(C) un procédé de production d'un composé de formule 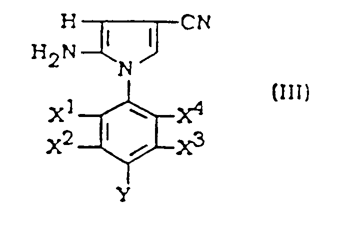 dans laquelle X¹, X², X³, X⁴ et Y ont la même définition que dans la revendication 1, dans lequel un dérivé de dicyanopropène de formule 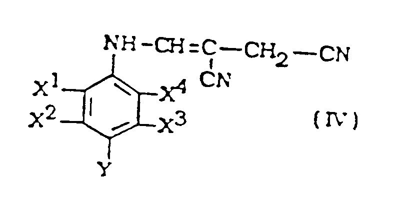 est amené à réagir avec un agent basique.

9. Procédé de préparation d'une composition destinée à combattre des arthropodes, des nématodes des plantes, des helminthes ou des protozoaires, qui comprend un composé de formule générale (I) tel que défini dans l'une quelconque des revendications 1 à 7 ou le composé dans lequel X⁴=X¹=Y=R¹=X=R²=R³=Cl en même temps que X³=X²=H, en association avec un ou plusieurs diluants ou véhicules compatibles, procédé qui comprend la formulation du composé de formule (I) ou du composé dans lequel X⁴=X¹=Y=R¹=X=R²=R³=Cl en même temps que X³=X²=H avec un ou plusieurs diluants ou véhicules comptabiles.

10. Procédé de préparation d'une composition destinée à être utilisée en médecine vétérinaire et dans l'exploitation du bétail ou dans le maintien de l'hygiène publique, contenant comme ingrédient actif au moins un composé de formule (I) tel que défini dans l'une quelconque des revendications 1 à 7 ou le composé dans lequel X⁴=X¹=Y=R¹=X=R²=R³=Cl en même temps que X³=X²=H, procédé qui comprend la formulation du composé de formule (I) du composé dans lequel X⁴=X¹=Y=R¹=X=R²=R³=Cl en même temps que X³=X²=H.

11. Procédé de préparation d'une composition insecticide, acaricide ou nématocide, qui comprend une quantité efficace d'un composé de formule (I) tel que défini dans l'une quelconque des revendications 1 à 7 ou le composé dans lequel X⁴=X¹=Y=R¹=X=R²=R³=Cl en même temps que X³=X²=H, procédé qui comprend la formulation du composé de formule (I) ou un composé dans lequel X⁴=X¹=Y=R¹=X=R²=R³=Cl en même temps que X³=X²=H.

12. Procédé suivant la revendication 11, dans lequel la composition comprend :
entre 0,05 % et 95 %, en poids, d'un ou plusieurs ingrédients tensio-actifs ; entre 1 % et 95 %, en poids, d'un ou plusieurs supports acceptables en agriculture ; entre 0,1 % et 50 %, en poids, d'un ou plusieurs agents tensio-actifs acceptables en agriculture.

13. Procédé d'utilisation en médecine vétérinaire et dans l'exploitation du bétail ou pour le maintien de l'hygiène publique, en vue de combattre des arthropodes, des helminthes ou des protozoaires qui sont des endoparasites ou des ectoparasites de vertébrés à sang chaud, qui comprend l'application à un site d'une quantité efficace d'un composé de formule (I) tel que défini dans l'une quelconque des revendications 1 à 7 ou du composé dans lequel X⁴=X¹=Y=R¹=X=R²=R³=Cl en même temps que X³=X²=H.

14. Procédé pour combattre des arthropodes et des nématodes parasites suivant la revendication 13, dans lequel le composé est appliqué au site où l'infestation d'arthropodes ou de nématodes doit être combattue, en une quantité de 0,005 kg à 15 kg du composé par hectare du site traité.

15. Procédé suivant la revendication (I), tel que défini dans l'une quelconque des revendications 1 à 7.

16. Composés de formules (III), (XXVIII) à (XXXII), (XXXIV) à (XXXVI), (XXXVIII), (XXXIX) et (XLI), dans lesquels les divers substituants ont la même définition que dans les revendications précédentes, à l'exclusion des composés dans lesquels R¹=R²=H, R³=amino, X=cyano, X³=X⁴=H et : X¹=méthyle, méthoxy ou chlore et X²=Y=H ; X²=méthyle, méthoxy ou chlore et X¹=Y=H ; Y=méthyle, méthoxy ou chlore et X¹=X²=H.

17. Composition telle que définie dans l'une quelconque des revendications 9 à 12.
